# EUROPEAN PATENT APPLICATION

(11) **EP 4 790 055 A2**
(43) Date of publication of application: **12.08.2026**
(21) Application number: 26152149.6
(22) Date of filing: 15.01.2026
(51) Int. Cl.: C07D 498/16, A61P 35/00, A61K 31/519

(54) **3-((5-(1H-INDAZOL-4-YL)-8,9-DIHYDRO-10H-7-OXA-1,3,6,10-TETRAAZACYCLOHEPTA[DE]NAPHTHALEN-10-YL)METHYL)PYRIDIN-2-AMINE DERIVATIVES AS RAS INHIBITORS FOR THE TREATMENT OF CANCER**

(30) Priority: 15.01.2025 US 202563745421 P; 09.04.2025 US 202563785862 P; 14.10.2025 US 202563899173 P
(71) Applicant: Kestrel Therapeutics Inc., Watertown, Massachusetts 02472 (US)
(72) Inventor: AHMAD, Omar K., Boston, 02114 (US); LAJKIEWICZ, Neil, Watertown, 02472 (US); FERNANDO, Dilinie P., Watertown, 02472 (US); RUI, Huan, Watertown, 02472 (US); ZHANG, Yun, Acton, 01720 (US)
(74) Representative: Carpmaels & Ransford LLP

(57) **Abstract**

The disclosure provides compounds of formulae I through IX, such as e.g. compounds of formula III:

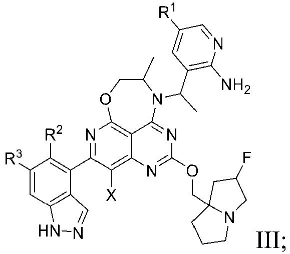

and their use in treating medical diseases or disorders, such as cancer. Pharmaceutical compositions and methods of making compounds of the disclosure are provided. The compounds are contemplated to be modulators of Ras (e.g., K-Ras).

## Description

### CROSS-REFERENCE

This application claims the benefit of and priority to U.S. Provisional Patent Application No. 63/745,421 filed on January 15, 2025, U.S. Provisional Patent Application No. 63/785,862 filed on April 9, 2025, and U.S. Provisional Patent Application No. 63/899,173 filed on October 14, 2025, the entire contents of which are hereby incorporated by reference herein.

### BACKGROUND

Ras proteins (e.g., K-Ras, H-Ras and N-Ras) play an important role in various human cancers and represent attractive targets for anticancer therapy. Dysregulation of Ras proteins by activating mutations, overexpression or upstream activation is commonly observed in human tumor cells, and activating mutations in Ras are often observed in human cancers. For example, activating mutations at codon 12 in Ras proteins function by inhibiting both GTPase-activating protein (GAP)-dependent and intrinsic hydrolysis rates of GTP, significantly altering the population of Ras mutant proteins to the "on" (GTP-bound) state (Ras(ON)), leading to oncogenic MAPK signaling. Ras proteins show a strong affinity for GTP, thereby allowing Ras to be activated even in the presence of low concentrations of this nucleotide. Mutations at codons 13 (e.g., G13D) and 61 (e.g., Q61K) of Ras are also responsible for oncogenic activity in some cancers.

For example, oncogenic K-Ras mutations that stabilize GTP binding and lead to constitutive activation of K-Ras and downstream signaling have been reported in various types of cancers. K-Ras mutations at codons 12, 13, 61 and other positions of the K-Ras primary amino acid sequence have been observed in patients with pancreatic, colorectal, non-small cell lung, and small cell lung adenocarcinomas.

Despite extensive research and discovery efforts by the pharmaceutical industry to develop inhibitors of Ras (e.g., K-Ras) for treating cancer, no such inhibitor has yet demonstrated sufficient safety and/or efficacy to obtain regulatory approval. Thus, an unmet need exists to develop new pan-Ras inhibitors, for example inhibitors of activating Ras mutants. that show safety and efficacy profiles necessary for treating Ras-mediated cancers and other conditions that are affected by, associated with, or would benefit from inhibition of Ras.

### SUMMARY

The disclosure is directed, in part, to compounds that inhibit Ras, for example, multiple mutated forms of Ras, for example, K-Ras. Also disclosed herein are pharmaceutical compositions comprising at least one disclosed compound and a pharmaceutically acceptable carrier. In some embodiments, the present disclosure provides a method of treating a disease or disorder characterized by aberrant Ras activity due to a Ras mutation (e.g., aberrant K-Ras activity due to a K-Ras mutation). In some embodiments, the disease or disorder is a cancer.

In some aspects, disclosed herein are compounds represented by Formula I: 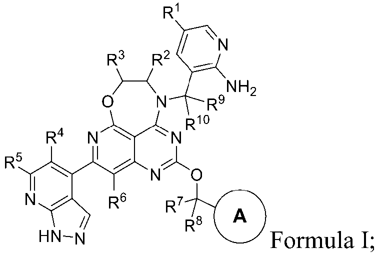 and pharmaceutically acceptable salts, stereoisomers, and tautomers thereof, wherein:
R¹ is selected from the group consisting of halogen, C₁-C₆alkoxy, C₃-C₄cycloalkyl, C₁-C₆alkyl, C₂-C₆alkenyl, and C₂-C₆alkynyl, wherein the alkoxy, cycloalkyl, alkyl, alkenyl, and alkynyl are optionally substituted with one or more halogens;
R² and R³ are each independently selected from the group consisting of C₁-C₆alkyl and hydrogen, wherein the alkyl is optionally substituted with one or more halogens or hydroxyl; or
R² and R³, together with the atoms to which they are attached, may be joined together to form a 4-7 membered heterocyclyl or a 5-7 membered cycloalkyl; wherein the heterocyclyl and cycloalkyl are optionally substituted with one or more substituents each independently selected from the group consisting of halogen, deuterium, hydroxyl, -NR^{a}R^{b}, C₁-C₆alkyl, and C₁-C₆alkoxy;
R⁴ and R⁵ are each independently selected from the group consisting of hydrogen, halogen, deuterium, hydroxyl, -CN, -NO₂, -NR^{a}R^{b}, C₁-C₆alkyl, C₂-C₆alkenyl, C₂-C₆alkynyl, C₁-C₆alkoxy, C₃-C₆cycloalkyl, phenyl, -C(=O)NR^{a}R^{b}, -NR^{a}(C=O)R^{b}, - O(C=O)NR^{a}R^{b}, -NR^{a}(C=O)OR^{b}, -NR^{a}(C=O)NR^{a}R^{b}, -(C=O)C₁-C₆alkyl, -(C=O)OC₁-C₆alkyl, -O(C=O)C₁-C₆alkyl, -O(C=O)OC₁-C₆alkyl, -SH, -SC₁-C₆alkyl, -S(O)C₁-C₆alkyl, - S(O)₂C₁-C₆alkyl, -S(O)₂NR^{a}R^{b}, and -NR^{a}S(O)₂C₁-C₆alkyl, wherein the alkyl, alkenyl, alkynyl, alkoxy, cycloalkyl, and phenyl are optionally substituted with one or more substituents each independently selected from the group consisting of halogen, deuterium, hydroxyl, oxo, -NR^{a}R^{b}, C₁-C₆alkyl, and C₁-C₆alkoxy;
R⁶ is selected from the group consisting of halogen, hydrogen, deuterium, and C₁-C₃alkyl;
R⁷ and R⁸ are each independently selected from the group consisting of hydrogen, deuterium, and C₁-C₃alkyl;
R⁹ and R¹⁰ are each independently selected from the group consisting of hydrogen, deuterium, and the C₁-C₂alkyl is optionally substituted with one or more halogens;
Ring A is selected from the group consisting of 4-12 membered heterocyclyl containing at least one ring nitrogen; wherein ring A is optionally substituted with one, two, or three substituents each independently selected from R^{A};
R^{A} is independently selected for each occurrence from the group consisting of halogen, deuterium, hydroxyl, oxo, -CN, -NR^{a}R^{b}, -COOH, C₁-C₆alkyl, C₁-C₆alkoxy, C₃-C₆cycloalkyl, =CHF, and =CH-C₁-C₆alkyl, wherein the alkyl is optionally substituted with one, two, or three substituents each independently selected from the group consisting of hydroxyl, halogen, and C₁-C₃alkoxy; and
R^{a} and R^{b} are independently selected for each occurrence from the group consisting of hydrogen and C₁-C₆alkyl, wherein the alkyl is optionally substituted with one or more substituents each independently selected from the group consisting of halogen, deuterium, hydroxyl, and C₁-C₆alkoxy; or
R^{a} and R^{b}, at each occurrence, together with the nitrogen to which they are attached, are joined together to form a 4-7 membered heterocyclyl optionally substituted by one or more substituents each independently selected from the group consisting of halogen, deuterium, hydroxyl, -NR^{a1}R^{b1}, C₁-C₆alkyl, and C₁-C₆alkoxy; wherein R^{a1} and R^{b1} are independently, at each occurrence, selected from the group consisting of hydrogen and C₁-C₆alkyl, wherein the alkyl is optionally substituted with one or more substituents which are each independently selected from the group consisting of halogen, deuterium, hydroxyl, and C₁-C₆alkoxy, or R^{a1} and R^{b1}, at each occurrence, together with the nitrogen to which they are attached, are joined together to form a 4-7 membered heterocyclyl.

In some aspects, disclosed herein are compounds represented by Formula II: 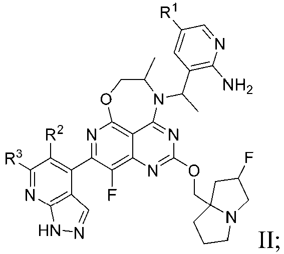 and pharmaceutically acceptable salts, stereoisomers, and tautomers thereof, wherein:
R¹ is selected from the group consisting of halogen, cyano, and C₁-C₆alkoxy; wherein the alkoxy is optionally substituted with one or more halogens;
R² is selected from the group consisting of C₁-C₄alkyl and C₃-C₆cycloalkyl, wherein the alkyl and cycloalkyl are optionally substituted with one, two, or three halogens; and
R³ is selected from the group consisting of halogen and C₁-C₄alkyl, wherein the alkyl is optionally substituted with one, two, or three halogens.

In some aspects, disclosed herein are compounds represented by Formula III: 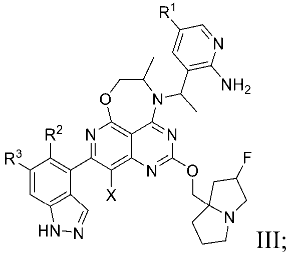 and pharmaceutically acceptable salts, stereoisomers, and tautomers thereof, wherein:
X is F or Cl;
R¹ is cyano, -C(O)NH₂, isopropyl, C₁-C₄alkoxy, -OCH₃, -OCF₃, or -OCHF₂, wherein the alkyl of the alkoxy is optionally -CH₂CN or a C₃-C₆cycloalkyl group;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens,
   or:
R¹ is fluoro;
R² is selected from the group consisting of C₁-C₄alkoxy and C₃-C₆cycloalkyl, wherein alkoxy and cycloalkyl are optionally substituted with one, two, or three halogens; and
R³ is selected from the group consisting of halogen and C₁-C₄alkyl.

In some aspects, disclosed herein are compounds represented by Formula III: 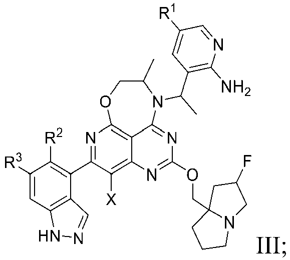 and pharmaceutically acceptable salts, stereoisomers, and tautomers thereof, wherein:
X is F or Cl;
R¹ is cyano, -C(O)NH₂, isopropyl, C₁-C₄alkoxy, -OCH₃, -OCF₃, or -OCHF₂, wherein the alkyl of the alkoxy is optionally -CH₂CN or a C₃-C₆cycloalkyl group;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens.

In some aspects, disclosed herein are compounds represented by Formula III: 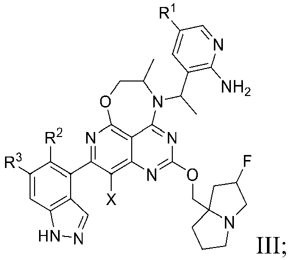 and pharmaceutically acceptable salts, stereoisomers, and tautomers thereof, wherein:
X is F or Cl;
R¹ is fluoro;
R² is selected from the group consisting of C₁-C₄alkoxy and C₃-C₆cycloalkyl, wherein alkoxy and cycloalkyl are optionally substituted with one, two, or three halogens; and
R³ is selected from the group consisting of halogen and C₁-C₄alkyl.

In some aspects, disclosed herein are compounds represented by Formula IIIa: 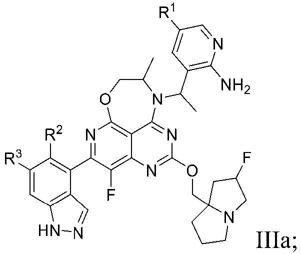 and pharmaceutically acceptable salts, stereoisomers, and tautomers thereof, wherein:
R¹ is cyano, -C(O)NH₂, isopropyl, C₁-C₄alkoxy, -OCH₃, -OCF₃, or -OCHF₂, wherein the alkyl of the alkoxy is optionally -CH₂CN, or a C₃-C₆cycloalkyl group, and wherein the alkyl may be substituted with one or more deuterium;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens;
   or:
R¹ is fluoro;
R² is selected from the group consisting of C₁-C₄alkoxy and C₃-C₆cycloalkyl, wherein the alkoxy and cycloalkyl are optionally substituted with one, two, or three halogens; and
R³ is selected from the group consisting of halogen and C₁-C₄alkyl.

In some aspects, disclosed herein are compounds represented by Formula IIIa: 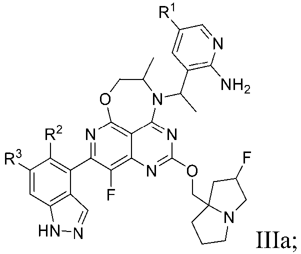 and pharmaceutically acceptable salts, stereoisomers, and tautomers thereof, wherein:
R¹ is cyano, -C(O)NH₂, isopropyl, C₁-C₄alkoxy, -OCH₃, -OCF₃, or -OCHF₂, wherein the alkyl of the alkoxy is optionally -CH₂CN, or a C₃-C₆cycloalkyl group, and wherein the alkyl may be substituted with one or more deuterium;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens.

In some aspects, disclosed herein are compounds represented by Formula IIIa: 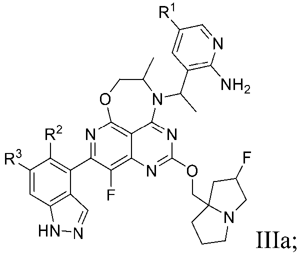 and pharmaceutically acceptable salts, stereoisomers, and tautomers thereof, wherein:
R¹ is fluoro;
R² is selected from the group consisting of C₁-C₄alkoxy and C₃-C₆cycloalkyl, wherein the alkoxy and cycloalkyl are optionally substituted with one, two, or three halogens; and
R³ is selected from the group consisting of halogen and C₁-C₄alkyl.

In some aspects, disclosed herein are compounds represented by Formula IV: 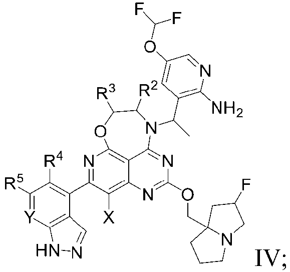 and pharmaceutically acceptable salts, stereoisomers, and tautomers thereof, wherein
X is F or Cl;
Y is N or CH;
R² and R³ are each independently selected from C₁-C₆ alkyl and hydrogen; and
R⁴ and R⁵ are independently selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogens, or one to three methyls.

In some aspects, disclosed herein are compounds represented by Formula V: 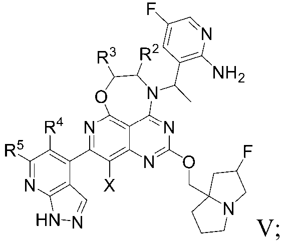 and pharmaceutically acceptable salts, stereoisomers, and tautomers thereof, wherein
X is F or Cl;
R² and R³ are each independently selected from C₁-C₆ alkyl and hydrogen; and
R⁴ and R⁵ are each independently selected from hydrogen, halogen, deuterium, C₁-C₆ alkyl, C₁-C₆ alkoxy, and C₃-C₆ cycloalkyl, wherein the alkyl, alkoxy, and cycloalkyl are optionally substituted with one to three halogen.

In some aspects, disclosed herein are compound represented by Formula VI: 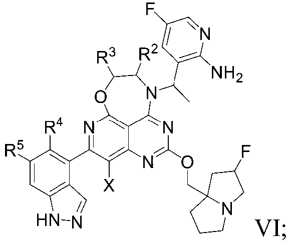
and pharmaceutically acceptable salts, stereoisomers, and tautomers thereof, wherein
X is F or Cl;
R² and R³ are each independently selected from C₁-C₆ alkyl and hydrogen;
R⁴ is C₁-C₆ alkoxy or C₃-C₆ cycloalkyl, optionally substituted with one to three halogens or one to three methyls; and
R⁵ is selected from hydrogen, deuterium, fluoro, and C₁-C₆ alkyl;
wherein the compound is not:

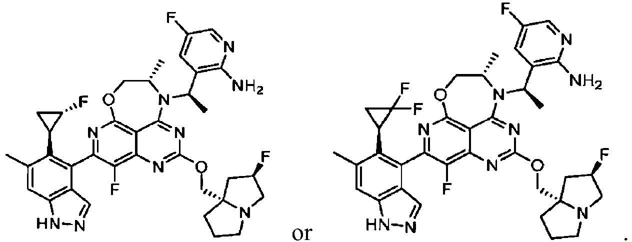

In some aspects, disclosed herein are compound represented by Formula VII: 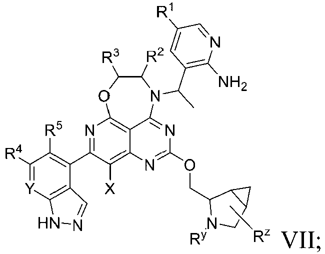 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with; or C₁-C₆ alkoxy substituted with one or more halogens;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one or more halogens, and
R⁵ is C₃-C₆ cycloalkyl optionally substituted with one or more halogens, or C₁-C₆ alkoxy substituted with one or more halogens;
wherein when R¹ is halogen, R⁵ is C₁-C₆ alkoxy substituted with one or more halogens, or C₄-C₆ cycloalkyl.

In some aspects, disclosed herein are compound represented by Formula VIII: 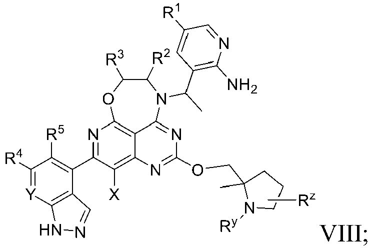 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with one or more halogens; or C₁-C₆ alkoxy substituted with one or more halogens;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one or more halogens, and
R⁵ is C₃-C₆ cycloalkyl optionally substituted with one or more halogens, or C₁-C₆ alkoxy substituted with one or more halogens;
wherein when R¹ is halogen, R⁵ is C₁-C₆ alkoxy substituted with one or more halogens, or C₄-C₆ cycloalkyl.

In some aspects, disclosed herein are compound represented by Formula IX: 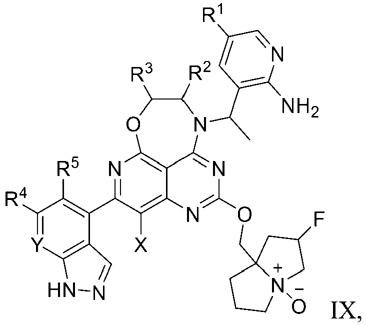 or a pharmaceutically acceptable salt, stereoisomer, or tautomer thereof, wherein:
X is F or Cl;
Y is N or CH;
R¹ is halogen, C₃-C₆ cycloalkyl optionally substituted with one to three halogen; or
C₁-C₆
   alkoxy substituted with one to three halogen;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen, and
R⁵ is C₃-C₆ cycloalkyl optionally substituted with one to three halogen, or C₁-C₆ alkoxy substituted with one to three halogen.

In some aspects, when R¹ is halogen, R⁵ is C₁-C₆ alkoxy substituted with one to three halogen, or C₄-C₆ cycloalkyl.

Also disclosed herein are pharmaceutical compositions comprising at least one compound of the disclosure or a pharmaceutically acceptable salt, stereoisomer, or tautomer thereof and at least one pharmaceutically acceptable excipient. In certain embodiments, the pharmaceutical compositions comprise at least one additional therapeutic agent.

Also disclosed herein is a compound of the disclosure or a pharmaceutically acceptable salt, stereoisomer, or tautomer thereof, for use as a medicament.

Further disclosed herein are methods of treating a patient suffering from a condition, disease, or disorder that is affected by, associated with, or would benefit from inhibition of Ras (e.g., K-Ras), comprising administering to the patient a therapeutically effective amount of a compound disclosed herein or a pharmaceutically acceptable salt, stereoisomer, or tautomer thereof, or a pharmaceutical composition thereof.

For example, disclosed herein are methods of treating a Ras protein-related (e.g., a K-Ras protein-related) disease or disorder in a patient in need thereof, comprising administering to the patient a therapeutically effective amount of a compound disclosed herein or a pharmaceutically acceptable salt, stereoisomer, or tautomer thereof, or a pharmaceutical composition thereof. For example, in some embodiments, the compounds and compositions disclosed herein may be used to treat a cancer having one or more Ras mutations (e.g., a K-Ras mutation). In some embodiments, the methods described herein may be useful to treat cancers (e.g., cancers having one or more Ras alterations (e.g., one or more amplifications or mutations) including, but not limited to, pancreatic cancer, colorectal cancer, multiple myeloma, lung adenocarcinoma, melanoma, endometrial cancer, uterine cancer, thyroid, acute myelocytic leukemia, bladder carcinoma, gastric cancer, cervical cancer, and head and neck squamous cell carcinoma.

Also disclosed herein are methods of inhibiting K-Ras G12C, K-Ras G12D, and K-Ras G12V in a subject in need thereof, comprising administering to the subject a therapeutically effective amount of the compound of the disclosure or a pharmaceutically acceptable salt, stereoisomer, or tautomer thereof.

Also disclosed herein are methods of inhibiting a Ras protein (e.g., a K-Ras protein) in a cell or tissue, comprising contacting the cell or tissue with a therapeutically effective amount of a compound disclosed herein or a pharmaceutically acceptable salt, stereoisomer, or tautomer thereof, or a pharmaceutical composition thereof.

Further disclosed herein is a compound of the disclosure or a pharmaceutically acceptable salt, stereoisomer, or tautomer thereof for use in the manufacture of a medicament for treating a Ras protein-related (e.g., a K-Ras protein-related) disease or disorder or inhibiting K-Ras in a patient in need thereof.

Further disclosed herein is a compound of the disclosure or a pharmaceutically acceptable salt, stereoisomer, or tautomer thereof for use in a method of treating a Ras protein-related (e.g., a K-Ras protein-related) disease or disorder or inhibiting K-Ras in a patient in need thereof. Further disclosed herein is a use of a compound of the disclosure or a pharmaceutically acceptable salt, stereoisomer, or tautomer thereof in the manufacture of a medicament for treating a Ras protein-related (e.g., a K-Ras protein-related) disease or disorder or inhibiting K-Ras in a patient in need thereof. Further disclosed herein is a use of a compound of the disclosure or a pharmaceutically acceptable salt, stereoisomer, or tautomer thereof in a method of treating a Ras protein-related (e.g., a K-Ras protein-related) disease or disorder or inhibiting K-Ras in a patient in need thereof.

Also disclosed herein is a process of preparing a compound of the disclosure or a pharmaceutically acceptable salt, stereoisomer, or tautomer thereof. Also disclosed herein is a compound of the disclosure or a pharmaceutically acceptable salt, stereoisomer, or tautomer thereof, which is obtained by or obtainable by this process.

### BRIEF DESCRIPTIONS OF THE DRAWINGS

The patent or application file contains at least one drawing executed in color. Copies of this patent or patent application publication with color drawing(s) will be provided by the Office upon request and payment of the necessary fee.

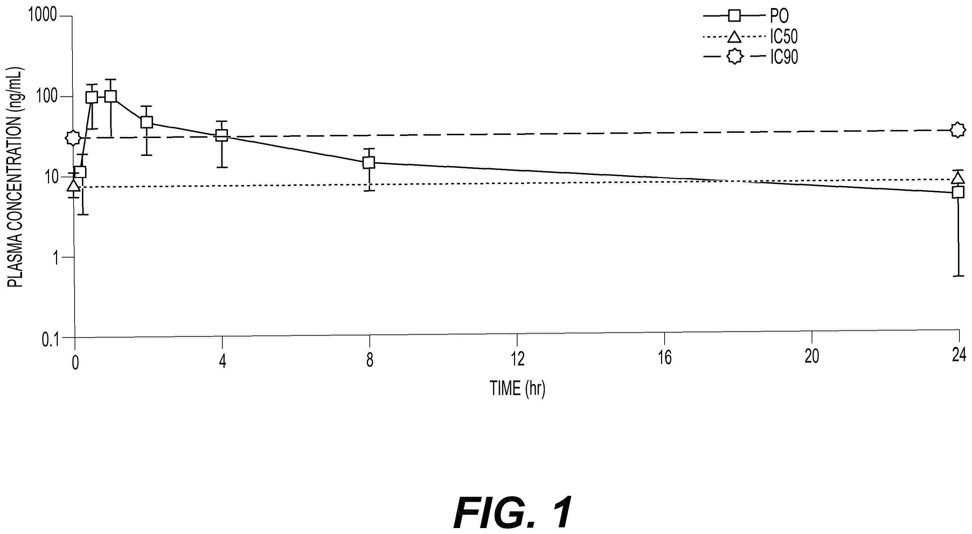

**Figure 1** shows the mean plasma concentration of compound 106 as a function of time. The mean plasma concentration was measured in beagle dogs orally administered a 1.5 mg/kg dose of compound 106. The IC₃₀ and IC₉₀ concentrations of compound 106 are also displayed. The mean plasma concentration of compound 106 exceeds its IC₅₀ and IC₉₀ for extended periods of time (~20 h and ~4 h respectively).

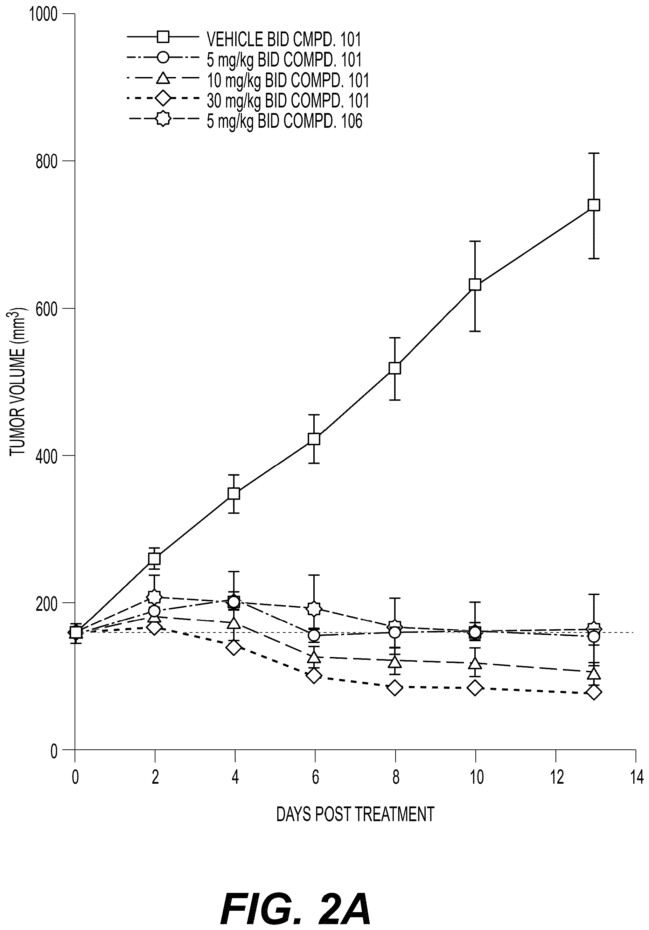

**Figure 2A** shows the effect of compounds of the disclosure on tumor volume over time in a GP2D KRAS G12D CRC model. Female (6-8 week old) Balb/c nude mice were administered a control (vehicle only), 5 mg/kg, 10 mg/kg, or 30 mg/kg dose of compound 101 twice a day (BID), or a 5 mg/kg dose of compound 106 twice a day (BID).

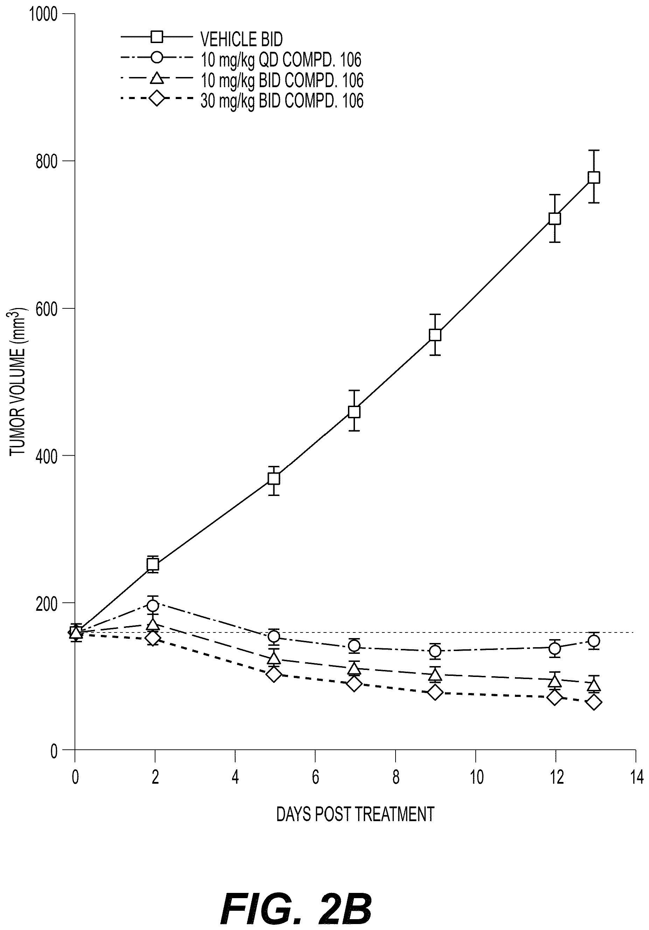

**Figure 2B** shows the effect of compounds of the disclosure on tumor volume over time in a GP2D KRAS G12D CRC xenograft tumor model. Female (6-8 week old) Balb/c nude mice were administered a control (vehicle only), a 10 mg/kg or 30 mg/kg dose of compound 106 twice a day, or a 10 mg/kg dose of compound 106 once a day.

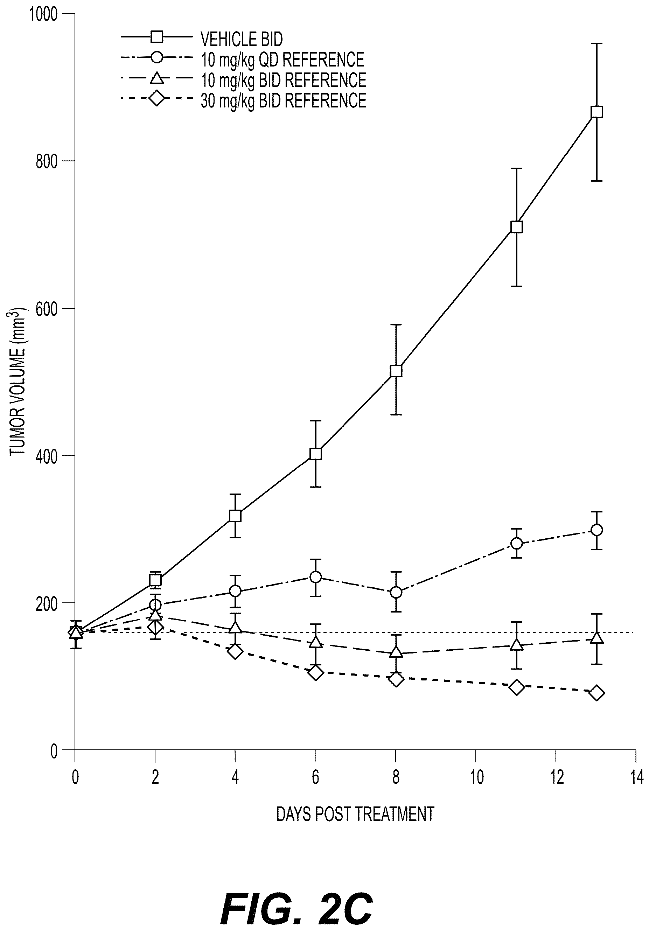

**Figure 2C** shows the effect of a reference compound (as defined in Example 49) on tumor volume over time in a GP2D KRAS G12D CRC xenograft tumor model. Female (6-8 week old) Balb/c nude mice were administered a control (vehicle only), a 10 mg/kg, or 30 mg/kg dose of the reference compound twice a day (BID), or a 10 mg/kg dose of the reference compound once a day (QD).

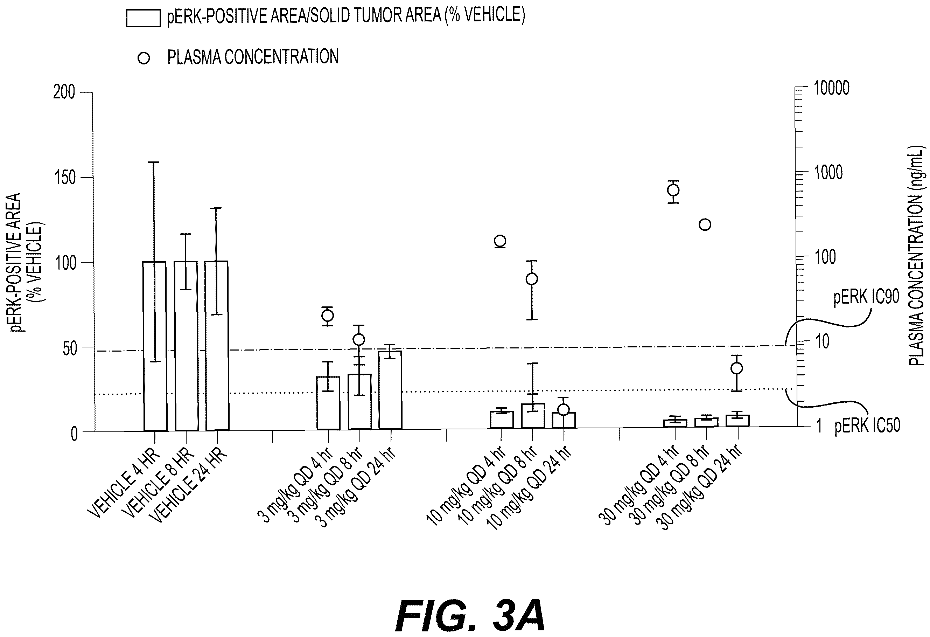

**Figure 3A** shows inhibition of phosphorylation of extracellular signal-regulated kinase (pERK) and plasma concentration in human cancer cells (GP2D KRAS G12D-driven CRC model) after administration of a control (vehicle only), or compound 106 at 4 h, 8 h, and 24 h after administration of a 3 mg/kg, 10 mg/kg, or 30 mg/kg dose once a day.

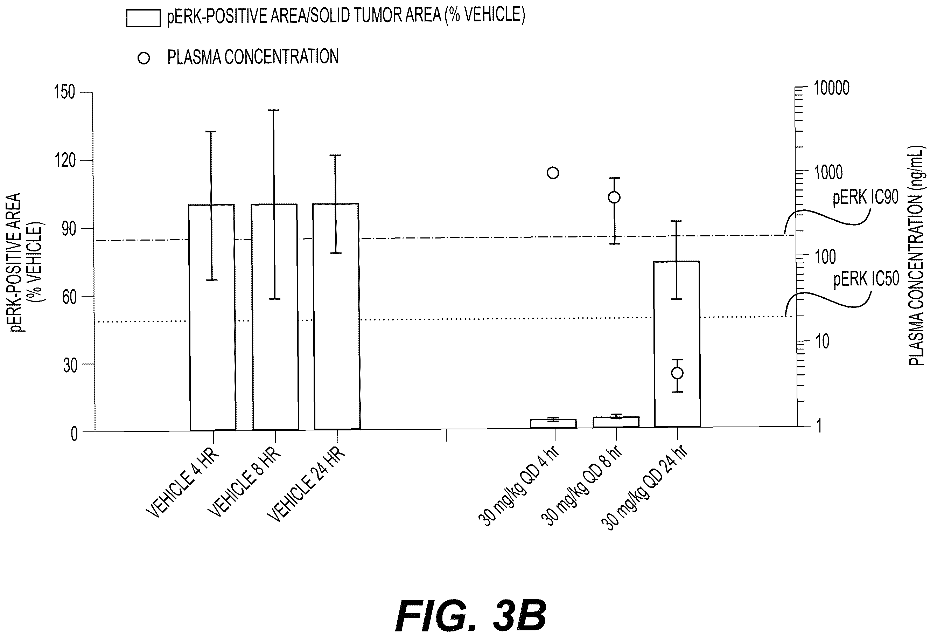

**Figure 3B** shows inhibition of phosphorylation of extracellular signal-regulated kinase (pERK) and plasma concentration in human cancer cells (GP2D KRAS G12D-driven CRC model) after administration of a control (vehicle only), or a reference compound (as defined in Example 50) at 4 h, 8 h, and 24 h after administration of a 3 mg/kg, 10 mg/kg, or 30 mg/kg dose once a day.

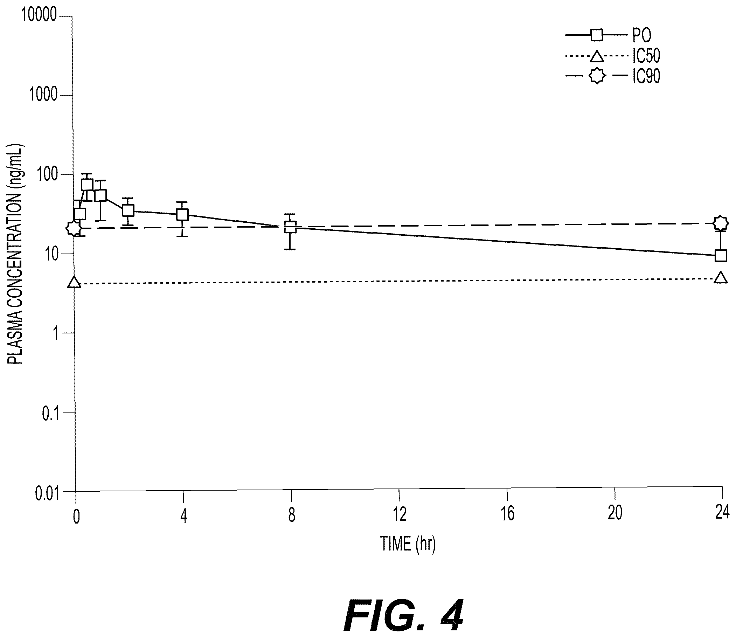

**Figure 4** shows the mean plasma concentration of compound 101 as a function of time. The mean plasma concentration was measured in beagle dogs orally administered a 1.5 mg/kg dose of compound 101. The IC₅₀ and IC₉₀ concentrations of compound 101 are also displayed. The mean plasma concentration (PO) of compound 101 exceeds its IC₅₀ and IC₉₀ for extended periods of time.

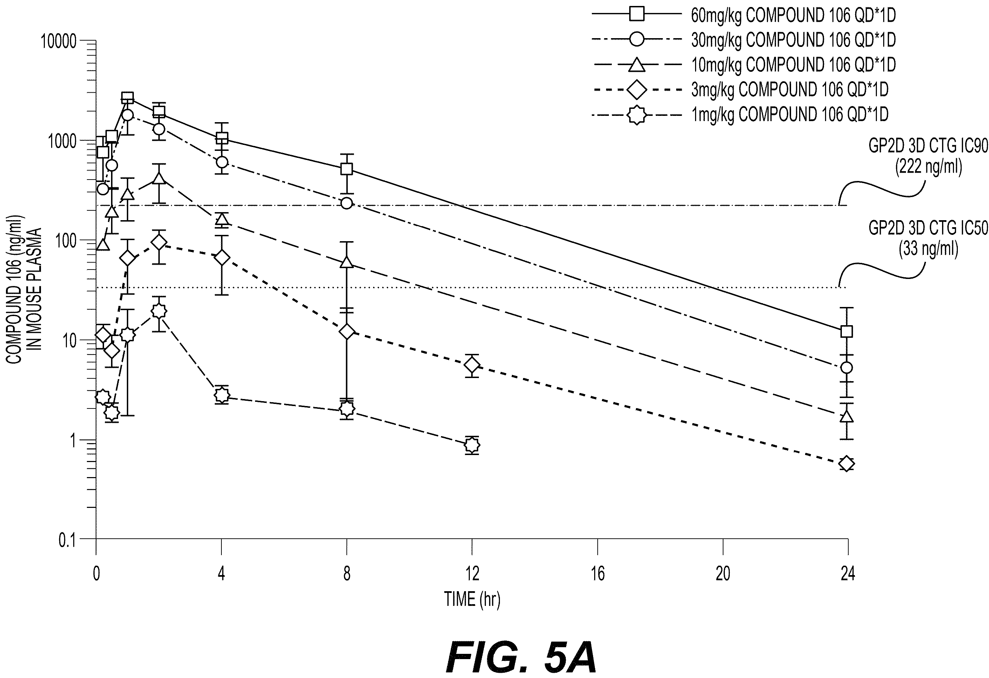

**Figure 5A** shows antiproliferative activities and plasma exposure of compound 106 measured in mouse plasma over time in a GP2D KRAS G12D CRC xenograft tumor model. The IC₅₀ and IC₉₀ concentrations of compound 106 are also displayed. Female (6-8 week old) Balb/c nude mice were administered varied doses of compound 106 once a day (QD).

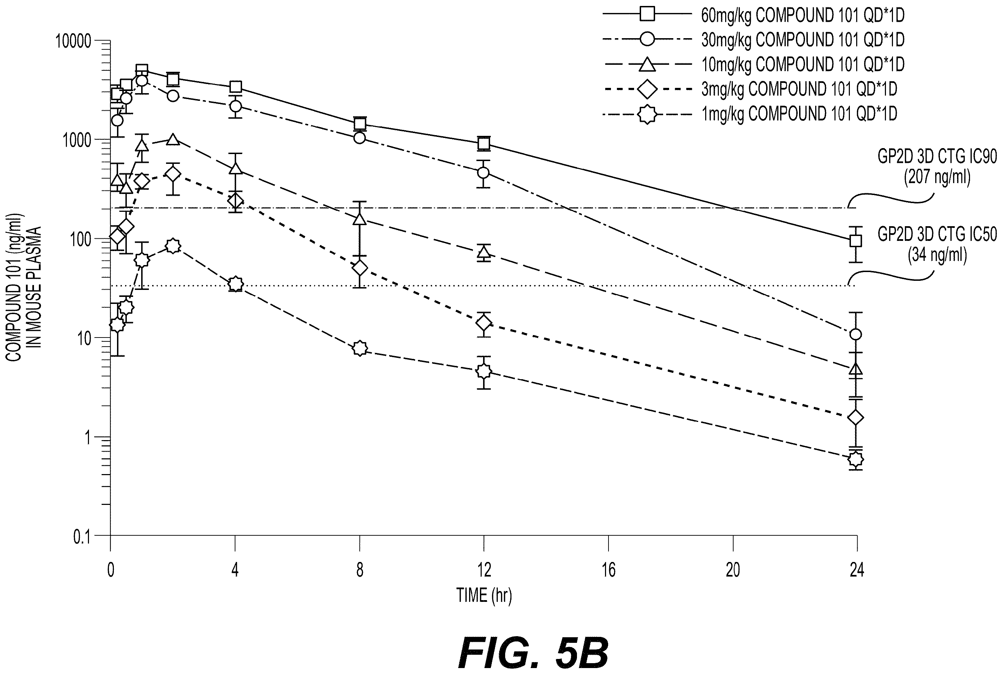

**Figure 5B** shows antiproliferative activities of compound 101 measured in mouse plasma over time in a GP2D KRAS G12D CRC xenograft tumor model. The IC₅₀ and IC₉₀ concentrations of compound 101 are also displayed. Female (6-8 week old) Balb/c nude mice were administered varied doses of compound 101 once a day (QD).

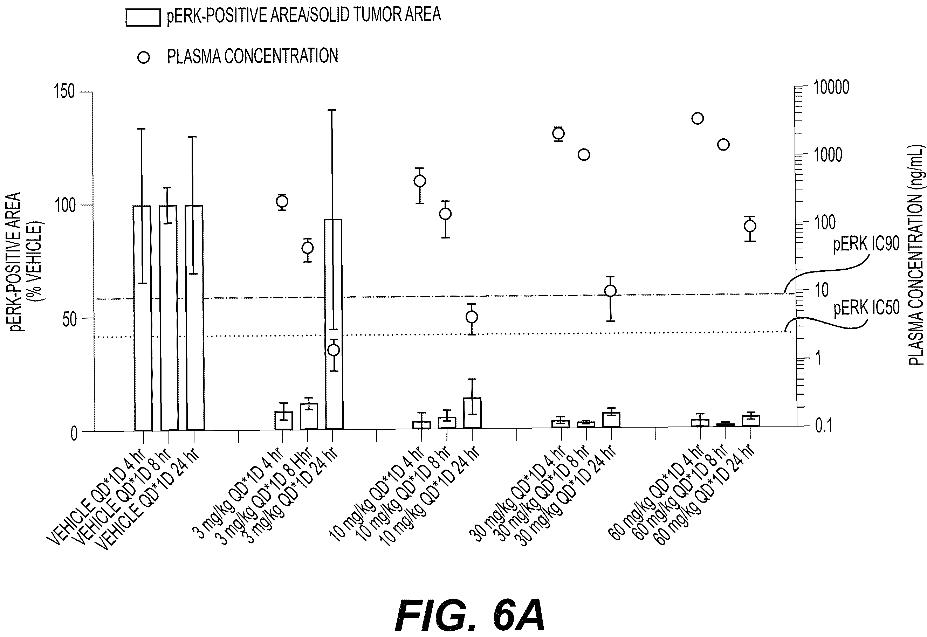

**Figure 6A** shows inhibition of phosphorylation of extracellular signal-regulated kinase (pERK) and plasma concentration in human cancer cells (GP2D KRAS G12D-driven CRC model) after administration of a control (vehicle only), or compound 101 at 4 h, 8 h, and 24 h after administration of a 3 mg/kg, 10 mg/kg, 30 mg/kg, or 60 mg/kg dose once a day (QD).

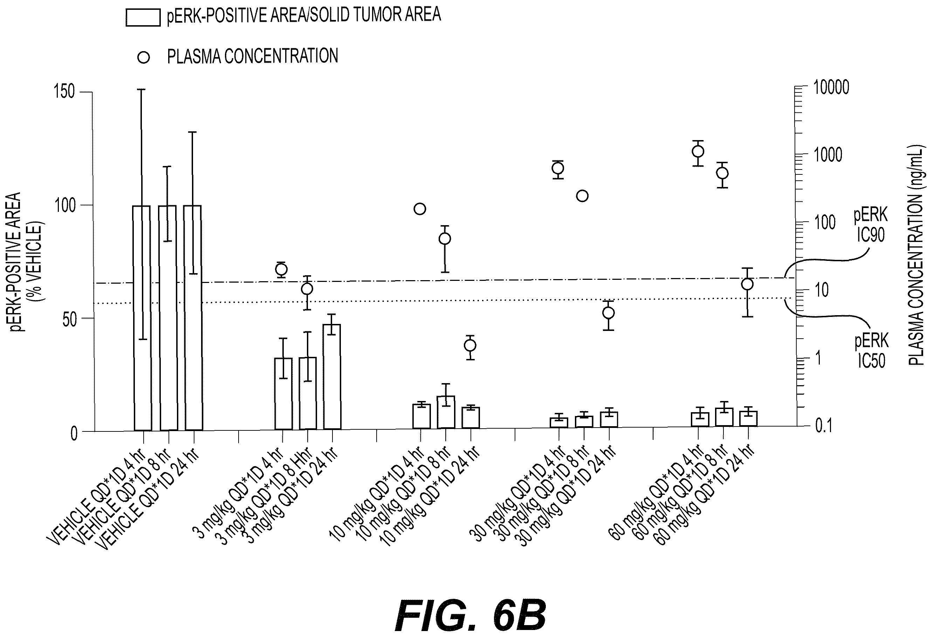

**Figure 6B** shows inhibition of phosphorylation of extracellular signal-regulated kinase (pERK) and plasma concentration in human cancer cells (GP2D KRAS G12D-driven CRC model) after administration of a control (vehicle only), or compound 106 at 4 h, 8 h, and 24 h after administration of a 3 mg/kg, 10 mg/kg, 30 mg/kg, or 60 mg/kg dose once a day (QD).

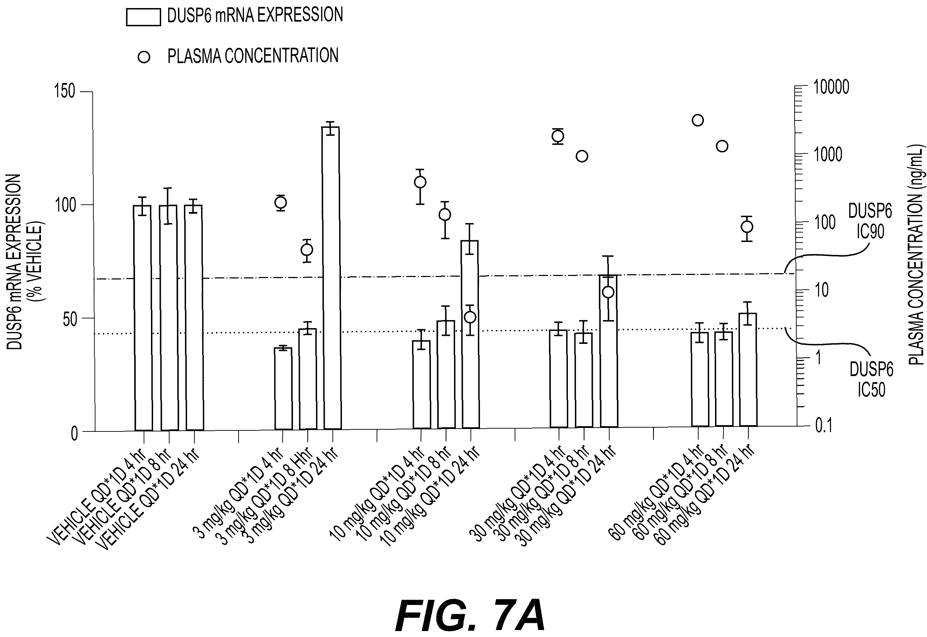

**Figure 7A** shows DUSP6 mRNA expression and plasma concentration in human cancer cells (GP2D KRAS G12D-driven CRC model) after administration of compound 101 at 4 h, 8 h, and 24 h after administration of a 3 mg/kg, 10 mg/kg, 30 mg/kg, or 60 mg/kg dose once a day (QD).

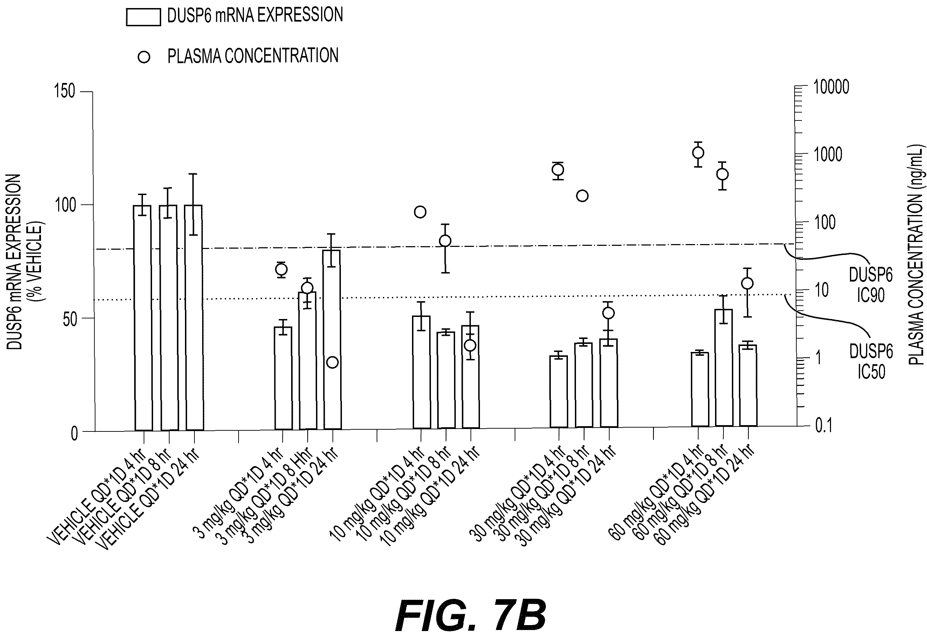

**Figure 7B** shows DUSP6 mRNA expression and plasma concentration in human cancer cells (GP2D KRAS G12D-driven CRC model) after administration of compound 106 at 4 h, 8 h, and 24 h after administration of a 3 mg/kg, 10 mg/kg, 30 mg/kg, or 60 mg/kg dose once a day (QD).

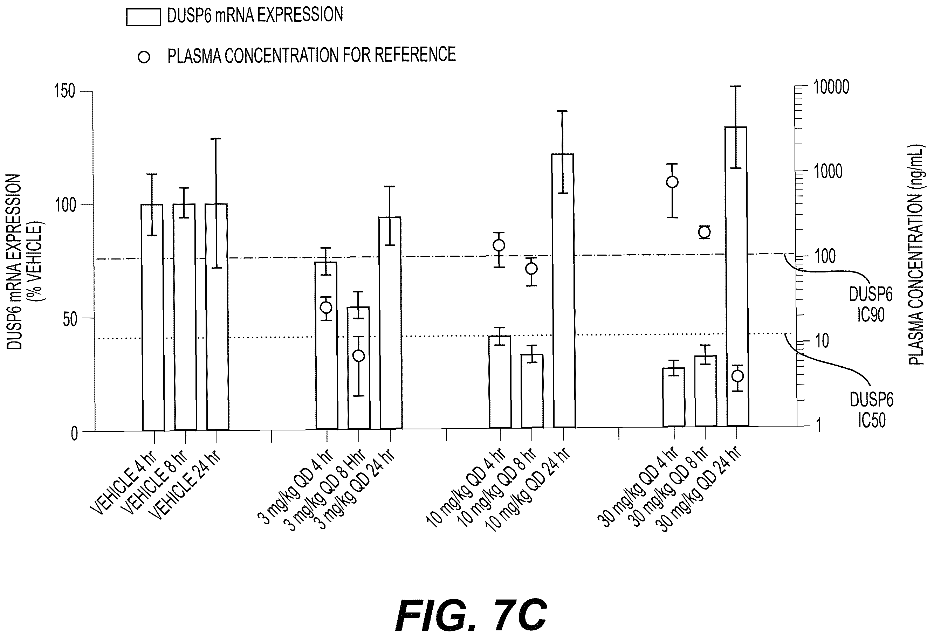

**Figure 7C** shows DUSP6 mRNA expression and plasma concentration in human cancer cells (GP2D KRAS G12D-driven CRC model) after administration of a reference compound (as presented in Example 50) at 4 h, 8 h, and 24 h after administration of a 3 mg/kg, 10 mg/kg, 30 mg/kg, or 60 mg/kg dose once a day (QD).

### DETAILED DESCRIPTION

The features and other details of the disclosure will now be more particularly described. Before further description of the present disclosure, certain terms employed in the specification, examples and appended claims are collected here. These definitions should be read in light of the remainder of the disclosure and as understood by a person of skill in the art. Unless defined otherwise, all technical and scientific terms used herein have the same meaning as commonly understood by a person of ordinary skill in the art.

### Definitions

The term "treating" includes any effect, e.g., lessening, reducing, modulating, or eliminating, that results in the improvement of the condition, disease, disorder and the like.

The term "alkyl" as used herein refers to a saturated straight or branched hydrocarbon. Exemplary alkyl groups include, but are not limited to, straight or branched hydrocarbons of 1-6, 1-4, or 1-3 carbon atoms, referred to herein as C₁₋₆alkyl, C₁₋₄alkyl, and C₁₋₃alkyl, respectively. Exemplary alkyl groups include, but are not limited to, methyl, ethyl, propyl, isopropyl, 2-methyl-1-butyl, 3-methyl-2-butyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, etc.

The term "alkenyl" as used herein refers to an unsaturated straight or branched hydrocarbon having at least one carbon-carbon double bond. Exemplary alkenyl groups include, but are not limited to, a straight or branched group of 2-6 or 3-4 carbon atoms, referred to herein as C₁-C₅alkenyl, C₂-C₆alkenyl, and C₃-C₄alkenyl, respectively. Exemplary alkenyl groups include, but are not limited to, vinyl, allyl, butenyl, pentenyl, etc.

The term "alkynyl" as used herein refers to an unsaturated straight or branched hydrocarbon having at least one carbon-carbon triple bond. Exemplary alkynyl groups include, but are not limited to, straight or branched groups of 2-6, or 3-6 carbon atoms, referred to herein as C₂₋6alkynyl, and C₃₋₆alkynyl, respectively. Exemplary alkynyl groups include, but are not limited to, ethynyl, propynyl, butynyl, pentynyl, hexynyl, methylpropynyl, etc.

The term "alkoxy" as used herein refers to a straight or branched alkyl group attached to oxygen (alkyl-O-). Exemplary alkoxy groups include, but are not limited to, alkoxy groups of 1-6 or 2-6 carbon atoms, referred to herein as C₁-C₅alkoxy, C₁-C₆alkoxy, and C₂-C₆alkoxy, respectively. Exemplary alkoxy groups include, but are not limited to methoxy, ethoxy, isopropoxy, etc.

The term "aryl" refers to a radical of a monocyclic or polycyclic (*e.g.,* bicyclic or tricyclic) 4n+2 aromatic ring system (*e.g.,* having 6, 10, or 14 p electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g.,* anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Examples of representative substituted aryls include the following 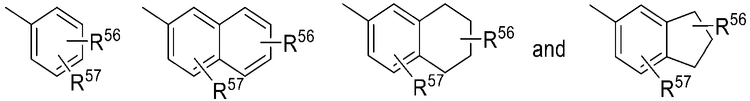 wherein one of R⁵⁶ and R⁵⁷ may be hydrogen and at least one of R⁵⁶ and R⁵⁷ is each independently selected from C₁-C₈ alkyl, C₁-C₈ haloalkyl, 4-10 membered heterocyclyl, alkanoyl, C₁-C₈ alkoxy, heteroaryloxy, alkylamino, arylamino, heteroarylamino, NR⁵⁸COR⁵⁹, NR⁵⁸SOR⁵⁹NR⁵⁸SO₂R⁵⁹, COOalkyl, COOaryl, CONR⁵⁸R⁵⁹, CONR⁵⁸OR⁵⁹, NR⁵⁸R⁵⁹, SO₂NR⁵⁸R⁵⁹, S-alkyl, SOalkyl, SO₂alkyl, Saryl, SOaryl, SO₂aryl; or R⁵⁶ and R⁵⁷ may be joined to form a cyclic ring (saturated or unsaturated) from 5 to 8 atoms, optionally containing one or more heteroatoms selected from the group N, O, or S.

The term "carbonyl" as used herein refers to the radical -C(O)-.

The term "cyano" as used herein refers to the radical -CN.

The terms "cycloalkyl" or a "carbocyclic group" as used herein refers to a saturated or partially unsaturated hydrocarbon group of, for example, 3-6, or 4-6 carbons, referred to herein as C₃-C₁₀cycloalkyl, C₃₋₆cycloalkyl or C₄₋₆cycloalkyl, respectively. Exemplary cycloalkyl groups include, but are not limited to, cyclohexyl, cyclopentyl, cyclopentenyl, cyclobutyl or cyclopropyl. In some embodiments, the cycloalkyl is a spiro cycloalkyl (e.g., spiro[2.2]pentan-1-yl). "Spiro cycloalkyl" refers to a polycyclic cycloalkyl with rings connected through one common carbon atom.

The terms "halo" or "halogen" as used herein refer to F, Cl, Br, or I.

The terms "haloalkyl" as used herein refers to an alkyl radical in which the alkyl group is substituted with one or more halogens. Typical haloalkyl groups include, but are not limited to, trifluoromethyl (i.e., CF₃), difluoromethyl, fluoromethyl, chloromethyl, dichloromethyl, dibromoethyl, tribromomethyl, tetrafluoroethyl, and the like. Exemplary haloalkyl groups include, but are not limited to, straight or branched hydrocarbons of 1-6, 1-4, or 1-3 carbon atoms substituted with a halogen (i.e., Cl, F, Br and I), referred to herein as C₁₋₆haloalkyl, C₁₋₄ haloalkyl, and C₁₋₃haloalkyl, respectively.

The term "hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, e.g., heteroalkyl, cycloalkyl, e.g., heterocyclyl, aryl, e.g., heteroaryl, cycloalkenyl, e.g., cycloheteroalkenyl, and the like having from 1 to 5, and particularly from 1 to 3 heteroatoms.

The terms "heteroaryl" or "heteroaromatic group" as used herein refers to an aromatic 5-10 membered ring system containing one or more heteroatoms, for example one to three heteroatoms, such as nitrogen, oxygen, and sulfur. The term may also be used to refer to a 5-7 membered monocyclic heteroaryl or an 8-10 membered bicyclic heteroaryl. Where possible, said heteroaryl ring may be linked to the adjacent radical though carbon or nitrogen. Examples of heteroaryl rings include but are not limited to furan, thiophene, pyrrole, pyrrolopyridine, indole, thiazole, oxazole, isothiazole, isoxazole, imidazole, benzoimidazole, imidazopyridine, pyrazole, triazole, pyridine or pyrimidine, etc.

The terms "heterocyclyl," "heterocycle," or "heterocyclic group" are art-recognized and refer to saturated or partially unsaturated 4-10 membered ring structures, whose ring structures include one to three heteroatoms, such as nitrogen, oxygen, and sulfur. Where possible, heterocyclyl rings may be linked to the adjacent radical through carbon or nitrogen. The term may also be used to refer to 4-10 membered saturated or partially unsaturated ring structures that are bridged, fused or spirocyclic ring structures, whose ring structures include one to three heteroatoms, such as nitrogen, oxygen, and sulfur. Examples of heterocyclyl groups include, but are not limited to, pyrrolidine, piperidine, morpholine, thiomorpholine, piperazine, oxetane, azetidine, tetrahydrofuran, dihydrofuran, dihydropyran, tetrahydropyran, etc. In some embodiments, the heterocycle is a spiro heterocycle (e.g., 2,8-diazaspiro[4.5]decane). In some embodiments, the heterocycle is a bridged heterocycle (e.g., octahydro-1H-4,7-methanoisoindole). "Spiro heterocyclyl," or "spiro heterocycle" refers to a polycyclic heterocyclyl with rings connected through one common atom (called a spiro atom), wherein the rings have one or more heteroatoms selected from the group consisting of N, O, and S(O)ₘ (wherein m is an integer of 0 to 2) as ring atoms.

The terms "hydroxy" and "hydroxyl" as used herein refers to the radical -OH.

The term "oxo" as used herein refers to the radical =O.

"Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, and general safety and purity standards as required by FDA Office of Biologics standards.

The term "pharmaceutically acceptable carrier" or "pharmaceutically acceptable excipient" as used herein refers to any and all solvents, dispersion media, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions.

The term "pharmaceutical composition" as used herein refers to a composition comprising at least one compound as disclosed herein formulated together with one or more pharmaceutically acceptable carriers.

"Individual," "patient," or "subject" are used interchangeably and include any animal, including mammals, preferably mice, rats, other rodents, rabbits, dogs, cats, swine, cattle, sheep, horses, or primates, and most preferably humans. The compounds of the disclosure can be administered to a mammal, such as a human, but can also be administered to other mammals such as an animal in need of veterinary treatment, *e.g.,* domestic animals (*e.g.,* dogs, cats, and the like), farm animals (*e.g.,* cows, sheep, pigs, horses, and the like) and laboratory animals (*e.g.,* rats, mice, guinea pigs, and the like). "Modulation" includes antagonism (*e.g.,* inhibition), inverse agonism, agonism, biased agonism, biased signal transduction, functionally selective agonism, partial antagonism and/or partial agonism.

In the present specification, the term "therapeutically effective amount" means the amount of the subject compound that will elicit the biological or medical response of a tissue, system or animal, (e.g., mammal or human) that is being sought by the researcher, veterinarian, medical doctor or other clinician. The compounds of the disclosure are administered in therapeutically effective amounts to treat a disease. Alternatively, a therapeutically effective amount of a compound is the quantity required to achieve a desired therapeutic and/or prophylactic effect.

The term "pharmaceutically acceptable salt(s)" as used herein refers to salts of acidic or basic groups that may be present in compounds used in the compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including, but not limited to, malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, *p*-toluenesulfonate and pamoate (i.e., 1,1'-methylene-bis-(2-hydroxy-3-naphthoate)) salts. Compounds included in the present compositions that are acidic in nature are capable of forming base salts with various pharmacologically acceptable cations. Examples of such salts include alkali metal or alkaline earth metal salts, particularly calcium, magnesium, sodium, lithium, zinc, potassium, and iron salts. Compounds included in the present compositions that include a basic or acidic moiety may also form pharmaceutically acceptable salts with various amino acids. The compounds of the disclosure may contain both acidic and basic groups; for example, one amino and one carboxylic acid group. In such a case, the compound can exist as an acid addition salt, a zwitterion, or a base salt.

The compounds of the disclosure may contain one or more chiral centers and, therefore, exist as stereoisomers. Stereoisomers of the compounds may include optical isomers such as R and S enantiomers, diastereomers, rotational isomers, atropisomers, and conformational isomers. For example, compounds of the invention containing one or more asymmetric carbon atoms may exist as two or more stereoisomers. The term "stereoisomers" when used herein consist of all enantiomers or diastereomers. These compounds may be designated by the symbols "(+)," "(-)," "R" or "S," depending on the configuration of substituents around the stereogenic carbon atom, but the skilled artisan will recognize that a structure may denote a chiral center implicitly. The present disclosure encompasses various stereoisomers of these compounds and mixtures thereof. Mixtures of enantiomers or diastereomers may be designated "(±)" in nomenclature, but the skilled artisan will recognize that a structure may denote a chiral center implicitly.

The compounds of the disclosure may contain one or more double bonds and, therefore, exist as geometric isomers resulting from the arrangement of substituents around a carbon-carbon double bond. The symbol - - - denotes a bond that may be a single, double or triple bond as described herein. Substituents around a carbon-carbon double bond are designated as being in the *"Z"* or "*E*" configuration wherein the terms "*Z*" and "*E*" are used in accordance with IUPAC standards. Unless otherwise specified, structures depicting double bonds encompass both the "*E*" and "*Z*" isomers. Substituents around a carbon-carbon double bond alternatively can be referred to as "cis" or "trans," where "cis" represents substituents on the same side of the double bond and "trans" represents substituents on opposite sides of the double bond.

Compounds of the disclosure may contain a carbocyclic or heterocyclic ring and therefore, exist as geometric isomers resulting from the arrangement of substituents around the ring. The arrangement of substituents around a carbocyclic or heterocyclic ring are designated as being in the *"Z"* or "*E*" configuration wherein the terms "*Z*" and "*E*" are used in accordance with IUPAC standards. Unless otherwise specified, structures depicting carbocyclic or heterocyclic rings encompass both "*Z*" and "*E*" isomers. Substituents around a carbocyclic or heterocyclic rings may also be referred to as "cis" or "trans", where the term "cis" represents substituents on the same side of the plane of the ring and the term "trans" represents substituents on opposite sides of the plane of the ring. Mixtures of compounds wherein the substituents are disposed on both the same and opposite sides of plane of the ring are designated "cis/trans."

Individual enantiomers and diastereomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well known methods, such as chiral-phase liquid chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art. Stereoselective syntheses encompass both enantio- and diastereoselective transformations and may involve the use of chiral auxiliaries. For examples, see Carreira and Kvaerno, Classics in Stereoselective Synthesis, Wiley-VCH: Weinheim, 2009.

The compounds disclosed herein can exist in solvated as well as unsolvated forms with pharmaceutically acceptable solvents such as water, ethanol, and the like, and it is intended that the disclosure embrace both solvated and unsolvated forms. In one embodiment, the compound is amorphous. In one embodiment, the compound is a single polymorph. In another embodiment, the compound is a mixture of polymorphs. In another embodiment, the compound is in a crystalline form.

The disclosure also embraces isotopically labeled compounds of the disclosure which are identical to those recited herein, except that one or more atoms are replaced by an atom having an atomic mass or mass number different from the atomic mass or mass number usually found in nature. Examples of isotopes that can be incorporated into compounds of the disclosure include isotopes of hydrogen, carbon, nitrogen, oxygen, phosphorus, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. For example, a compound of the disclosure may have one or more H atom replaced with deuterium.

Certain isotopically labeled disclosed compounds (*e.g.,* those labeled with ³H and ¹⁴C) are useful in compound and/or substrate tissue distribution assays. Tritiated (*i.e.,* ³H) and carbon-14 (*i.e.,* ¹⁴C) isotopes are particularly preferred for their ease of preparation and detectability. Further, substitution with heavier isotopes such as deuterium (*i.e.,* ²H) may afford certain therapeutic advantages resulting from greater metabolic stability (*e.g.,* increased *in vivo* half-life or reduced dosage requirements) and hence may be preferred in some circumstances. Isotopically labeled compounds of the disclosure can generally be prepared by following procedures analogous to those disclosed in the examples herein by substituting an isotopically labeled reagent for a non-isotopically labeled reagent.

The term "prodrug" refers to compounds that are transformed *in vivo* to yield a disclosed compound or a pharmaceutically acceptable salt, hydrate or solvate of the compound. The transformation may occur by various mechanisms (such as by esterase, amidase, phosphatase, oxidative and or reductive metabolism) in various locations (such as in the intestinal lumen or upon transit of the intestine, blood or liver). Prodrugs are well known in the art (for example, see Rautio, Kumpulainen, et al, Nature Reviews Drug Discovery 2008, 7, 255). For example, if a compound of the disclosure or a pharmaceutically acceptable salt, hydrate or solvate of the compound contains a carboxylic acid functional group, a prodrug can comprise an ester formed by the replacement of the hydrogen atom of the acid group with a group such as (C₁₋₈)alkyl, (C₂₋₁₂)alkylcarbonyloxymethyl, 1-(alkylcarbonyloxy)ethyl having from 4 to 9 carbon atoms, 1-methyl-1-(alkylcarbonyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁₋₂)alkylamino(C₂₋₃)alkyl (such as β-dimethylaminoethyl), carbamoyl-(C₁₋₂)alkyl, N,N-di(C₁₋₂)alkylcarbamoyl-(C₁₋₂)alkyl and piperidino-, pyrrolidino- or morpholino(C₂₋₃)alkyl.

Similarly, if a compound of the disclosure contains an alcohol functional group, a prodrug can be formed by the replacement of the hydrogen atom of the alcohol group with a group such as (C₁₋₆)alkylcarbonyloxymethyl, 1-((C₁₋₆)alkylcarbonyloxy)ethyl, 1-methyl-1-((C₁₋₆)alkylcarbonyloxy)ethyl (C₁₋₆)alkoxycarbonyloxymethyl, N-(C₁₋₆)alkoxycarbonylaminomethyl, succinoyl, (C₁₋₆)alkylcarbonyl, α-amino(C₁₋₄)alkylcarbonyl, arylalkylcarbonyl and α-aminoalkylcarbonyl, or α-aminoalkylcarbonyl-α-aminoalkylcarbonyl, where each α -aminoalkylcarbonyl group is independently selected from the naturally occurring L-amino acids, P(O)(OH)₂, -P(O)(O(C₁₋₆)alkyl)₂ or glycosyl (the radical resulting from the removal of a hydroxyl group of the hemiacetal form of a carbohydrate).

If a compound of the disclosure incorporates an amine functional group, a prodrug can be formed, for example, by creation of an amide or carbamate, an N-alkylcarbonyloxyalkyl derivative, an (oxodioxolenyl)methyl derivative, an N-Mannich base, imine or enamine. In addition, a secondary amine can be metabolically cleaved to generate a bioactive primary amine, or a tertiary amine can metabolically cleaved to generate a bioactive primary or secondary amine. For examples, see Simplicio, et al., Molecules 2008, 13, 519 and references therein.

### I. Compounds

The disclosure is directed, in part, to compounds that inhibit Ras, for example, multiple mutated forms of Ras, for example, K-Ras. In some embodiments, the present disclosure provides a method of treating a disease or disorder characterized by aberrant Ras activity due to a Ras mutant (e.g., aberrant K-Ras activity due to a K-Ras mutant). In some embodiments, the disease or disorder is a cancer.

For example, disclosed herein is a compound represented by Formula I: 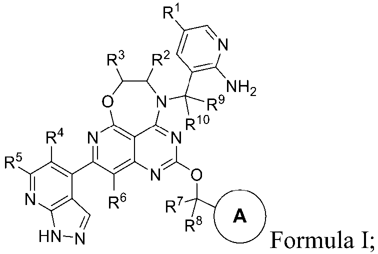 or a pharmaceutically acceptable salt, stereoisomer, or tautomer thereof, wherein:
R¹ is selected from the group consisting of halogen, C₁-C₆alkoxy, C₃-C₄cycloalkyl, C₁-C₆alkyl, C₂-C₆alkenyl, and C₂-C₆alkynyl; wherein the alkoxy, cycloalkyl, alkyl, alkenyl, and alkynyl are optionally substituted with one or more halogens;
R² and R³ are each independently selected from the group consisting of C₁-C₆alkyl and hydrogen, wherein the C₁-C₆alkyl is optionally substituted with one or more halogens or hydroxyl; or
R² and R³, together with the atoms to which they are attached, may be joined together to form a 4-7 membered heterocyclyl or a 5-7 membered cycloalkyl; wherein the heterocyclyl and cycloalkyl are optionally substituted with one or more substituents each independently selected from the group consisting of halogen, deuterium, hydroxyl, -NR^{a}R^{b}, C₁-C₆alkyl, and C₁-C₆alkoxy;
R⁴ and R⁵ are each independently selected from the group consisting of hydrogen, halogen, deuterium, hydroxyl, -CN, -NO₂, -NR^{a}R^{b}, C₁-C₆alkyl, C₂-C₆alkenyl, C₂-C₆alkynyl, C₁-C₆alkoxy, C₃-C₆cycloalkyl, phenyl, -C(=O)NR^{a}R^{b}, -NR^{a}(C=O)R^{b}, -O(C=O)NR^{a}R^{b}, - NR^{a}(C=O)OR^{b}, -NR^{a}(C=O)NR^{a}R^{b}, -(C=O)C₁-C₆alkyl, -(C=O)OC₁-C₆alkyl, -O(C=O)C₁-C₆alkyl, -O(C=O)OC₁-C₆alkyl, -SH, -SC₁-C₆alkyl, -S(O)C₁-C₆alkyl, -S(O)₂C₁-C₆alkyl, - S(O)₂NR^{a}R^{b}, and -NR^{a}S(O)₂C₁-C₆alkyl, wherein the alkyl, alkenyl, alkynyl, alkoxy, cycloalkyl, and phenyl are optionally substituted with one or more substituents each independently selected from the group consisting of halogen, deuterium, hydroxyl, oxo, - NR^{a}R^{b}, C₁-C₆alkyl, and C₁-C₆alkoxy;
R⁶ is selected from the group consisting of halogen, hydrogen, deuterium, and C₁-C₃alkyl;
R⁷ and R⁸ are each independently selected from the group consisting of hydrogen, deuterium, and C₁-C₃alkyl;
R⁹ and R¹⁰ are each independently selected from the group consisting of hydrogen, deuterium, and the C₁-C₂alkyl is optionally substituted with one or more halogens;
Ring A is selected from the group consisting of 4-12 membered heterocyclyl containing at least one ring nitrogen; wherein ring A is optionally substituted with one, two, or three substituents each independently selected from R^{A};
R^{A} is independently selected for each occurrence from the group consisting of halogen, deuterium, hydroxyl, oxo, -CN, -NR^{a}R^{b}, -COOH, C₁-C₆alkyl, C₁-C₆alkoxy, C₃-C₆cycloalkyl, =CHF, and =CH-C₁-C₆alkyl; wherein the C₁-C₆alkyl is optionally substituted with one, two, or three substituents each independently selected from the group consisting of hydroxyl, halogen, and C₁-C₃alkoxy; and
R^{a} and R^{b} are independently selected for each occurrence from the group consisting of hydrogen and C₁-C₆alkyl, wherein the C₁-C₆alkyl is optionally substituted with one or more substituents each independently selected from the group consisting of halogen, deuterium, hydroxyl, and C₁-C₆alkoxy; or
R^{a} and R^{b}, together with the nitrogen to which they are attached, may be joined together to form a 4-7 membered heterocyclyl optionally substituted by one or more substituents each independently selected from the group consisting of halogen, deuterium, hydroxyl, -NR^{a1}R^{b1}, C₁-C₆alkyl, and C₁-C₆alkoxy; wherein R^{a1} and R^{b1} are independently, at each occurrence, selected from the group consisting of hydrogen and C₁-C₆alkyl, wherein the alkyl is optionally substituted with one or more substituents which are each independently selected from the group consisting of halogen, deuterium, hydroxyl, and C₁-C₆alkoxy, or R^{a1} and R^{b1}, at each occurrence, together with the nitrogen to which they are attached, are joined together to form a 4-7 membered heterocyclyl.

In some embodiments, a compound of the present disclosure may be represented by Formula IA, for example:

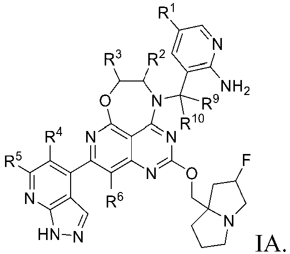

For example, in some embodiments, R¹ is selected from the group consisting of fluoro, chloro, -OCH₃, -OCHF₂, and -OCF₃. In some embodiments, R² is selected from the group consisting of, for example, -CH₃ and hydrogen. In further embodiments, R⁴ and R⁵ are each independently selected from the group consisting of , for example, halogen, hydroxyl, cyano, -NH₂, -C(O)NH₂, C₁-C₄alkyl, C₂-C₄alkynyl, C₁-C₄alkoxy, and C₃-C₆cycloalkyl, wherein the alkyl, alkynyl, alkoxy, and cycloalkyl are optionally substituted with one, two, or three halogens or -CN.

In some embodiments, R⁴ is selected from the group consisting of C₁-C₄alkyl and C₃-C₆cycloalkyl, wherein the alkyl and cycloalkyl are optionally substituted with one, two, or three halogens. For example, in certain embodiments R⁴ is selected from the group consisting of -CF₃,

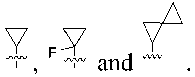

In some embodiments, R⁵ is selected from the group consisting of halogen and C₁-C₄alkyl, wherein the alkyl is optionally substituted with one, two, or three halogens. For example, in certain embodiments R⁵ is selected from the group consisting of chloro, fluoro, and -CH₃.

In additional embodiments, the moiety 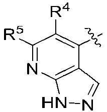 is selected from the group consisting of, for example:

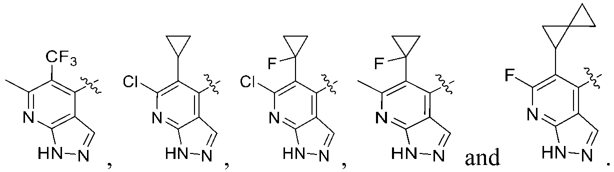

In further embodiments, R⁶ is, for example, fluoro. In some embodiments, R⁹ is hydrogen and R¹⁰ is -CH₃.

Also disclosed herein is a compound represented by Formula II: 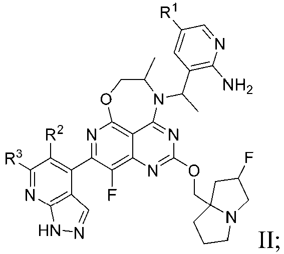 or a pharmaceutically acceptable salt, stereoisomer, or tautomer thereof, wherein:
R¹ is selected from the group consisting of halogen, cyano, and C₁-C₆alkoxy; wherein the alkoxy is optionally substituted with one or more halogens;
R² is selected from the group consisting of C₁-C₄alkyl and C₃-C₆cycloalkyl, wherein the alkyl and cycloalkyl are optionally substituted with one, two, or three halogens; and
R³ is selected from the group consisting of halogen and C₁-C₄alkyl, wherein the alkyl is optionally substituted with one, two, or three halogens.

In some embodiments, the compound of Formula II is represented by Formula II-1:

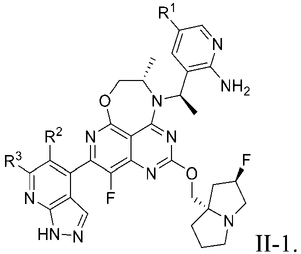

In some embodiments, R¹ is selected from the group consisting of, for example, fluoro, chloro, -OCHF₂, and -OCF₃. In some embodiments, R¹ is cyano or -C(O)NH₂. In some embodiments, R¹ is isopropyl. In some embodiments, R² is selected from the group consisting of, for example, -CF₃, 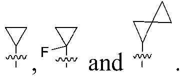 In some embodiments, R³ is selected from the group consisting of, for example, chloro, fluoro, and -CH₃.

In further embodiments, the moiety 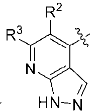 is selected from the group consisting of, for example:

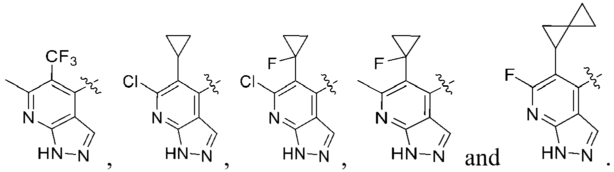

In some aspects, the compound represented by Formula III: 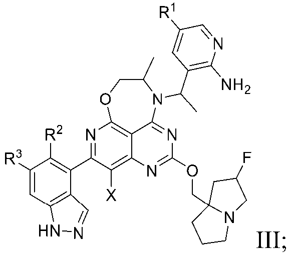 or a pharmaceutically acceptable salt, stereoisomer, or tautomer thereof, wherein:
X is F or Cl;
R¹ is cyano, -C(O)NH₂, isopropyl, C₁-C₄alkoxy, -OCH₃, -OCF₃, or -OCHF₂, wherein the alkyl of the alkoxy is optionally -CH₂CN or a C₃-C₆cycloalkyl group;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens, or -CN; and
R³ is selected from the group consisting of halogen and C₁-C₄alkyl, wherein the alkyl is optionally substituted with one, two, or three halogens,
   or:
R¹ is fluoro;
R² is selected from the group consisting of C₁-C₄alkoxy and C₃-C₆cycloalkyl, wherein alkoxy and cycloalkyl are optionally substituted with one, two, or three halogens, or -CN; and
R³ is selected from the group consisting of halogen and C₁-C₄alkyl.

In some embodiments, R¹ is cyano, -C(O)NH₂, isopropyl, C₁-C₄alkoxy, -OCH₃, - OCF₃, -OCHF₂, -OCH₂CN, or -O C₃-C₆cycloalkyl.

In some aspects, the compound represented by Formula III: 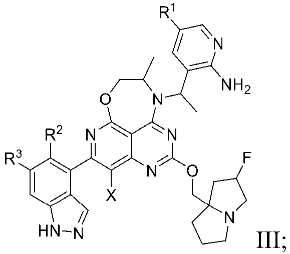 or a pharmaceutically acceptable salt, stereoisomer, or tautomer thereof, wherein:
X is F or Cl;
R¹ is cyano, -C(O)NH₂, isopropyl, or -OCHF₂;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens.

In some aspects, the compound represented by Formula III: 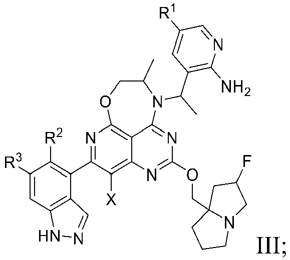 or a pharmaceutically acceptable salt, stereoisomer, or tautomer thereof, wherein:
R¹ is fluoro;
R² is selected from the group consisting of C₁-C₄alkoxy and C₃-C₆cycloalkyl, wherein alkoxy and cycloalkyl are optionally substituted with one, two, or three halogens; and
R³ is selected from the group consisting of halogen and C₁-C₄alkyl.

In some embodiments, the compound of Formula III is represented by Formula III-1:

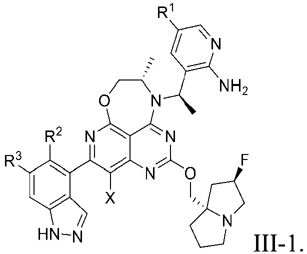

In some aspects, disclosed herein is a compound represented by Formula IIIa: 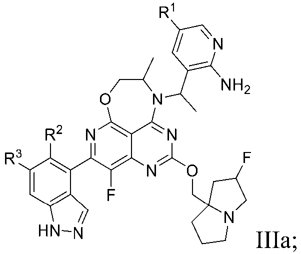 or a pharmaceutically acceptable salt, stereoisomer, or tautomer thereof, wherein:
R¹ is cyano, -C(O)NH₂, isopropyl, C₁-C₄alkoxy, -OCH₃, -OCF₃, or -OCHF₂, wherein the alkyl of the alkoxy is optionally -CH₂CN, or a C₃-C₆cycloalkyl group, and wherein the alkyl may be substituted with one or more deuterium;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens; and
R³ is selected from the group consisting of halogen and C₁-C₄alkyl, wherein the alkyl is optionally substituted with one, two, or three halogens;
   or:
R¹ is fluoro;
R² is selected from the group consisting of C₁-C₄alkoxy and C₃-C₆cycloalkyl, wherein the alkoxy and cycloalkyl are optionally substituted with one, two, or three halogens or -CN; and
R³ is selected from the group consisting of halogen and C₁-C₄alkyl.

In some embodiments, R¹ is cyano, -C(O)NH₂, isopropyl, C₁-C₄alkoxy, -OCH₃, -OCF₃, -OCHF₂, -OCH₂CN, or -O C₃-C₆cycloalkyl.

In some aspects, disclosed herein is a compound represented by Formula IIIa: 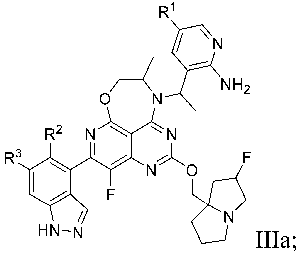 or a pharmaceutically acceptable salt, stereoisomer, or tautomer thereof, wherein:
R¹ is cyano, -C(O)NH₂, isopropyl, C₁-C₄alkoxy, -OCH₃, -OCF₃, or -OCHF₂, wherein the alkyl of the alkoxy is optionally -CH₂CN, or a C₃-C₆cycloalkyl group, and wherein the alkyl may be substituted with one or more deuterium;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens.

In some embodiments, R¹ is cyano, -C(O)NH₂, isopropyl, C₁-C₄alkoxy, -OCH₃, -OCF₃, -OCHF₂, -OCH₂CN, or -O C₃-C₆cycloalkyl.

In some aspects, disclosed herein is a compound represented by Formula IIIa: 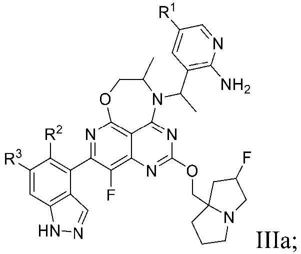 or a pharmaceutically acceptable salt, stereoisomer, or tautomer thereof, wherein:
R¹ is fluoro;
R² is selected from the group consisting of C₁-C₄alkoxy and C₃-C₆cycloalkyl, wherein the alkoxy and cycloalkyl are optionally substituted with one, two, or three halogens or -CN; and
R³ is selected from the group consisting of halogen and C₁-C₄alkyl.

In some aspects, disclosed herein is a compound represented by Formula IIIa: 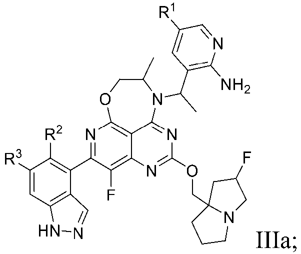 or a pharmaceutically acceptable salt, stereoisomer, or tautomer thereof, wherein:
R¹ is -OCHF₂;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens;
   or:
R¹ is fluoro;
R² is selected from the group consisting of C₁-C₄alkoxy and C₃-C₆cycloalkyl, wherein the alkoxy and cycloalkyl are optionally substituted with one, two, or three halogens or -CN; and
R³ is selected from the group consisting of halogen and C₁-C₄alkyl.

In some embodiments, R¹ is cyano, -C(O)NH₂, isopropyl, C₁-C₄alkoxy, -OCH₃, -OCF₃, -OCHF₂, -OCH₂CN, or -O C₃-C₆cycloalkyl.

In some aspects, disclosed herein is a compound represented by Formula IIIa: 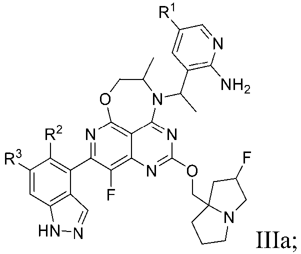 or a pharmaceutically acceptable salt, stereoisomer, or tautomer thereof, wherein:
R¹ is -OCHF₂;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens or -CN; and
R³ is selected from the group consisting of halogen and C₁-C₄alkyl, wherein the alkyl is optionally substituted with one, two, or three halogens.

In some aspects, disclosed herein is a compound represented by Formula IIIa: 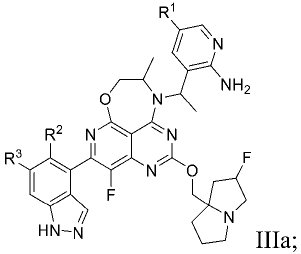 or a pharmaceutically acceptable salt, stereoisomer, or tautomer thereof, wherein:
R¹ is fluoro;
R² is selected from the group consisting of C₁-C₄alkoxy and C₃-C₆cycloalkyl, wherein the alkoxy and cycloalkyl are optionally substituted with one, two, or three halogens or -CN; and
R³ is selected from the group consisting of halogen and C₁-C₄alkyl.

In some embodiments, the compound of Formula III is represented by Formula IIIa-1:

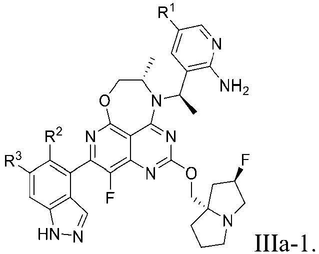

For example, in some embodiments R¹ is -OCHF₂.

For example, in some embodiments R¹ is -OCF₃.

In some embodiments, R² is selected from the group consisting of, for example, - CF₃, 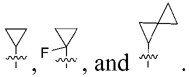 In additional embodiment, R³ is selected from the group consisting of, for example, chloro, fluoro, and -CH₃.

In some embodiments, R¹ is, for example, fluoro. In further embodiments, R² is selected from the group consisting of, for example, -OCHF₂, 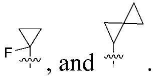 In certain embodiments, R³ is selected from the group consisting of, for example, fluoro, and -CH₃. In further embodiments, the moiety 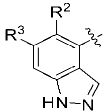 is selected from the group consisting of, for example

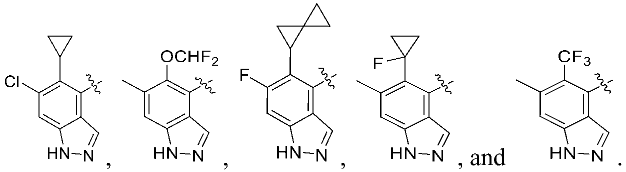

In some embodiments, R¹ is cyano or -C(O)NH₂. In some embodiments, R¹ is isopropyl. In some embodiments, R² is CF₃

In some aspects, disclosed herein is a compound 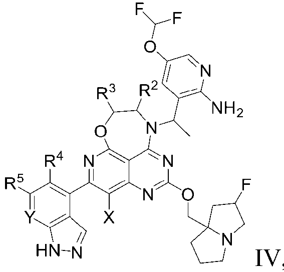 or a pharmaceutically acceptable salt, stereoisomer, or tautomer thereof, wherein
X is F or Cl;
Y is N or CH;
R² and R³ are each independently selected from C₁-C₆ alkyl and hydrogen; and
R⁴ and R⁵ are independently selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one or more halogens, or one to three methyls.

In some embodiments, the compound of Formula IV is represented by Formula IV-1:

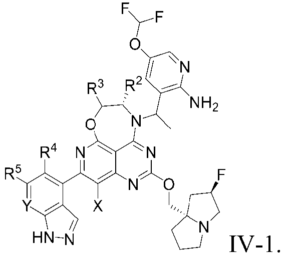

In some embodiments, the compound of Formula IV is represented by Formula IV-2:

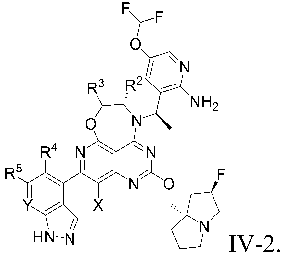

In some embodiments, the compound of Formula IV is represented by Formula IV-a1 or Formula IV-b1: 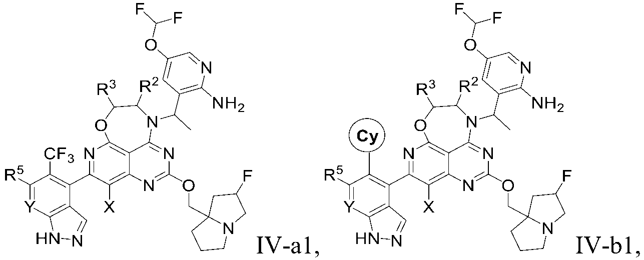 wherein 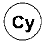 is a C₃-C₆ cycloalkyl, optionally substituted with one or more halogens , or one to three methyls.

In some embodiments, the compound of Formula IV is represented by Formula IV-a2 or Formula IV-b2: 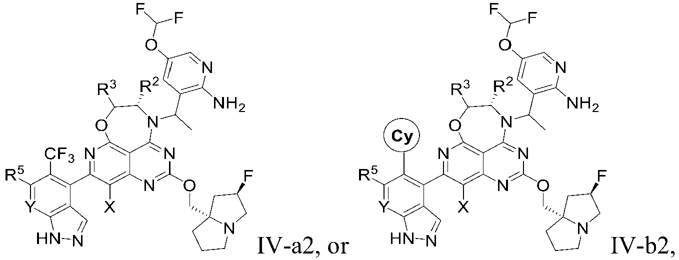 wherein 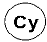 is a C₃-C₆ cycloalkyl, optionally substituted with one or more halogens, or one to three methyls.

In some embodiments, the compound of Formula IV is represented by Formula IV-a3 or Formula IV-b3: 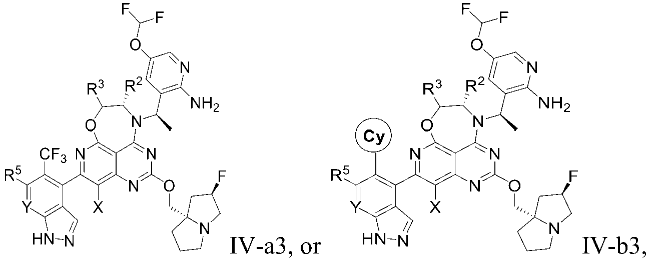 wherein 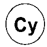 is a C₃-C₆ cycloalkyl, optionally substituted with one or more halogens.

In some aspects, disclosed herein is a compound represented by Formula V: 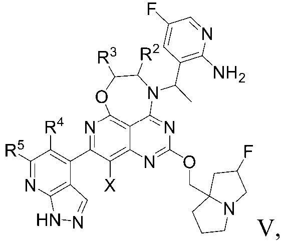 or a pharmaceutically acceptable salt, stereoisomer, or tautomer thereof, wherein
X is F or Cl;
R² and R³ are each independently selected from C₁-C₆ alkyl and hydrogen; and
R⁴ and R⁵ are each independently selected from hydrogen, halogen, deuterium, C₁-C₆ alkyl, C₁-C₆ alkoxy, and C₃-C₆ cycloalkyl, wherein the alkyl, alkoxy, and cycloalkyl are optionally substituted with one or more halogens.

In some embodiments, the compound of Formula V is represented by Formula V-1:

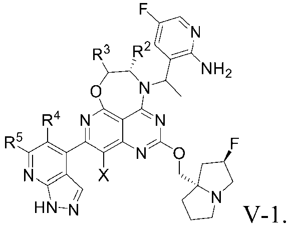

In some embodiments, the compound of Formula V is represented by Formula V-2:

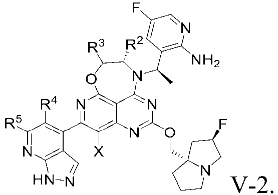

In some embodiments, the compound of Formula V is represented by Formula V-a, V-b, or V-c: 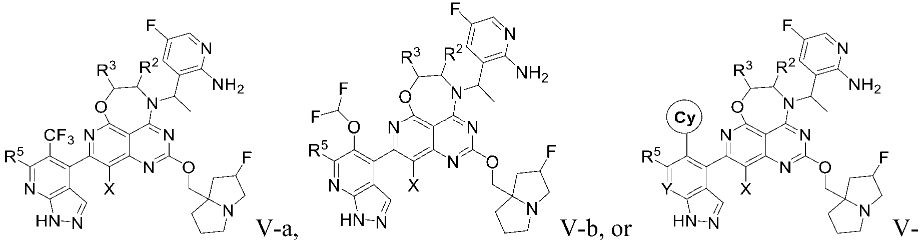 c, wherein 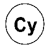 is a C₃-C₆ cycloalkyl, optionally substituted with one or more halogens.

In some embodiments, the compound of Formula V is represented by Formula V-a1, V-bl, or V-c1: 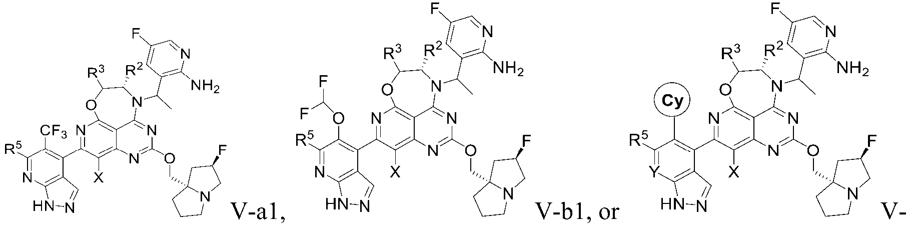 c1, wherein 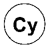 is a C₃-C₆ cycloalkyl, optionally substituted with one or more halogens.

In some embodiments, the compound of Formula V is represented by Formula V-a2, V-b2, or V-c2: 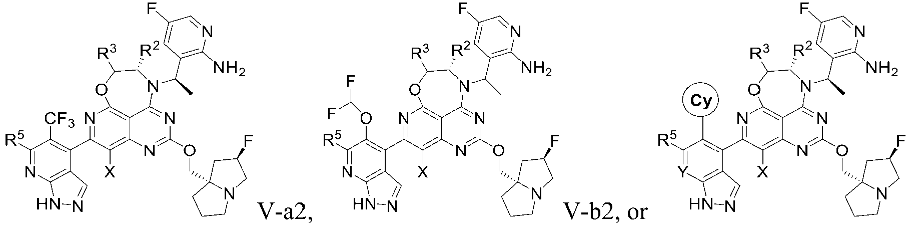 V-c2, wherein 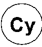 is a C₃-C₆ cycloalkyl, optionally substituted with one or more halogens.

In some embodiments, 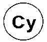 is 1-fluorocyclopropyl. In some embodiments, 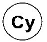 is spiro[2.2]pentan-1-yl. In some embodiments, 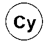 is cyclopropyl. In some embodiments, 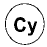 is 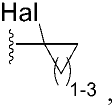 wherein 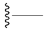 is the point of attachment to indole, and Hal is a halogen. In some embodiments, the halogen is fluoro.

In some aspects, disclosed herein is a compound represented by Formula VI: 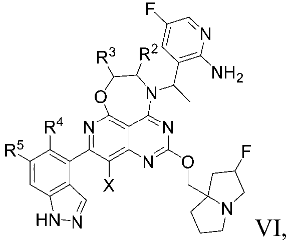 or a pharmaceutically acceptable salt, stereoisomer, or tautomer thereof, wherein
X is F or Cl;
R² and R³ are each independently selected from C₁-C₆ alkyl and hydrogen;
R⁴ is C₁-C₆ alkoxy or C₃-C₆ cycloalkyl, optionally substituted with one or more halogens or one to three methyls; and
R⁵ is selected from hydrogen, deuterium, fluoro, and C₁-C₆ alkyl;
wherein the compound is not:

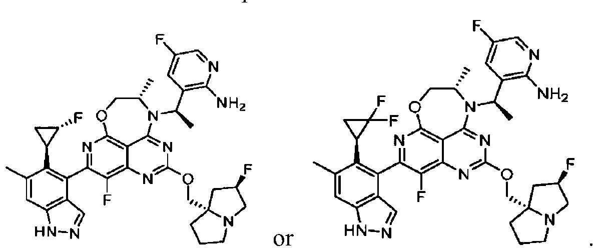

In some embodiments, the compound of Formula VI is represented by Formula VI-1:

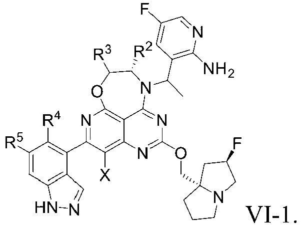

In some embodiments, the compound of Formula VI is represented by Formula VI-2:

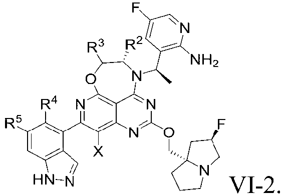

In some embodiments, R⁴ is halogen, C₁-C₆ alkyl, C₁-C₆ alkoxy, or C₃-C₆ cycloalkyl wherein the alkyl, alkoxy, and cycloalkyl are optionally substituted with one or more halogens. In some embodiments, R⁴ is halogen, C₁-C₆ alkyl, or C₃-C₆ cycloalkyl, wherein the alkyl and cycloalkyl are optionally substituted with one or more halogens.

In some embodiments, R⁴ is C₁-C₆ alkoxy or C₄-C₆ cycloalkyl optionally substituted with one or more halogens. In some embodiments, R⁴ is C₃-C₆ cycloalkyl optionally substituted with one halogen. In some embodiments, R⁴ is 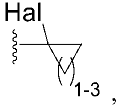 wherein 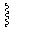 is the point of attachment to indole, and Hal is a halogen. In some embodiments, the halogen is fluoro.

In some embodiments, R⁵ is -C₁-C₃ alkyl. In some embodiments, R⁵ is -CH₃. In some embodiments, R⁵ is -Cl or -F.

In some embodiments, R³ is H.

In some embodiments, R² is -C₁-C₃ alkyl, for example -CH₃.

In another aspect is a compound represented by Formula VII: 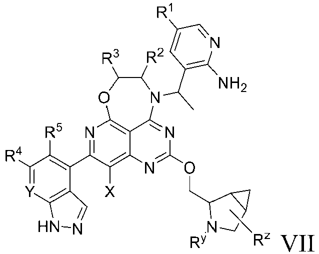 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen, and
R⁵ is C₃-C₆ cycloalkyl optionally substituted with one to three halogen, or C₁-C₆ alkoxy substituted with one to three halogen;
wherein when R¹ is halogen, R⁵ is C₁-C₆ alkoxy substituted with one to three halogen, or C₄-C₆ cycloalkyl

In some embodiments, the compound is represented by Formula VII-1 or VII-2:

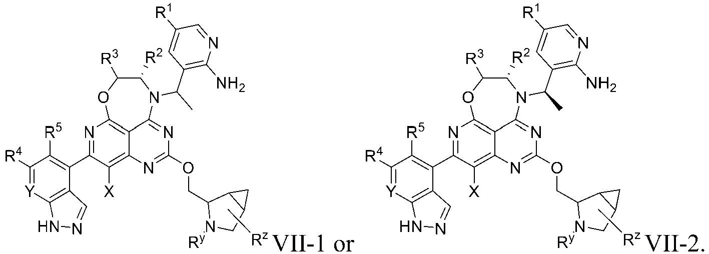

In some embodiments, the compound is represented by Formula VII-3, VII-4, or VII-5:

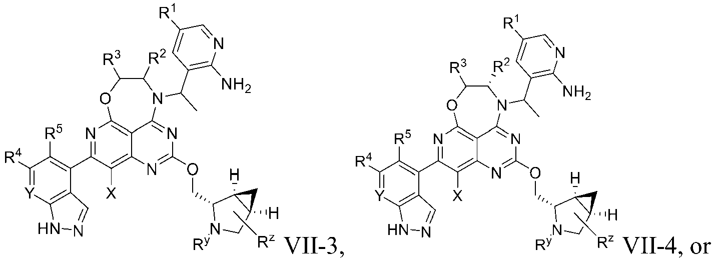

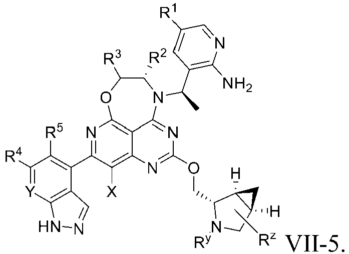

In some embodiments, the compound is represented by Formula VIII: 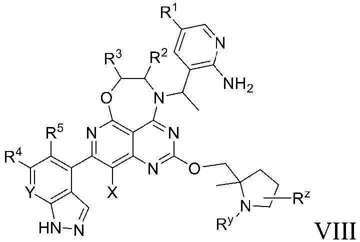 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen, and
R⁵ is C₃-C₆ cycloalkyl optionally substituted with one to three halogen, or C₁-C₆ alkoxy substituted with one to three halogen.

In some embodiments, when R¹ is halogen, R⁵ is C₁-C₆ alkoxy substituted with one to three halogen, or C₄-C₆ cycloalkyl.

In some embodiments, the compound is represented by Formula VIII-1 or VIII-2:

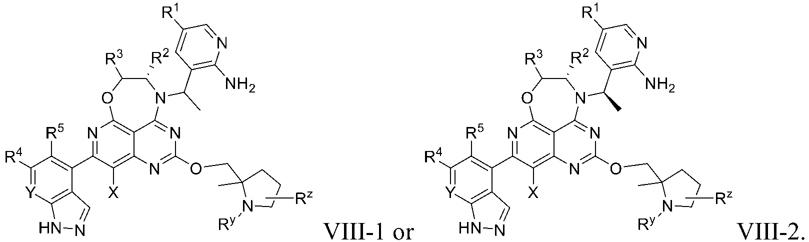

In some embodiments, the compound is represented by Formula VIII-3, VIII-4, or VIII-5: 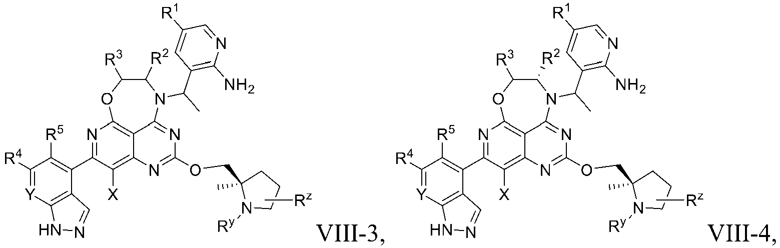 or

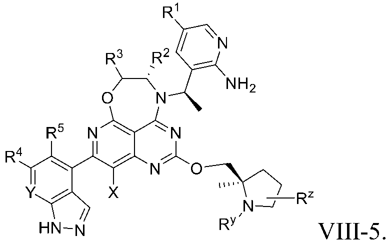

In some embodiments, suitable compounds of the present disclosure are: 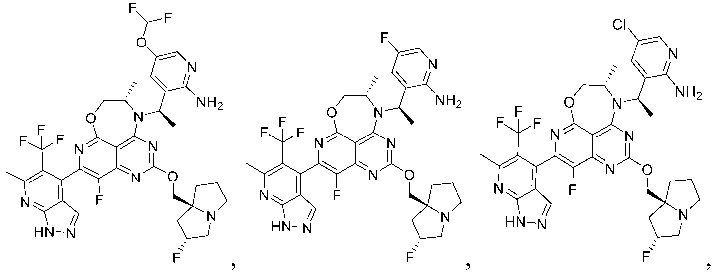 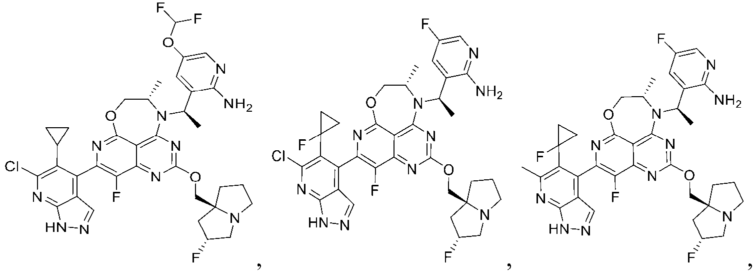 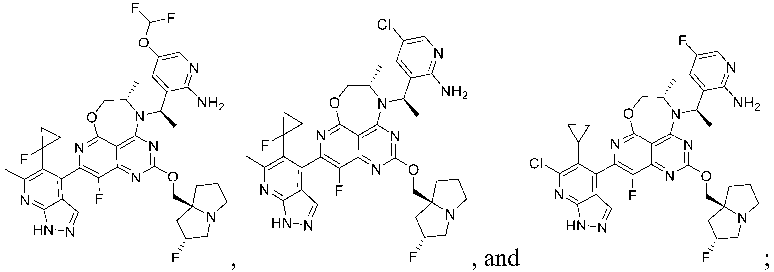 or a pharmaceutically acceptable salt, stereoisomer, or tautomer thereof.

In another aspect, is a compound represented by Formula IX: 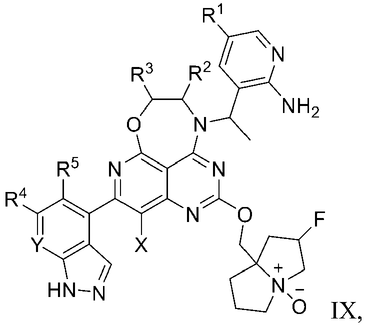 or a pharmaceutically acceptable salt, stereoisomer, or tautomer thereof, wherein:
X is F or Cl;
Y is N or CH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen; and
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen, and
R⁵ is C₃-C₆ cycloalkyl optionally substituted with one to three halogen, or C₁-C₆ alkoxy substituted with one to three halogen;
wherein when R¹ is halogen, R⁵ is C₁-C₆ alkoxy substituted with one to three halogen, or C₄-C₆ cycloalkyl.

In some embodiments, suitable compounds of the present disclosure are: 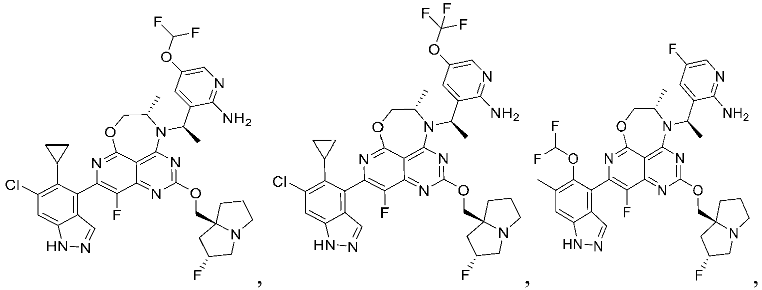 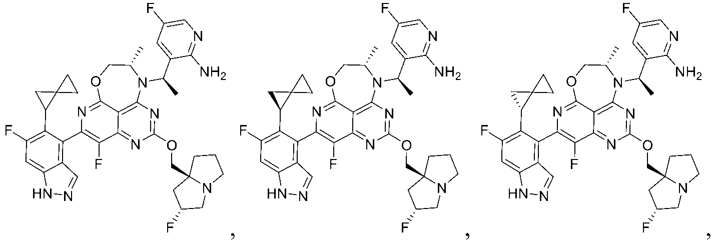 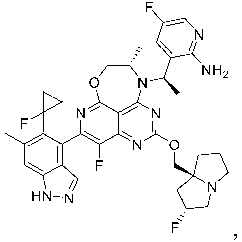 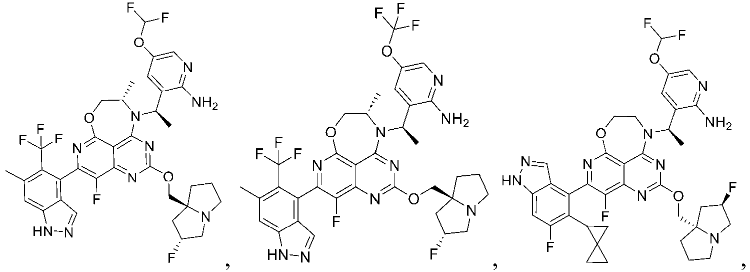 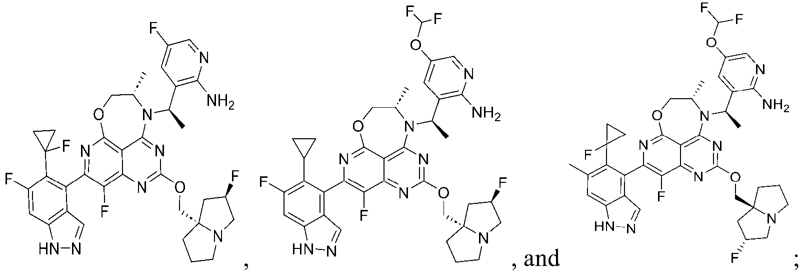 or a pharmaceutically acceptable salt, stereoisomer, or tautomer thereof.

In some embodiments, suitable compounds of the present disclosure are: 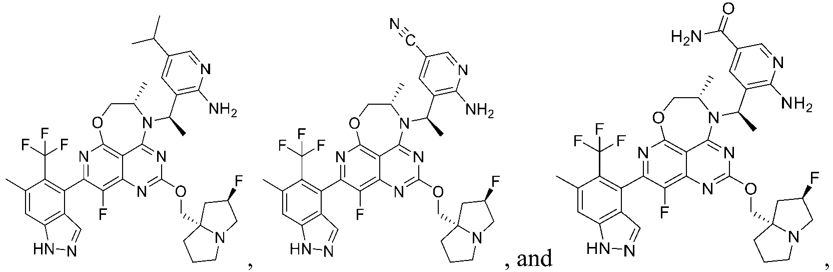 or a pharmaceutically acceptable salt or tautomer thereof.

In some embodiments, a compound of the disclosure is a pan-KRAS inhibitor (i.e., an inhibitor of K-Ras G12C, K-Ras G12D, and K-Ras G12V). In some embodiments, metabolism of a compound of the disclosure results in fewer species of metabolites than other K-Ras inhibitors. Without wishing to be bound by theory, fewer metabolites may result in lower toxicity of certain compounds of the disclosure compared to other K-Ras inhibitors.

In some embodiments, a compound of the present disclosure is 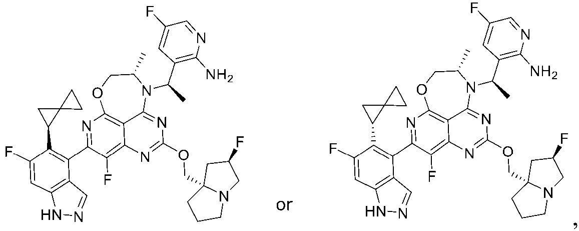 or pharmaceutically acceptable salt or tautomer thereof. In some embodiments, the compound is the compound eluting first (i.e., after about 26 min) from a chiral high-pressure liquid chromatography (HPLC) (e.g., using a first mobile phase comprising hexane with 0.2% isopropylamine and a second mobile phase comprising ethanol and dichloromethane in a 1:1 ratio). In some embodiments, the compound is the compound eluting second (i.e., after about 30 min) from a chiral HPLC (e.g., using a first mobile phase comprising hexane with 0.2% isopropylamine and a second mobile phase comprising ethanol and dichloromethane in a 1:1 ratio). In some embodiments, the compound has an nuclear magnetic resonance (NMR) spectrum as shown in Example 12. In some embodiments, the compound has a nuclear magnetic resonance (NMR) spectrum as shown in Example 13. In some embodiments, the compound of Formula VII is represented by Formula VII-1 or VII-2:

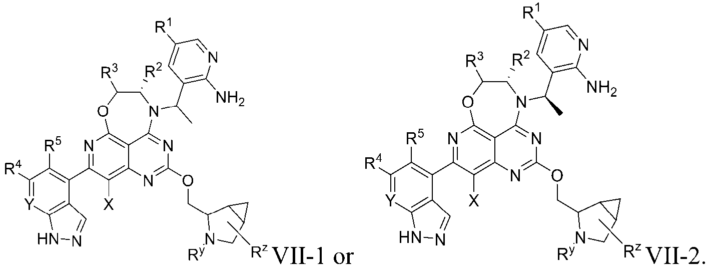

In some embodiments, the compound of Formula VII is represented by Formula VII-3, VII-4, or VII-5: 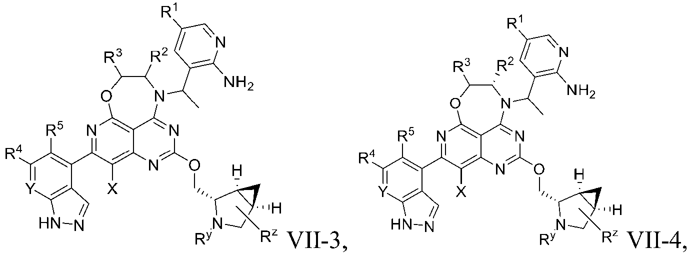 or

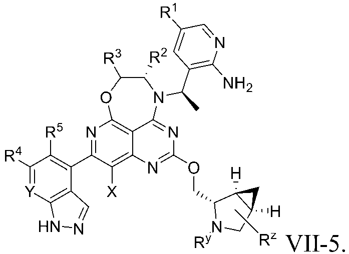

In some embodiments, the compound of Formula VIII is represented by Formula VIII-1 or VIII-2:

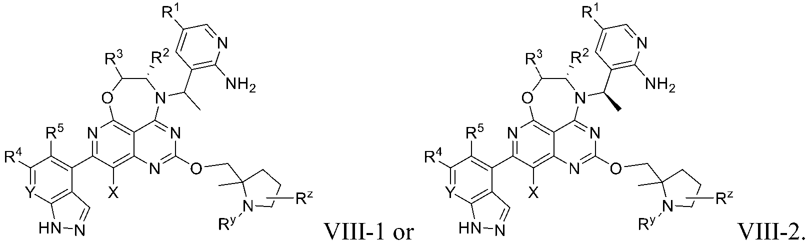

In some embodiments, the compound of Formula VIII is represented by Formula VIII-3, VIII-4, or VIII-5: 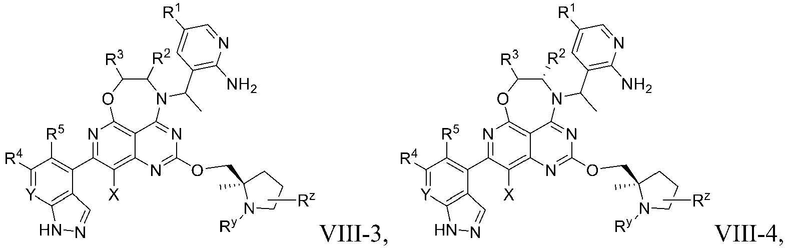 or

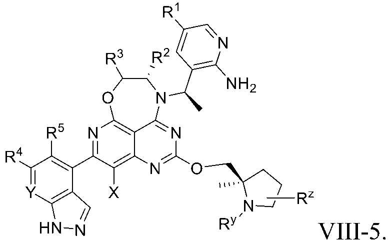

In some embodiments, Y is CH. In some embodiments, Y is N. In some embodiments, X is F.

In some embodiments, R^{z} is H. In some embodiments, R^{z} is halogen or OH. In some embodiments, R^{z} is Cl or F.

In some embodiments, R^{y} is C₁-C₃ alkyl. In some embodiments, R^{y} is -CH₃.

In some embodiments, R¹ is unsubstituted C₃-C₆ cycloalkyl. In some embodiments, the C₃-C₆ cycloalkyl of R¹ is cyclopropyl. In some embodiments, R¹ is C₁-C₆ alkoxy substituted with two or three halogen atoms. In some embodiments, R¹ is C₁-C₃ alkoxy substituted with two or three halogen atoms. In some embodiments, R¹ is methoxy substituted with two or three halogen atoms. In some embodiments, R¹ is -OCHF₂. In some embodiments, R¹ is -OCF₃.

In some embodiments, R² and R³ are each independently selected from C₁-C₃ alkyl and hydrogen. In some embodiments, R² and R³ are each independently selected from -CH₃ and hydrogen. In some embodiments, R³ is hydrogen. In some embodiments, R² is - CH₃.

In some embodiments, R⁴ is halogen. In some embodiments, R⁴ is chloro. In some embodiments, R⁴ is unsubstituted C₁-C₆ alkyl. In some embodiments, R⁴ is unsubstituted C₁-C₃ alkyl. In some embodiments, R⁴ is -CH₃.

In some embodiments, R⁵ is C₃-C₆ cycloalkyl optionally substituted with one or more substituents. In some embodiments, R⁵ is cyclopropyl optionally substituted with one or more substituents. In some embodiments, R⁵ is cyclopropyl optionally substituted with one or more halogens. In some embodiments, R⁵ is cyclopropyl substituted with one halogen atom. In some embodiments, the halogen is fluoro. In some embodiments, R⁵ is unsubstituted cyclopropyl. In some embodiments, R⁵ is methoxy optionally substituted with one or more substituents. In some embodiments, R⁵ is methoxy optionally substituted with one or more halogens. In some embodiments, R⁵ is -OCHF₂. In some embodiments, R⁵ is - OCF₃.

In some embodiments, suitable compounds of the present disclosure are compounds of Table 1 or a pharmaceutically acceptable salt or tautomer thereof.

**Table 1. Exemplary compounds.**

| Comp. No. | Structure |
|---|---|
| 101 | 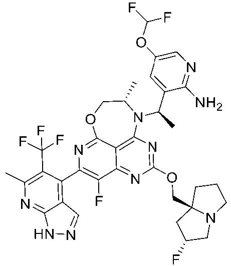 |
| 102 | 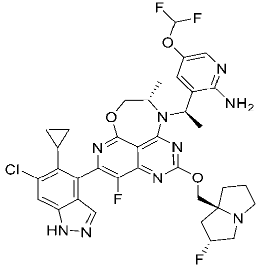 |
| 103 | 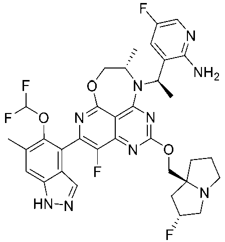 |
| 104 | 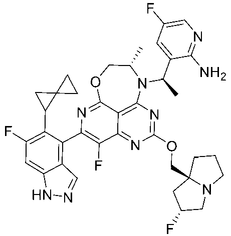 |
| 105 | 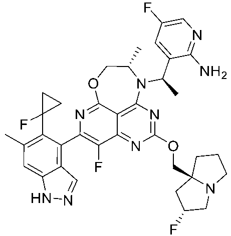 |
| 106 | 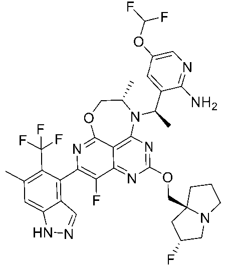 |
| 107 | 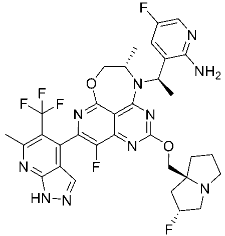 |
| 108 | 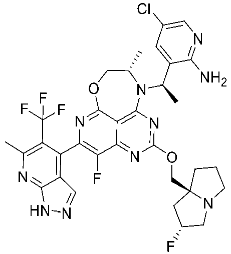 |
| 109 | 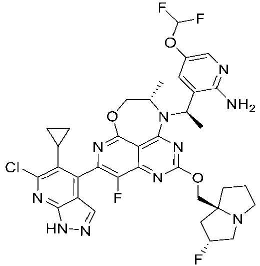 |
| 110 | 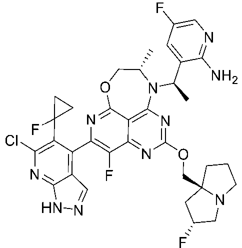 |
| 111 | 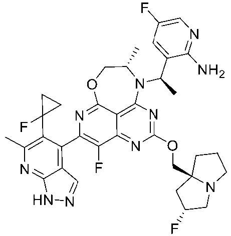 |
| 112 | 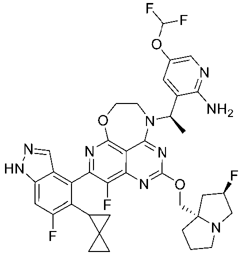 |
| 113 | 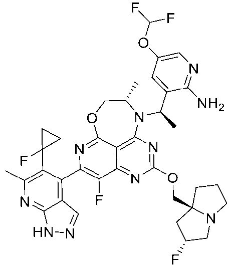 |
| 114 | 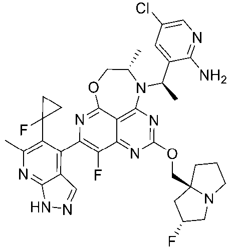 |
| 115 | 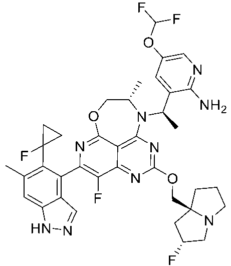 |
| 116 | 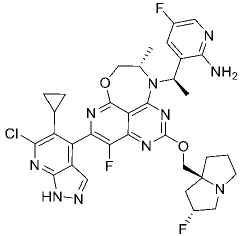 |
| 117 | 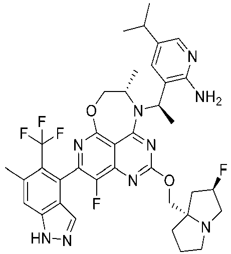 |
| 118 | 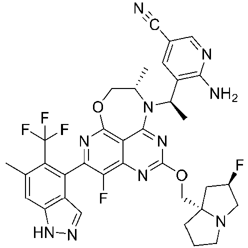 |
| 119 | 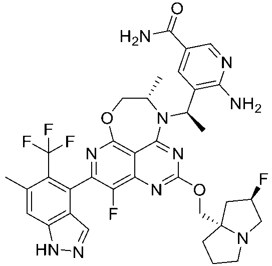 |
| 120 | 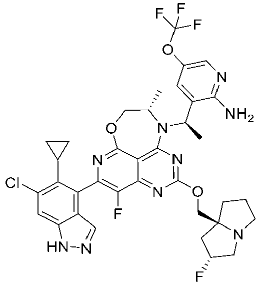 |
| 121 | 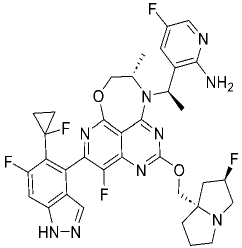 |
| 122 | 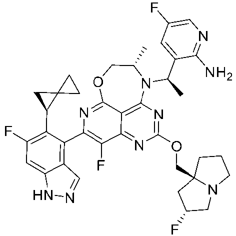 |
| 123 | 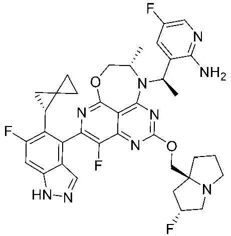 |
| 124 | 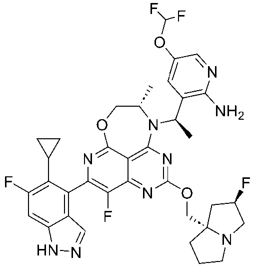 |
| 125 | 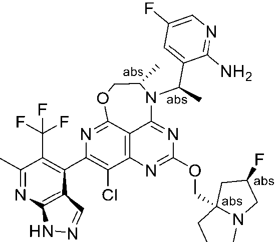 |
| 126 | 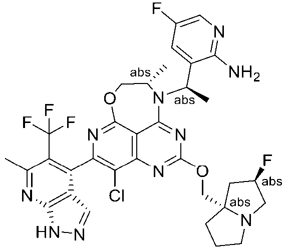 |
| 127 | 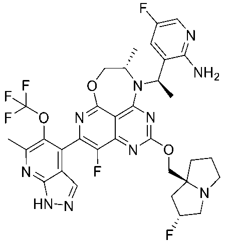 |
| 128 | 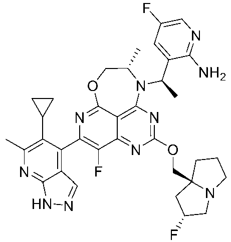 |
| 129 | 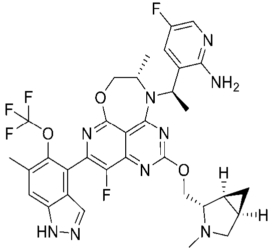 |
| 130 | 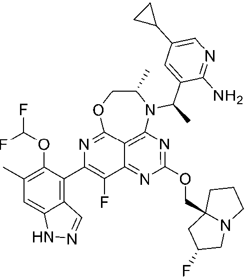 |
| 131 | 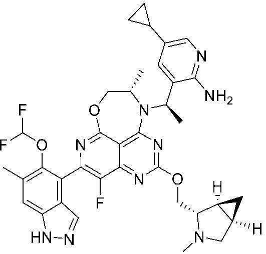 |
| 132 | 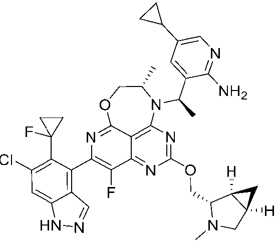 |
| 133 | 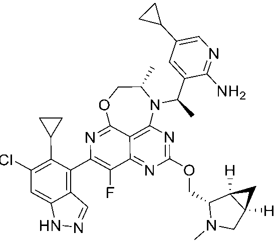 |
| 134 | 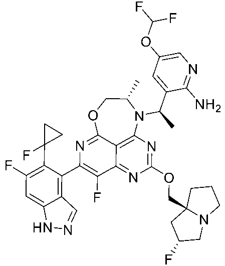 |
| 135 | 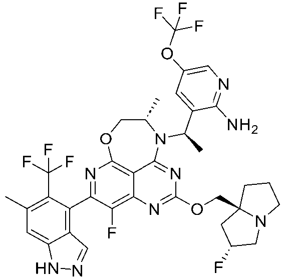 |
| 136 | 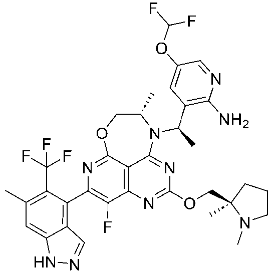 |
| 137 | 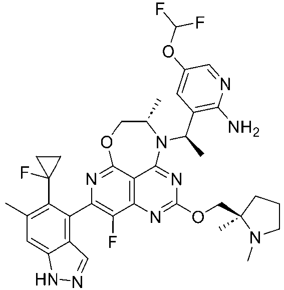 |
| 138 | 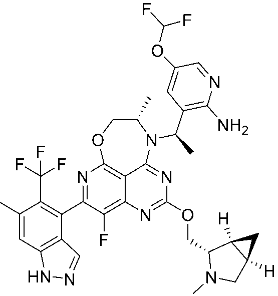 |
| 139 | 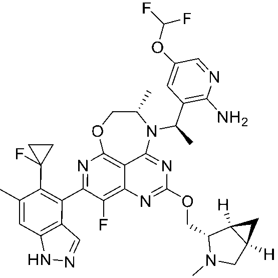 |
| 140 | 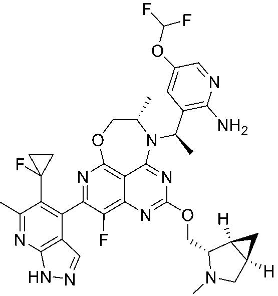 |
| 141 | 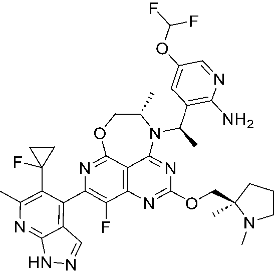 |
| 142 | 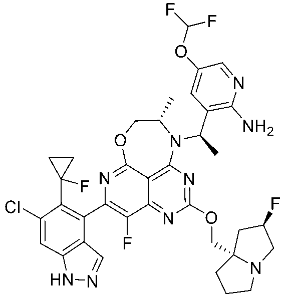 |
| 143 | 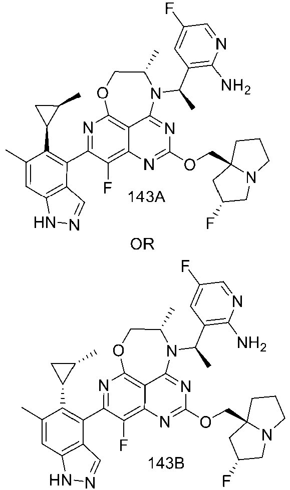 |
| 144 | 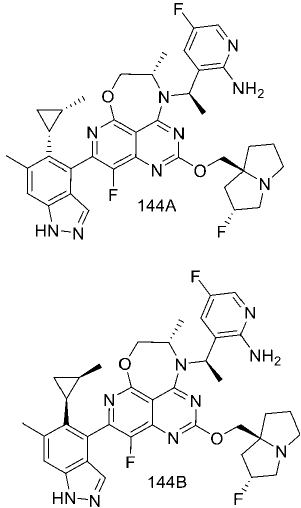 |
| 145 | 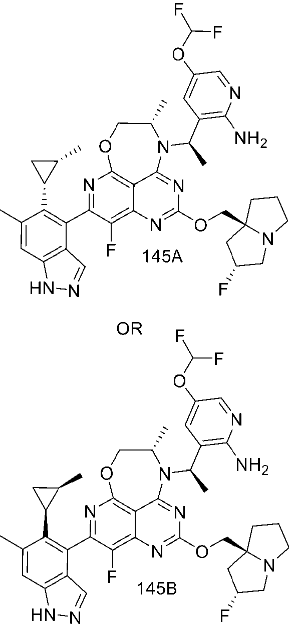 |
| 146 | 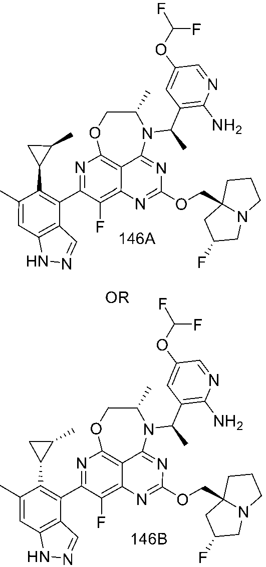 |
| 147 | 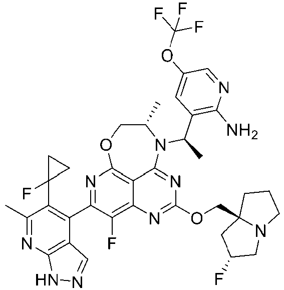 |
| 148 | 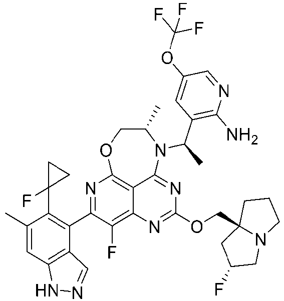 |
| 149 | 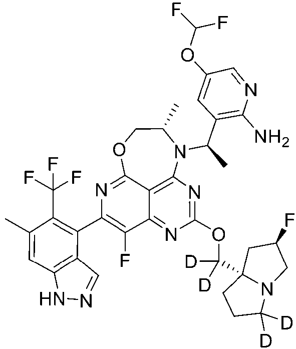 |
| 150 | 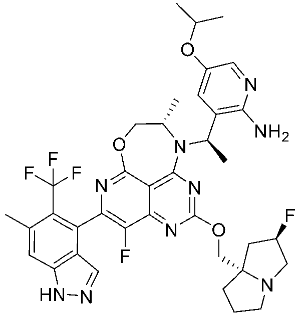 |
| 151 | 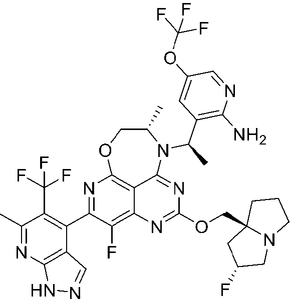 |
| 153 | 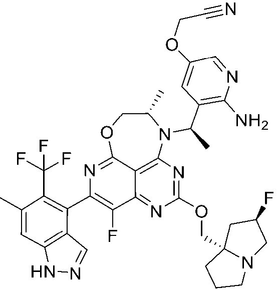 |
| 154 | 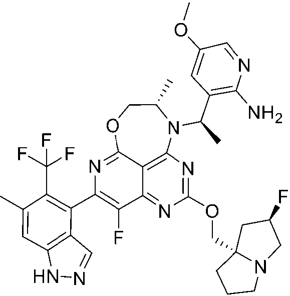 |
| 155 | 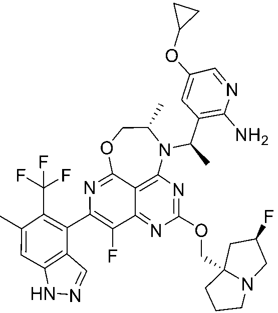 |
| 156 | 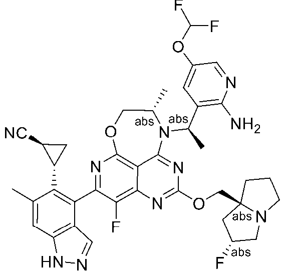 |
| 157 | 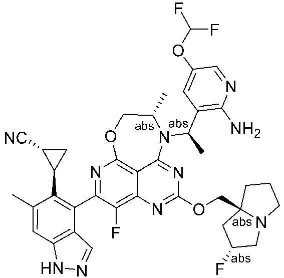 |
| 158 | 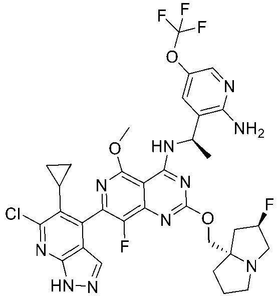 |
| 159 | 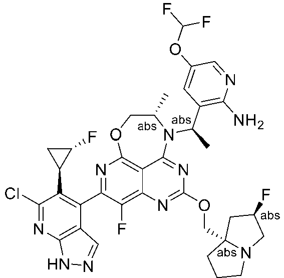 |
| 160 | 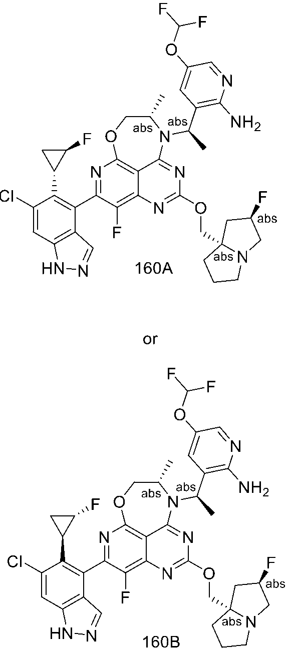 |
| 161 | 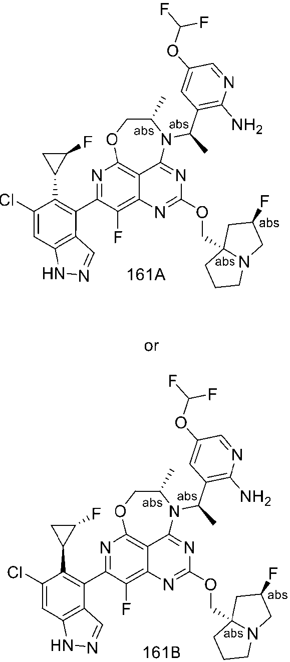 |
| 162 | 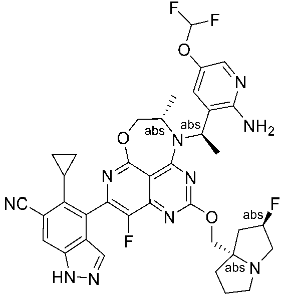 |
| 163 | 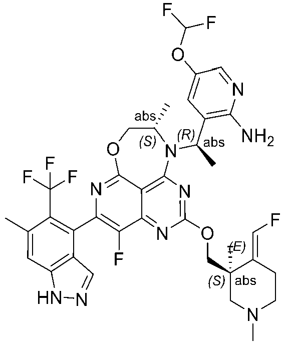 |
| 164 | 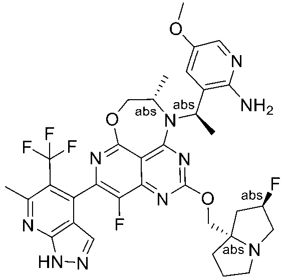 |
| 165 | 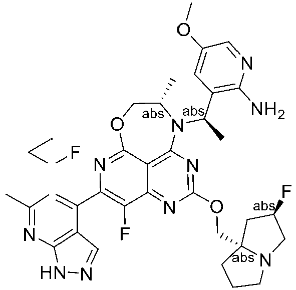 |
| 166 | 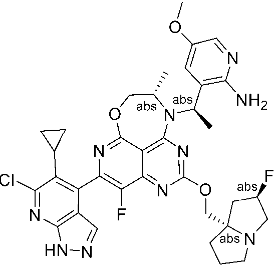 |
| 167 | 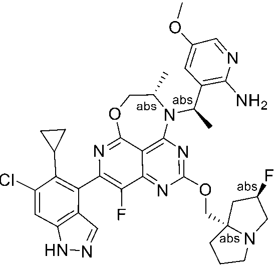 |
| 168 | 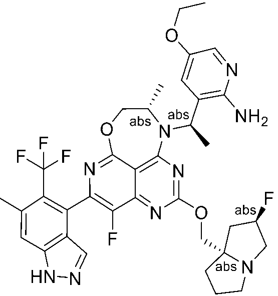 |
| 169 | 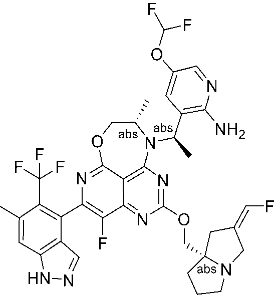 |
| 170 | |
| | 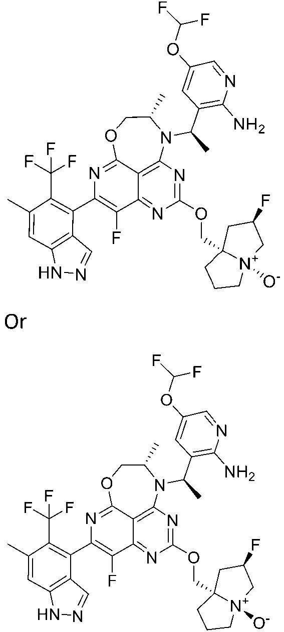 |
| 171 | 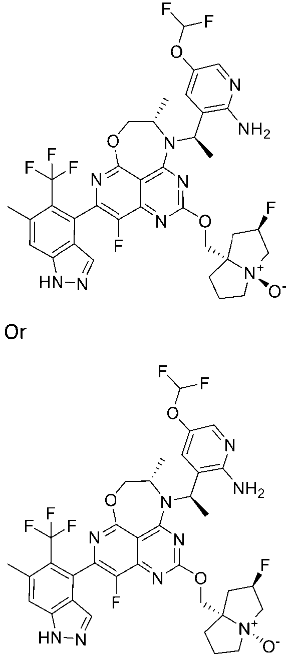 |
| 172 | 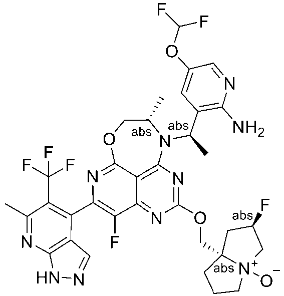 |
| 173 | 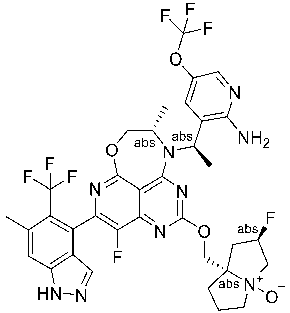 |
| 174 | 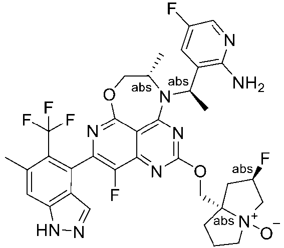 |

Procedures for making compounds described herein are provided in the examples below. In the reactions described below, it may be necessary to protect reactive functional groups (such as hydroxyl, amino, thio or carboxyl groups) to avoid their unwanted participation in the reactions. The incorporation of such groups, and the methods required to introduce and remove them are known to those skilled in the art (for example, see Greene, Wuts, Protective Groups in Organic Synthesis. 2nd Ed. (1999)). The deprotection step may be the final step in the synthesis such that the removal of protecting groups affords compounds as disclosed herein. Starting materials used in the following scheme can be purchased or prepared by methods described in the chemical literature, or by adaptations thereof, using methods known by those skilled in the art. The order in which the steps are performed can vary depending on the groups introduced and the reagents used, but would be apparent to those skilled in the art.

Compounds disclosed herein, or any of the intermediates described in the schemes above, can be further derivatized by using one or more standard synthetic methods known to those skilled in the art. Such methods can involve substitution, oxidation or reduction reactions. These methods can also be used to obtain or modify disclosed compounds or any preceding intermediates by modifying, introducing or removing appropriate functional groups.

Where it is desired to obtain a particular enantiomer of a disclosed compound, this may be produced from a corresponding mixture of enantiomers by employing any suitable conventional procedure for resolving enantiomers known to those skilled in the art. For example, diastereomeric derivatives (such as salts) can be produced by reaction of a mixture of enantiomers of a disclosed compound (such a racemate) and an appropriate chiral compound (such as a chiral base). The diastereomers can then be separated by any conventional means such as crystallization or chromatography, and the desired enantiomer recovered (such as by treatment with an acid in the instance where the diastereomer is a salt). Alternatively, a racemic mixture of esters can be resolved by kinetic hydrolysis using a variety of biocatalysts (for example, see Patel Stereoselective Biocatalysts, Marcel Decker; New York 2000).

In another resolution process a racemate of disclosed compounds can be separated using chiral High Performance Liquid Chromatography. Alternatively, a particular enantiomer can be obtained by using an appropriate chiral intermediate in one of the processes described above. Chromatography, recrystallisation and other conventional separation procedures may also be used with intermediates or final products where it is desired to obtain a particular geometric isomer of the disclosure.

In an alternative embodiment, disclosed compounds may also comprise one or more isotopic substitutions. For example, hydrogen may be ²H (D or deuterium) or ³H (T or tritium); carbon may be, for example, ¹³C or ¹⁴C; oxygen may be, for example, ¹⁸O; nitrogen may be, for example, ¹⁵N, and the like. In some embodiments, a particular isotope (e.g., ³H, ¹³C, ¹⁴C, ¹⁸O, or ¹⁵N) can represent at least 1%, at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, at least 99%, or at least 99.9% of the total isotopic abundance of an element that occupies a specific site of the compound.

### II. Methods

In some aspects, disclosed herein are methods of treating a patient suffering from a condition, disease, or disorder that is affected by, associated with, or would benefit from inhibition of Ras (e.g., K-Ras), comprising administering to the patient a therapeutically effective amount of a compound disclosed herein, or a pharmaceutical composition thereof. For example, disclosed herein are methods of treating a Ras protein-related (e.g., a K-Ras protein-related) disease or disorder in a patient in need thereof, comprising administering to the patient a therapeutically effective amount of a compound disclosed herein, or a pharmaceutical composition thereof.

In some embodiments, the present disclosure provides a method of treating a disease or disorder characterized by aberrant Ras activity due to a Ras mutant (e.g., aberrant K-Ras, H-Ras, or N-Ras activity due to a K-Ras mutation, a H-Ras mutation, or a N-Ras mutation). In some embodiments, for example, the compounds and compositions disclosed herein may be used to treat a cancer having one or more Ras mutations (e.g., a K-Ras mutation, a H-Ras mutation, or a N-Ras mutation). The K-Ras mutation may be a K-Ras G12C mutation, K-Ras G12D mutation, or a K-Ras G12V mutation and the cancer may be a K-Ras G12C mutated cancer, K-Ras G12D mutated cancer, or a K-Ras G12V mutated cancer.

For example, the methods described herein may be useful to treat cancers including, but not limited to, lung, prostate, breast, brain, skin, cervical carcinomas, and testicular carcinomas. For example, in some embodiments, the cancers that may be treated by the compounds, compositions and methods disclosed herein are, but are not limited to, tumor types such as astrocytic, breast, cervical, colorectal, endometrial, esophageal, gastric, head and neck, hepatocellular, laryngeal, lung, oral, ovarian, prostate and thyroid carcinomas and sarcomas. In some embodiments, these cancers are cancers having one or more Ras mutations (e.g., a K-Ras mutation, a H-Ras mutation, or a N-Ras mutation).

In certain embodiments, the compounds, compositions and methods disclosed herein can be used to treat cancers, including tumors, related to the cardiovascular system. Non-limiting examples contemplated herein include, e.g., angiosarcoma, fibrosarcoma, rhabdomyosarcoma, liposarcoma, myxoma, rhabdomyoma, fibroma, lipoma and teratoma. In some embodiments, these cancers are cancers having one or more Ras mutations (e.g., a K-Ras mutation, a H-Ras mutation, or a N-Ras mutation).

In further embodiments, the compounds, compositions and methods disclosed herein can be used to treat cancers, including tumors, of the lung. Non-limiting examples contemplated herein include, e.g., bronchogenic carcinoma (for example, squamous cell, undifferentiated small cell, undifferentiated large cell, adenocarcinoma), alveolar (for example, bronchiolar) carcinoma, bronchial adenoma, sarcoma, lymphoma, chondromatous hamartoma, and mesothelioma. In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat small cell lung cancer. In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat non-small cell lung cancer (NSCLC). In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat cancers, including tumors, of the gastrointestinal system. Non-limiting examples contemplated herein include, e.g., esophageal cancer (for example, squamous cell carcinoma, adenocarcinoma, leiomyosarcoma, lymphoma), stomach cancer (for example, carcinoma, lymphoma, and leiomyosarcoma), pancreatic cancer (for example, ductal adenocarcinoma, insulinoma, glucagonoma, gastrinoma, carcinoid tumors, vipoma), small bowel cancer (for example, adenocarcinoma, lymphoma, carcinoid tumors, Kaposi's sarcoma, leiomyoma, hemangioma, lipoma, neurofibroma, fibroma), and large bowel cancer (for example, adenocarcinoma, tubular adenoma, villous adenoma, hamartoma, leiomyoma). In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat pancreatic cancers, including tumors of the pancreas. In some embodiments, the pancreatic cancer is a ductal adenocarcinoma, insulinoma, glucagonoma, gastrinoma, carcinoid tumors, or vipoma. In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat cancers, including tumors, of the genitourinary tract. Non-limiting examples contemplated herein include, e.g., kidney cancer (for example, adenocarcinoma, Wilm's tumor (nephroblastoma), lymphoma, leukemia), bladder and urethral cancer (for example, squamous cell carcinoma, transitional cell carcinoma, adenocarcinoma), prostate cancer (for example, adenocarcinoma, sarcoma), and testicular cancer (for example, seminoma, teratoma, embryonal carcinoma, teratocarcinoma, choriocarcinoma, sarcoma, interstitial cell carcinoma, fibroma, fibroadenoma, adenomatoid tumors, lipoma). In some embodiments, these cancers are cancers having one or more Ras mutations (e.g., a K-Ras mutation, a H-Ras mutation, or a N-Ras mutation).

In still some embodiments, the compounds, compositions and methods disclosed herein can be used to treat cancers, including tumors, of the liver. Non-limiting examples contemplated herein include, e.g., hepatoma (for example, hepatocellular carcinoma), cholangiocarcinoma, hepatoblastoma, angiosarcoma, hepatocellular adenoma and hemangioma. In some embodiments, these cancers are cancers having one or more Ras mutations (e.g., a K-Ras mutation, a H-Ras mutation, or a N-Ras mutation).

In further embodiments, the compounds, compositions and methods disclosed herein can be used to treat cancers, including tumors, of the biliary tract. Non-limiting examples contemplated herein include, e.g., gall bladder carcinoma, ampullary carcinoma, and cholangiocarcinoma.

In certain embodiments, the compounds, compositions and methods disclosed herein can be used to treat cancers, including tumors, of the bone. Non-limiting examples contemplated herein include, e.g., osteogenic sarcoma (for example, osteosarcoma), fibrosarcoma, malignant fibrous histiocytoma, chondrosarcoma, Ewing's sarcoma, malignant lymphoma (for example, reticulum cell sarcoma), multiple myeloma, malignant giant cell tumor chordoma, osteochronfroma (for example, osteocartilaginous exostoses), benign chondroma, chondroblastoma, chondromyxofibroma, osteoid osteoma and giant cell tumors.

In some embodiments, the compounds, compositions and methods disclosed herein can be used to treat cancers, including tumors, of the nervous system. Non-limiting examples contemplated herein include, e.g., cancer and/or tumors of the skull (for example, osteoma, hemangioma, granuloma, xanthoma, osteitis deformans), cancer and/or tumors of the meninges (for example, meningioma, meningiosarcoma, gliomatosis), brain cancer (for example, astrocytoma, medulloblastoma, glioma, ependymoma, germinoma, pinealoma, glioblastoma multiform, oligodendroglioma, schwannoma, retinoblastoma, congenital tumors), and cancer and/or tumors of the spinal cord (for example, neurofibroma, meningioma, glioma, sarcoma). In some embodiments, these cancers are cancers having one or more Ras mutations (e.g., a K-Ras mutation, a H-Ras mutation, or a N-Ras mutation).

In some embodiments, the compounds, compositions and methods disclosed herein can be used to treat cancers, including tumors, of the gynecological system. Non-limiting examples contemplated herein include, e.g., cancers and/or tumors of the uterus (for example, endometrial carcinoma), cancers and/or tumors of the cervix (for example, cervical carcinoma, pre-tumor cervical dysplasia), cancers and/or tumors of the ovaries (for example, ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), cancers and/or tumors of the vulva (for example, squamous cell carcinoma, intraepithelial carcinoma, adenocarcinoma, fibrosarcoma, melanoma), cancers and/or tumors of the vagina (for example, clear cell carcinoma, squamous cell carcinoma, botryoid sarcoma (embryonal rhabdomyosarcoma), and cancers and/or tumors of the fallopian tubes (carcinoma). In some embodiments, these cancers are cancers having one or more Ras mutations (e.g., a K-Ras mutation, a H-Ras mutation, or a N-Ras mutation).

In further embodiments, the compounds, compositions and methods disclosed herein can be used to treat hematologic cancers, including tumors. Non-limiting examples contemplated herein include, e.g., cancers and/or tumors of the blood (for example, myeloid leukemia (acute and chronic), acute lymphoblastic leukemia, chronic lymphocytic leukemia, myeloproliferative diseases, multiple myeloma, myelodysplastic syndrome), Hodgkin's disease, and non-Hodgkin's lymphoma (malignant lymphoma). In some embodiments, these cancers are cancers having one or more Ras mutations (e.g., a K-Ras mutation, a H-Ras mutation, or a N-Ras mutation).

In certain embodiments, the compounds, compositions and methods disclosed herein can be used to treat cancers, including tumors, of the skin. Non-limiting examples contemplated herein include, e.g., malignant melanoma, basal cell carcinoma, squamous cell carcinoma, Kaposi's sarcoma, moles dysplastic nevi, lipoma, angioma, dermatofibroma, keloids, and psoriasis. In some embodiments, the compounds, compositions and methods disclosed herein can be used to treat cancers, including tumors, of the adrenal glands, e.g., neuroblastoma. In some embodiments, these cancers are cancers having one or more Ras mutations (e.g., a K-Ras mutation, a H-Ras mutation, or a N-Ras mutation).

In some embodiments, the methods described herein can be used to treat cancers including, but not limited to, pancreatic cancer, colorectal cancer, multiple myeloma, lung adenocarcinoma, melanoma, endometrial cancer, uterine cancer, thyroid, acute myelocytic leukemia, bladder carcinoma, gastric cancer, cervical cancer, and head and neck squamous cell carcinoma. In some embodiments, these cancers are cancers having one or more Ras mutations (e.g., a K-Ras mutation, a H-Ras mutation, or a N-Ras mutation).

In some embodiments, the methods described herein can be used to treat pancreatic cancer, colorectal cancer, or non-small cell lung cancer. In some embodiments, these cancers are cancers having one or more Ras mutations (e.g., a K-Ras mutation, a H-Ras mutation, or a N-Ras mutation).

In some embodiments, the methods described herein can be used to treat pancreatic cancer. In some embodiments, these cancers are cancers having one or more Ras mutations (e.g., a K-Ras mutation, a H-Ras mutation, or a N-Ras mutation).

In some embodiments, the methods described herein can be used to treat colorectal cancer. In some embodiments, these cancers are cancers having one or more Ras mutations (e.g., a K-Ras mutation, a H-Ras mutation, or a N-Ras mutation).

In some embodiments, the methods described herein can be used to treat endometriosis, or an inflammatory and/or autoimmune disease (e.g., a nonmalignant syndrome of autoimmunity and abnormal leukocyte homeostasis). See, e.g., Adashek et al. Genome Med. 2020; 12: 16; Niemela et al. Blood. 2011; 117(10):2883-6; Nosan et al. Croat Med J. 2013; 54(6): 574-578; and Messina et al. Small GTPases 11.5 (2020): 312-319. In some embodiments, the endometriosis, or the inflammatory and/or autoimmune disease (e.g., a nonmalignant syndrome of autoimmunity and abnormal leukocyte homeostasis) is characterized by aberrant Ras activity due to a Ras mutant (e.g., aberrant K-Ras, H-Ras, or N-Ras activity due to a K-Ras mutation, a H-Ras mutation, or a N-Ras mutation).

In some embodiments, the methods described herein can be used to treat a RASopathy (e.g., a genetic syndrome caused by a germline mutation in a gene that encodes a component or regulator of the RAS/MAPK pathway). In some embodiments, the RASopathy is selected from the group consisting of neurofibromatosis type 1, Noonan syndrome, Noonan syndrome with multiple lentigines, capillary malformation-arteriovenous malformation syndrome, Costello syndrome, cardio-facio-cutaneous syndrome, and Legius syndrome.

In particular, in certain embodiments, the disclosure provides a method of treating the medical indications contemplated herein comprising administering to a patient in need thereof a therapeutically effective amount of a compound described herein.

In some embodiments, the present disclosure provides a method for inhibiting K-Ras G12C, K-Ras G12D, or K-Ras G12V in a subject in need thereof, comprising administering to the subject a therapeutically effective amount of the compound of the disclosure or a pharmaceutically acceptable salt, stereoisomer, or tautomer thereof. In some embodiments, the method is a method of inhibiting K-Ras G12C and K-Ras G12D. In some embodiments, the method is a method of inhibiting K-Ras G12C and K-Ras G12V. In some embodiments, the method is a method of inhibiting K-Ras G12D and K-Ras G12V. In some embodiments, the method is a method of inhibiting K-Ras G12C, K-Ras G12D and K-Ras G12V. In some embodiments, the subject is a subject having cancer (e.g., a Ras-mutated cancer). In some embodiments, the subject is a subject having a K-Ras-mutated cancer, e.g., a K-Ras G12C-mutated cancer, a K-Ras G12D-mutated cancer, or a K-Ras G12V-mutated cancer.

In some embodiments, the Ras protein is wild-type (Ras^{wt}). Accordingly, in some embodiments, a compound of the present invention is employed in a method of treating a patient having a cancer comprising a Ras^{wt} (e.g., K-Ras^{wt}, H-Ras^{wt} or N-Ras^{wt}). In some embodiments, the Ras protein is Ras amplification (e.g., K-Ras^{amp}). Accordingly, in some embodiments, a compound of the present invention is employed in a method of treating a patient having a cancer comprising a Ras^{amp} (K-Ras^{amp}, H-Ras^{amp} or N-Ras^{amp}).

In some embodiments, the cancer comprises a Ras mutation, such as a Ras mutation described herein. In some embodiments, the mutation is selected from a K-Ras mutation (for example, G12D, G12V, G12C, G13D, G12R, G12A, Q61H, G12S, A146T, G13C, Q61L, Q61R, K117N, A146V, G12F, Q61K, L19F, Q22K, V14I, A59T, A146P, G13R, G12L, or G13V, and combinations thereof); a H-Ras mutation (for example, Q61 R, G13R, Q61K, G12S, Q61L, G12D, G13V, G13D, G12C, K117N, A59T, G12V, G13C, Q61H, G13S, A18V, D119N, G13N, A146T, A66T, G12A, A146V, G12N, or G12R, and combinations thereof); and a N-Ras mutation (for example, Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T, and combinations thereof); or a combination of any thereof.

In some embodiments, the cancer comprises a K-Ras mutation selected from the group consisting of, for example, G12C, G12D, G13C, G12V, G13D, G12R, G12S, Q61H, Q61K and Q61L. In some embodiments, the cancer comprises an N-Ras mutation selected from the group consisting of, for example, G12C, Q61H, Q61K, Q61L, Q61P and Q61R. In still some embodiments, the cancer comprises an H-Ras mutation selected from the group consisting of, for example, Q61H and Q61L. In further embodiments, the cancer comprises a Ras mutation selected from the group consisting of, for example, G12C, G13C, G12A, G12D, G13D, G12S, G13S, G12V and G13V. In certain embodiments, the cancer comprises at least two Ras mutations selected from the group consisting of, for example, G12C, G13C, G12A, G12D, G13D, G12S, G13S, G12V and G13V.

In some embodiments, the cancer comprises a K-Ras mutation selected from the group consisting of, for example, A11_G12delinsGC, A11_G12delinsGR, A11_G12delinsHV, A11_G12delinsTD, A11_G12delinsVD, A11_G12delinsVV, A11_G12dup, A11D, A11delinsGP, A11G, A11P, A11S, A11T, A11V, A146E, A146P, A146S, A146T, A146V, A18D, A18G, A18S, A18V, A59_G60delinsGV, A59E, A59G, A59S, A59T, A59V, A66_G75dup, D119E, D119H, D119N, D33E, D33H, D33N, D33Y, D54_E62dup, E31Q, E62_E63insDDTAGQE, E62_E63insDE, E62_Q70dup, E62A, E62G, E62K, E63A, E63D, E63G, E63K, F156L, F28C, F28I, F28L, F28S, F28V, G10_A11insTG, G10dup, G12_G13delinsAC, G12_G13delinsAR, G12_G13delinsCC, G12_G13delinsCD, G12_G13delinsDC, G12_G13delinsVC, G12_G13delinsVD, G12A, G12C, G12D, G12E, G12F, G12H, G12I, G12L, G12N, G12R, G12S, G12T, G12V, G12W, G12Y, G13_V14delinsCG, G13_V14delinsDI, G13A, G13C, G13D, G13E, G13F, G13H, G13P, G13R, G13S, G13V, G13Y, G60D, G60R, G60S, G60V, K117I, K117N, K117Q, K117R, K117T, K117Y, K147E, K147N, K147Q, K147R, K147T, K5_L6delinsNI, K5E, K5I, K5N, K5Q, K5R, K5T, L19F, L19S, L19V, L52_G60dup, M67I, M67K, M67L, M67V, N116_K117delinsTN, N116H, N116S, P34L, P34Q, P34R, P34S, P34T, Q22E, Q22H, Q22K, Q22L, Q22R, 61_E62delinsRK, Q61_S65dup, Q61A, Q61D, Q61E, Q61H, Q61K, Q61L, Q61P, Q61R, R68G, R68K, R68S, R68T, R68W, S65_A66delinsLDQY, S65_A66insKRSTV, S65_Y71dup, S65C, S65G, S65I, S65N, S65R, T144A, T144I, T144K, T144P, T50_E63dup, T50A, T50I, T50P, T58_Y64dup, T58A, T58I, T58S, T74A, T74P, V14A, V14G, V14I, V14L, V44_Q61dup, V9_G10dup, Y40C, Y40H, Y64_S65delinsAQEG, Y64_S65insMDILDTAGQEEY, Y64_S65insQQVKRST, Y64_S65insVS, Y64C, Y64D, Y64H, Y64N, Y71_M72delinsSV, Y71C, and Y71D. In some embodiments, the cancer comprises a K-Ras mutation selected from the group consisting of, for example, A146E, A146P, A146S, A146T, A146V, A59E, A59G, A59S, A59T, A59V, G12A, G12C, G12D, G12E, G12F, G12H, G12I, G12L, G12N, G12R, G12S, G12T, G12V, G12W, G12Y, G13A, G13C, G13D, G13E, G13F, G13H, G13P, G13R, G13S, G13V, G13Y, K117I, K117N, K117Q, K117R, K117T, K117Y, Q61A, Q61D, Q61E, Q61H, Q61K, Q61L, Q61P, and Q61R.

In some embodiments, the cancer comprises an N-Ras mutation selected from the group consisting of, for example A11_G12delinsDD, A11S, A146S, A146T, A146V, A59D, A59G, A59S, A59T, A59V, E153D, E153K, E153Q, G12A, G12C, G12D, G12H, G12I, G12N, G12R, G12S, G12V, G13C, G13D, G13E, G13F, G13H, G13I, G13L, G13N, G13R, G13S, G13V, G60E, G60R, G60V, 124F, I24N, I24T, K117M, K117R, Q61_E62delinsHK, Q61_E62delinsKK, Q61E, Q61H, Q61I, Q61K, Q61L, Q61P, Q61R, T50A, T50I, T58A, and T58I, T58K. In some embodiments, the cancer comprises an N-Ras mutation selected from the group consisting of, for example G12A, G12C, G12D, G12H, G12I, G12N, G12R, G12S, G12V, G13C, G13D, G13E, G13F, G13H, G13I, G13L, G13N, G13R, G13S, G13V, Q61E, Q61H, Q611, Q61K, Q61L, Q61P, and Q61R.

In some embodiments, the cancer comprises an H-Ras mutation selected from the group consisting of, for example A146T, A146V, A59G, A59T, F28I, F28L, G12A, G12C, G12D, G12F, G12I, G12N, G12R, G12S, G12V, G13C, G13D, G13dup, G13E, G13I, G13N, G13R, G13S, G13V, K117N, K117R, K117T, P34F, P34L, Q61E, Q61H, Q61K, Q61L, and Q61R. In some embodiments, the cancer comprises an H-Ras mutation selected from the group consisting of, for example G12A, G12C, G12D, G12F, G12I, G12N, G12R, G12S, G12V, G13C, G13D, G13dup, G13E, G131, G13N, G13R, G13S, G13V, Q61E, Q61H, Q61K, Q61L, and Q61R.

In some embodiments, a compound disclosed herein may inhibit more than one Ras mutant. For example, in some embodiments a disclosed compound may inhibit both K-Ras G12C and K-Ras G13C. In some embodiments, disclosed compound may inhibit both N-Ras G12C and K-Ras G12C. In still some embodiments, a disclosed compound may inhibit both N-Ras G12C and K-Ras G12C. In further embodiments, a disclosed compound may inhibit both K-Ras G12C and K-Ras G12D. In certain embodiments, a disclosed compound may inhibit both K-Ras G12V and K-Ras G12C. In some embodiments, a disclosed compound may inhibit both K-Ras G12V and K-Ras G12S.

In some embodiments, a compound disclosed herein inhibits Ras^{wt} in addition to one or more additional Ras mutations (e.g., K, H or N-Ras^{wt} and K-Ras G12D, G12V, G12C, G13D, G12R, G12A, Q61H, G12S, A146T, G13C, Q61L, Q61R, K117N, A146V, G12F, Q61 K, L 19F, Q22K, V14I, A59T, A146P, G13R, G12L, or G13V; K, H, or N-Ras^{wt} and H-Ras Q61R, G13R, Q61K, G12S, Q61L, G12D, G13V, G13D, G12C, K117N, A59T, G12V, G13C, Q61 H, G13S, A18V, D119N, G13N, A146T, A66T, G12A, A146V, G12N, or G12R; or K, H, or N-Ras^{wt} and N-Ras Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T).

In some embodiments, a compound disclosed herein inhibits Ras^{amp} in addition to one or more additional Ras mutations (e.g., K-, H- or N-Ras^{amp} and K-Ras G12D, G12V, G12C, G13D, G12R, G12A, Q61H, G12S, A146T, G13C, Q61L, Q61R, K117N, A146V, G12F, Q61K, L19F, Q22K, V14I, A59T, A146P, G13R, G12L, or G13V; K-, H- or N-Ras^{amp} and H-Ras Q61R, G13R, Q61K, G12S, Q61L, G12D, G13V, G13D,G12C, K117N, A59T, G12V, G13C, Q61H ,G13S, A18V, D119N, G13N, A146T, A66T, G12A, A146V, G12N, or G12R; or K-, H- or N-Ras^{amp} and N-Ras Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T).

In some embodiments, a cancer comprises a Ras mutation and an STK11^{LOF}, a KEAP1, an EPHA5 or an NF1 mutation. In some embodiments, the cancer is non-small cell lung cancer and comprises a K-Ras G12C mutation. In some embodiments, the cancer is non-small cell lung cancer and comprises a K-Ras G12C mutation and an STK11^{LOF} mutation. In some embodiments, the cancer is non-small cell lung cancer and comprises a K-Ras G12C mutation and an STK11^{LOF} mutation. In further embodiments, a cancer comprises a K-Ras G13C Ras mutation and an STK11^{LOF}, a KEAP1, an EPHA5 or an NF1 mutation. In certain embodiments, the cancer is non-small cell lung cancer and comprises a K-Ras G12D mutation. In some embodiments, the cancer is non-small cell lung cancer and comprises a K-Ras G12V mutation. In further embodiments, the cancer is colorectal cancer and comprises a K-Ras G12C mutation. In some embodiments, the cancer is pancreatic cancer and comprises a K-Ras G12D mutation. In some embodiments, the cancer is pancreatic cancer and comprises a K-Ras G12V mutation. In still some embodiments, the cancer is endometrial cancer and comprises a K-Ras G12C mutation. In certain embodiments, the cancer is lung cancer, colorectal cancer, or pancreactic cancer and comprises a K-Ras G12D mutation. In further embodiments, the cancer is lung cancer or pancreactic cancer and comprises a K-Ras G12D mutation. In some embodiments, the cancer is lung cancer and comprises a K-Ras G12D mutation. In some embodiments, the cancer is colorectal cancer and comprises a K-Ras G12D mutation. In some embodiments, the cancer is gastric cancer and comprises a K-Ras G12C mutation. In addition, a disclosed compound may inhibit Ras^{wt} (e.g., K-, H- or N-Ras^{wt}) or Ras^{amp} (e.g., K-, H- or N-Ras^{amp}).

Also disclosed herein are methods of inhibiting a Ras protein (e.g., a K-Ras protein) in a cell or tissue, comprising contacting the cell or tissue with a therapeutically effective amount of a compound disclosed herein, or a pharmaceutical composition thereof. In some embodiments, the Ras protein is a mutated Ras protein. In some embodiments, the method is an *ex vivo* method. Further disclosed herein are methods of inhibiting a Ras protein (e.g., a K-Ras protein) in a patient, comprising administering to the patient a therapeutically effective amount of a compound disclosed herein, or a pharmaceutical composition thereof.

The compounds described herein can be administered in combination with one or more additional therapeutic agents to treat a disorder described herein. For clarity, contemplated herein are both a fixed composition comprising a disclosed compound and another therapeutic agent such as disclosed herein, and methods of administering, separately a disclosed compound and a disclosed therapeutic. For example, provided in the present disclosure is a pharmaceutical composition comprising a compound described herein, one or more additional therapeutic agents, and a pharmaceutically acceptable excipient. In some embodiments, a disclosed compound and one additional therapeutic agent is administered. In some embodiments, a disclosed compound as defined herein and two additional therapeutic agents are administered. In some embodiments, a disclosed compound as defined herein and three additional therapeutic agents are administered. Combination therapy can be achieved by administering two or more therapeutic agents, each of which is formulated and administered separately. For example, a disclosed compound and an additional therapeutic agent can be formulated and administered separately. Combination therapy can also be achieved by administering two or more therapeutic agents in a single formulation, for example a pharmaceutical composition comprising a disclosed compound as one therapeutic agent and one or more additional therapeutic agents. For example, a disclosed compound and an additional therapeutic agent can be administered in a single formulation. Other combinations are also encompassed by combination therapy. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or weeks of each other. In some cases, even longer intervals are possible. While in many cases it is desirable that the two or more agents used in a combination therapy be present in within the patient's body at the same time, this need not be so.

Combination therapy can also include two or more administrations of one or more of the agents used in the combination using different sequencing of the component agents. For example, if agent X and agent Y are used in a combination, one could administer them sequentially in any combination one or more times, e.g., in the order X-Y-X, X-X-Y, Y-X-Y, Y-Y-X, X-X-Y-Y, etc.

In some embodiments, the additional therapy is the administration of side-effect limiting agents (e.g., agents intended to lessen the occurrence or severity of side effects of treatment). For example, in some embodiments, the compounds of the present invention can also be used in combination with a therapeutic agent that treats nausea. Examples of agents that can be used to treat nausea include: dronabinol, granisetron, metoclopramide, ondansetron, and prochlorperazine, or pharmaceutically acceptable salts thereof.

In some embodiments, the one or more additional therapies includes a non-drug treatment (e.g., surgery or radiation therapy). In some embodiments, the one or more additional therapies includes a therapeutic agent (e.g., a compound or biologic that is an anti-angiogenic agent, signal transduction inhibitor, antiproliferative agent, glycolysis inhibitor, or autophagy inhibitor). In some embodiments, the one or more additional therapies includes a non-drug treatment (e.g., surgery or radiation therapy) and a therapeutic agent (e.g., a compound or biologic that is an anti-angiogenic agent, signal transduction inhibitor, antiproliferative agent, glycolysis inhibitor, or autophagy inhibitor). In some embodiments, the one or more additional therapies includes two therapeutic agents. In still some embodiments, the one or more additional therapies includes three therapeutic agents. In some embodiments, the one or more additional therapies includes four or more therapeutic agents.

Examples of non-drug treatments include, but are not limited to, radiation therapy, cryotherapy, hyperthermia, surgery (e.g., surgical excision of tumor tissue), and T cell adoptive transfer (ACT) therapy. In some embodiments, the compounds of the invention may be used as an adjuvant therapy after surgery. In some embodiments, the compounds of the invention may be used as a neo-adjuvant therapy prior to surgery.

A therapeutic agent may be a compound used in the treatment of cancer or symptoms associated with cancer. For example, a therapeutic agent may be a steroid. Non-limiting examples contemplated herein include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, fiucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts or derivatives thereof.

In further embodiments, a therapeutic agent contemplated herein may be a biologic (e.g., cytokine (e.g., interferon or an interleukin such as IL-2)) used in treatment of cancer or symptoms associated therewith. In some embodiments, the biologic is an immunoglobulin-based biologic, e.g., a monoclonal antibody (e.g., a humanized antibody, a fully human antibody, an Fe fusion protein, or a functional fragment thereof) that agonizes a target to stimulate an anti-cancer response or antagonizes an antigen important for cancer. Also contemplated herein are antibody-drug conjugates.

In some embodiments, a therapeutic agent contemplated herein may be a T-cell checkpoint inhibitor. In one embodiment, the checkpoint inhibitor is an inhibitory antibody (e.g., a monospecific antibody such as a monoclonal antibody). The antibody may be, e.g., humanized or fully human. In some embodiments, the checkpoint inhibitor is a fusion protein, e.g., an Fe-receptor fusion protein. In some embodiments, the checkpoint inhibitor is an agent, such as an antibody, which interacts with a checkpoint protein. In some embodiments, the checkpoint inhibitor is an agent, such as an antibody, which interacts with the ligand of a checkpoint protein. In some embodiments, the checkpoint inhibitor is an inhibitor (e.g., an inhibitory antibody or small molecule inhibitor) of CTLA-4 (e.g., an anti-CTLA-4 antibody or fusion a protein). In some embodiments, the checkpoint inhibitor is an inhibitor or antagonist (e.g., an inhibitory antibody or small molecule inhibitor) of PD-1. In some embodiments, the checkpoint inhibitor is an inhibitor or antagonist (e.g., an inhibitory antibody or small molecule inhibitor) of PD-L 1. In some embodiments, the checkpoint inhibitor is an inhibitor or antagonist (e.g., an inhibitory antibody or Fe fusion or small molecule inhibitor) of PD-L2 (e.g., a PD-L2/lg fusion protein). In some embodiments, the checkpoint inhibitor is an inhibitor or antagonist (e.g., an inhibitory antibody or small molecule inhibitor) of B7-H3, B7-H4, BTLA, HVEM, TIM3, GAL9, LAG3, VISTA, KIR, 2B4, CD160, CGEN-15049, CHK 1, CHK2, A2aR, B-7 family ligands, or a combination thereof. In some embodiments, the checkpoint inhibitor is pembrolizumab, nivolumab, ipilimumab, tremelimumab, or lirilumab. In some embodiments, a therapeutic agent may be an anti-TIGIT antibody, such as etigilimab.

In some embodiments, a therapeutic agent contemplated herein may be an anti-cancer agent. Non-limiting examples contemplated herein include, but are not limited to, mitotic inhibitors, intercalating antibiotics, growth factor inhibitors, cell cycle inhibitors, enzymes, topoisomerase inhibitors, biological response modifiers, alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Further anti-cancer agents include leucovorin (LV), irenotecan, oxaliplatin, capecitabine, paclitaxel, and doxetaxel. In some embodiments, the one or more additional therapies includes two or more anti-cancer agents, for example, to be administered in combination or administered separately.

Other non-limiting examples of anti-cancer agents include, e.g., imatinib mesylate, carfilzornib, bortezornib, bicalutarnide, gefitinib, alkylating agents such as thiotepa and cyclosphosphamide; alkyl sulfonates such as busulfan, improsulfan and piposulfan; aziridines such as benzodopa, carboquone, and meturedopa; uredopaethylenimines and methylamelamines including altretamine, triethylenemelamine, triethylenephosphoramide, triethiylenethiophosphoramide and trimethylolomelamine; acetogenins (e.g., bullatacin and bullatacinone); camptothecin and synthetic analogues (e.g., topotecan); bryostatin; callystatin; adozelesin; carzelesin; bizelesin; cryptophycins (e.g., cryptophycin 1 and cryptophycin 8); dolastatin; duocarmycin and synthetic analogues; eleutherobin; pancratistatin; sarcodictyin A; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrsureas such as carmustine, chlorozotocin, fotemustine, lomustine, nimustine, and ranimustine; antibiotics such as the enediyne antibiotics (e.g., calicheamicin, such as calicheamicin gammall and calicheamicin omegall; dynemicin such as dynemicin A; bisphosphonates such as clodronate; an esperamicin; neocarzinostatin chromophore and related chromoprotein enediyne antiobiotic chromophores, aclacinomysins, actinomycin, authramycin, azaserine, bleomycins, cactinomycin, calicheamicin, carabicin, caminomycin, carminomycin, carzinophilin, chromomycins, dactinomycin, daunorubicin, detorubicin, 6-diazo- 5-oxo-L-norleucine, adriamycin (doxorubicin), morpholino-doxorubicin, cyanomorpholino-doxorubicin, 2-pyrrolino-doxorubicin, deoxydoxorubicin, epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone such as epothilone B; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; razoxane; rhizoxin; sizofiran; spirogermanium; tenuazonic acid; triaziquone; 2,2',2"-trichlorotriethylamine; trichothecenes such as T-2 toxin, verracurin A, roridin A and anguidine; urethane; vindesine; dacarbazine; mannomustine; mitobronitol; mitolactol; pipobroman; gacytosine; arabinoside; cyclophosphamide; thiotepa; toxoids (e.g., paclitaxel and doxetaxel; chloranbucil; tamoxifen; raloxifene; aromatase inhibiting 4(5)-irnidazoles; 4-tiydroxytamoxifen; trioxifene; keoxifene; onapristone; torernifene; flutamide, nilutarnide, bicalutarnide, leuprnlide, goserelin; chlorarnbucil; gemcitabine; 6-thioguanine; mercaptopurine; platinum coordination complexes such as cisplatin, oxaliplatin and carboplatin; vinblastine; platinum; etoposide; ifosfamide; mitoxantrone; vincristine; vinorelbine; novantrone; teniposide; edatrexate; daunomycin; aminopterin; ibandronate; irinotecan; topoisomerase inhibitors; difluoromethylornithine; retinoids such as retinoic acid; esperamicins; capecitabine; and pharmaceutically acceptable salts of any of the aforementioned agents.

Additional non-limiting examples of anti-cancer agents include trastuzumab, bevacizumab, cetuximab, rituximab, avicine, abagovomab, acridine carboxamide, adecatumumab, 17-N-allylamino-17-demethoxygeldanamycin, alpharadin, alvocidib, 3-aminopyridine-2-carboxaldehyde thiosemicarbazone, amonafide, anthracenedione, anti-CD22 immunotoxins, antineoplastics (e.g., cell-cycle nonspecific antineoplastic agents, and other antineoplastics described herein), antitumorigenic herbs, apaziquone, atiprimod, azathioprine, belotecan, bendamustine, biricodar, brostallicin, bryostatin, buthionine sulfoximine, calyculin, dichloroacetic acid, discodermolide, elsamitrucin, enocitabine, eribulin, exatecan, exisulind, ferruginol, forodesine, fosfestrol, imexon, imiquimod, indolocarbazole, irofulven, laniquidar, larotaxel, lenalidomide, lucanthone, lurtotecan, mafosfamide, mitozolomide, nafoxidine, nedaplatin, olaparib, ortataxel, pawpaw, pixantrone, proteasome inhibitors, rebeccamycin, resiquimod, rubitecan, SN-38, salinosporamide A, sapacitabine, swainsonine, talaporfin, tariquidar, tegafur-uracil, temodar, tesetaxel, triplatin tetranitrate, tris(2-chloroethyl)amine, troxacitabine, uramustine, vadimezan, vinflunine, and zosuquidar.

Further non-limiting examples of anti-cancer agents include natural products such as vinca alkaloids (e.g., vinblastine, vincristine, and vinorelbine), epidipodophyllotoxins (e.g., etoposide and teniposide), antibiotics (e.g., dactinomycin (actinomycin D), daunorubicin, and idarubicin), anthracyclines, mitoxantrone, bleomycins, plicamycin (mithramycin), mitomycin, antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexaamethylmelaamine and thiotepa), CDK inhibitors (e.g., abemaciclib, ribociclib, palbociclib; seliciclib, dinaciclib), alkyl sulfonates (e.g., busulfan), nitrosoureas (e.g., carmustine and analogs, and streptozocin), trazenes-dacarbazinine, antiproliferative/antimitotic antimetabolites such as folic acid analogs, pyrimidine analogs (e.g., fluorouracil, floxuridine, and cytarabine), purine analogs and related inhibitors (e.g., mercaptopurine, thioguanine, pentostatin, and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, romidepsin, and panobinostat), mTOR inhibitors (e.g., vistusertib, temsirolimus, everolimus, ridaforolimus, and sirolimus), KSP(Eg5) inhibitors, DNA binding agents, Pl3K delta and gamma inhibitors, copanlisib, alpelisib and idelalisib; multi-kinase inhibitors (e.g., sorafenib), hormones (e.g., estrogen), goserelin, leuprolide, triptorelin, IKK inhibitors, p38MAPK inhibitors, anti-IL-6, telomerase inhibitors, aurora kinase inhibitors, cell surface monoclonal antibodies, elotuzumab, HSP90 inhibitors, P13K /Akt inhibitors, Akt inhibitors, PKC inhibitors (e.g., enzastaurin), Torcl/2 specific kinase inhibitors, ER/UPR targeting agents, cFMS inhibitors, JAK1/2 inhibitors, PARP inhibitors (e.g., olaparib and veliparib), and BCL-2 antagonists. In some embodiments, a contemplated anti-cancer agent is selected from, for example, mechlorethamine, camptothecin, ifosfamide, tamoxifen, raloxifene, gemcitabine, sorafenib, or any analog or derivative variant of the foregoing.

In some embodiments, the anti-cancer agent is a HER2 inhibitor, for example, monoclonal antibody such as trastuzumab and pertuzumab; or a small molecule tyrosine kinase inhibitor such as gefitinib, erlotinib, pilitinib, canertinib, or lapatinib. In some embodiments, a contemplated anti-cancer agent is an ALK inhibitor, for example, ceritinib, crizotinib, alectinib, brigatinib, entrectinib, ensartinib, or lorlatinib. In some embodiments, a contemplatd anti-cancer agent is a SHP2 inhibitor an SOS1 inhibitor, a Raf inhibitor, a MEK inhibitor, an ERK inhibitor, a Pl3K inhibitor, a PTEN inhibitor, an AKT inhibitor, or an mTOR inhibitor (e.g., mTORC1 inhibitor or mTORC2 inhibitor).

In some embodiments, the antibody-drug conjugate including the microtubule inhibitor is belantamab mafodotin, brentuximab vedotin, cofetuzumab pelidotin, disitamab vedotin, enfortumab vedotin (e.g., enfortumab vedotin-ejfv, or a biosimilar thereof), mirvetuximab soravtansine (e.g., mirvetuximab soravtansine-gynx, or a biosimilar thereof), polatuzumab vedotin, telisotuzumab vedotin, tisotumab vedotin, trastuzumab emtansine (e.g., ado-trastuzumab emtansine, or a biosimilar thereof), tusamitamab ravtansine, upifitamab rilsodotin, zilovertamab vedotin, Alpha-Her2-pAF1-AS-269, BAT-8001, TAA-013, biosimilars thereof, or a combination thereof. In some embodiments, the antibody-drug conjugate including the microtubule inhibitor is enfortumab vedotin (e.g., enfortumab vedotin-ejfv, or a biosimilar thereof). In some embodiments, the antibody-drug conjugate including the microtubule inhibitor is mirvetuximab soravtansine (e.g., mirvetuximab soravtansine-gynx, or a biosimilar thereof). In some embodiments, the antibody-drug conjugate including the microtubule inhibitor is trastuzumab emtansine (e.g., ado-trastuzumab emtansine, or a biosimilar thereof). In some embodiments, the antibody-drug conjugate including the topoisomerase inhibitor is datopotamab deruxtecan, patritumab deruxtecan, sacituzumab govitecan (e.g., sacituzumab govitecan-hziy, or a biosimilar thereof), trastuzumab deruxtecan (fam-trastuzumab deruxtecan- nxki, or a biosimilar thereof), or a combination thereof. In some embodiments, the antibody- drug conjugate including the topoisomerase inhibitor is sacituzumab govitecan (e.g., sacituzumab govitecan-hziy, or a biosimilar thereof). In some embodiments, the antibody-drug conjugate including the topoisomerase inhibitor is trastuzumab deruxtecan (e.g., fam- trastuzumab deruxtecan-nxki, or a biosimilar thereof).

In some embodiments, the BCL-XL inhibitor or degrader is foselutoclax (UBX-1325), , navitoclax, obatoclax, pelcitoclax, mirzotamab clezutoclax (ABBV-155), ABBV-637, APG- 1252-12A, AZD-0466, DT-2216, PA-15227, UBX-1967, XZ-739, 753-B, or a combination thereof.

In some embodiments, the CDK4/6 inhibitor is abemaciclib, birociclib, dalpiciclib, lerociclib, milciclib, palbociclib, ribociclib (e.g., ribociclib succinate), riviciclib, roniciclib, trilaciclib (e.g., trilaciclib dihydrochloride), BPI-16350, FCN-437, SPH-4336, or a combination thereof. In some embodiments, the CDK4/6 inhibitor is palbociclib or ribociclib (e.g., ribociclib succinate).

In some embodiments, the chemotherapy is gemcitabine and paclitaxel. In some embodiments, the BCL-XL inhibitor or degrader is foselutoclax (UBX-1325), , navitoclax, obatoclax, pelcitoclax, mirzotamab clezutoclax (ABBV-155), ABBV-637, APG- 1252-12A, AZD-0466, DT-2216, PA-15227, UBX-1967, XZ-739, 753-B, or a combination thereof.

In some embodiments, the EGFR inhibitor is abivertinib, afatinib (e.g., afatinib dimaleate), alflutinib (e.g., alflutinib mesylate), almonertinib (e.g., almonertinib mesylate), befotertinib, brigatinib, canertinib, dacomitinib (e.g., dacomitinib monohydrate), dovitinib, erlotinib (e.g., erlotinib hydrochloride), gefitinib, icotinib, lapatinib (e.g., lapatinib ditosylate monohydrate), larotinib, lazertinib, limertinib, mobocertinib (e.g., mobocertinib succinate), nazartinib, neratinib (e.g., neratinib maleate), olmutinib, osimertinib (e.g., osimertinib mesylate), pelitinib, poziotinib, pyrotinib (e.g., pyrotinib maleate), ruserontinib (SKLB-1028), sapitinib, sunvozertinib, sutetinib, tesevatinib, vandetanib, varlitinib, zipalertinib, zorifertinib, BIBW-2948, BPI-7711, HA-121-28, SH-1028, or a combination thereof. In some embodiments, the EGFR inhibitor is an EGFR-targeting biologic. In some embodiments, the EGFR-targeting biologic is amivantamab (e.g., amivantamab-vmjw, or a biosimilar thereof), cetuximab (e.g., ERBITUX^{®} (cetuximab), or a biosimilar thereof (e.g., CMAB-009, CPGJ-602, or KL-140)), cetuximab sarotalocan (AKALUX^{®} (cetuximab sarotalocan), or a biosimilar thereof), depatuxizumab, duligotuzumab, futuximab, imgatuzumab, modotuximab, necitumumab (e.g., PORTRAZZA^{®} (necitumumab), or a biosimilar thereof), nimotuzumab (e.g., BIOMAb EGFR^{®} (nimotuzumab), or a biosimilar thereof), panitumumab (e.g., VECTIBIX^{®} (panitumumab), or a biosimilar thereof), tomuzotuximab, zalutumumab, EMD-55900, EMD-82633, GC-1118, HLX-07, ICR-62, SCT-200, SI-B-001, biosimilars thereof, or a combination thereof. In some embodiments, the EGFR-targeting biologic is cetuximab or panitumumab. In some embodiments, the EGFR-targeting biologic is cetuximab. In some embodiments, the EGFR- targeting biologic is panitumumab.

In some embodiments, a contemplated anti-cancer agent is an additional Ras inhibitor or a Ras vaccine, or another therapeutic modality designed to directly or indirectly decrease the oncogenic activity of Ras. For example, in some embodiments a contemplated anti-cancer agent is an additional Ras inhibitor. In some embodiments, the Ras inhibitor targets Ras in its active, or GTP-bound state (Ras(ON)). In some embodiments, the Ras inhibitor targets Ras in its inactive, or GDP-bound state. In some embodiments, the Ras inhibitor is an inhibitor of K-Ras G12C. In some embodiments, the Ras inhibitor is an inhibitor of K-Ras G12C. In some embodiments, the Ras inhibitor is a K-Ras G12V inhibitor.

In certain embodiments, the methods described herein further comprises administering to the patient one or more additional therapeutic agents that treats a disease or disorder that is affected by, associated with, or would benefit from inhibition of Ras protein (e.g., K-Ras).

The methods described herein include administering to the patient a therapeutically effective amount of at least one compound as described herein, which is optionally formulated in a pharmaceutical composition. In various embodiments, a therapeutically effective amount of at least one compound described herein present in a pharmaceutical composition is the only therapeutically active compound in a pharmaceutical composition. In certain embodiments, the method further comprises administering to the patient an additional therapeutic agent that treats a cancer, or that treats a disease or disorder that is affected by, associated with, or would benefit from inhibition of Ras, e.g., K-Ras.

In some embodiments, administering the compound(s) described herein to the patient allows for administering a lower dose of the additional therapeutic agent as compared to the dose of the additional therapeutic agent alone that is required to achieve similar results in treating, ameliorating, and/or preventing cancer, or in treating, ameliorating, and/or preventing a disease or disorder that is affected by, associated with, or would benefit from inhibition of Ras (e.g., K-Ras) in the patient. For example, in certain embodiments, the compound(s) described herein enhance(s) the activity of the additional therapeutic compound, thereby allowing for a lower dose of the additional therapeutic compound to provide the same effect.

In particular, in certain embodiments, the disclosure provides a method of treating the above medical indications comprising administering a subject in need thereof a therapeutically effective amount of a compound described herein.

Where a method of treating a patient suffering from a condition, disease or disorder (e.g., a condition, disease, or disorder that is affected by, associated with, or would benefit from inhibition of Ras, e.g., K-Ras) comprising administering to the patient a therapeutically effective amount of a compound disclosed herein, or a pharmaceutical composition thereof, is disclosed, so too is a compound disclosed herein, or a pharmaceutical composition thereof, for use in such a method.. It should be understood that references herein to methods of treatment (e.g., methods of treating a patient suffering from a condition, disease, or disorder that is affected by, associated with, or would benefit from inhibition of Ras, e.g., K-Ras) comprising administering to the patient a therapeutically effective amount of a compound disclosed herein, or a pharmaceutical composition thereof, should also be interpreted as references to:
- the use a compound disclosed herein, or a pharmaceutical composition thereof, in the manufacture of medicaments (e.g., medicaments for treating a patient suffering from a condition, disease, or disorder that is affected by, associated with, or would benefit from inhibition of Ras, e.g., K-Ras);
- the use a compound disclosed herein, or a pharmaceutical composition thereof in methods of treatment (e.g., methods of treating a patient suffering from a condition, disease, or disorder that is affected by, associated with, or would benefit from inhibition of Ras, e.g., K-Ras); and/or
- a compound disclosed herein, or a pharmaceutical composition thereof, for use in methods of treatment (e.g., methods of treating a patient suffering from a condition, disease, or disorder that is affected by, associated with, or would benefit from inhibition of Ras, e.g., K-Ras).

### III. Pharmaceutical Compositions and Kits

Another aspect of the disclosure provides pharmaceutical compositions comprising compounds as disclosed herein formulated together with a pharmaceutically acceptable carrier. In particular, the present disclosure provides pharmaceutical compositions comprising compounds as disclosed herein formulated together with one or more pharmaceutically acceptable carriers. These formulations include those suitable for oral, rectal, topical, intranasal, buccal, parenteral (e.g., subcutaneous, intramuscular, intradermal, or intravenous) rectal, vaginal, or aerosol administration, although the most suitable form of administration in any given case will depend on the degree and severity of the condition being treated and on the nature of the particular compound being used. For example, disclosed compositions may be formulated as a unit dose, and/or may be formulated for oral or subcutaneous administration.

Exemplary pharmaceutical compositions of this disclosure may be used in the form of a pharmaceutical preparation, for example, in solid, semisolid or liquid form, which contains one or more of the compounds of the disclosure, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The active object compound is included in the pharmaceutical composition in an amount sufficient to produce the desired effect upon the process or condition of the disease.

For preparing solid compositions such as tablets, the principal active ingredient may be mixed with a pharmaceutical carrier, e.g., conventional tableting ingredients such as corn starch, lactose, sucrose, sorbitol, talc, stearic acid, magnesium stearate, dicalcium phosphate or gums, and other pharmaceutical diluents, e.g., water, to form a solid preformulation composition containing a homogeneous mixture of a compound of the disclosure, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules.

In solid dosage forms for oral administration (capsules, tablets, pills, dragees, powders, granules and the like), the subject composition is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the subject composition moistened with an inert liquid diluent. Tablets, and other solid dosage forms, such as dragees, capsules, pills and granules, may optionally be scored or prepared with coatings and shells, such as enteric coatings and other coatings well known in the pharmaceutical-formulating art.

Compositions for inhalation or insufflation include solutions and suspensions in pharmaceutically acceptable, aqueous or organic solvents, or mixtures thereof, and powders. Liquid dosage forms for oral administration include pharmaceutically acceptable emulsions, microemulsions, solutions, suspensions, syrups and elixirs. In addition to the subject composition, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, cyclodextrins and mixtures thereof.

Suspensions, in addition to the subject composition, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing a subject composition with one or more suitable non-irritating excipients or carriers comprising, for example, cocoa butter, polyethylene glycol, a suppository wax or a salicylate, and which is solid at room temperature, but liquid at body temperature and, therefore, will melt in the body cavity and release the active agent.

Dosage forms for transdermal administration of a subject composition include powders, sprays, ointments, pastes, creams, lotions, gels, solutions, patches and inhalants. The active component may be mixed under sterile conditions with a pharmaceutically acceptable carrier, and with any preservatives, buffers, or propellants which may be required.

The ointments, pastes, creams and gels may contain, in addition to a subject composition, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to a subject composition, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane.

Compositions and compounds of the present disclosure may alternatively be administered by aerosol. This is accomplished by preparing an aqueous aerosol, liposomal preparation or solid particles containing the compound. A non-aqueous (e.g., fluorocarbon propellant) suspension could be used. Sonic nebulizers may be used because they minimize exposing the agent to shear, which may result in degradation of the compounds contained in the subject compositions. Ordinarily, an aqueous aerosol is made by formulating an aqueous solution or suspension of a subject composition together with conventional pharmaceutically acceptable carriers and stabilizers. The carriers and stabilizers vary with the requirements of the particular subject composition, but typically include non-ionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Pharmaceutical compositions of this disclosure suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required particle size in the case of dispersions, and by the use of surfactants.

In another aspect, the disclosure provides enteral pharmaceutical formulations including a disclosed compound and an enteric material; and a pharmaceutically acceptable carrier or excipient thereof. Enteric materials refer to polymers that are substantially insoluble in the acidic environment of the stomach, and that are predominantly soluble in intestinal fluids at specific pHs. The small intestine is the part of the gastrointestinal tract (gut) between the stomach and the large intestine, and includes the duodenum, jejunum, and ileum. The pH of the duodenum is about 5.5, the pH of the jejunum is about 6.5 and the pH of the distal ileum is about 7.5. Accordingly, enteric materials are not soluble, for example, until a pH of about 5.0, of about 5.2, of about 5.4, of about 5.6, of about 5.8, of about 6.0, of about 6.2, of about 6.4, of about 6.6, of about 6.8, of about 7.0, of about 7.2, of about 7.4, of about 7.6, of about 7.8, of about 8.0, of about 8.2, of about 8.4, of about 8.6, of about 8.8, of about 9.0, of about 9.2, of about 9.4, of about 9.6, of about 9.8, or of about 10.0. Exemplary enteric materials include cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate butyrate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (e. g. , Eudragit L30D55, Eudragit FS30D, Eudragit L100, Eudragit S100, Kollicoat EMM30D, Estacryl 30D, Coateric, and Aquateric). The solubility of each of the above materials is either known or is readily determinable *in vitro.* The foregoing is a list of possible materials, but one of skill in the art with the benefit of the disclosure would recognize that it is not comprehensive and that there are other enteric materials that would meet the objectives of the present disclosure.

The disclosure also provides kits for use by a e.g., a consumer in need of treatment of a disease or disorder described herein. Such kits include a suitable dosage form such as those described above and instructions describing the method of using such dosage form to mediate, reduce or prevent inflammation. The instructions would direct the consumer or medical personnel to administer the dosage form according to administration modes known to those skilled in the art. Such kits could advantageously be packaged and sold in single or multiple kit units. An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, *e.g.,* in the form of numbers next to the tablets or capsules whereby the numbers correspond with the days of the regimen which the tablets or capsules so specified should be ingested. Another example of such a memory aid is a calendar printed on the card, *e.g.,* as follows "First Week, Monday, Tuesday, ... etc.... Second Week, Monday, Tuesday, ... " etc. Other variations of memory aids will be readily apparent. A "daily dose" can be a single tablet or capsule or several pills or capsules to be taken on a given day. Also, a daily dose of a first compound can consist of one tablet or capsule while a daily dose of the second compound can consist of several tablets or capsules and vice versa. The memory aid should reflect this.

In some embodiments, compounds described herein are represented by any one of formulae I-IX.

In some embodiments, compounds described herein are represented by Formula IV, 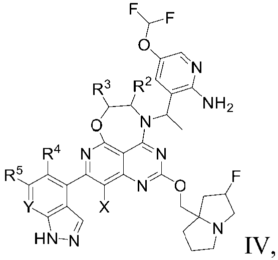 or a pharmaceutically acceptable salt, stereoisomer, or tautomer thereof, wherein
X is F or Cl;
Y is N or CH;
R² and R³ are each independently selected from C₁-C₆ alkyl and hydrogen; and
R⁴ and R⁵ are independently selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen, or one to three methyls.

In some embodiments, compounds of Formula IV are synthesized according to a general synthesis scheme:

### General Synthesis Scheme for compounds of Formula IV.

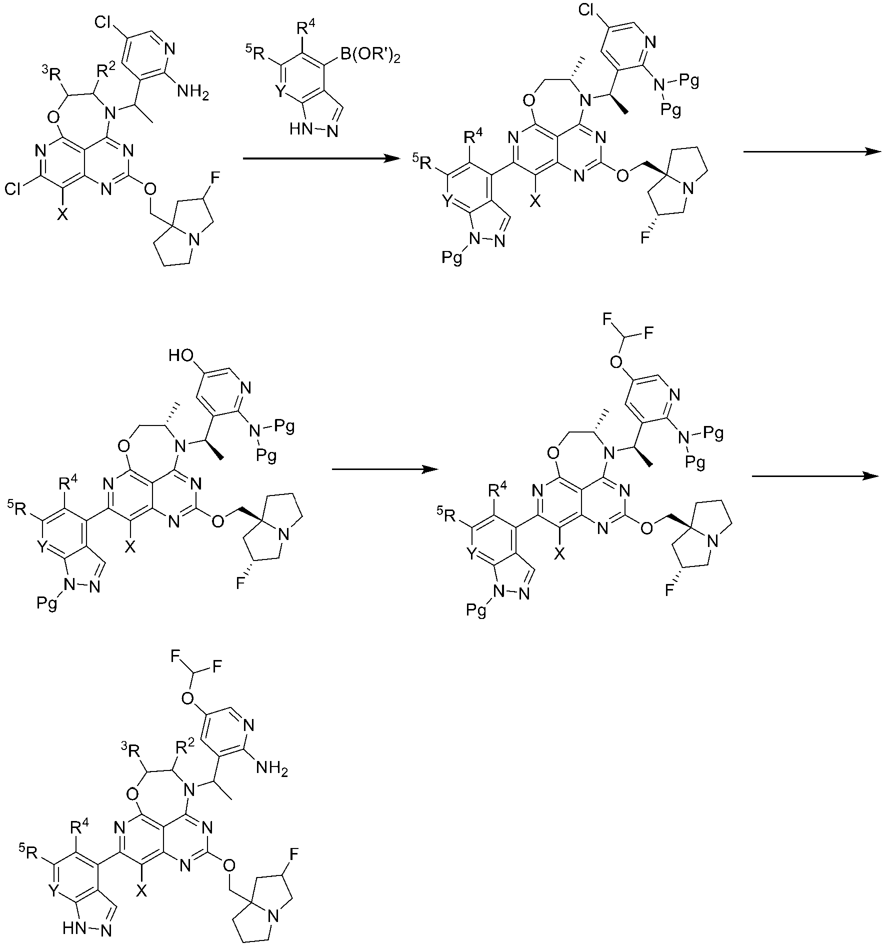

### EXAMPLES

The compounds described herein can be prepared in a number of ways based on the teachings contained herein and synthetic procedures known in the art. In the description of the synthetic methods described below, it is to be understood that all proposed reaction conditions, including choice of solvent, reaction atmosphere, reaction temperature, duration of the experiment and workup procedures, can be chosen to be the conditions standard for that reaction, unless otherwise indicated. It is understood by one skilled in the art of organic synthesis that the functionality present on various portions of the molecule should be compatible with the reagents and reactions proposed. Substituents not compatible with the reaction conditions will be apparent to one skilled in the art, and alternate methods are therefore indicated. The starting materials for the examples are either commercially available or are readily prepared by standard methods from known materials.

### HPLC Conditions:

**HPLC Method A:** Phenomenex Luna C18 150 * 25 mm * 10 um column; mobile phase: (H₂O (0.225% FA)-MeCN);
**HPLC Method B:** XBridge Prep OBD C18 Column, 30*150 mm, 5µm; mobile Phase A: Water (10mmol/L NH₄HCO₃+0.05%NH₃H₂O), mobile Phase B: MeCN; Flow rate: 60 mL/min;
**HPLC Method C:** SANTAI, SW-8222-040-SP, Spherical C18, 20-45 µm, 100 Å column ; mobile phase A: water (0.1% FA), mobile phase B: MeCN;
**HPLC Method D:** SANTAI, SW-5222-080-SP, Spherical C18, 20-45 µm, 100 Å column ; mobile phase A: water (0.1% FA), mobile Phase B: MeCN;
**HPLC Method E:** SANTAI, SW-5222-040-SP, Spherical C 18, 20-45µm, 100A column; mobile phase A: water (0.1% FA), mobile Phase B: MeCN;
**HPLC Method F:** XBridge Prep OBD C18 Column, 25*150 mm, 10µm; mobile Phase A: Water (10mmol/L NH₄HCO₃), mobile Phase B: MeCN;
**HPLC Method G:** SANTAI, SW-8222-330-SP, Spherical C 18 Column, 20-45µm, 100A; mobile phase A: water (0.1% FA), mobile phase B: MeCN.

### Synthesis of Intermediates:

### Synthesis of 3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine (Intermediate 1)

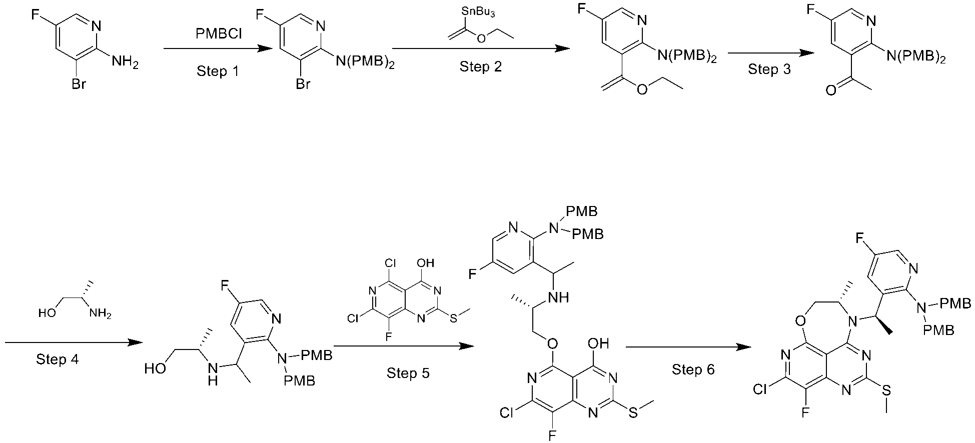

### Step 1: Synthesis of 3-bromo-5-fluoro-N,N-bis(4-methoxybenzyl)pyridin-2-amine

To a solution of 3-bromo-5-fluoropyridin-2-amine (150 g, 0.77 mol) in DMF (2 L) was added NaH (77.3 g, 1.93 mol, 60% purity) in portions at 0 °C under N₂ and the mixture was stirred at 0 °C for 1 h. PMBCl (266 g, 1.70 mol) was added dropwise at 0 °C under N₂ and the reaction mixture was stirred at rt for 18 h. The mixture was cooled and quenched with sat. NH₄Cl (2 L) dropwise and extracted with EtOAc (2 L x 2). The combined organic layers were washed with brine (500 mL x 3), dried over Na₂SO₄, filtered and the filtrate was concentrated. The residue was purified by silica gel chromatography, eluting with 0~25% EtOAc/PE to give the title compound (240 g, 72.0%) as a yellow oil.
LCMS m/z = 431/433 [M+H]+

### Step 2: Synthesis of 3-(1-ethoxyvinyl)-5-fluoro-N,N-bis(4-methoxybenzyl)pyridin-2-amine

To a solution of the compound from step 1 (120 g, 278 mmol) in DMF (1.5 L) was added tributyl(1-ethoxyvinyl)tin (151 g, 417 mmol), TEA (84.2 g, 834 mmol) and Pd(PPh₃)₂Cl₂ (8.7 g, 11.2 mmol) and the reaction mixture was stirred at 80 °C for 18 h under N₂. The mixture was poured into water (2 L) and extracted with EtOAc (2 L x 2). The combined organic layers were washed with brine (500 mL x 3), dried over Na₂SO₄, filtered and the filtrate was concentrated. The residue was purified by silica gel chromatography, eluting with 0~20% THF/PE to give the title compound (151 g, 64.2%) as a yellow gum.
LCMS m/z = 423 [M+H]+

### Step 3: Synthesis of 1-(2-(bis(4-methoxybenzyl)amino)-5-fluoropyridin-3-yl)ethan-1-one

To a solution of the compound from step 2 (150 g, 355 mmol) in dioxane (1.5 L) was added HCl/dioxane (200 mL, 4 M) and the reaction was stirred at rt for 1 h. The mixture was concentrated and the residue was diluted with sat. NaHCO₃ (300 mL) and EtOAc (2 L). The EtOAc layer was washed with brine (500 mL), dried over Na₂SO₄, filtered and the filtrate was evaporated to give the title compound (130 g, 92.8%) as yellow oil. LCMS m/z = 395 [M+H]+

### Step 4: Synthesis of (2S)-2-((1-(2-(bis(4-methoxybenzyl)amino)-5-fluoropyridin-3-yl)ethyl)amino)propan-1-ol

To a solution of the compound from step 3 (150 g, 380.3 mmol) and (S)-2-aminopropan-1-ol (85.7 g, 1.14 mol) in dioxane (1.5 L) was added Ti(i-PrO)₄ (162 g, 570 mmol) and 4Å molecular sieves (100 g, 380.3 mmol) and the mixture was stirred at 110 °C for 18 h under N₂. The mixture was cooled to 0 °C and NaBH₄ (28.8 g, 760 mmol) was added in portions while keeping the internal temperature below 20 °C. After the addition, the mixture was stirred at rt for 2 h. The mixture was cooled to 0 °C, sat. NaHCO₃ (200 mL) was added dropwise under N₂ and the mixture was stirred at rt for 1 h. The resulting mixture was poured into water (2 L) and filtered through a pad of Celite^{®}. The filter cake was rinsed with EtOAc (1 L x 2). The separated organic layers were washed with brine (500 mL), dried over Na₂SO₄, filtered and the filtrate was concentrated. The residue was purified by silica gel chromatography, eluting with 0~100% THF/PE to give the title compound (130 g, 75.4%) as a yellow gum. LCMS m/z = 454 [M+H]+

### Step 5: Synthesis of 5-((2S)-2-((1-(2-(bis(4-methoxybenzyl)amino)-5-fluoropyridin-3-yl)ethyl)amino)propoxy)-7-chloro-8-fluoro-2-(methylthio)pyrido[4,3-d]pyrimidin-4-ol

To a solution of the compound from step 4 (80.9 g, 178 mmol) in THF (1.5 L) was added NaH (25 g, 625 mmol, 60% purity) in portions at 0 °C under N₂. The mixture was stirred at 0 °C for 30 min. 5,7-dichloro-8-fluoro-2-(methylthio)pyrido[4,3-d]pyrimidin-4(3H)-one (50 g, 178.5 mmol) was added in portions at 0 °C under N₂ and the reaction mixture was stirred at 60 °C for 2.5 h. The mixture was cooled to 0 °C and sat. NH₄Cl (500 mL) was added dropwise, while keeping the internal temperature below 15 °C. The mixture was extracted with EtOAc (1 L x 2). The combined organic layers were washed with brine (500 mL), dried over Na₂SO₄, filtered and the filtrate was concentrated *in vacuo.* The residue was purified by silica gel chromatography, eluting with 0-40% of THF in PE to give the title compound (74 g, 59.4%) as a yellow solid.

### Step 6: Synthesis of 3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine

To a solution of the compound from step 5 (70 g, 100.4 mmol) in DCE (1.5 L) was added DIPEA (38.9 g, 301 mmol) and BOP-Cl (38.3 g, 150 mmol) and the reaction mixture was stirred at 90 °C for 18 h. The mixture was washed with sat. NaHCO₃ (1 L), brine (500 mL x 2), dried over Na₂SO₄, filtered and the filtrate was concentrated *in vacuo.* The residue was purified by silica gel chromatography (ISCO^{®}; 330 g SepaFlash^{®} Silica Column, Eluent of 0~30% THF/PE gradient @ 100 mL/min) followed by prep-SFC (column: REGIS (s,s) WHELK-O1 (250mm*50mm,10um); mobile phase: CO₂ / EtOH (0.1%NH₃H₂O) (45:55) isocratic elution mode) to give the title compound (28 g, 41.1%) as a yellow solid. ^{1HNMR} (400 MHz, DMSO-d₆) δ ppm 8.41 (d, 1H), 7.97 (dd, 1H), 6.89 - 6.98 (m, 1 H), 6.70 (d, 4H), 6.54 (d, 4H), 4.38 - 4.49 (m, 1H), 4.21 - 4.32 (m, 1H), 4.09 (d, 2H), 3.96 - 4.03 (m, 1H), 3.83 (d, 2H), 3.64 (s, 6H), 2.66 (s, 3H), 1.39 (br d, 3H), -0.13 (d, 3H)

### Synthesis of 3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine (Intermediate 2)

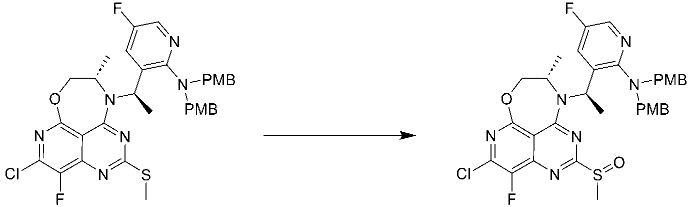

To a mixture of the compound from Intermediate 1 (26 g, 34.45 mmol) in DCM (250 mL) was added m-CPBA (7.69 g, 37.90 mmol, 85% purity) and the mixture was stirred at rt for 30 min. The reaction mixture was quenched with sat. NaHCO₃ (100 mL) and sat. Na₂SO₃ (50 mL), then extracted with DCM (200mL x 3). The combined organic phase was washed with brine (200 mL), dried over MgSO₄, filtered and concentrated under reduced pressure to give the title compound (24 g, 90.2%). LCMS m/z = 695 [M+H]+

### Synthesis of 3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine (Intermediate 3)

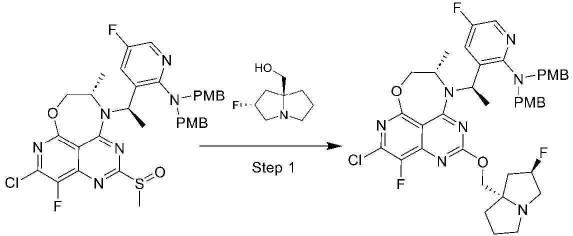

A mixture of Intermediate 2 (260 mg, 0.374 mmol) and ((2R,7aS)-2-fluoro-hexahydropyrrolizin-7a-yl)methanol (178.6 mg, 1.12 mmol) in toluene (20 mL) was stirred at rt for 1 h, then cooled down to 0°C. NaOtBu (107.8 mg, 1.12 mmol) was added and the reaction was stirred at 0°C for 15 min. The mixture was concentrated *in vacuo* and the residue was purified by silica gel column (DCM:MeOH=30:1) to afford the title compound (160 mg, 54.1%) as a white solid. LCMS m/z = 790 [M+H]+

### Synthesis of 5-chloro-3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine (Intermediate 4)

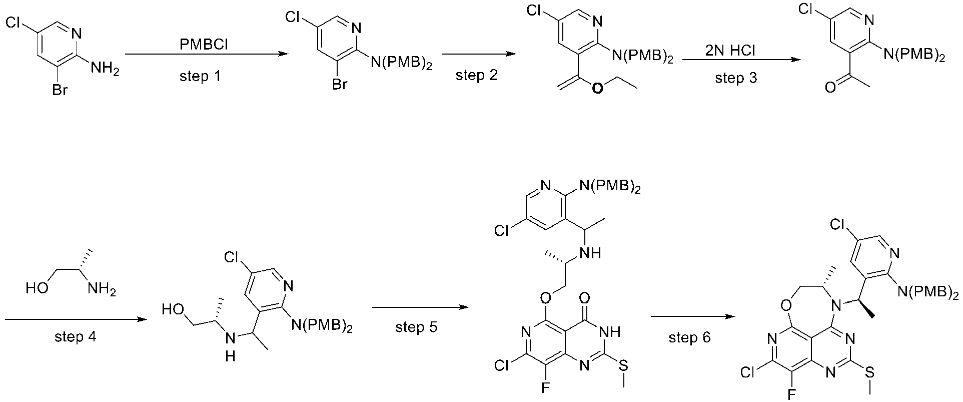

### Steps 1 and 2: Synthesis of 3-bromo-5-chloro-N,Nbis(4-methoxybenzyl)pyridin-2-amine

The title compound was obtained as a yellow oil, from 3-bromo-5-chloropyridin-2-amine, PMBCl and tributyl(1-ethoxyethenyl)stannane, following a similar procedure to that described in Intermediate 1, steps 1 and 2. LCMS m/z = 439 [M+H]+

### Step 3: Synthesis of 1-(2-(bis(4-methoxybenzyl)amino)-5-chloropyridin-3-yl)ethan-1-one

A solution of the title compound from steps 1 and 2 (21 g, 47.8 mmol) and 1M HCl (768.8 mL) in EtOAc (730 mL) was stirred at rt for 2h. The mixture was extracted with EtOAc (2 x 500 mL) and the combined organic layers were washed with NaHCO₃ (3 x 100 mL) and brine, dried over anhydrous Na₂SO₄, filtered and the filtrate was concentrated *in vacuo.* The residue was purified by silica gel column chromatography, eluting with PE / EtOAc (5:1) to afford the title compound (17 g, 86.5%) as a yellow oil. LCMS m/z = 411 [M+H]+

### Step 4: Synthesis of (2S)-2-((1-(2-(bis(4-methoxybenzyl)amino)-5-chloropyridin-3-yl)ethyl)amino)propan-1-ol

Ti(i-PrO)₄ (47.04 g, 165.5 mmol) was added to a solution of the title compound from step 3 (17 g, 41.4 mmol) and (2S)-2-aminopropan-1-ol (9.32 g, 124.1 mmol) in EtOH (300 mL) and the resulting mixture was stirred at 80°C overnight. NaBH₄ (4.70 g, 124.1 mmol) and HOAc (3.73 g, 62.1 mmol) were added at 0°C, and the reaction mixture was stirred at 0°C for 1h. The reaction was quenched with water/ice (100 mL) at 0°C, the resulting mixture was filtered and the filter cake was washed with EtOAc (4 x 500 mL). The filtrate was concentrated under reduced pressure and the residue was purified by silica gel column chromatography, eluting with PE / EtOAc (1:8) to afford the title compound (16.85 g, 86.7%) as a yellow oil. LCMS m/z = 470 [M+H]+

### Step 5: Synthesis of 5-((2S)-2-((1-(2-(bis(4-methoxybenzyl)amino)-5-chloropyridin-3-yl)ethyl)amino)propoxy)-7-chloro-8-fluoro-2-(methylthio)pyrido[4,3-d]pyrimidin-4(3H)-one

The title compound was obtained as a brown solid (6 g, 32.9%) from the title compound from step 4, following a similar procedure to that described in Intermediate 1, step 5. LCMS m/z = 713 [M+H]+

### Step 6: Synthesis of 5-chloro-3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

DIEA (3.80 g, 29.4 mmol) was added to a solution of the title compound from step 5 (7 g, 9.81 mmol) and BOPCl (7.49 g, 29.4 mmol) in CHCl₃ (120 mL) and the resulting mixture was stirred at 80°C for 20 h. The reaction was quenched with ice-water at 0°C and the mixture was extracted with DCM (3 x 100 mL). The combined organic extracts were washed with brine, dried over anhydrous Na₂SO₄, filtered and the filtrate was concentrated *in vacuo.* The residue was purified by silica gel column chromatography, eluting with PE / EtOAc (2:1) to afford the title compound (1.7 g, 22.4%) as a yellow solid.
LCMS m/z = 695 [M+H]+

### Synthesis of 5-chloro-3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine (Intermediate 5)

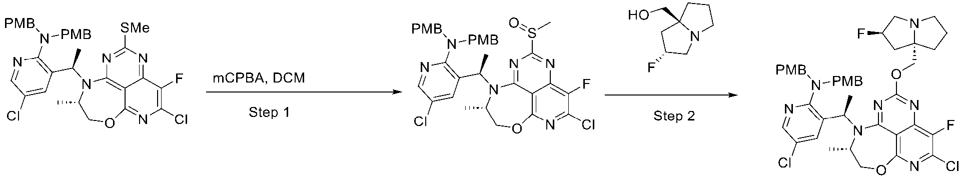

### Step 1: Synthesis of 5-chloro-3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-NN-bis(4-methoxybenzyl)pyridin-2-amine

To a solution of Intermediate 4 (1 g, 1.44 mmol) in DCM (25 mL) was added m-CPBA (0.38 g, 1.58 mmol) at 0 °C and the reaction mixture was stirred at rt for 1 h. The reaction was quenched with aq. sodium thiosulfate (20 mL) and extracted with DCM (3 x 20 mL). The organic layer was washed with aq. NaHCO₃ solution (3 x 20 mL), dried over Na₂SO₄, filtered and evaporated under reduced pressure to obtain the title compound (1 g, 96 %) as a pale yellow solid. LCMS m/z = 711 [M+H]⁺

### Step 2: Synthesis of 5-chloro-3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of the title compound from step 1 (500 mg, 0.71 mmol) in toluene (6 mL) was added ((2R,7aS)-2-fluorotetrahydro-1H-pyrrolizin-7a(5H)-yl)methanol (559 mg, 3.51 mmol) at rt and the reaction mixture was heated to 100 °C and stirred for 24 h. The cooled reaction was diluted with water (15 mL) and extracted with EtOAc (3 x 20 mL). The combined organic layers were dried over Na₂SO₄, filtered and concentrated *in vacuo.* The crude product was purified by column chromatography on silica gel using a gradient of 0-10% MeOH in DCM to obtain the title compound (320 mg, 52.5 %) as a light yellow solid. LCMS m/z = 807 [M+H]⁺

### Synthesis of 3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine (Intermediate 6)

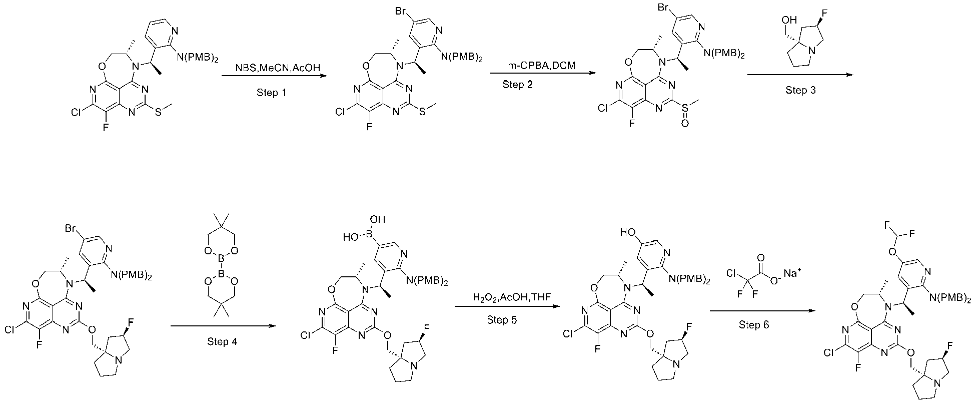

### Step 1: Synthesis of 5-bromo-3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 4 (600 mg, 0.907 mmol) in MeCN (30 mL) was added NBS (161 mg, 0.907 mmol) and HOAc (1 mL) at 0°C and the mixture was stirred at rt for 15 mins. The mixture was neutralized to pH 7 with aq. NaHCO₃ and extracted with EtOAc. The organic extract was washed with brine, dried and concentrated *in vacuo.* The crude product was purified by prep-TLC (PE:EtOAc-2:1) to afford the title compound (640 mg, 95.3%) as white solid. LCMS m/z = 739, 741 [M+H]⁺

### Step 2: Synthesis of 5-bromo-3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of the title compound from step 1 (650 mg, 0.878 mmol) in DCM (40 mL) was added m-CPBA (230 mg, 1.05 mmol, 80%) at 0°C and the reaction mixture was stirred at rt for 1h. The reaction was quenched with NaHSO₃, then extracted with DCM. The organic extract was washed with brine, dried and evaporated under reduced pressure to afford the title compound (670 mg, crude). LCMS m/z = 755, 757 [M+H]⁺

### Step 3: Synthesis of 5-bromo-3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 540 mg, 77.4%, from the title compound from step 2 and ((2R,7aS)-2-fluorotetrahydro-1H-pyrrolizin-7a(5H)-yl)methanol, following a similar procedure to that described in Intermediate 3. LCMS m/z = 850, 852 [M+H]⁺

### Step 4: Synthesis of (6-(bis(4-methoxybenzyl)amino)-5-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-yl)boronic acid

To a solution of the title compound from step 3 (400 mg, 0.47 mmol) and 2-(5,5-dimethyl-1,3,2-dioxaborinan-2-yl)-5,5-dimethyl-1,3,2-dioxaborinane (159.2 mg, 0.705 mmol) in dioxane (20 mL) was added KOAc (138.4 mg, 1.41 mmol) and Pd(dppf)Cl₂.DCM (38.4 mg, 0.047 mmol) and the reaction mixture was purged with N₂ for 4 min, then warmed to 80°C and stirred for 8h. The reaction was diluted with EtOAc (80 mL), then washed with brine (2 x 60 mL), the organic layer was dried and evaporated to afford the title compound (400 mg, crude) as brown solid. LCMS m/z = 816 [M+H]⁺

### Step 5: Synthesis of 6-(bis(4-methoxybenzyl)amino)-5-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

To a mixture of the title compound from step 4 (240 mg, 0.294 mmol) in THF (20 mL) was added H₂O₂ (0.41 mL, 13.52 mmol) and HOAc (1.01 mL, 17.64 mmol) sequentially at 0°C and the mixture was stirred at rt overnight. The reaction was diluted with EtOAc, washed with brine, the organic layer was dried and concentrated *in vacuo.* The residue was purified by reverse-phase column chromatography (C18, Mobile Phase A: water (0.5% TFA), Mobile Phase B: MeCN) to afford the title compound (200 mg, 86.3 %) as light yellow solid. LCMS m/z = 788 [M+H]⁺

### Step 6: Synthesis of 3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine

To a mixture of the title compound from step 5 (50 mg, 0.063 mmol) and sodium chlorodifluoroacetate (14.51 mg, 0.095 mmol) in DMF (5 mL) was added Cs₂CO₃ (62.0 mg, 0.19 mmol) the mixture was purged with N₂ for 3 min, then stirred at 50°C for 8h under N₂. The reaction was diluted with EtOAc, washed with brine, dried and concentrated *in vacuo.* The residue was purified by silica gel column chromatography (PE:EtOAc=1:2) to afford the title compound (22 mg, 41.4 %) as white solid. LCMS m/z = 838 [M+H]+

### Synthesis of 3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine (Intermediate 6)

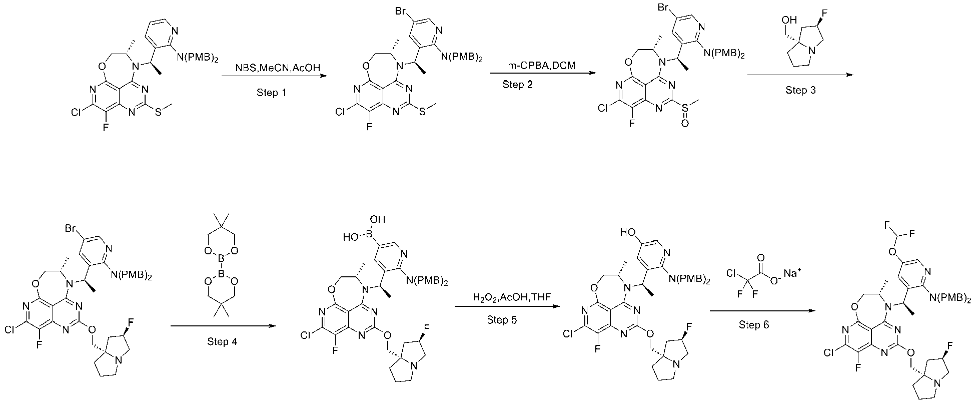

### Step 1: Synthesis of 5-bromo-3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 21 (600 mg, 0.907 mmol) in MeCN (30 mL) was added NBS (161 mg, 0.907 mmol) and HOAc (1 mL) at 0°C and the mixture was stirred at rt for 15 min. The mixture was neutralized to pH 7 with aq. NaHCO₃ and extracted with EtOAc. The organic extract was washed with brine, dried and concentrated *in vacuo.* The crude product was purified by prep-TLC (PE:EtOAc=2:1) to afford the title compound (640 mg, 95.3%) as white solid. LCMS m/z = 739, 741 [M+H]⁺

### Step 2: Synthesis of 5-bromo-3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of the title compound from step 1 (650 mg, 0.878 mmol) in DCM (40 mL) was added m-CPBA (230 mg, 1.05 mmol, 80%) at 0°C and the reaction mixture was stirred at rt for 1h. The reaction was quenched with aq. NaHSO₃, then extracted with DCM. The organic extract was washed with brine, dried and evaporated under reduced pressure to afford the title compound (670 mg, crude). LCMS m/z = 755, 757 [M+H]⁺

### Step 3: Synthesis of 5-bromo-3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid (540 mg, 77.4%) from the title compound from step 2 and ((2R,7aS)-2-fluorotetrahydro-1H-pyrrolizin-7a(5H)-yl)methanol, following a similar procedure to that described in Intermediate 3. LCMS m/z = 850, 852 [M+H]⁺

### Step 4: Synthesis of (6-(bis(4-methoxybenzyl)amino)-5-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-yl)boronic acid.

To a solution of the title compound from step 3 (400 mg, 0.47 mmol) and 2-(5,5-dimethyl-1,3,2-dioxaborinan-2-yl)-5,5-dimethyl-1,3,2-dioxaborinane (159.2 mg, 0.705 mmol) in dioxane (20 mL) was added KOAc (138.4 mg, 1.41 mmol) and Pd(dppf)Cl₂•DCM (38.4 mg, 0.047 mmol) and the reaction mixture was purged with N₂ for 4 min, then warmed to 80°C and stirred for 8h. The reaction was diluted with EtOAc (80 mL) then washed with brine (2 x 60 mL). The organic layer was dried and evaporated to afford the title compound (400 mg, crude) as brown solid. LCMS m/z = 816 [M+H]⁺

### Step 5: Synthesis of 6-(bis(4-methoxybenzyl)amino)-5-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

To a mixture of the title compound from step 4 (240 mg, 0.294 mmol) in THF (20 mL) was added H₂O₂ (25% w/w in water, 0.41 mL, 13.52 mmol) and HOAc (1.01 mL, 17.64 mmol) sequentially at 0°C and the mixture was stirred at rt overnight. The reaction was diluted with EtOAc and washed with brine. The organic layer was dried and concentrated *in vacuo.* The residue was purified by reverse-phase column chromatography (C18, Mobile Phase A: water (0.5% TFA), Mobile Phase B: MeCN) to afford the title compound (200 mg, 86.3%) as a light-yellow solid. LCMS m/z = 788 [M+H]⁺

### Step 6: Synthesis of 3-((R)-1-((S)-5-chloro-4-fluoro-2((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine.

To a mixture of the title compound from step 5 (50 mg, 0.063 mmol) and sodium chlorodifluoroacetate (14.51 mg, 0.095 mmol) in DMF (5 mL) was added Cs₂CO₃ (62.0 mg, 0.19 mmol). The mixture was purged with N₂ for 3 min, then stirred at 50°C for 8h under N₂. The reaction was diluted with EtOAc, washed with brine, dried and concentrated *in vacuo.* The residue was purified by silica gel column chromatography (PE:EtOAc=1:2) to afford the title compound (22 mg, 41.4 %) as a white solid. LCMS m/z = 838 [M+H]+

### Synthesis of 6-chloro-5-cyclopropyl-1-(tetrahydro-2H-pyran-2-yl)-4-(4,4,5,5-tetramethyl-1,3,2-dioxaborolan-2-yl)-1H-indazole (Intermediate 7)

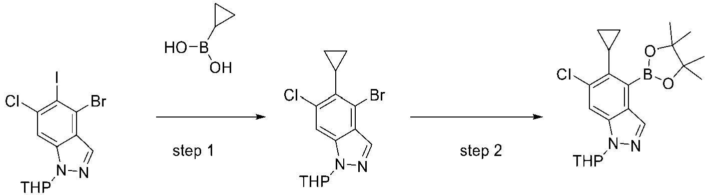

### Step 1: Synthesis of 4-bromo-6-chloro-5-cyclopropyl-1-(oxan-2-yl)indazole

A mixture of 4-bromo-6-chloro-5-iodo-1-(oxan-2-yl)indazole (2 g, 4.53 mmol), cyclopropylboronic acid (467 mg, 5.44 mmol), K₃PO₄ (2.88 g, 13.59 mmol) and Pd(dppf)Cl₂.DCM (739.9 mg, 0.91 mmol) in dioxane (15 mL) and water (5 mL) was stirred at 100°C for 2 h under N₂. The mixture was cooled to rt and diluted with water. The resulting mixture was extracted with EtOAc (3 x 50mL), the combined organic layers were washed with brine (3 x 50 mL), dried over anhydrous Na₂SO₄, filtered and the filtrate was concentrated *in vacuo.* The residue was purified by silica gel column chromatography, eluting with PE/EtOAc (1:5) to afford the title compound (0.52 g, 32.3%) as a white solid.
LCMS m/z = 357 [M+H]+

### Step 2: Synthesis of 6-chloro-5-cyclopropyl-1-(oxan-2-yl)-4-(4,4,5,5-tetramethyl-1,3,2-dioxaborolan-2-yl)indazole

A mixture of the title compound from step 1 (0.38 g, 1.07 mmol), bis(pinacolato)diboron (325.6 mg, 1.28 mmol), KOAc (314.6 mg, 3.20 mmol) and Pd(dppf)Cl₂•DCM (87.25 mg, 0.107 mmol) in dioxane (5 mL) was stirred at 100°C for 2 h under N₂. The mixture was allowed to cool to rt, then concentrated *in vacuo.* The residue was purified by silica gel column chromatography, eluting with Hexane:EtOAc (1:5) to afford the title compound (0.1 g, 23.2%) as a colorless oil. LCMS m/z = 403 [M+H]+

### Synthesis of 6-chloro-5-cyclopropyl-4-(5,5-dimethyl-1,3,2-dioxaborinan-2-yl)-1-(tetrahydro-2H-pyran-2-yl)-1H-indazole (Intermediate 8)

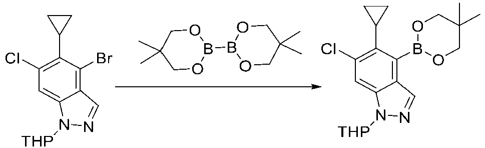

A mixture of 4-bromo-6-chloro-5-cyclopropyl-1-(oxan-2-yl)indazole (Intermediate 7, step 1, 600 mg, 1.69 mmol), 2-(5,5-dimethyl-1,3,2-dioxaborinan-2-yl)-5,5-dimethyl-1,3,2-dioxaborinane (571.6 mg, 2.53 mmol), KOAc (1074.3 mg, 5.06 mmol) and Pd(dppf)Cl₂.DCM (137.8 mg, 0.169 mmol) in dioxane (20 mL) was degassed with N₂ (x3). The reaction mixture was stirred at 100°C for 5h under N₂. The cooled mixture was concentrated *in vacuo* and the residue was purified by silica gel column chromatography (PE:EtOAc-5:1) to afford the title compound (300 mg, 55.5%) as white solid. LCMS: m/z = 321[M-C₅H₈+H]+

### Synthesis of 5-cyclopropyl-4-(5,5-dimethyl-1,3,2-dioxaborinan-2-yl)-6-fluoro-1-(tetrahydro-2H-pyran-2-yl)-1H-indazole (Intermediate 9)

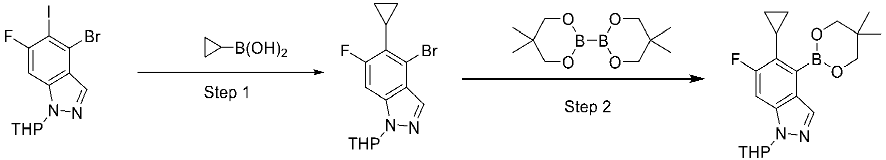

### Step 1: Synthesis of 4-bromo-5-cyclopropyl-6-fluoro-1-(tetrahydro-2H-pyran-2-yl)-1H-indazole

To a solution of 4-bromo-6-fluoro-5-iodo-1-tetrahydropyran-2-yl-indazole (4 g, 9.41 mmol) and cyclopropylboronic acid (1.62 g, 18.8 mmol) in dioxane (80 mL) and H₂O (16 mL) was added Pd(dppf)Cl₂ (688 mg, 941 µmol) and K₂CO₃ (3.90 g, 28.2 mmol) and the reaction mixture was stirred at 90 °C for 16 h under N₂. The solution was diluted with water (30 mL) and extracted with EtOAc (30 mL x 2). The organic layer was concentrated and the residue was purified by silica gel column chromatography (hexane: EtOAc=1:0 to 95:5) to give the title compound (1.4 g, 43.8%) as a yellow oil. LCMS m/z = 340 [M+H]+

### Step 2: Synthesis of 5-cyclopropyl-4-(5,5-dimethyl-1,3,2-dioxaborinan-2-yl)-6-fluoro-1-(tetrahydro-2H-pyran-2-yl)-1H-indazole

To a solution of the title compound from step 1 (1.4 g, 4.13 mmol) and 2-(5,5-dimethyl-1,3,2-dioxaborinan-2-yl)-5,5-dimethyl-1,3,2-dioxaborinane (2.80 g, 12.3 mmol) in dioxane (28 mL) was added Pd(dppf)Cl₂.DCM (674 mg, 825 µmol) and KOAc (1.22 g, 12.3 mmol) and the mixture was stirred at 100°C for 16 h under N₂. The reaction mixture was concentrated *in vacuo* and the residue was purified by silica gel column chromatography (PE: EtOAc=100:01 to 95:5). The residue was purified by prep-HPLC (column: Welch Ultimate XB-CN 250*70mm*10 um; mobile phase: [Hexane-EtOH];gradient:1%-15% B over 15.0 min) and the product containing fractions were lyophilized to give the title compound (1.2 g, 78.1%) as a colorless oil. ¹HNMR (400 MHz, DMSO-d₆) δ = 7.98 (s, ¹H), 7.49 (d, 1H), 5.76 - 5.74 (m, 1H), 3.85 (s, 5H), 3.72 (d, 1H), 2.41 - 2.31 (m, 1H), 2.01 (ddd, 2H), 1.91 (dd, 1H), 1.76 - 1.66 (m, 1H), 1.61 - 1.51 (m, 2H), 1.09 - 1.04 (m, 6H), 0.89 (t, 2H), 0.59 - 0.53 (m, 2H)

### Synthesis of 6-methyl-1-(tetrahydro-2H-pyran-2-yl)-4-(4,4,5,5-tetramethyl-1,3,2-dioxaborolan-2-yl)-5-(trifluoromethyl)-1H-indazole (Intermediate 10)

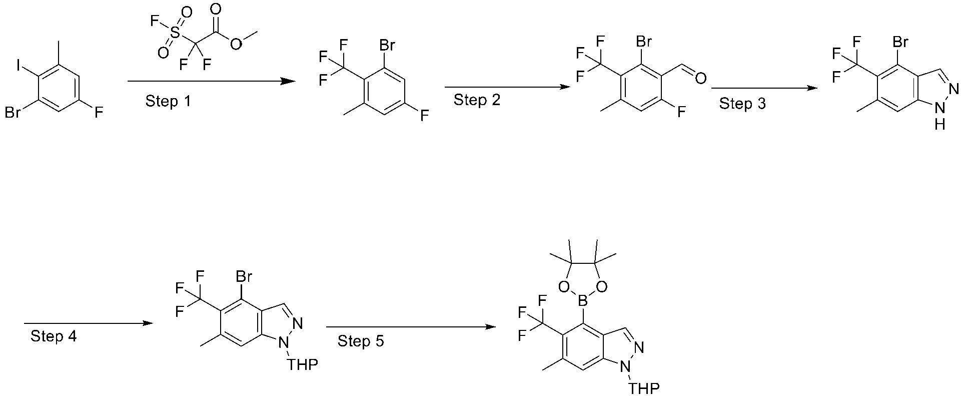

### Step 1: Synthesis of 1-bromo-5-fluoro-3-methyl-2-(trifluoromethyl)benzene

To a solution of 1-bromo-5-fluoro-2-iodo-3-methylbenzene (14 g, 44.5 mmol) in DMF (140 mL) was added CuI (72.0 g, 377.9 mmol) and methyl 2,2-difluoro-2-(fluorosulfonyl)acetate (72.6 g, 377.9 mmol) and the reaction mixture was stirred at 70 °C for 12 h under N₂. The reaction mixture was filtered, the filtrate was diluted with H₂O (200 mL) and extracted with PE (200 mL x 2). The combined organic layers were washed with brine (50 mL x 2), dried over Na₂SO₄, filtered and evaporated under reduced pressure to give the title compound (9 g, 78.8%) as a yellow oil. ^{1HNMR} (400 MHz, CDCl₃) δ: 7.33 - 7.31 (m, 1H), 6.95 (dd, 1H), 2.56 - 2.53 (m, 3H).

### Step 2: Synthesis of 2-bromo-6-fluoro-4-methyl-3-(trifluoromethyl)benzaldehyde

To a solution of the title compound from step 1 (5 g, 19.5 mmol) in THF (85 mL) was added LDA (2 M in THF, 19.45 mL) under N₂ at -65 °C and the mixture was stirred for 0.5 h. DMF (4.27 g, 58.4 mmol) was added dropwise at -65 °C and the reaction mixture was stirred for 1 h. The reaction was quenched with NH₄Cl (50 mL) at 0 °C and then extracted with EtOAc (50 mL x 3). The combined organic layers were washed with brine (50 mL x 2), dried over Na₂SO₄, filtered and concentrated *in vacuo.* The residue was purified by silica gel chromatography, eluting with 0 to 50% EtOAc/PE gradient, to give the title compound (3.3 g, 59.5%) as a yellow oil. ¹HNMR (400 MHz, CDCl₃) δ: 10.35 (s, 1H), 7.08 (d, 1H), 2.64 - 2.60 (m, 3H).

### Step 3: Synthesis of 4-bromo-6-methyl-5-(trifluoromethyl)-1H-indazole

To a solution of the title compound from step 2 (5 g, 17.5 mmol) in DMSO (50 mL) was added NH₂NH₂•H₂O (17.56 g, 17.0 mL) and the reaction mixture was stirred at 60 °C for 2 h. The pH of the reaction mixture was adjusted to 7 using 1M HCl, the mixture diluted with H₂O (100 mL) and extracted with EtOAc (100 mL x 2). The combined organic layers were washed with brine (50 mL x 3), dried over Na₂SO₄, filtered and concentrated *in vacuo* to give the title compound (4.6 g, crude) as a yellow solid. ¹HNMR (400 MHz, CDCl₃) δ 10.99 (br s, 1H), 8.19 (s, 1H), 7.31 (s, 1H), 2.66 - 2.63 (m, 3H).

### Step 4: Synthesis of 4-bromo-6-methyl-1-(tetrahydro-2H-pyran-2-yl)-5-(trifluoromethyl)-1H-indazole

To a solution of the title compound from step 3 (4.6 g, 16.48 mmol) in DCM (100 mL) was added TsOH (567.7 mg, 3.30 mmol), followed by DHP (5.55 g, 65.94 mmol) in MeCN (20 mL) and the reaction mixture was stirred at 25 °C for 12 h. The reaction mixture was concentrated *in vacuo* and the residue was purified by silica gel chromatography eluting with 0 to 50% EtOAc/PE gradient @ 100 mL/min to give the title compound (5 g, 83.5%) as a yellow oil. ¹HNMR (400 MHz, CDCl₃) δ: 8.11 (s, 1H), 7.43 (s, 1H), 5.69 (dd, 1H), 4.03 - 3.99 (m, 1H), 3.79 - 3.72 (m, 1H), 2.71 - 2.65 (m, 3H), 2.56 - 2.47 (m, 1H), 2.19 - 2.07 (m, 2H), 1.81 - 1.67 (m, 3H).

### Step 5: Synthesis of 6-methyl-1-(tetrahydro-2H-pyran-2-yl)-4-(4, 4, 5, 5-tetramethyl-1, 3, 2-dioxaborolan-2-yl)-5-(trifluoromethyl)-1H-indazole

The title compound was obtained as a white solid, 1.9 g, 36.7%, from the title compound from step 4 and bis(pinacolato)diboron, following a similar procedure to that described in Intermediate 8. ¹HNMR (400 MHz, CDCl₃) δ: 8.06 (s, 1H), 7.49 (s, 1H), 5.72 (dd, 1H), 3.98 (d, 1H), 3.77 - 3.71 (m, 1H), 2.62 (s, 3H), 2.57 - 2.48 (m, 1H), 2.18 - 2.15 (m, 1H), 2.08 - 2.04 (m, 1H), 1.80 - 1.66 (m, 3H), 1.45 (s, 12H), 1.27 (s, 1H).

### Synthesis of 4-(5,5-dimethyl-1,3,2-dioxaborinan-2-yl)-1-(4-methoxybenzyl)-6-methyl-5-(trifluoromethyl)-1H-pyrazolo[3,4-b]pyridine (Intermediate 11)

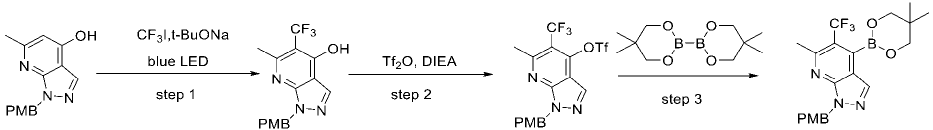

### Step 1: Synthesis of 1-(4-methoxybenzyl)-6-methyl-5-(trifluoromethyl)-1H-pyrazolo[3,4-b]pyridin-4-ol

A solution of 1-(4-methoxybenzyl)-6-methyl-1H-pyrazolo[3,4-b]pyridin-4-ol (1 g, 3.71 mmol), trifluoroiodomethane (5.82 g, 7.43 mmol 25%w/w in DMF) and t-BuONa (0.71 g, 7.43 mmol) in DMF (20 mL) was stirred at rt under LED (25W; 450 nm) irradiation overnight under N₂. The reaction was quenched with ice-water (50 mL) at rt and the mixture was extracted with EtOAc (3 x 20mL). The combined organic layers were washed with brine (20 mL), dried over Na₂SO₄ filtered and the filtrate was concentrated *in vacuo.* The residue was purified by reverse phase column chromatography (Column: C18; Mobile Phase A: Water (0.1% TFA), Mobile Phase B: MeCN; Flow rate: 60 mL/min) to afford the title compound (300 mg, 24.0%) as a brown solid. LCMS m/z = 338 [M+H]+

### Step 2: Synthesis of 1-(4-methoxybenzyl)-6-methyl-5-(trifluoromethyl)-1H-pyrazolo[3,4-b]pyridin-4-yl trifluoromethanesulfonate

Trifluoromethanesulfonic anhydride (606.4 mg, 2.15 mmol) was added to a solution of the title compound from step 1 (290 mg, 0.860 mmol) and DIEA (666.7 mg, 5.16 mmol) in DCM (3 mL) and THF (1 mL) at 0°C and the resulting mixture was stirred at rt for 2h under N₂. The reaction was quenched by the addition of water (20 mL) at rt and the resulting mixture was extracted with DCM (3 x 10mL). The combined organic layers were washed with brine (20 mL), dried over Na₂SO₄, filtered and the filtrate was concentrated *in vacuo.* The residue was purified by Prep-TLC (PE / EtOAc 7:1) to afford the title compound (120 mg, 29.7%) as a white solid. LCMS m/z = 470 [M+H]⁺.

### Step 3: Synthesis of 4-(5,5-dimethyl-1,3,2-dioxaborinan-2-yl)-1-(4-methoxybenzyl)-6-methyl-5-(trifluoromethyl)-1H-pyrazolo[3, 4-b]pyridine

The title compound was obtained as a white solid, 100 mg, 90.3%, from the title compound from step 2 and 2-(5,5-dimethyl-1,3,2-dioxaborinan-2-yl)-5,5-dimethyl-1,3,2-dioxaborinane, following a similar procedure to that described in Intermediate 8. LCMS m/z = 366 (M-C₅H₈+H]+

### Synthesis of (5-(1-fluorocyclopropyl)-6-methyl-1-(tetrahydro-2H-pyran-2-yl)-1H-indazol-4-yl)boronic acid (Intermediate 12)

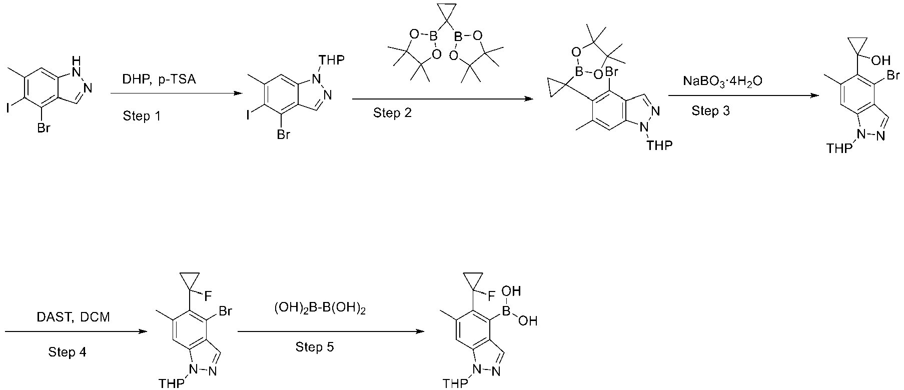

### Step 1: Synthesis of 4-bromo-5-iodo-6-methyl-1-(tetrahydro-2H-pyran-2-yl)-1H-indazole

To a solution of 4-bromo-5-iodo-6-methyl-1H-indazole (2.2 g, 6.53 mmol) in THF (22 mL) was added DHP (1.19 mL, 13.1 mmol) followed by p-toluene sulfonic acid monohydrate (0.25 g, 1.31 mmol) and the reaction mixture was stirred at 60 °C for 6h. The reaction mixture was diluted with water (50 mL) and extracted with EtOAc (2 x 20 mL). The combined organic layer was washed with brine (30 mL), dried over Na₂SO₄, filtered and concentrated *in vacuo.* The crude was purified by column chromatography on silica gel using a gradient of 0-100% EtOAc in hexane as eluents to afford the title compound (2 g, 64.2 %) as an off-white solid. LCMS m/z = 421 [M+H]⁺

### Step 2: Synthesis of 4-bromo-6-methyl-1-(tetrahydro-2H-pyran-2-yl)-5-(1-(4,4,5,5-tetramethyl-1,3,2-dioxaborolan-2-yl)cyclopropyl)-1H-indazole

To a solution of the title compound from step 1 (1 g, 2.38 mmol) and 2,2'-(cyclopropane-1,1-diyl)bis(4,4,5,5-tetramethyl-1,3,2-dioxaborolane) (1.75g, 5.94 mmol) in dioxane (12 mL) was added a solution of KOH (0.34 g, 5.94 mmol) in water (3 mL), the mixture was degassed with N₂ for 3 min, then PdCl₂(dppf) •DCM (0.19 g, 0.24 mmol) was added and the reaction mixture was stirred for 6 h at 100 °C. The cooled reaction mixture was diluted with water (20 mL) and extracted with EtOAc (3 x 20 mL). The combined organic layer was washed with brine (20 mL), dried over Na₂SO₄, filtered and concentrated *in vacuo.* The crude was purified by column chromatography on silica gel using a gradient of 0-100% EtOAc in hexane to afford the title compound (700 mg, 62.2 %) as an off-white solid. LCMS m/z = 462 [M+H]+

### Step 3: Synthesis of 1-(4-bromo-6-methyl-1-(tetrahydro-2H-pyran-2-yl)-1H-indazol-5-yl)cyclopropan-1-ol

Sodium perborate tetrahydrate (6.0 g, 39.0 mmol) was added to a solution of the title compound from step 2 (900 mg, 1.95 mmol) in THF (18 mL) and water (18 mL) and the reaction mixture was stirred at 25 °C for 16h. The reaction mixture was diluted with water (50 mL) and extracted with EtOAc (2 x 20 mL). The combined organic layer was washed with sat. aq. Na₂S₂O₃ solution (40 mL), then brine (20 mL), dried over anhydrous Na₂SO₄, filtered and concentrated *in vacuo* to afford the title compound (400 mg, 55.4 %) as colorless oil. LCMS m/z = 351 [M+H]+

### Step 4: Synthesis of 4-bromo-5-(1-fluorocyclopropyl)-6-methyl-1-(tetrahydro-2H-pyran-2-yl)-1H-indazole

DAST (0.30 mL, 2.28 mmol) was added to a solution of the title compound from step 3 (400 mg, 1.14 mmol) in DCM (6 mL) at -78 °C and the reaction mixture was stirred at -78 °C for 0.5 h. The reaction mixture was quenched with sat. NaHCO₃ (20 mL) and extracted with DCM (3 x 20 mL). The combined organic layer was washed with brine (20 mL), dried over Na₂SO₄, filtered and concentrated *in vacuo.* The crude was purified by column chromatography on silica gel using a gradient of 0-100% EtOAc in hexane to afford the title compound (290 mg, 70.6 %) as colorless oil. LCMS m/z = 355 [M+H]+

### Step 5: Synthesis of (5-(1-fluorocyclopropyl)-6-methyl-1-(tetrahydro-2H-pyran-2-yl)-1H-indazol-4-yl)boronic acid

A solution of the title compound from step 4 (250 mg, 0.71 mmol) and tetrahydroxydiboron (190 mg, 2.12 mmol) in MeOH (2.5 mL) was degassed with N₂ for 2 min. TEA (0.40 mL, 2.83 mmol) and cataCXium^{®} A Pd G2 (95 mg, 0.14 mmol) were added and the resulting reaction mixture was stirred at 60 °C for 2h. The reaction mixture was filtered through a filter pad and washed with EtOAc (2 x 20 mL). The filtrate was concentrated *in vacuo* and the crude was purified by column chromatography on silica gel using a gradient of 0-100% EtOAc in hexane to afford the title compound (150 mg, 66.2 %) as pale brown foam. LCMS m/z = 320 [M+H]+

### Synthesis of 4-(5,5-dimethyl-1,3,2-dioxaborinan-2-yl)-6-fluoro-5-(1-fluorocyclopropyl)-1-(tetrahydro-2H-pyran-2-yl)-1H-indazole (Intermediate 13)

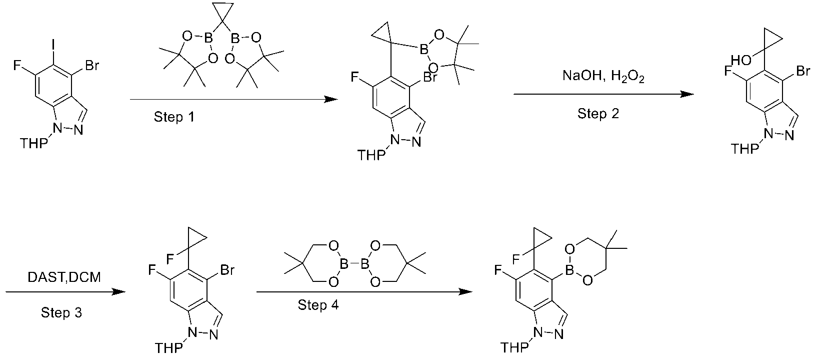

### Step 1: Synthesis of 4-bromo-6-fluoro-1-(tetrahydro-2H-pyran-2-yl)-5-(1-(4,4,5,5-tetramethyl-1,3,2-dioxaborolan-2-yl)cyclopropyl)-1H-indazole

To a mixture of 4-bromo-6-fluoro-5-iodo-1-tetrahydropyran-2-yl-indazole (6.60 g, 15.5 mmol) and 2,2'-(cyclopropane-1,1-diyl)bis(4,4,5,5-tetramethyl-1,3,2-dioxaborolane) (9.13 g, 31.0 mmol) in dioxane (130 mL) and H₂O (26 mL) was added Pd(dppf)Cl₂.DCM (1.27 g, 1.55 mmol) and K₂CO₃ (6.44 g, 46.5 mmol) and the mixture was stirred at 120 °C for 16 h under N₂. The reaction mixture was concentrated *in vacuo,* and the residue was purified by silica gel column chromatography (PE / EtOAc=1/0 to 14/1) to give the title compound (3.23 g, 42%) as colorless oil. LCMS m/z = 465 [M+H]+

### Step 2: Synthesis of 1-(4-bromo-6-fluoro-1-(tetrahydro-2H-pyran-2-yl)-1H-indazol-5-yl)cyclopropan-1-ol

To a mixture of the title compound from step 1 (3.23 g, 6.94 mmol) in THF (30 mL) and MeOH (3 mL) was added NaOH (555 mg, 13.9 mmol) at 0 °C and the mixture stirred at 0 °C for 5 min. H₂O₂ (5.73 g, 55.5 mmol, 33% purity) was added at 0 °C and the reaction mixture was stirred at 25 °C for 1 h. The reaction mixture was quenched with saturated Na₂SO₃ soln. (50 mL) at 0 °C and the mixture extracted with EtOAc (100 mL x 3). The combined organic layers were washed with saturated brine (50 mL), dried over anhydrous Na₂SO₄, filtered and evaporated under reduced pressure to give the title compound (2.92 g, crude) as yellow oil. LCMS m/z = 355 [M+H]+

### Step 3: Synthesis of 4-bromo-6-fluoro-5-(1-fluorocyclopropyl)-1-(tetrahydro-2H-pyran-2-yl)-1H-indazole

The title compound was obtained as a yellow oil, 1.18 g, 34%, from the title compound from step 2, following a similar procedure to that described in Intermediate 12, step 4. LCMS m/z = 274 [M+H]+

### Step 4: Synthesis of 4-(5,5-dimethyl-1,3,2-dioxaborinan-2-yl)-6-fluoro-5-(1-fluorocyclopropyl)-1-(tetrahydro-2H-pyran-2-yl)-1H-indazole

The title compound was obtained as a yellow solid, 1.16 g, 89%, from the title compound from step 3 and (5,5,5',5'-tetramethyl-2,2'-bi(1,3,2-dioxaborinane) following a similar procedure to that described in Intermediate 8. LCMS m/z = 323 [M+H]+

### Synthesis of 4-(5,5-dimethyl-1,3,2-dioxaborinan-2-yl)-6-fluoro-5-(spiro[2.2]pentan-1-yl)-1-(tetrahydro-2H-pyran-2-yl)-1H-indazole (Intermediate 14)

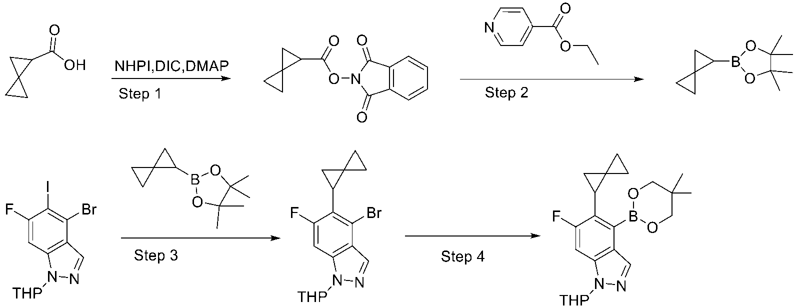

### Step 1: Synthesis of 1,3-dioxoisoindolin-2-yl spiro[2.2]pentane-1-carboxylate

To a stirred mixture of spiro[2.2]pentane-1-carboxylic acid (860 mg, 7.67 mmol) and 2-hydroxy-2,3-dihydro-1H-isoindole-1,3-dione (1.38 g, 8.44 mmol) in DCM (5 mL) was added DMAP (937 mg, 7.67 mmol) at 0°C. N,N'-Diisopropylcarbodiimide (1.07 g, 8.44 mmol) was added dropwise over 2 min and the reaction mixture was stirred at rt for 3h. The resulting mixture was filtered and the filter cake was washed with DCM (3x10 mL). The filtrate was washed with aq. NaHCO₃ (3x10 mL) and aq. NaHSO₄ (10%), then evaporated under reduced pressure to afford the title compound (1.9 g) as a yellow solid. LCMS m/z = 258 [M+H]+

### Step 2: Synthesis of 4, 4, 5, 5-tetramethyl-2-(spiro[2.2]pentan-1-yl)-1, 3, 2-dioxaborolane

To a stirred solution of the title compound from step 1 (1.9 g, 7.39 mmol) and ethyl isonicotinate (111.7 mg, 0.74 mmol) in EtOAc (30 mL) was added bis(pinacolato)diboron (4.13 g, 16.25 mmol) and the resulting mixture was stirred at 85°C overnight under N₂. The mixture was allowed to cool to rt and concentrated *in vacuo.* The residue was triturated with hexane, the resulting mixture was filtered and the filter cake was washed with hexane (2x10 mL). The filtrate was concentrated *in vacuo* and the residue was purified by silica gel column chromatography, eluting with DCM / PE (1:1) to afford the title compound (300 mg, 20.9%) as a colorless oil. ¹HNMR (400 MHz, CDCl₃) δ 1.16 (m, 12H), 1.02 (m, 1H), 0.97 (m, 1H), 0.85 - 0.63 (m, 4H), 0.29 (m, 1H).

### Step 3: Synthesis of 4-bromo-6-fluoro-5-(spiro[2.2]pentan-1-yl)-1-(tetrahydro-2H-pyran-2-yl)-1H-indazole

A solution of 4-bromo-6-fluoro-5-iodo-1-(tetrahydro-2H-pyran-2-yl)-1H-indazole (100 mg, 0.235 mmol), the title compound from step 2 (91.3 mg, 0.47 mmol), K₂CO₃ (97.55 mg, 0.705 mmol) and Pd(dppf)Cl₂ (34.4 mg, 0.047 mmol) in dioxane (2 mL) and H₂O (0.4 mL) was stirred at 100°C for 6h under N₂. The reaction was quenched with water at rt and the resulting mixture was extracted with EtOAc (3 x 5mL). The combined organic layers were washed with brine (10 mL), dried over anhydrous Na₂SO₄, filtered and the filtrate was concentrated *in vacuo.* The residue was purified by Prep-TLC (PE / EtOAc 5:1) to afford the title compound (30 mg, 34.9%) as a yellow solid. LCMS m/z = 365 [M+H]+

### Step 4: Synthesis of 4-(5,5-dimethyl-1,3,2-dioxaborinan-2-yl)-6-fluoro-5-(spiro[2.2]pentan-1-yl)-1-(tetrahydro-2H-pyran-2-yl)-1H-indazole

A solution of the title compound from step 3 (55 mg, 0.151 mmol), 2-(5,5-dimethyl-1,3,2-dioxaborinan-2-yl)-5,5-dimethyl-1,3,2-dioxaborinane (68.03 mg, 0.302 mmol), Pd(dppf)Cl₂ (22.04 mg, 0.030 mmol) and KOAc (44.34 mg, 0.453 mmol) in dioxane (1 mL) was stirred at 100°C for 1h under N₂. The reaction was quenched with water at rt and the resulting mixture was extracted with EtOAc (3 x 5mL). The combined organic layers were washed with brine (10 mL), dried over anhydrous Na₂SO₄, filtered and the filtrate was concentrated *in vacuo.* The residue was purified by Prep-TLC (PE / EtOAc 5:1) to afford the title compound (53 mg, 88.4%) as a yellow solid. LCMS m/z = 399 [M+H]+

### Synthesis of (5-(1-fluorocyclopropyl)-1-(4-methoxybenzyl)-6-methyl-1H-pyrazolo[3,4-b]pyridin-4-yl)boronic acid (Intermediate 15)

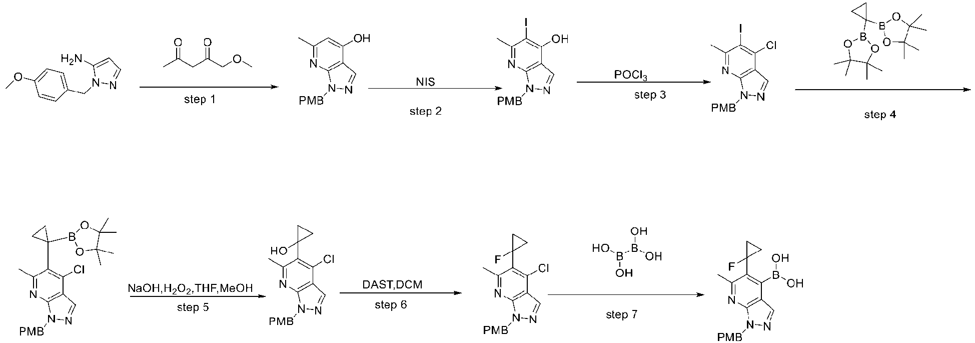

### Step 1: Synthesis of 1-(4-methoxybenzyl)-6-methyl-1H-pyrazolo[3,4-b]pyridin-4-ol

A solution of 1-(4-methoxybenzyl)-1H-pyrazol-5-amine (5 g, 24.6 mmol) in 1-methoxypentane-2,4-dione (50 mL) was stirred at 190 °C for 16 h under N₂. The mixture was allowed to cool to rt, then diluted with water (50 mL). The resulting mixture was extracted with EtOAc (3 x 50mL), the combined organic layers were washed with brine (2 x 20 mL), dried over anhydrous Na₂SO₄, filtered and the filtrate was concentrated *in vacuo.* The crude product was purified by reverse phase column chromatography (column, C18 silica gel; mobile phase, MeCN in Water (0.1% TFA), 20% to 50% gradient in 10 min) to afford the title compound (2.4 g, 36.2%) as a yellow solid. LCMS m/z = 270 [M+H]+

### Step 2: Synthesis of 5-iodo-1-(4-methoxybenzyl)-6-methyl-1H-pyrazolo[3, 4-b]pyridin-4-ol

A mixture of the title compound from step 1 (4.4 g, 16.3 mmol) and N-iodosuccinimide (5.51 g, 24.51 mmol) in MeCN (50 mL) was stirred at rt for 2 h under N₂. The resulting mixture was concentrated *in vacuo* and the residue was diluted with water (20 mL). The mixture was extracted with EtOAc (3 x 20 mL), the combined organic layers were washed with brine (2 x 10 mL), dried over anhydrous Na₂SO₄, filtered and the filtrate was concentrated *in vacuo.* The residue was purified by reverse-phase column chromatography (column, C18 silica gel; mobile phase, MeCN in Water (0.1% TFA), 20% to 60% gradient in 20 min) to afford the title compound (2 g, 31.0%) as a yellow solid. LCMS m/z = 396 [M+H]+

### Step 3: Synthesis of 4-chloro-5-iodo-1-(4-methoxybenzyl)-6-methyl-1H-pyrazolo[3,4-b]pyridine

A solution of the title compound from step 2 (2 g, 5.06 mmol) in POCl₃ (20 mL) was stirred at 100 °C for 1 h under N₂. The mixture was allowed to cool to rt then concentrated *in vacuo.* The reaction was quenched with water (20mL) and the mixture was extracted with EtOAc (3 x 20mL). The combined organic layers were washed with brine (2 x 10 mL), dried over anhydrous Na₂SO₄, filtered and the filtrate was concentrated *in vacuo.* The residue was purified by Prep-TLC (DCM / MeOH 15:1) to afford the title compound (1.4 g, 66.9%) as a yellow solid. LCMS m/z = 414 [M+H]+

### Step 4: Synthesis of 4-chloro-1-(4-methoxybenzyl)-6-methyl-5-(1-(4,4, 5, 5-tetramethyl-1, 3,2-dioxaborolan-2-yl)cyclopropyl)-1H-pyrazolo[3,4-b]pyridine

A mixture of the title compound from step 3 (1.4 g, 3.39 mmol), 2,2'-(cyclopropane-1,1-diyl)bis(4,4,5,5-tetramethyl-1,3,2-dioxaborolane) (1.49 g, 5.08 mmol), Cs₂CO₃ (2.21 g, 6.77 mmol) and Pd(dppf)Cl₂ (495.3 mg, 0.68 mmol) in dioxane (16 mL) and H₂O (2 mL) was stirred at 80°C for 16 h under N₂. The mixture was allowed to cool down to rt, the resulting mixture was filtered and the filter cake was washed with EtOAc (3x10 mL). The filtrate was concentrated *in vacuo* and the residue was purified by Prep-TLC (PE / EA 5:1) to afford the title compound (360 mg, 23.4%) as a yellow solid. LCMS m/z = 454 [M+H]+

### Step 5: Synthesis of 1-(4-chloro-1-(4-methoxybenzyl)-6-methyl-1H-pyrazolo[3, 4-b]pyridin-5-yl)cyclopropan-1-ol

To a solution of the title compound from step 4 (360 mg, 0.793 mmol) in THF (5 mL) and MeOH (0.5 mL) was added NaOH (63.4 mg, 1.59 mmol) and the mixture cooled to 0°C. H₂O₂ (53.97 mg, 1.59 mmol) was added dropwise at 0 °C under N₂ and the reaction mixture was stirred at °C for 30 min. The reaction was quenched with sat. NaHCO₃ (aq.) (10 mL) at 0 °C and the mixture extracted with DCM (3 x 20 mL). The combined organic layers were washed with brine (2 x 10 mL), dried over anhydrous Na₂SO₄, filtered and the filtrate was concentrated *in vacuo.* The residue was purified by Prep-TLC (DCM / MeOH 15:1) to afford the title compound (200 mg, 73.3%) as a light yellow solid. LCMS M/z = 344 [M+H]+

### Step 6: Synthesis of 4-chloro-5-(1-fluorocyclopropyl)-1-(4-methoxybenzyl)-6-methyl-1H-pyrazolo[3,4-b]pyridine

To a stirred solution of the title compound from step 4 (200 mg, 0.582 mmol) in DCM (5 mL) was added DAST (281.3 mg, 1.75 mmol) dropwise at -78 °C under N₂ and the reaction mixture was stirred at -78 °C for 30 min. The reaction mixture was allowed to warm to rt then quenched with water (10 mL). The resulting mixture was extracted with DCM (3 x 20 mL), the combined organic layers were washed with brine (2 x 10 mL), dried over anhydrous Na₂SO₄, filtered and the filtrate was concentrated *in vacuo.* The residue was purified by Prep-TLC (DCM / MeOH 15:1) to afford the title compound (160 mg, 79.5%) as a light yellow solid. LCMS m/z = 346 [M+H]+

### Step 7: Synthesis of (5-(1-fluorocyclopropyl)-1-(4-methoxybenzyl)-6-methyl-1H-pyrazolo[3,4-b]pyridin-4-yl)boronic acid

A mixture of the title compound from step 6 (100 mg, 0.289 mmol), tetrahydroxydiboron (51.85 mg, 0.578 mmol), TEA (117.1 mg, 1.16 mmol) and cataCXium^{®} A Pd G3 (21.1 mg, 0.029 mmol) in MeOH (5 mL) was stirred at rt for 2 h under N₂. The reaction was quenched with water (10 mL) at 0 °C. The resulting mixture was extracted with EtOAc (3 x 20mL), the combined organic layers were washed with brine (2 x 10 mL), dried over anhydrous Na₂SO₄, filtered and the filtrate was concentrated *in vacuo.* The residue was purified by Prep-TLC (DCM / MeOH 15:1) to afford the title compound (75 mg, 73.0%) as a white solid. LCMS m/z = 356 [M+H]+

### Synthesis of 4-(5,5-dimethyl-1,3,2-dioxaborinan-2-yl)-5-(1-fluorocyclopropyl)-1-(4-methoxybenzyl)-6-methyl-1H-pyrazolo[3,4-b]pyridine (Intermediate 16)

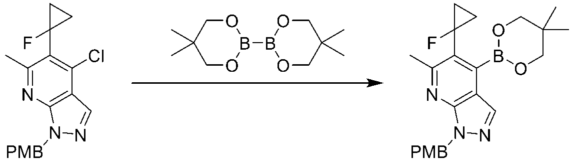

The title compound was obtained as a colorless oil, 180 mg, 66.5%, from 4-chloro-5-(1-fluorocyclopropyl)-1-(4-methoxybenzyl)-6-methyl-1H-pyrazolo[3,4-b]pyridine (Intermediate 15, step 6) and 5,5,5',5'-tetramethyl-2,2'-bi(1,3,2-dioxaborinane), following a similar procedure to that described in Intermediate 8. LCMS: m/z = 356 [M+H]+

### Synthesis of (5-(difluoromethoxy)-6-methyl-1-(tetrahydro-2H-pyran-2-yl)-1H-indazol-4-yl)boronic acid (Intermediate 17)

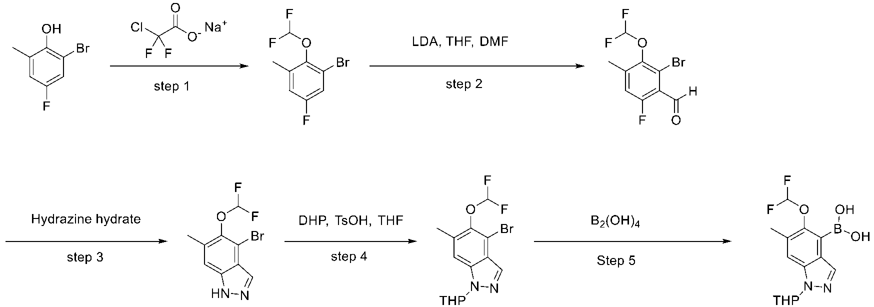

### Step 1: Synthesis of 1-bromo-2-(difluoromethoxy)-5-fluoro-3-methylbenzene

To a solution of 2-bromo-4-fluoro-6-methylphenol (1 g, 4.88 mmol) in DMF (10 mL) was added K₂CO₃ (2.02 g, 14.63 mmol) followed by sodium chlorodifluoroacetate (1.12 g, 7.32 mmol) and the reaction mixture was stirred at 100 °C for 3 h. The cooled reaction mixture was diluted with water (50 mL), extracted with EtOAc (3 x 50 mL), the combined organic layer was dried over anhydrous Na₂SO₄, filtered and concentrated *in vacuo.* The crude compound was purified by silica column chromatography using a gradient of 0-100% EtOAc/hexane to afford the title compound (600 mg, 44.4 %) as an off white solid. ¹HNMR (400 MHz, CDCl₃): δ 7.21 (dd, 1H), 7.07 (dd, 1H), 6.51 (t, 1H), 2.39 (s, 3H).

### Step 2: Synthesis of 2-bromo-3-(difluoromethoxy)-6-fluoro-4-methylbenzaldehyde

To a solution of the title compound from step 1 (800 mg, 3.14 mmol) in dry THF (10 mL) was added LDA (2.35 mL, 2M in THF, 4.71 mmol) at -78 °C and the mixture stirred for 40 min at -78 °C. DMF (1.70 mL, 22.0 mmol) was added at -78 °C and the reaction mixture stirred at -78 °C for 30 min. The reaction was quenched with ice-cold water (20 mL), extracted with EtOAc (3 x 50 mL), the combined organic layer was dried over anhydrous Na₂SO₄, filtered, and concentrated *in vacuo.* The crude compound was purified by silica column chromatography eluting with 0-100% EtOAc in hexane to afford the title compound (350 mg, 39.4 %) as an off white solid. ¹HNMR (400 MHz, CDCl₃): δ 10.32 (s, 1H), 7.09 (d, 1H), 6.56 (t, 1H), 2.47 (s, 3H).

### Step 3: Synthesis of 4-bromo-5-(difluoromethoxy)-6-methyl-1H-indazole

To a solution of the title compound from step 2 (1 g, 3.53 mmol) in DMSO (10 mL) was added hydrazine hydrate (0.88 g, 17.67 mmol) and the reaction mixture was stirred at 120 °C for 6 h. The reaction mixture was cooled to rt, diluted with water (50 mL) and stirred for 15 min. The resulting solid was filtered off and dried under vacuum to afford the title compound (700 mg, 59.4 %) as an off white solid. LCMS m/z = 277 [M+H]+

### Step 4: Synthesis of 4-bromo-5-(difluoromethoxy)-6-methyl-1-(tetrahydro-2H-pyran-2-yl)-1H-indazole

The title compound was obtained as an off-white solid, 750 mg, 76%, from the title compound from step 3, following a similar procedure to that described in Intermediate 12, step 1. LCMS m/z = 361 [M+H]+

### Step 5: Synthesis of (5-(difluoromethoxy)-6-methyl-1-(tetrahydro-2H-pyran-2-yl)-1H-indazol-4-yl)boronic acid

To a solution of the title compound from step 4 (1 g, 2.77 mmol) in MeOH (15 ml) was added tetrahydroxydiboron (0.99 g, 11.07 mmol) and TEA (1.16 ml, 8.31 mmol), then cataCXium^{®} A Pd G2 (0.20 g, 0.28 mmol) was added under degassing with N₂. The reaction mixture was stirred for 1 h at 25 °C. The mixture was filtered through a Celite^{®} pad, washing through with MeOH (3 x 20 mL). The combined filtrate was evaporated under reduced pressure to give the title compound (800 mg, 62.6 %) as a light brown gum. LCMS m/z = 328 [M+H]+

### Synthesis of 4-(5,5-dimethyl-1,3,2-dioxaborinan-2-yl)-5-(1-fluorocyclopropyl)-6-methyl-1-(tetrahydro-2H-pyran-2-yl)-1H-indazole (Intermediate 18)

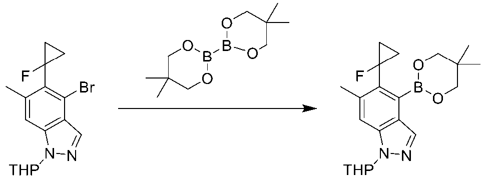

The title compound was obtained as a yellow solid, 3.0 g, 94%, from Intermediate 12, step 4 and 2-(5,5-dimethyl-1,3,2-dioxaborinan-2-yl)-5,5-dimethyl-1,3,2-dioxaborinane, following a similar procedure to that described in Intermediate 8. 1H NMR (400 MHz, CDCl₃) δ = 8.11 (s, 1H), 7.45 (s, 1H), 5.79 - 5.54 (m, 1H), 4.08 - 3.95 (m, 1H), 3.91 (s, 4H), 3.83 - 3.73 (m, 1H), 2.71 (s, 3H), 2.62 - 2.49 (m, 1H), 2.21 - 1.99 (m, 2H), 1.84 - 1.66 (m, 3H), 1.53 - 1.41 (m, 2H), 1.17 (s, 6H), 0.99 - 0.96 (m, 2H)

### Synthesis of ((1S,2S,5R)-3-methyl-3-azabicyclo[3.1.0]hexan-2-yl)methanol (Intermediate 19)

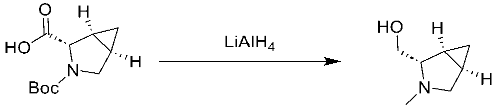

Solution 1: (1S,25,5R)-3-(tert-butoxycarbonyl)-3-azabicyclo[3.1.0]hexane-2-carboxylic acid (2 g, 8.80 mmol) in 2-MeTHF (20 mL). Solution 2: LiAlH₄ (2.5 M, THF, 14.08 mL)

Solution 1 was pumped at 4.7 mL/min to the flow reactor (CSTRs, 40.0 mL, 80.0°C).

Solution 2 was pumped at 3.4 mL/min to the same flow reactor. The residence time of the flow reactor was 10.0 min. The reaction mixture was quenched by Na₂SO₄•10H₂O (10 g) at 0 °C and the mixture was filtered. The filtrate was evaporated under reduced pressure to give the title compound (1.05 g, 89.1%) as a white solid. ¹H NMR (400 MHz, DMSO-d₆) δ = 4.43 (s, 1H), 3.51 - 3.43 (m, 1H), 3.31 - 3.24 (m, 1H), 2.82 (dd, 1H), 2.66 (dd, 1H), 2.54 (d, 1H), 2.28 (s, 3H), 1.39 - 1.29 (m, 2H), 0.49 (dt, 1H), 0.33 (q, 1H).

### Synthesis of 6-chloro-5-cyclopropyl-1-(tetrahydro-2H-pyran-2-yl)-1H-pyrazolo[3,4-b]pyridin-4-yl trifluoromethanesulfonate (Intermediate 20)

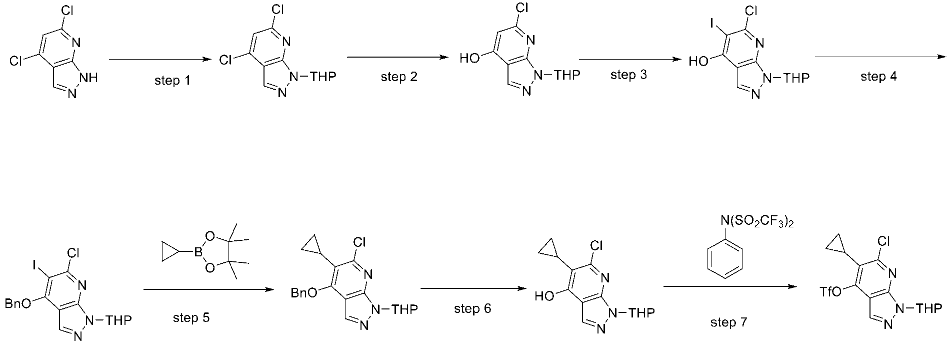

### Step 1: Synthesis of 4, 6-dichloro-1-(tetrahydro-2H-pyran-2-yl)-1H-pyrazolo[3, 4-b]pyridine

A solution of 4,6-dichloro-1H-pyrazolo[3,4-b]pyridine (4 g, 21.28 mmol), dihydropyran (1.88 g, 22.34 mmol) and TsOH (0.73 g, 4.26 mmol) in DCM (50 mL) was stirred at rt for 2h under N₂. The residue was purified by silica gel column chromatography, eluting with PE / EtOAc (5:1) to afford the title compound (4 g, 69.1%) as a white solid. LCMS m/z = 272 [M+H]+

### Step 2: Synthesis of 6-chloro-1-(tetrahydro-2H-pyran-2-yl)-1H-pyrazolo[3,4-b]pyridin-4-ol

To a solution of the title compound from step 1 (5 g, 19.71 mmol) in dioxane (100 mL) and H₂O (50 mL) was added KOH (1.55 g, 27.56 mmol), t-BuBrettPhos (1.78 g, 3.68 mmol) and Pd₂(dba)₃ (1.68 g, 1.84 mmol). The mixture was purged with N₂ (4 x) and the reaction mixture was stirred at 80°C for 1h. The cooled mixture was concentrated and the residue was purified by reverse-phase chromatography using a C18 silica gel column (aq. NH₄HCO₃/MeCN, 0% to 30% gradient in 30 min) to afford the title compound (2.5 g, 53.6%) as a light yellow solid. LCMS m/z = 254 [M+H]⁺

### Step 3: Synthesis of 6-chloro-5-iodo-1-(tetrahydro-2H-pyran-2-yl)-1H-pyrazolo[3, 4-b]pyridin-4-ol

To a solution of the title compound from step 2 (266 mg, 1.05 mmol) in MeCN (20 mL) was added NIS (259.5 mg, 1.15 mmol) and the mixture was stirred at rt for 1h. The mixture was concentrated and the residue was purified by reverse-phase chromatography using a C18 silica gel column (aq. NH₄HCO₃/MeCN, 0% to 30% gradient in 30 min) to afford the title compound (240 mg, 60.3%) as an off-white solid. LCMS m/z = 380 [M+H]⁺

### Step 4: Synthesis of 4-(benzyloxy)-6-chloro-5-iodo-1-(tetrahydro-2H-pyran-2-yl)-1H-pyrazolo[3,4-b]pyridine

To a mixture of the title compound from step 3 (900 mg, 2.37 mmol) in DMF (50 mL) and K₂CO₃ (983.1 mg, 7.11 mmol) was added (bromomethyl)benzene (366.1 µL, 3.08 mmol) and the reaction mixture was stirred at 50°C for 2h. The reaction was diluted with EtOAc, washed with brine (3x), dried and concentrated *in vacuo.* The residue was purified by silica gel column (PE:EtOAc=3:1) to afford the title compound (850 mg, 76.3%) as a white solid. LCMS m/z = 470 [M+H]+

### Step 5: Synthesis of 4-(benzyloxy)-6-chloro-5-cyclopropyl-1-(tetrahydro-2H-pyran-2-yl)-1H-pyrazolo[3,4-b]pyridine

To a mixture of the title compound from step 4 (800 mg, 1.70 mmol) and 2-cyclopropyl-4,4,5,5-tetramethyl-1,3,2-dioxaborolane (0.62 mL, 3.42 mmol) in dioxane (50 mL) and water (10 mL) was added Pd(dppf)Cl₂.DCM (139.1 mg, 0.17 mmol) and K₂CO₃ (706.2 mg, 5.11 mmol). The mixture was purged with N₂ (3x), and the reaction was stirred at 80°C overnight. The cooled mixture was concentrated and the residue was purified by silica gel column (PE:EtOAc=5:1) to afford the title compound (500 mg, 76.5%) as a white solid.

### Step 6: Synthesis of 6-chloro-5-cyclopropyl-1-(tetrahydro-2H-pyran-2-yl)-1H-pyrazolo[3,4-b]pyridin-4-ol

To a mixture of the title compound from step 5 (600 mg, 1.56 mmol) in 2,2,2-trifluoroethan-1-ol (100 mL) was added PtO₂ (400 mg, 1.76 mmol) and the mixture purged with N₂ (3x), then H₂ (3x). The reaction mixture was stirred under H₂ for 4 h, then filtered and the filtrate was concentrated. The residue was purified by reverse-phase chromatography using a C18 silica gel column (0.5% TFA in water/MeCN, 10% to 50% gradient in 30 min) to afford the title compound (260 mg, 56.6%) as a white solid. LCMS m/z = 294 [M+H]⁺

### Step 7: Synthesis of 6-chloro-5-cyclopropyl-1-(tetrahydro-2H-pyran-2-yl)-1H-pyrazolo[3, 4-b]pyridin-4-yl trifluoromethanesulfonate

To a mixture of the title compound from step 6 (100 mg, 0.34 mmol) in DCM (4 mL) was added DIPEA (177.9 µL, 1.02 mmol), DMAP (4.16 mg, 0.034 mmol) and 1,1,1-trifluoro-N-phenyl-N-(trifluoromethane)sulfonylmethanesulfonamide (182.4 mg, 0.51 mmol) at 0°C and the reaction mixture was stirred at rt for 10 min. The mixture was concentrated *in vacuo* and the residue was purified by prep TLC (PE:EtOAc=10:1) to afford the title compound (90 mg, 62.1%) as a colorless oil. LCMS m/z = 426 [M+H]+

### Synthesis of 3-((R)-1-((S)-5-chloro-4-fluoro-9-methyl-2-(methylthio)-8,9-dihydro-10H-7-oxa-1,3,6,10-tetraazacyclohepta|de]naphthalen-10-yl)ethyl)-N,N-bis(4-methoxybenzyl)pyridin-2-amine (Intermediate 21)

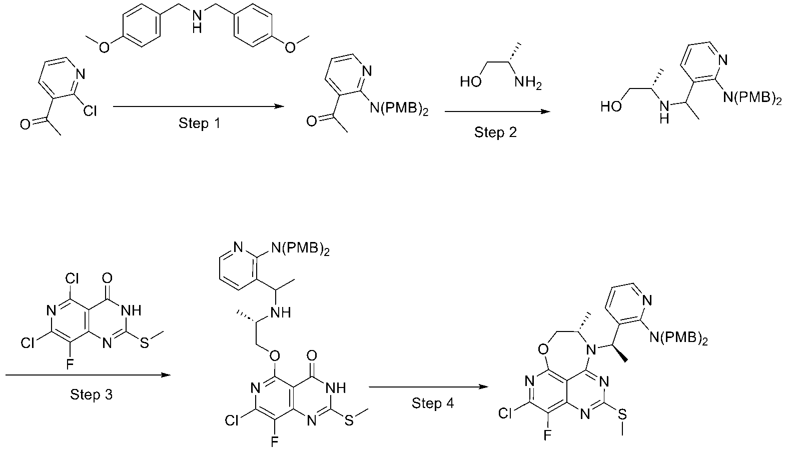

### Step 1: Synthesis of 1-(2-(bis(4-methoxybenzyl)amino)pyridin-3-yl)ethan-1-one

DIPEA (16.8 mL, 96 mmol) and bis(4-methoxybenzyl)amine (12.41 g, 48.2 mmol) were added to a well-stirred solution of 1-(2-chloropyridin-3-yl)ethan-1-one (5 g, 32.1 mmol) in DMSO (50 mL) and the reaction mixture was stirred at 120 °C for 24 h. The mixture was diluted with EtOAc (200 mL) and washed with water (3 x 100 mL). The organic layer was dried over Na₂SO₄, filtered and concentrated under reduced pressure. The crude product was purified by column chromatography on silica gel, using a gradient of 0-100 % EtOAc in hexane, to afford the title compound (8 g, 62.0 %) as a pale-yellow liquid.
LCMS m/z = 377 [M+H]+

### Step 2: Synthesis of (2S)-2-((1-(2-(bis(4-methoxybenzyl)amino)pyridin-3-yl)ethyl)amino)propan-1-ol

(S)-2-Aminopropan-1-ol (4.94 mL, 63.8 mmol), titanium(IV)isopropoxide (9.44 mL, 31.9 mmol) and 4 Å molecular sieves were added to a solution of the compound from step 1 (8 g, 21.25 mmol) in dioxane (80 mL) and the reaction mixture was heated to 100 °C and stirred for 16 h. NaBH₄ (2.41 g, 63.8 mmol) was added to the cooled reaction and the reaction mixture was stirred at rt for 2 h. The reaction was quenched with aq. NaHCO₃ solution (40 mL) and extracted with EtOAc (3 x 50 mL). The combined organic layers were dried over Na₂SO₄, filtered and concentrated under reduced pressure. The crude material was purified by silica gel column chromatography using a gradient of 0-15% MeOH in DCM, to afford the title compound (7.8 g, 82 %) as a pale-yellow liquid. LCMS m/z = 436 [M+H]+

### Step 3: Synthesis of 5-((2S)-2-((1-(2-(bis(4-methoxybenzyl)amino)pyridin-3-yl)ethyl)amino)propoxy)-7-chloro-8-fluoro-2-(methylthio)pyrido[4,3-d]pyrimidin-4(3H)-one

NaHMDS (1M in THF, 21.42 mL, 21.42 mmol) was added dropwise to a stirred solution of 5,7-dichloro-8-fluoro-2-(methylsulfanyl)-3H-pyrido[4,3-d]pyrimidin-4-one (3 g, 10.71 mmol) and the title compound from step 2 (5.13 g, 11.8 mmol) in THF (30 mL) and DMF (6 mL) at 0 °C and the reaction mixture was stirred at rt for 12 h under N₂. The reaction was quenched with ice-cold water (50 mL) and extracted with EtOAc (3 x 100 mL). The combined organic layers were dried over Na₂SO₄, filtered and concentrated under reduced pressure. The crude was purified by silica gel column chromatography, using a gradient of 0-15% MeOH in DCM to afford the title compound (2.4 g, 33%) as a pale yellow solid. LCMS m/z = 679 [M+H]+

### Step 4: Synthesis of 3-(1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

DIPEA (2.30 mL, 13.25 mmol) and BOP-Cl (1.34 g, 5.30 mmol) were added to a well-stirred solution of the title compound from step 3 (3 g, 4.42 mmol) in CHCl₃ (200 mL). A reflux condenser was attached and the reaction mixture was stirred at 80 °C for 16 h. The mixture was concentrated under reduced pressure and the crude product was purified by silica gel column chromatography using a gradient of 0-100 % EtOAc in hexane to afford the title compound (910 mg, 28.6 %) as a pale yellow solid. LCMS m/z = 661 [M+H]+

### Synthesis of 3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine (Intermediate 22)

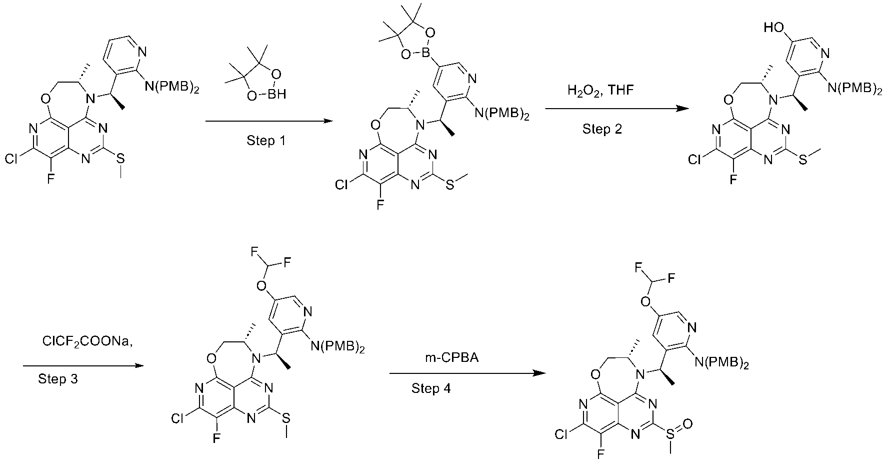

### Step 1: Synthesis of 3-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)-5-(4,4,5,5-tetramethyl-1,3,2-dioxaborolan-2-yl)pyridin-2-amine

A solution of Intermediate 21 (10 g, 15.1 mmol), 4,4,5,5-tetramethyl-1,3,2-dioxaborolane (5.81 g, 45.3 mmol, 6.58 mL), 4,4'-di-tert-butyl-2,2'-dipyridyl (405 mg, 1.51 mmol) and (1,5-cyclooctadiene)(methoxy)iridium(I) dimer (501 mg, 756 µmol) in THF (100 mL) was stirred at 80 °C under N₂ for 16 h. The solution was diluted with water (150 mL) and extracted with EtOAc (200 mL x 2). The combined organic layers were evaporated under reduced pressure to give the title compound (12 g, crude) as a yellow solid. LCMS m/z = 787 [M+H]+

### Step 2: Synthesis of 6-(bis(4-methoxybenzyl)amino)-5-((R)-1-((S)-5-chloro-4-fluoro-9-methyl-2-(methylthio)-8,9-dihydro-10H-7-oxa-1,3,6,10-tetraazacyclohepta[de]naphthalen-10-yl)ethyl)pyridin-3-ol

Solution 1: The title compound from step 1 (12 g, 15.24 mmol) in THF (120 mL).

Solution 2: H₂O₂ (6.66 mL, 76.2 mmol, 33% purity).

Solution 1 was pumped at 5.57 mL/min to a flow reactor (SS, Static mixer, 0.93 mL, 25.0°C), (PFA, Coils reactor, 3.175 mm (1/8"), 21.37 mL, 25.0°C). Solution 2 was pumped at 5.58 mL/min to the flow reactor, with residence time of 2.0 min. On completion, the reaction mixture was quenched by saturated aq. Na₂SO₃ (200 mL) at 0 °C and extracted with EtOAc (200 mL x 2). The combined organic layers were washed with saturated brine (150 mL), dried over anhydrous Na₂SO₄, filtered and concentrated under reduced pressure. The residue was purified by prep-HPLC (column: Welch Ultimate XB-SiOH 250 * 70 mm * 10 µm; mobile phase: [Heptane - EtOH (0.1% NH₃H₂O)]; gradient: 5% - 45% B over 15.0 min) and then prep-HPLC (Method A, Gradient: 55% - 85% B over 15.0 min) to give the title compound (6.7 g, 64.9%) as a yellow solid. LCMS m/z = 677 [M+H]+

### Step 3: Synthesis of 3-((R)-1-((S)-5-chloro-4-fluoro-9-methyl-2-(methylthio)-8,9-dihydro-10H-7-oxa-1,3,6,10-tetraazacycloheptalde[naphthalen-10-yl)ethyl)-5-(difluoromethoxy)-N,N-bis(4-methoxybenzyl)pyridin-2-amine

The title compound was obtained as a yellow solid (0.27 g, 62.8%) from the title compound from step 2 and sodium chlorodifluoroacetate, following a similar procedure to that described in Intermediate 6, step 6. LCMS m/z = 727 [M+H]+

### Step 4: Synthesis of 3-((1R)-1-((9S)-5-chloro-4-fluoro-9-methyl-2-(methylsulfinyl)-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine

To a solution of the title compound from Step 3 (0.27 g, 371 µmol) in DCM (2.7 mL) was added m-CPBA (82.9 mg, 408 µmol, 85% purity) at 0 °C and the reaction mixture was stirred at 20 °C for 0.5 h. The reaction mixture was quenched by addition of saturated aq. Na₂SO₃ solution (20 mL) and extracted with DCM (20 mL x 3). The combined organic layers were washed with saturated brine (10 mL), dried over anhydrous Na₂SO₄, filtered and evaporated under reduced pressure, to give the title compound (0.27 g, crude) as a yellow solid. LCMS m/z = 743 [MH]+

### Synthesis of 3-((R)-1-((S)-5-chloro-2-(((R)-1,2-dimethylpyrrolidin-2-yl)methoxy)-4-fluoro-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine (Intermediate 23)

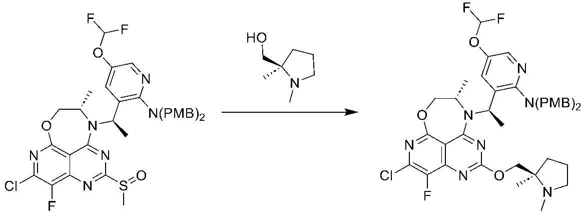

To a solution of (R)-(1,2-dimethylpyrrolidin-2-yl)methanol (93.8 mg, 726 µmol) in THF (2 mL) was added t-BuOLi (2.2 M, THF, 330 µL) at 0 °C and the mixture was stirred at 0 °C for 15 min. A solution of Intermediate 22 (0.27 g, 363 µmol) in THF (3 mL) was added and the reaction mixture stirred at 15 °C for 0.5 h. The solution was quenched with saturated NH₄Cl solution (15 mL) and extracted with EtOAc (20 mL x 2). The combined organic layers were washed with saturated brine (15 mL), dried over Na₂SO₄, filtered and concentrated *in vacuo.* The residue was purified by prep-HPLC (column: Waters Xbridge Prep OBD C18 150 * 40 mm * 10 um; mobile phase: [H₂O (10 mM NH₄HCO₃)-MeCN]; gradient: 63% - 93% B over 14.0 min) to give the title compound (150 mg, 51.0%) as a white solid. LCMS m/z = 808 [M+H]+.

### Synthesis of 3-((R)-1-((S)-5-chloro-4-fluoro-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine (Intermediate 24)

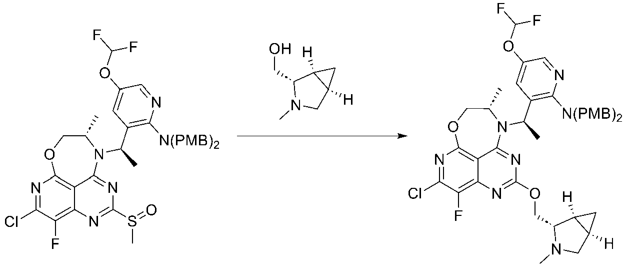

To a solution of Intermediate 19 (256 mg, 2.02 mmol) in THF (5 mL) was added t-BuOLi (2.2 M, THF, 1.22 mL) at 0 °C and the mixture was stirred at 0 °C for 15 min. Intermediate 22 (1 g, 1.35 mmol) in THF (10 mL) was added and the reaction mixture was stirred at 20 °C for 0.5 h. The reaction mixture was quenched with aq. NH₄Cl (30 mL) and extracted with EtOAc (10 mL x 3). The combined organic layers were dried over anhydrous Na₂SO₄, filtered and concentrated under reduced pressure. The residue was purified by reverse-phase HPLC (column: SANTAI, SW-8222-040-SP, Spherical C 18, 20 - 45µm, 100A; mobile phase: water (0.1% FA - MeCN; MeCN%: 1% - 30%, 20 min) and lyophilized to give the title compound (520 mg, 46.0%) as a white solid. LCMS m/z = 806 [M+H]+.

### Synthesis of (6-chloro-5-(1-fluorocyclopropyl)-1-(tetrahydro-2H-pyran-2-yl)-1H-indazol-4-yl)boronic acid (Intermediate 25)

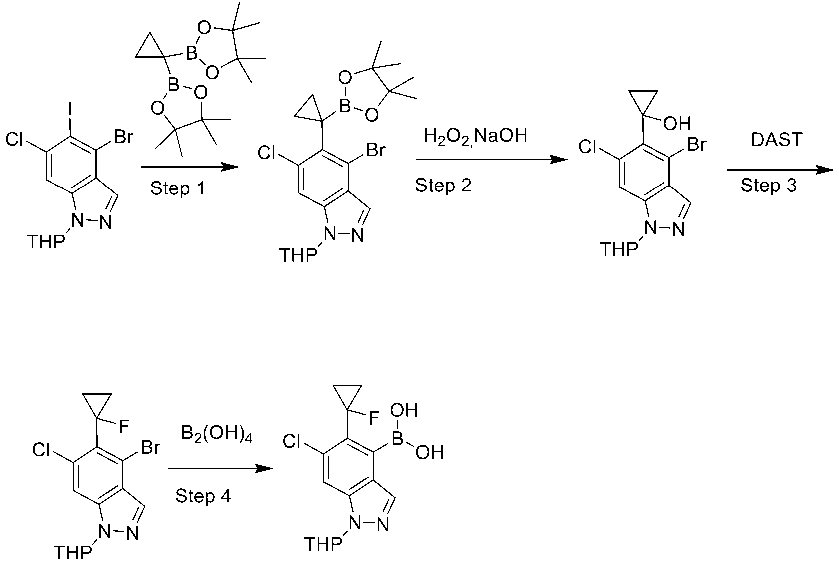

### Step 1: Synthesis of 4-bromo-6-chloro-1-(tetrahydro-2H-pyran-2-yl)-5-(1-(4,4,5,5-tetramethyl-1,3,2-dioxaborolan-2-yl)cyclopropyl)-1H-indazole

To a solution of 4-bromo-6-chloro-5-iodo-1-(tetrahydro-2H-pyran-2-yl)-1H-indazole (2.5 g, 5.66 mmol) and 2,2'-(cyclopropane-1,1-diyl)bis(4,4,5,5-tetramethyl-1,3,2-dioxaborolane) (2.50 g, 8.49 mmol) in dioxane (100 mL) and H₂O (20 mL) was added Pd(dppf)Cl₂ (414.3 mg, 566.3 µmol) and Na₂CO₃ (1.80 g, 17.0 mmol) and the reaction mixture was stirred at 100 °C for 36 h under N₂. The reaction mixture was quenched with water (100 mL) and extracted with EtOAc (150 mL x 3). The combined organic layers were washed with saturated brine (20 mL), dried over anhydrous Na₂SO₄, filtered and concentrated under reduced pressure. The residue was purified by column chromatography (SiO₂, hexanes: EtOAc = 100:0 to 90:10) to give the title compound (3.2 g, 34%) as a colorless oil. LCMS: *m*/*z* = 483 [M+2H]⁺

### Step 2: Synthesis of 1-(4-bromo-6-chloro-1-(tetrahydro-2H-pyran-2-yl)-1H-indazol-5-yl)cyclopropan-1-ol

To a solution of the title compound from step 1 (3.55 g, 7.37 mmol) in THF (40 mL) and MeOH (4 mL) was added NaOH (589.6 mg, 14.74 mmol) at 0 °C and the mixture was stirred at 0 °C for 5 min. H₂O₂ (1.52 g, 14.74 mmol, 33% purity) was added and the reaction mixture was stirred at 20 °C for 1 h. The reaction mixture was quenched by addition of saturated Na₂SO₃ (30 mL) at 0 °C and extracted with EtOAc (30 mL x 3). The combined organic layers were washed with saturated brine (30 mL), dried over anhydrous Na₂SO₄, filtered and concentrated under reduced pressure to give the title compound (3 g, crude) as a yellow oil. LCMS: *m*/*z* = 289 [M+H]⁺

### Step 3: Synthesis of 4-bromo-6-chloro-5-(1-fluorocyclopropyl)-1-(tetrahydro-2H-pyran-2-yl)-1H-indazole

To a solution of the title compound from step 2 (3 g, 7.10 mmol) in DCM (60 mL) was added DAST (3.43 g, 21.31 mmol) at -78 °C and the reaction mixture was stirred at -78 °C for 0.5 h under N₂. The reaction mixture was quenched by addition of saturated NaHCO₃ aq. solution (50 mL) and extracted with DCM (30 mL x 3). The combined organic layers were washed with saturated brine (20 mL), dried over anhydrous Na₂SO₄, filtered and concentrated under reduced pressure. The residue was purified by column chromatography (SiO₂, PE: EtOAc = 100:0 to 80:20) to give the title compound (2.16 g, 74 %) as a yellow oil. LCMS: *m*/*z* = 291 [M+H]⁺

### Step 4: Synthesis of (6-chloro-5-(1-fluorocyclopropyl)-1-(tetrahydro-2H-pyran-2-yl)-1H-indazol-4-yl)boronic acid

To a solution of the title compound from step 3 (2.16 g, 5.78 mmol) and tetrahydroxydiboron (777.4 mg, 8.67 mmol) in MeOH (40 mL) was added TEA (2.34 g, 23.12 mmol) and CataCXium Pd G3 (421.0 mg, 578 µmol) and the reaction mixture was stirred at 25 °C for 4 h under N₂. The reaction mixture was filtered and the filtrate was concentrated under reduced pressure. The residue was purified by prep-HPLC (Method G, Gradient: 1%-50% B over 20 min) to give the title compound (1.5 g, 76%) as a white solid.
LCMS: *m*/*z* = 339 [M+H]⁺

### Synthesis of rac-(6-methyl-5-((1R,2S)-2-methylcyclopropyl)-1-(tetrahydro-2H-pyran-2-yl)-1H-indazol-4-yl)boronic acid (Intermediate 26)

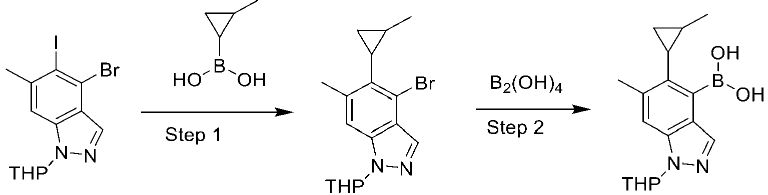

### Step 1: Synthesis of rac-4-bromo-6-methyl-5-((1R,2S)-2-methylcyclopropyl)-1-(tetrahydro-2H-pyran-2-yl)-1H-indazole

To a solution of 4-bromo-5-iodo-6-methyl-1-tetrahydropyran-2-yl-indazole (1 g, 2.37 mmol) and rac-((1R,2S)-2-methylcyclopropyl)boronic acid (284 mg, 2.85 mmol) in dioxane (20 mL) and H₂O (4 mL) was added Pd(dppf)Cl₂ (173 mg, 237 µmol) and KOH (333 mg, 5.94 mmol) under N₂ and the reaction mixture was stirred at 100 °C for 12 h. The reaction mixture was diluted with H₂O (20 mL) and extracted with EtOAc (2 × 20 mL). The combined organic phases were dried over Na₂SO₄, filtered and concentrated *in vacuo.* The residue was purified by prep HPLC (column: SANTAI, SW-8222-080-SP, Spherical C 18, 20 45 µm 100A ; mobile phase A: water (0.1% FA) mobile phase B: MeCN%: 30% - 50%, 20 min) to give the title compound (500 mg, 60%) as a yellow solid. LCMS: *m*/*z* = 265 [M+H]⁺

### Step 2: Synthesis of rac-(6-methyl-5-((1R,2S)-2-methylcyclopropyl)-1-(tetrahydro-2H-pyran-2-yl)-1H-indazol-4-yl)boronic acid

The title compound was obtained as a white solid, 250 mg, 56%, from the title compound from step 1, following a similar procedure to that described in Intermediate 25, step 4. LCMS: *m*/*z* = 315 [M+H]⁺

### Synthesis of ((2R,7aS)-2-fluorotetrahydro-1H-pyrrolizin-7a(5H)-yl-5,5-d2)methan-d2-ol (Intermediate 27)

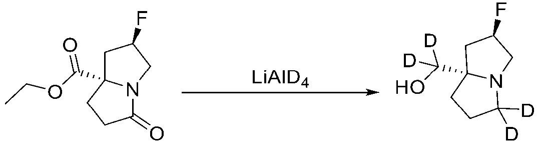

LiAlD₄ (300mg, 6.9 mmol) was added slowly at rt to a solution of ethyl (2R,7aS)-2-fluoro-5-oxotetrahydro-1H-pyrrolizine-7a(5H)-carboxylate (500 mg, 2.3 mmol) in THF and the reaction mixture was stirred at 80°C for 2 h under N₂. The reaction was quenched with D₂O (1 mL), the resulting mixture filtered and the solid washed with THF. The filtrate was evaporated under reduced pressure to give the title compound (250 mg, crude) as colorless oil. LCMS m/z = 164 [M+H]⁺

### Synthesis of 6-(bis(4-methoxybenzyl)amino)-5-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)pyridin-3-ol (Intermediate 28)

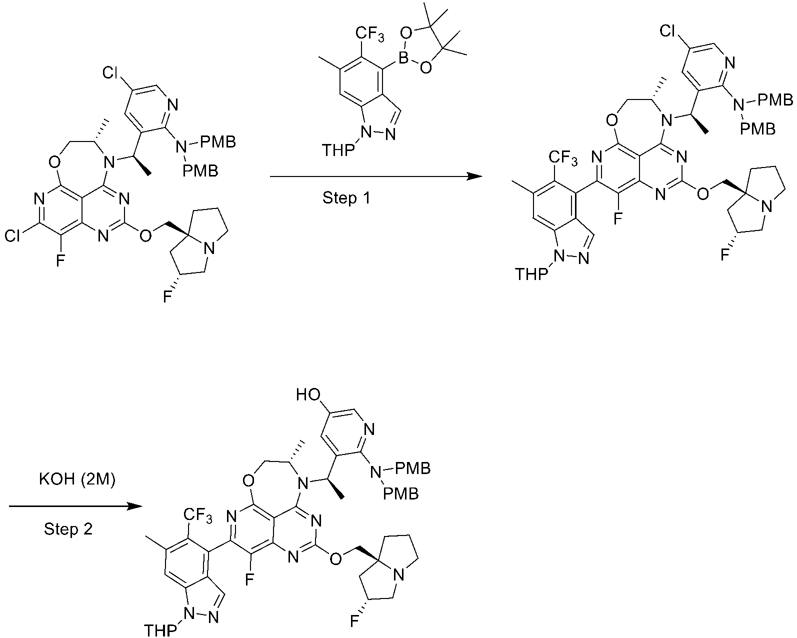

### Step 1: Synthesis of 5-chloro-3-((1R)-1-((9S)-4-fluoro-2-(((2R, 7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was prepared from Intermediate 5 and Intermediate 10, following a similar procedure to that described in Example 15, step 1. The crude product was purified by prep-HPLC (column: Phenomenex luna C18 (250 x 70 mm, 10 um); mobile phase: [H₂O (0.225% FA)-MeCN]; gradient: 45% - 75% B over 15.0 min) and lyophilized to give the title compound (0.75 g, 52.7%) as a yellow solid. LCMS: *m*/*z* = 1054 [M+H]⁺

### Step 2: Synthesis of 6-(bis(4-methoxybenzyl)amino)-5-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)pyridin-3-ol

To a solution of the title compound from step 1 (4.75 g, 4.14 mmol) in dioxane (95 mL) was added KOH (2 M in water, 10.3 mL), Pd₂(dba)₃ (379 mg, 414 µmol) and t-BuBrettphos (200 mg, 414 µmol). The mixture was stirred at 100 °C for 3 h under N₂. The reaction mixture was quenched by addition of water (200 mL) and extracted with EtOAc (200 mL x 3). The combined organic layers were washed with saturated brine (10 mL), dried over anhydrous Na₂SO₄, filtered and concentrated under reduced pressure. The residue was purified by prep-HPLC (column: Phenomenex luna c18 250 mm x 100 mm x 10 um; mobile phase: [H₂O (0.225% FA) - MeCN]; gradient: 30%-50% B over 20.0 min) and lyophilized to give a yellow solid. This was dissolved in DCM (50 mL), the pH was adjusted to 9 using NH₃ (aq) and the mixture was extracted with DCM (30 mL x 3). The combined organic layers were concentrated and lyophilized to give the title compound (4.5 g, 50%) as a yellow solid. LCMS: *m*/*z* = 1037 [M+H]⁺

### Example 1: Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine (Compound 107)

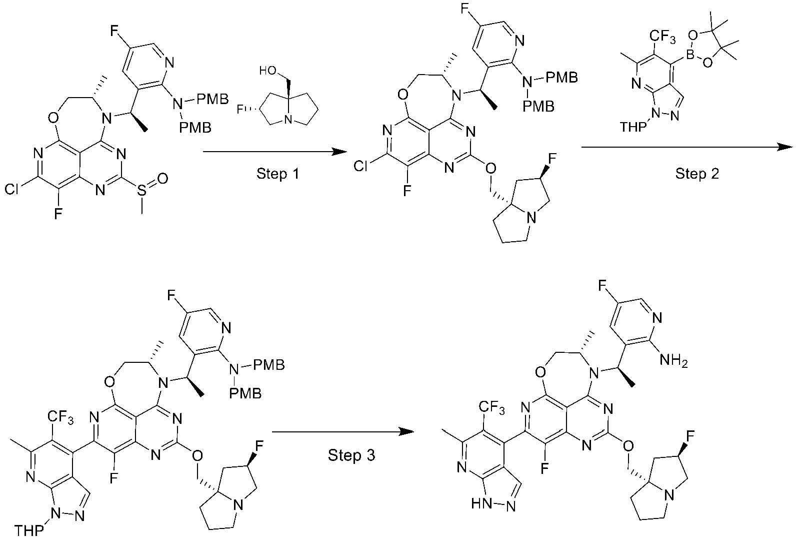

### Step 1: Synthesis of 3-((R)-1-((S)-5-chloro-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine

A mixture of Intermediate 2 (260 mg, 0.374 mmol) and ((2R,7aS)-2-fluoro-hexahydropyrrolizin-7a-yl)methanol (178.6 mg, 1.12 mmol) in toluene (20 mL) was stirred at rt for 1 h, then cooled down to 0°C. NaOtBu (107.8 mg, 1.12 mmol) was added and the reaction was stirred at 0°C for 15 min. The mixture was concentrated *in vacuo* and the residue was purified by silica gel column (DCM:MeOH=30:1) to afford the title compound (160 mg, 54.1%) as white solid. LCMS m/z = 790 [M+H]+

### Step 2: Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of the compound from step 1 and 6-methyl-1-(tetrahydro-2H-pyran-2-yl)-4-(4,4,5,5-tetramethyl-1,3,2-dioxaborolan-2-yl)-5-(trifluoromethyl)-1H-pyrazolo[3,4-b]pyridine in dioxane and H₂O was added CataCXium A Pd G3 and K₃PO₄ under N₂, the mixture purged with N₂ and the reaction was warmed to 80 °C and stirred for 6 h. The mixture was concentrated *in vacuo* and the residue purified by reverse-phase flash chromatography to afford the title compound as white solid.

### Step 3: Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine

To a solution of the compound from step 2 in TFA was added trifluoromethanesulfonic acid, then the mixture was stirred at rt. The reaction mixture was concentrated under reduced pressure to remove solvent. The residue was diluted with H₂O, adjusted to pH=8 by careful addition of saturated NaHCO₃ solution, and extracted with EtOAc. The combined organic layers were washed with brine, dried over MgSO₄, filtered and concentrated under reduced pressure to give a residue which was purified by prep-HPLC to afford the title compound as a solid.

### Example 1a: Alternative Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine (Compound 107)

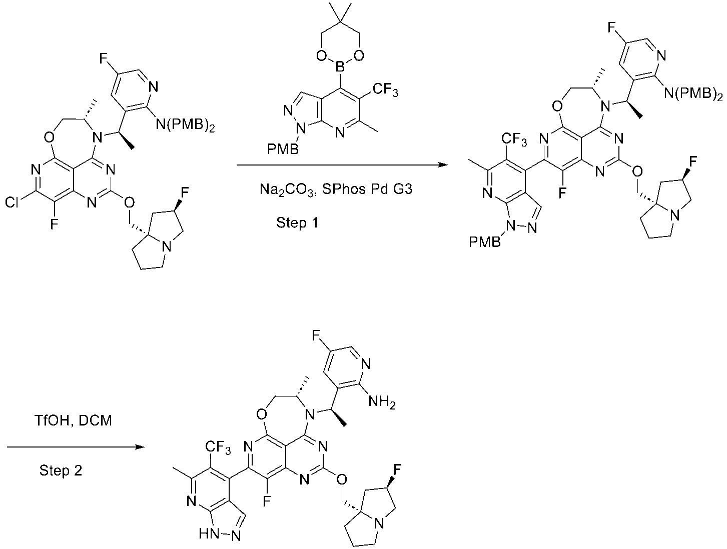

To a solution of Intermediate 3 (45 mg, 0.06 mmol) and Intermediate 11 (49.3 mg, 0.12 mmol) in a mixture of toluene (3 mL) and water (0.8 mL) was added Na₂CO₃ (18.11 mg, 0.17 mmol) and the mixture was purged with N₂ for 5 min. SPhos Pd G3 (22.2 mg, 0.028 mmol) was added and the mixture was degassed with N₂ then stirred at 120 °C for 1 h under microwave irradiation. The reaction mixture was diluted with water (10 mL) and extracted with EtOAc (3 x 15 mL). The combined organic layers were washed with brine (10 mL), dried over anhydrous Na₂SO₄, filtered and concentrated *in vacuo.* The crude was purified by silica gel column chromatography using a gradient of 0-100% EtOAc in hexane followed by 0-20% MeOH in DCM, to afford the title compound (50 mg, 69.4 %) as brown gum. LCMS m/z = 1075 [M+H]⁺

### Step 2: Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine

To a solution of the title compound from step 1 (50 mg, 0.05 mmol) in DCM (3.5 mL) was added triflic acid (69.8 mg, 0.46 mmol) at 0 °C and the reaction mixture was stirred for 1 h at rt. The reaction was diluted with DCM (10 mL), quenched with aq. NaHCO₃ solution (8 mL, pH was adjusted to 8) and stirred at rt for 10 min. The layers were separated, the aqueous layer was extracted with 10% MeOH/DCM (2 x 10 mL) and the combined organic layers were washed with brine (10 mL), dried over Na₂SO₄, filtered and concentrated *in vacuo.* The crude was purified by prep HPLC purification (column: Xtimate C18 (250*21.0 mm),5um, mobile phase A:10 mM NH₄HCO₃ in H₂O, mobile phase B:100% MeCN, gradient 0-100% MeCN, flow rate:18 mL/min). The product containing fractions were lyophilized to afford the title compound (8 mg, 23.1 %) as an off-white solid. LCMS m/z = 716 [M+H]+. ¹HNMR (400 MHz, DMSO-d₆): δ 13.00 (br s, 1H), 8.00 (d, 1H), 7.93 (s, 1H), 7.71-7.68 (m, 1H), 6.55-6.51 (m, 1H), 5.68-5.59 (m, 2H), 5.36-5.22 (m, 1H), 4.56-4.52 (m, 1H), 4.40-4.31 (m, 1H), 4.15-4.10 (m, 3H), 3.14-3.08 (m, 2H), 3.03-3.02 (m, 1H), 2.84-2.80 (m, 4H), 2.17-2.03 (m, 3H), 1.90-1.79 (m, 3H), 1.63-1.61 (d, 3H), 0.64 (dd, 3H)

### Example 2: 5-Chloro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine (Compound 108)

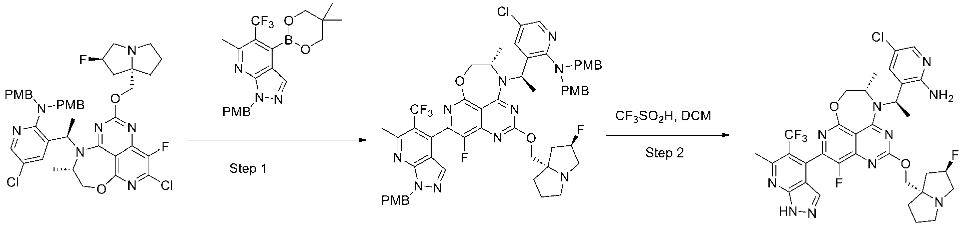

### Step 1: Synthesis of 5-chloro-3-((1R)-1-((9S)-4-fluoro-2-(((2R, 7aS)-2-fluorotetrahydro-1H-pyrrolizin-7a(5H)-yl)methoxy)-5-(1-(4-methoxybenzyl)-6-methyl-5-(trifluoromethyl)-1H-pyrazolo[3,4-b]pyridin-4-yl)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a brown gum, 45 mg, 51.7%, from Intermediate 5 and Intermediate 11, following a similar procedure to that described in Example 1 (Alternative synthesis), step 1. LCMS m/z = 1091 [M+H]⁺

### Step 2: Synthesis of 5-chloro-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine

The title compound was obtained as an off-white solid, 6 mg, 19.4%, from the title compound from step 1, following a similar procedure to that described in Example 1 (Alternative Synthesis), step 2. LCMS m/z = 731 [M+H]^{+ 1}HNMR (400 MHz, DMSO-d₆): δ 14.25-14.10 (m, 1H), 8.03-8.03 (m, 1H), 7.94-7.94 (m, 1H), 7.76 (s, 1H), 6.54-6.48 (m, 1H), 5.91-5.86 (m, 2H), 5.36-5.22 (m, 1H), 4.58-4.52 (m, 1H), 4.40-4.30 (m, 1H), 4.18-4.12 (m, 3H), 3.11-3.08 (m, 2H), 3.05-3.02 (m, 1H), 2.85-2.83 (m, 4H), 2.17-2.16 (m, 1H), 2.15-2.03 (m, 2H), 1.87-1.79 (m, 3H), 1.62 (d, 3H), 0.64 (dd, 3H),

### Example 3: Synthesis of 5-chloro-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 114)

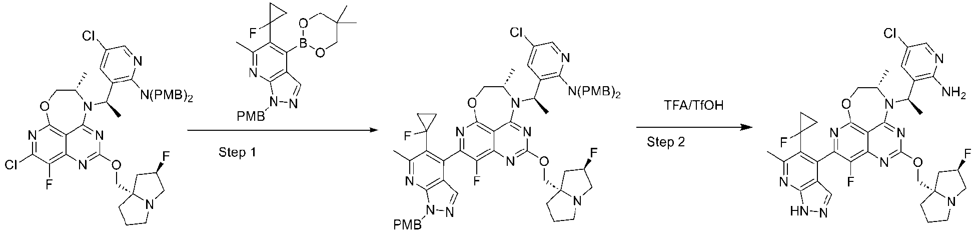

### Step 1: Synthesis of 5-chloro-3-((1R)-1-((9S)-4-fluoro-5-(5-(1-fluorocyclopropyl)-1-(4-methoxybenzyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 5 (130 mg, 161 µmol) and Intermediate 16, 88.6 mg, 209 µmol) in dioxane (2.5 mL) and H₂O (0.25 mL) was added Na₂CO₃ (51.2 mg, 483 µmol) and cataCXium^{®} A Pd G₃ (23.4 mg, 32.2 µmol) and the reaction mixture was stirred at 80 °C for 2 h under N₂. The reaction was quenched by addition of water (10 mL) and extracted with EtOAc (10 mL x 3). The combined organic layers were washed with saturated brine (10 mL), dried over anhydrous Na₂SO₄, filtered and concentrated *in vacuo.* The residue was purified by prep-HPLC (Method A, gradient:42%-62% B over 10.0 min) and lyophilized to give the title compound (75 mg, 37.0%) as a white solid. LCMS m/z = 1082 [M+H]+

### Step 2: Synthesis of 5-chloro-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine formate

A solution of the title compound from step 1 (75 mg, 59.6 µmol) in TFA (1 mL) and TfOH (0.1 mL) was stirred at 20 °C for 1 h. The reaction was diluted with DCM (10 mL) and then adjusted to pH 8-9 with saturated NaHCO₃ solution at 0 °C. The mixture was extracted with DCM (10 mLx2) and the combined organic layers were concentrated *in vacuo.* The residue was purified by prep-HPLC (Method A, gradient: 15%-45% B over 11.0 min) and lyophilized to give the title compound (14.0 mg, 31.8%) as a white solid.

LCMS: m/z = 721 [M+H]+ ¹HNMR (400 MHz, DMSO-d₆) δ = 13.88 - 13.62 (m, 1H), 8.14 (s, 1H), 8.02 (d, 1H), 7.85 (s, 1H), 7.76 (d, 1H), 6.52 (d, 1H), 5.92 (s, 2H), 5.47 - 5.18 (m, 1H), 4.65 - 4.49 (m, 1H), 4.35 (d, 1H), 4.20 - 4.10 (m, 3H), 3.18 - 3.02 (m, 3H), 2.87 (s, 4H), 2.21 - 2.00 (m, 3H), 1.90 - 1.76 (m, 3H), 1.63 (d, 3H), 1.34 - 1.07 (m, 2H), 0.77 - 0.56 (m, 5H).

### Example 4: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine (Compound 101)

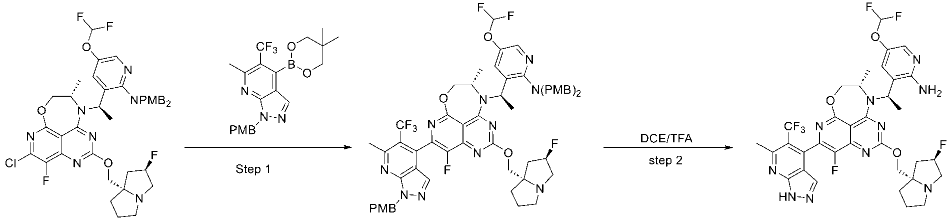

### Step 1: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-5-(1-(4-methoxybenzyl)-6-methyl-5-(trifluoromethyl)-1H-pyrazolo[3,4-b]pyridin-4-yl)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

A mixture of Intermediate 6 (18 mg, 0.021 mmol), Intermediate 11 (13.95 mg, 0.032 mmol), cataCXium^{®} A Pd G3 (3.13 mg, 0.004 mmol) and K₃PO₄ (13.67 mg, 0.063 mmol) in dioxane (1 mL) and H₂O (0.2 mL) was stirred for 40 min at 80°C under N₂. The reaction was diluted with water (10 mL) at rt. The resulting mixture was extracted with EtOAc (3 x 5mL), the combined organic layers were washed with brine (20 mL), dried over anhydrous Na₂SO₄, filtered and the filtrate was concentrated *in vacuo.* The residue was purified by Prep-TLC (DCM / MeOH 15:1) to afford the title compound (7 mg, 29.0%) as a yellow solid. LCMS m/z = 1123 [M+H]+

### Step 2: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine formate

A solution of the title compound from step 1 (7 mg, 0.006 mmol) in DCE (1 mL) and TFA (1 mL) was stirred at 80°C for 1 h under N₂. The reaction was diluted with water (10 mL) at rt and the resulting mixture was extracted with DCM (3 x 5mL). The combined organic layers were washed with brine (20 mL), dried over anhydrous Na₂SO₄, filtered and the filtrate was concentrated *in vacuo.* The crude product was purified by Prep-HPLC (Column: Xselect CSH C18 OBD Column 30*150mm 5µm; Mobile Phase A: Water (0.1% FA), Mobile Phase B: MeCN; Flow rate: 60 mL/min; Gradient: 18% B to 45% B in 7 min) to afford the title compound (1.2 mg, 22.0%) as a white solid. LCMS m/z = 763 [M+H]+ ¹HNMR (400 MHz, MeOD) δ: 7.96 (d, 1H), 7.87 - 7.72 (m, 2H), 6.84 - 6.71 (m, 2H), 5.59 (d, 1H), 4.74 (s, 2H), 4.68 (d, 1H), 4.39 (dd, 1H), 4.23 - 4.13 (m, 1H), 3.99 - 3.79 (m, 2H), 3.48 - 3.47 (m, 1H), 2.89 (dd, 2H), 2.77 (dd, 1H), 2.72 - 2.58 (m, 2H), 2.45 (d, 1H), 2.41 - 2.28 (m, 3H), 2.18 (s, 1H), 1.72 (d, 3H), 0.80 (dd, 3H).

### Example 5: Synthesis of 3-((1R)-1-((9S)-5-(6-chloro-5-cyclopropyl-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)pyridin-2-amine (Compound 102)

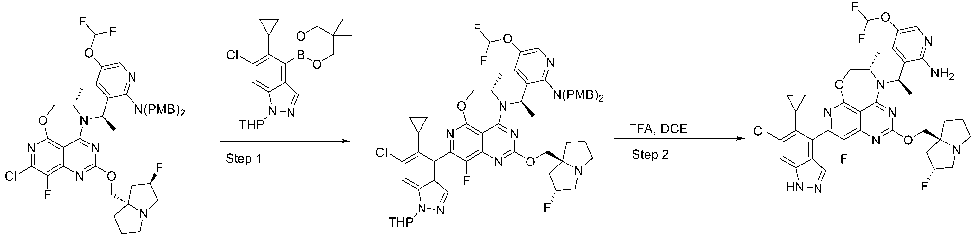

### Step 1: Synthesis of 3-((1R)-1-((9S)-5-(6-chloro-5-cyclopropyl-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine

The title compound was obtained as an off-white solid, 20 mg, 34.5%, from Intermediate 6 and Intermediate 8, following a similar procedure to that described in Example 4, step 1. LCMS m/z = 1078 [M+H]+

### Step 2: Synthesis of 3-((1R)-1-((9S)-5-(6-chloro-5-cyclopropyl-1H-indazol-4-yl)-4-fluoro-2-(((2R, 7aS)-2-fluorotetrahydro- 1H-pyrrolizin-7a(5H)-yl)methoxy)-9-methyl-8, 9-dihydro-10H-7-oxa-1,3,6,10-tetraazacyclohepta[de]naphthalen-10-yl)ethyl)-5-(difluoromethoxy)pyridin-2-amine

To a solution of the title compound from step 1 (20 mg, 0.019 mmol) in DCE (1 mL) was added TFA (1 mL) and the reaction mixture was stirred at 60°C for 4 h. The mixture was concentrated *in vacuo* and the residue was purified by prep-HPLC (Method B, Gradient: 44% B to 69% B in 7 min) to give the title compound (5.4 mg, 38.6%) as white solid. LCMS m/z = 754 [M+H]+ ¹HNMR (400 MHz, DMSO-d₆) δ 13.10 - 12.98 (m, 1H), 7.75 - 7.69 (m, 1H), 7.60 - 7.69 (m, 2H), 7.45 - 7.40 (m, 1H), 7.11 - 6.70 (m, 1H), 6.38 - 6.31 (m, 1H), 6.65 - 6.56 (m, 2H), 5.19 - 4.99 (m, 1H), 4.41 - 4.30 (m, 1H), 4.19 - 4.13 (m, 1H), 4.00 - 3.87 (m, 3H), 2.93 - 2.78 (m, 3H), 2.68 - 2.58 (m, 1H), 2.00 - 1.94 (m, 1H), 1.84 - 1.54 (m, 6H), 1.47 - 1.39 (m, 3H), 0.72 - 0.54 (m, 2H), 0.47 - 0.37 (m, 3H), 0.08 - - 0.14 (m, 2H)

### Example 6: Synthesis of 5-(difluoromethoxy)-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 113)

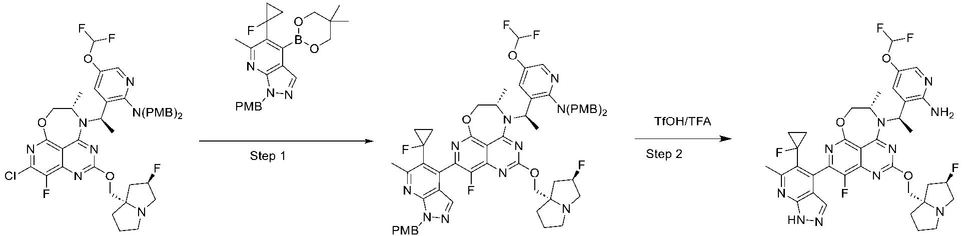

### Step 1: Synthesis of 5-(difluoromethoxy)-3-((1R)-1-((9S)-4-fluoro-5-(5-(1-fluorocyclopropyl)-1-(4-methoxybenzyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 220 mg, 66.1%, from Intermediate 6 and Intermediate 16 following a similar procedure to that described in Example 3, step 1. LCMS: m/z = 1114 [M+H]+

### Step 2: Synthesis of 5-(difluoromethoxy)-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine formate

The title compound was obtained as a white solid, 81.5 mg, 68.3%, from the title compound from step 1, following a similar procedure to that described in Example 3, step 2. LCMS: m/z = 753 [M+H]+ ¹HNMR (400 MHz, DMSO-d₆) δ = 13.89 - 13.66 (m, 1H), 8.16 (s, 1H), 7.93 (d, 1H), 7.85 (d, 1H), 7.63 (d, 1H), 7.33 - 6.90 (m, 1H), 6.53 (s, 1H), 5.80 (s, 2H), 5.44 - 5.19 (m, 1H), 4.63 - 4.51 (m, 1H), 4.36 (d, 1H), 4.22 - 4.07 (m, 3H), 3.15 - 3.00 (m, 3H), 2.92 - 2.79 (m, 4H), 2.21 - 2.00 (m, 3H), 1.91 - 1.75 (m, 3H), 1.63 (d, 3H), 1.37 - 1.04 (m, 2H), 0.79 - 0.56 (m, 5H).

### Example 7: Synthesis of 5-fluoro-3-((1R)-1-((9S)-4-fluoro-5-(6-fluoro-5-(1-fluorocyclopropyl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 121)

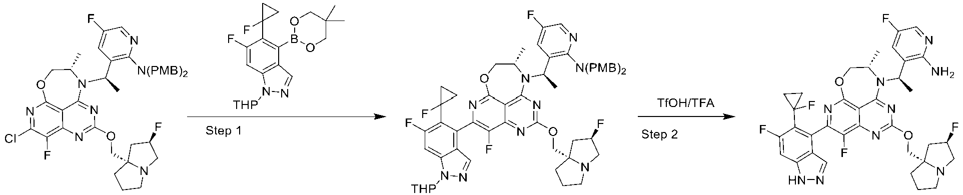

### Step 1: Synthesis of 5-fluoro-3-((1R)-1-((9S)-4-fluoro-5-(6-fluoro-5-(1-fluorocyclopropyl)-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid, 140 mg, 51%, from Intermediate 3 and Intermediate 13, following a similar procedure to that described in Example 3, step 1. LCMS: m/z = 1033 [M+H]+

### Step 2: Synthesis of 5-fluoro-3-((1R)-1-((9S)-4-fluoro-5-(6-fluoro-5-(1-fluorocyclopropyl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine

The title compound was obtained as a white solid, 33.6 mg, 35%, from the title compound from step 1, following a similar procedure to that described in Example 3, step 2. LCMS: m/z = 708 [M+H]^{+ 1}HNMR (400 MHz, DMSO-d₆) δ 13.55 - 13.33 (m, 1H), 8.14 (s, 1H), 8.01 (d, 1H), 7.83 (d, 1H), 7.71 (dd, 1H), 7.59 (d, 1H), 6.53 (s, 1H), 5.66 (s, 2H), 5.47 - 5.21 (m, 1H), 4.56 (dd, 1H), 4.36 (d, 1H), 4.24 (d, 2H), 4.17 - 4.09 (m, 1H), 3.28 - 3.02 (m, 3H), 2.99 - 2.83 (m, 1H), 2.28 - 2.04 (m, 3H), 1.88 (dd, 3H), 1.63 (d, 3H), 1.27 - 1.00 (m, 2H), 0.78 - 0.55 (m, 5H).

### Example 8: Synthesis of 3-((1R)-1-((9S)-5-(5-(difluoromethoxy)-6-methyl-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoropyridin-2-amine (Compound 103).

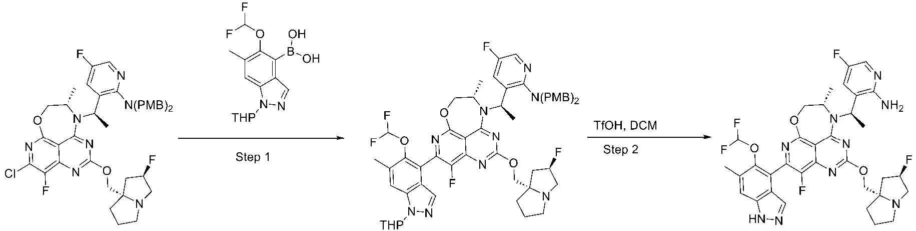

### Step 1: Synthesis of 3-((1R)-1-((9S)-5-(5-(difluoromethoxy)-6-methyl-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine

To a solution of Intermediate 3 (150 mg, 0.190 mmol) and Intermediate 17 (136 mg, 0.42 mmol) in dioxane (8 mL) and water (2 mL) at rt was added K₃PO₄ (121 mg, 0.57 mmol) and the mixture degassed for 5 min with N₂. CataCXium^{®} A Pd G3 (10.37 mg, 0.02 mmol) was added at rt, the reaction mixture degassed for 2 min, then stirred at 110 °C for 1h under microwave irradiation. The reaction mixture was quenched with water (10 mL), extracted with EtOAc (3 x 30 mL), the combined organic layer was dried over anhydrous Na₂SO₄, filtered, and concentrated *in vacuo.* The crude compound was purified by prep-HPLC [column: Redisep Rf Gold ^{®} reversed-phase C18-teledyne), [10 mm aq. NH₄HCO₃ : MeCN). The product containing fractions were lyophilized to afford the title compound (90 mg, 45.5 %) as an off white solid. LCMS m/z = 1037 [M+H]+

### Step 2: Synthesis of 3-((1R)-1-((9S)-5-(5-(difluoromethoxy)-6-methyl-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoropyridin-2-amine

The title compound was obtained as an off-white solid, 19 mg, 30.4%, from the title compound from step 1, following a similar procedure to that described in Example 1 (Alternative synthesis), step 2. LCMS m/z = 712 [M+H]+ ¹HNMR (400 MHz, DMSO-d₆): δ 13.25 (s, 1H), 8.00 (s, 1H), 7.82 (s, 1H), 7.70 (dd, 1H), 7.62 (s, 1H), 6.99-6.62 (m, 1H), 6.56-6.51 (m, 1H), 5.63 (s, 2H), 5.37-5.23 (m, 1H), 4.58-4.53 (m, 1H), 4.38-4.35 (m, 1H), 4.16-4.10 (m, 3H), 3.16-3.09 (m, 2H), 3.06-3.03 (m, 2H), 2.45 (s, 3H), 2.18-2.15 (m, 1H), 2.08-2.04 (m, 2H), 1.87-1.80 (m, 3H), 1.63 (d, 3H), 0.61 (d, 3H).

### Example 9: Synthesis of 5-fluoro-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 105)

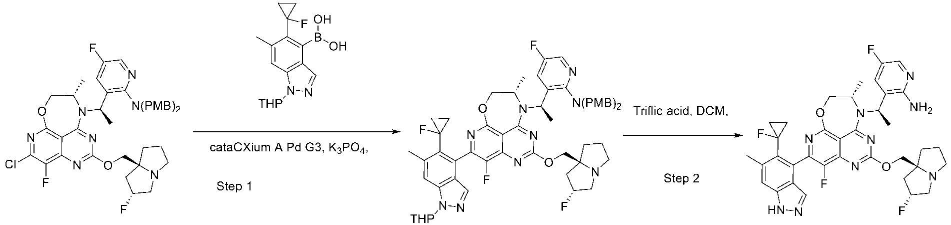

### Step 1: Synthesis of 5-fluoro-3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

A mixture of Intermediate 3 (120 mg, 0.15 mmol), Intermediate 12 (97 mg, 0.31 mmol) and K₃PO₄ (97 mg, 0.46 mmol) in a mixture of dioxane (8 mL) and water (2 mL) was degassed with N₂ for 5 min. CataCXium^{®} A Pd G3 (11.06 mg, 0.02 mmol) was added and the reaction mixture was stirred at 100 °C for 1h under microwave irradiation. The reaction mixture was diluted with water (20 mL) and extracted with EtOAc (3 x 20 mL). The combined organic layer was washed with brine (30 mL), dried over anhydrous Na₂SO₄, filtered and concentrated *in vacuo.* The crude was purified by column chromatography on silica gel using a gradient of 0-100% acetone in hexane to afford the title compound (130 mg, 45.9 %) as pale brown solid. LCMS m/z = 1029 [M+H]⁺

### Step 2: Synthesis of 5-fluoro-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine

The title compound was obtained as a white solid, 10.5 mg, 14.9%, from the title compound from step 1, following a similar procedure to that described in Example 1, step 2 (Alternative synthesis). LCMS m/z = 705 [M+H]+ ¹HNMR (400 MHz, DMSO-d₆): δ 13.24-13.17 (m, 1H), 8.01 (d, 1H), 7.72-7.66 (m, 2H), 7.56 (d, 1H), 6.56-6.53 (m, 1H), 5.66 (s, 2H), 5.35-5.30 (m, 1H), 4.57-4.53 (m, 1H), 4.36 (d, 1H), 4.18-4.11 (m, 3H), 3.12-3.03 (m, 3H), 2.84-2.83 (m, 1H), 2.69 (d, 3H), 2.18-2.04 (m, 3H), 1.85-1.75 (m, 3H), 1.63 (d, 3H), 1.47-1.44 (m, 1H), 1.15-1.01 (m, 1H), 0.67-0.62 (m, 5H).

### Example 10: Synthesis of 5-fluoro-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 111)

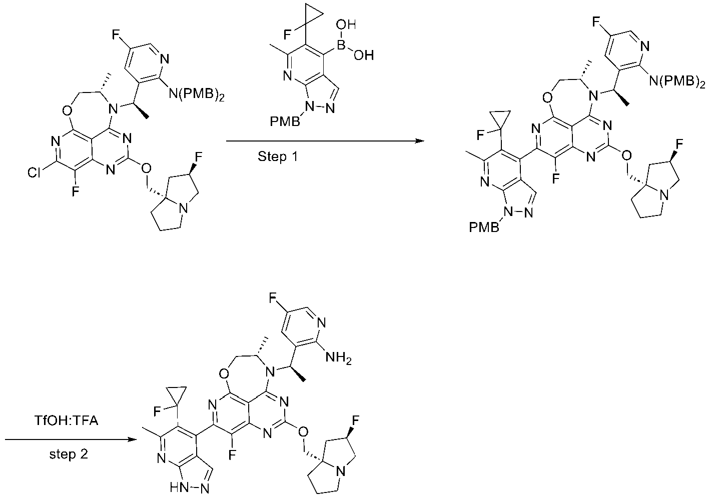

### Step 1: Synthesis of 5-fluoro-3-((1R)-1-((9S)-4-fluoro-5-(5-(1-fluorocyclopropyl)-1-(4-methoxybenzyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 40 mg, 37.1%, from Intermediate 3 and Intermediate 15, following a similar procedure to that described in Example 4, step 1. LCMS m/z = 1066 [M+H]+

### Step 2: Synthesis of 5-fluoro-3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine

A solution of the title compound from step 1 (40 mg, 0.038 mmol) in TFA (4 mL) and TfOH (0.4 mL) was stirred at rt for 1 h under N₂. The reaction was diluted with sat. NaHCO₃ (aq.) (10 mL) at 0°C and the mixture was extracted with DCM (3 x 20 mL). The combined organic layers were washed with brine (2 x 10 mL), dried over anhydrous Na₂SO₄, filtered and the filtrate was concentrated *in vacuo.* The crude product was purified by Prep-HPLC (Method B, Gradient: 31% B to 51% B in 7 min) to afford the title compound (9.0 mg, 34.0%) as a white solid. LCMS m/z = 705 [M+H]+ ¹HNMR (400 MHz, DMSO-d₆) δ 13.78 (d, 1H), 8.01 (d, 1H), 7.85 - 7.83 (m, 1H), 7.71 - 7.69 (m, 1H), 6.53 (s, 1H), 5.66 (s, 2H), 5.30 (d, 1H), 4.57 - 4.54(m, 1H), 4.38 - 4.35 (m, 1H), 4.14 (s, 3H), 3.15 - 3.10 (m, 2H), 3.02 (s, 1H), 2.86 - 2.81 (m, 4H), 2.15 - 2.11 (m, 1H), 2.08 (s, 1H), 2.04 - 2.01 (m, 1H), 1.86 - 1.78 (m, 3H), 1.63 (d, 3H), 1.31 - 1.15 (m, 2H), 0.63 - 0.53 (m, 5H).

### Example 11: Synthesis of 5-fluoro-3-((1R)-1-((9S)-4-fluoro-5-(6-fluoro-5-(spiro[2.2]pentan-1-yl)-1H-indazol-4-yl)-2-(((2R,7aS)-2-fluorotetrahydro-1H-pyrrolizin-7a(5H)-yl)methoxy)-9-methyl-8,9-dihydro-10H-cyclohepta[de]naphthalen-10-yl)ethyl)pyridin-2-amine (Compound 104)

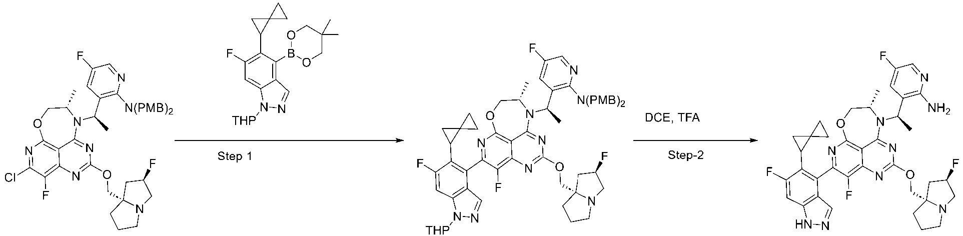

### Step 1: Synthesis of 5-fluoro-3-((1R)-1-((9S)-4-fluoro-5-(6-fluoro-5-(spiro[2.2]pentan-1-yl)-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-cyclohepta[de]naphthalen-10-yl)ethyl)-N,N-bis(4-methoxybenzyl)pyridin-2-amine

The title compound was obtained as a yellow solid, 30 mg, 45.6%, from Intermediate 3 and Intermediate 14 following a similar procedure to that described in Example 4, step 1. LCMS m/z = 1041 [M+H]+

### Step 2: Synthesis of 5-fluoro-3-((1R)-1-((9S)-4-fluoro-5-(6-fluoro-5-(spiro[2.2]pentan-1-yl)-1H-indazol-4-yl)-2-(((2R,7aS)-2-fluorotetrahydro-1H-pyrrolizin-7a(5H)-yl)methoxy)-9-methyl-8, 9-dihydro-10H-cyclohepta[de]naphthalen-10-yl)ethyl)pyridin-2-amine formate

The title compound was obtained as a white solid, 3.8 mg, 27.6%, from the title compound from step 1, following a similar procedure to that described in Example 4, step 2. LCMS m/z = 716 [M+H]+ ¹HNMR (400 MHz, DMSO-d₆) δ 13.21 (d, 1H), 8.00 (d, 1H), 7.85 - 7.66 (m, 2H), 7.43 (d, 1H), 6.53 (d, 1H), 5.65 (d, 2H), 5.31 (d, 1H), 4.55 (d, 1H), 4.36 (d, 1H), 4.23 - 4.08 (m, 3H), 3.14 (s, 3H), 2.86 (s, 1H), 2.18 (s, 2H), 2.09 (d, 2H), 1.82 (s, 3H), 1.63 (d, 3H),1.24 (s, 5H), 0.82 (dd, 1H), 0.61 (s, 2H), 0.56 - 0.45 (m, 1H).

### Example 12: Synthesis of 5-fluoro-3-((R)-1-((5S,9S)-4-fluoro-5-(6-fluoro-5-((S)-spiro[2.2]pentan-1-yl)-1H-indazol-4-yl)-2-(((2R,7aS)-2-fluorotetrahydro-1H-pyrrolizin-7a(5H)-yl)methoxy)-9-methyl-8,9-dihydro-10H-cyclohepta[de]naphthalen-10-yl)ethyl)pyridin-2-amine (Compound 122)

### And Example 13: 5-fluoro-3-((R)-1-((5S,9S)-4-fluoro-5-(6-fluoro-5-((R)-spiro[2.2]pentan-1-yl)-1H-indazol-4-yl)-2-(((2R,7aS)-2-fluorotetrahydro-1H-pyrrolizin-7a(5H)-yl)methoxy)-9-methyl-8,9-dihydro-10H-cyclohepta[de]naphthalen-10-yl)ethyl)pyridin-2-amine(Compound 123)

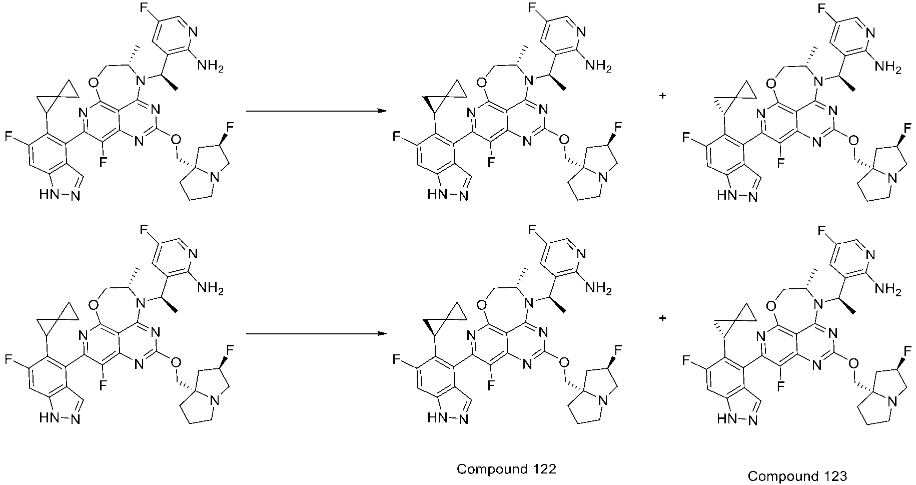

Example 11 (20 mg 0.028 mmol) was purified by chiral HPLC (Column: CHIRALPAK IK 3*25 cm, 5 µm; Mobile Phase A: Hex (0.2% isopropylamine), Mobile Phase B: EtOH: DCM=1:1 at 40 mL/min; Gradient: 20% B isocratic over 36 min) to give Peak 1 (25.78 min), Example 12, 4.6 mg, 23% as a white solid and Peak 2 (30.25 min), Example 13, 3.4 mg, 17% as a white solid.
**Example 12:** LCMS m/z = 716 [M+H]+. ¹HNMR (400 MHz, DMSO-d₆) δ 13.29 - 13.21 (m, 1H), 8.01 - 7.98 (m, 1H), 7.83 - 7.67 (m, 2H), 7.46 - 7.39 (m, 1H), 6.55 - 6.48 (m, 1H), 5.71 - 5.62 (m, 2H), 5.38 - 5.20 (m, 1H), 4.59 - 4.53 (m, 1H), 4.39 - 4.33 (m, 1H), 4.18 - 4.10 (m, 3H), 3.13 - 2.78 (m, 4H), 2.37 - 2.12 (m, 1H), 2.19 - 1.99 (m, 3H), 1.88 - 1.74 (m, 3H), 1.66 - 1.58 (m, 3H), 1.34 - 1.28 (m, 1H), 1.18 - 1.13 (m, 2H), 0.77 - 0.44 (m, 6H) **Example 13:** LCMS m/z = 716 [M+H]+. ¹HNMR (400 MHz, DMSO-d₆) δ 13.27 - 13.17 (m, 1H), 8.02 - 7.97 (m, 1H), 7.83 - 7.67 (m, 2H), 7.48 - 7.39 (m, 1H), 6.55 - 6.48 (m, 1H), 5.68 - 5.60 (m, 2H), 5.38 - 5.20 (m, 1H), 4.61 - 4.50 (m, 1H), 4.39 - 4.33 (m, 1H), 4.19 - 4.07 (m, 3H), 3.16 - 2.78 (m, 4H), 2.45 - 2.24 (m, 1H), 2.19 - 1.99 (m, 3H), 1.88 - 1.74 (m, 3H), 1.66 - 1.58 (m, 3H), 1.34 - 1.28 (m, 1H), 1.20 - 1.13 (m, 2H), 0.86 - 0.49 (m, 6H)

### Example 14: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine (Compound 106)

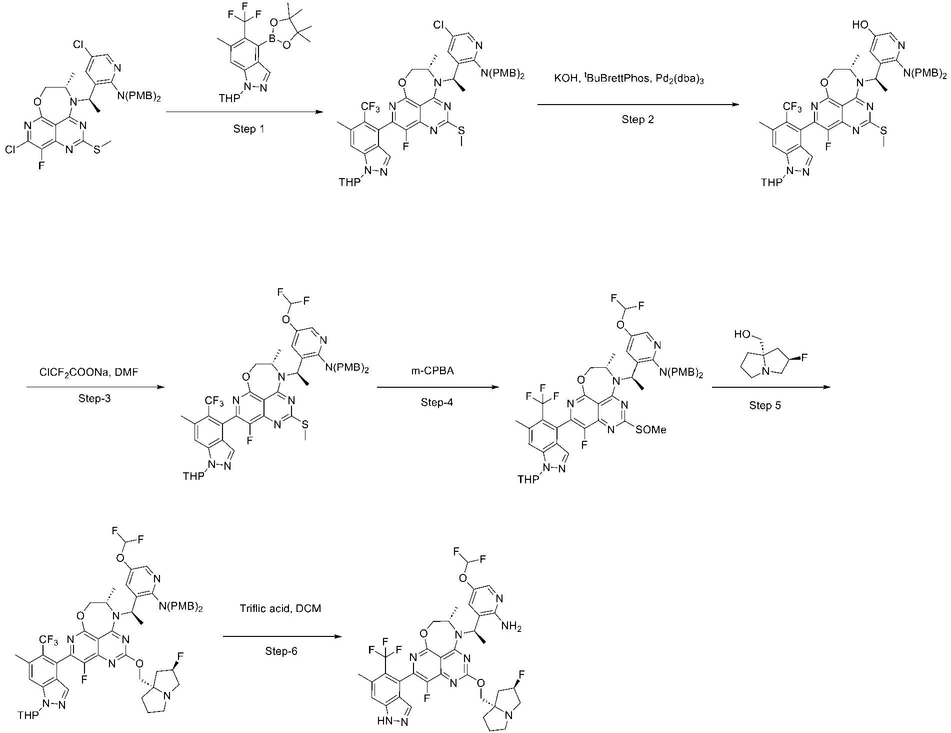

### Step 1: Synthesis of 5-chloro-3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

Two batches of product were prepared.

To a solution of Intermediate 4 (200 mg, 0.29 mmol) and Intermediate 10 (142 mg, 0.35 mmol) in dioxane (8 mL) and water (2 mL) was added K₃PO₄ (183 mg, 0.86 mmol) and the reaction mixture was degassed for 5 min with N₂. CataCXium^{®} A Pd G3 (10.47 mg, 0.014 mmol) was added and the reaction mixture was stirred at 110 °C under microwave irradiation for 2 h. The cooled reaction was diluted with water (10 mL) and the mixture extracted with EtOAc (2 x 20 mL). The combined organic layer was dried over Na₂SO₄, filtered and concentrated *in vacuo.* The crude was purified by column chromatography on silica gel using a gradient of 0-100% EtOAc in hexane, to obtain the title compound (192 mg, 60.9 %) as a light yellow solid. LCMS m/z = 944 [M+H]+

### Step 2: Synthesis of 6-(bis(4-methoxybenzyl)amino)-5-((1R)-1-((9S)-4-fluoro-9-methyl-5-(6-methyl-1-(tetrahydro-2H-pyran-2-yl)-5-(trifluoromethyl)-1H-indazol-4-yl)-2-(methylthio)-8,9-dihydro-10H-7-oxa-1,3,6,10-tetraazacyclohepta[de]naphthalen-10-yl)ethyl)pyridin-3-ol

A stirred solution of the title compound from step 1 (380 mg, 0.40 mmol) and KOH (158 mg, 2.82 mmol) in dioxane (10 mL) and water (2 mL) was degassed for 5 min with N₂, then t-BuBrettPhos (39.0 mg, 0.08 mmol) followed by Pd₂(dba)₃ (36.9 mg, 0.04 mmol) were added at rt. The reaction mixture was heated at 100 °C for 10 h. The reaction was diluted with water (10 mL) and extracted with EtOAc (2 x 20 mL). The combined organic layer was dried over Na₂SO₄, filtered and concentrated *in vacuo.* The crude was purified by column chromatography on silica gel using a gradient of 0-100% EtOAc in hexane, to obtain the title compound (236 mg, 53.4 %) as an off-white solid. LCMS m/z = 926 [M+H]+

### Step 3: Synthesis of 5-(difluoromethoxy)-3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

Cs₂CO₃ (497 mg, 1.52 mmol) and sodium chlorodifluoroacetate (194 mg, 1.27 mmol) were added to a solution of the title compound from step 2 (235 mg, 0.25 mmol) in DMF (5 ml) and the reaction mixture was heated to 100 °C and stirred for 16 h. The reaction mixture was diluted with water (10 mL) and extracted with EtOAc (2 x 15 mL). The combined organic layer was dried over Na₂SO₄, filtered, and concentrated *in vacuo.* The crude product was purified by column chromatography on silica gel using a gradient of 0-100% EtOAc in hexane, to obtain the title compound (106 mg, 40.4 %) as an off-white solid.
LCMS m/z = 976 [M+H]+

### Step 4: Synthesis of 5-(difluoromethoxy)-3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylsulfinyl)-8,9-dihydro-10H-7-oxa-1,3,6,10-tetraazacyclohepta[de]naphthalen-10-yl)ethyl)-N,N-bis(4-methoxybenzyl)pyridin-2-amine

To a stirred solution of the title compound from step 3 (80 mg, 0.08 mmol) in DCM (2 ml) was added m-CPBA (23.60 mg, 0.1 mmol) at 0 °C and the reaction mixture was stirred at rt for 1 h. The reaction mixture was quenched with aq. sodium thiosulfate (10 mL) and extracted with DCM (2x 25 mL). The combined organic layer was washed with aq. NaHCO₃ solution (20 mL), dried over Na₂SO₄, filtered and evaporated under reduced pressure to obtain the title compound (82 mg, crude) as a pale yellow solid. LCMS m/z = 992 [M+H]+

### Step 5: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of the title compound from step 4 (160 mg, 0.16 mmol) and ((2R,7aS)-2-fluorotetrahydro-1H-pyrrolizin-7a(5H)-yl)methanol (77 mg, 0.48 mmol) in toluene (5 ml) was added NaOtBu (23.27 mg, 0.24 mmol) at 0 °C and the reaction mixture was stirred at rt for 1 h. The reaction was diluted with EtOAc (30 mL) and washed with water (2 x 10 mL). The organic layer was dried over Na₂SO₄, filtered, and concentrated *in vacuo.* The crude was purified by reverse phase C-18 chromatography (column: RediSep-gold, C1-8, mobile phase A: 10mm aq. NH₄HCO₃, mobile phase B: MeCN, using gradient 0 to 100 % MeCN, flow rate:12 mL/min) and the pure fractions were lyophilized to obtain the title compound (82 mg, 41.7 %) as an off-white solid. LCMS m/z = 1087 [M+H]+

### Step 6: Synthesis of 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine

Triflic acid (58.0 mg, 0.38 mmol) was added to a solution of the title compound from step 5 (70 mg, 0.064 mmol) in DCM (2 ml) at 0 °C and the reaction mixture was stirred at rt for 1 h. The reaction was diluted with DCM (20 mL), quenched with aq. NaHCO₃ solution (pH adjusted to 8 - 9) and extracted with 10% MeOH in DCM (2x 20 mL). The combined organic layer was dried over Na₂SO₄, filtered, and concentrated *in vacuo.* The crude was purified by prep-HPLC reverse phase purification (column: Xtimate C18(19*250mm) 5um, mobile phase A: 10mm aq. NH₄HCO₃, mobile phase B: MeCN, using gradient 0 to 100 % MeCN, flow rate:16 mL/min) and the pure fraction was lyophilized to obtain the title compound (16.9 mg, 33.2 %) as an off-white solid. LCMS m/z = 763 [M+H]+. ¹HNMR (400 MHz, DMSO-d₆): δ 13.5-13.4 (m, 1H), 7.94 (d, 1H), 7.75 - 7.65 (m, 2H), 7.64 - 7.58 (m, 1H), 7.11 (t, 1H), 6.58 - 6.43 (m, 1H), 5.95 - 5.71 (m, 2H), 5.40 - 5.20 (m, 1H), 4.62 - 4.48 (m, 1H), 4.40 - 4.29 (m, 1H), 4.19 - 4.05 (m, 3H), 3.16 - 3.01 (m, 3H), 2.86 - 2.78 (m, 1H), 2.69 - 2.59 (m, 3H), 2.21 - 1.98 (m, 3H), 1.93 - 1.71 (m, 3H), 1.63 (d, 3H), 0.63 (dd, 3H) ppm.

### Example 15: Synthesis of 3-((1R)-1-((9S)-5-(5-cyclopropyl-6-fluoro-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)pyridin-2-amine (Compound 124)

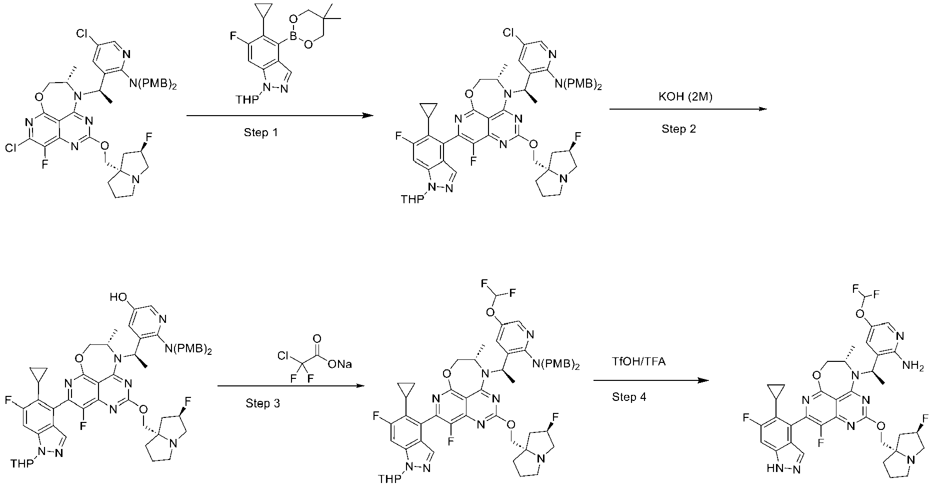

### Step 1: Synthesis of 5-chloro-3-((1R)-1-((9S)-5-(5-cyclopropyl-6-fluoro-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 5 (500 mg, 619 µmol) and Intermediate 9 (461 mg, 1.24 mmol) in dioxane (10 mL) and H₂O (1 mL) was added cataCXium^{®} A Pd G3 (45.1 mg, 61.9 µmol) and Cs₂CO₃ (605 mg, 1.86 mmol) and the reaction mixture was stirred at 80 °C for 0.5 h under N₂. The reaction mixture was quenched with water (30 mL) and extracted with EtOAc (20 mL x 3). The combined organic layers were washed with saturated brine (10 mL), dried over anhydrous Na₂SO₄, filtered and concentrated *in vacuo.* The crude product was purified by reverse-phase HPLC (0.1% FA condition) and lyophilized to give the title compound (550 mg, 41.7%) as a white solid. LCMS: m/z = 1030 [M+H]+

### Step 2: Synthesis of 6-(bis(4-methoxybenzyl)amino)-5-((1R)-1-((9S)-5-(5-cyclopropyl-6-fluoro-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

To a solution of the title compound from step 1 (500 mg, 485 µmol) in dioxane (10 mL) was added KOH (2 M, 1.21 mL), Pd₂(dba)₃ (44.4 mg, 48.5 µmol) and tBuBrettPhos (23.5 mg, 48.5 µmol) and the reaction mixture was stirred at 100 °C for 4 h under N₂. The reaction mixture was quenched with water (30 mL) and extracted with EtOAc (10 mL x 3). The combined organic layers were washed with saturated brine (10 mL), dried over anhydrous Na₂SO₄, filtered and concentrated *in vacuo.* The residue was purified by prep-HPLC (Method A, gradient: 30%-60% B over 15.0 min) and lyophilized to give the title compound (320 mg, 65.1%) as a yellow solid. ¹HNMR (400 MHz, DMSO-d₆) δ = 10.02 - 9.58 (m, 1H), 8.01 (s, 1H), 7.69 (d, 1H), 7.35 (d, 1H), 6.79 - 6.65 (m, 7H), 6.58 (d, 1H), 5.84 (d, 1H), 5.33 - 5.05 (m, 1H), 4.60 - 4.42 (m, 1H), 4.34 - 4.21 (m, 2H), 4.17 - 3.59 (m, 16H), 3.12 - 2.99 (m, 3H), 2.86 - 2.75 (m, 1H), 2.42 - 2.29 (m, 1H), 2.15 - 1.89 (m, 5H), 1.84 - 1.66 (m, 5H), 1.64 - 1.50 (m, 2H), 1.22 (d, 3H), 0.87 - 0.60 (m, 1H), 0.48 - 0.27 (m, 3H), 0.19 - 0.09 (m, 2H)

### Step 3: Synthesis of 3-((1R)-1-((9S)-5-(5-cyclopropyl-6-fluoro-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine

To a solution of the title compound from step 2 (300 mg, 296 µmol) and sodium chlorodifluoroacetate (67.7 mg, 444 µmol) in DMF (3 mL) was added Cs₂CO₃ (289 mg, 889 µmol) and the reaction mixture was stirred at 80 °C for 1 h. The reaction mixture was filtered and the filtrate concentrated *in vacuo.* The residue was purified by prep-HPLC (Method A, gradient: 40%-70% B over 15.0 min) and lyophilized to give the title compound (130 mg, 33.8%) as a yellow oil. LCMS: m/z = 1063 [M+H]+

### Step 4: Synthesis of 3-((1R)-1-((9S)-5-(5-cyclopropyl-6-fluoro-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)pyridin-2-amineformate

The title compound was obtained as a white solid, 33.5 mg, 33.3%, from the title compound from step 3, following a similar procedure to that described in Example 3, step 2. LCMS: m/z = 738 [M+H]+ ¹HNMR (400 MHz, DMSO-d₆) δ = 13.31 - 13.12 (m, 1H), 11.13 - 10.86 (m, 1H), 7.94 (d, 1H), 7.81 - 7.68 (m, 1H), 7.65 (d, 1H), 7.43 (d, 1H), 7.34 - 6.90 (m, 1H), 6.55 (d, 1H), 5.82 (d, 2H), 5.66 - 5.41 (m, 1H), 4.86 - 4.41 (m, 3H), 4.40 - 4.31 (m, 1H), 4.17 (s, 1H), 4.03 - 3.46 (m, 3H), 3.30 - 3.15 (m, 1H), 2.49 - 1.78 (m, 7H), 1.63 (d, 3H), 0.83 - 0.55 (m, 5H), 0.43 - 0.09 (m, 2H).

### Example 16: Synthesis of 5-(difluoromethoxy)-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 115)

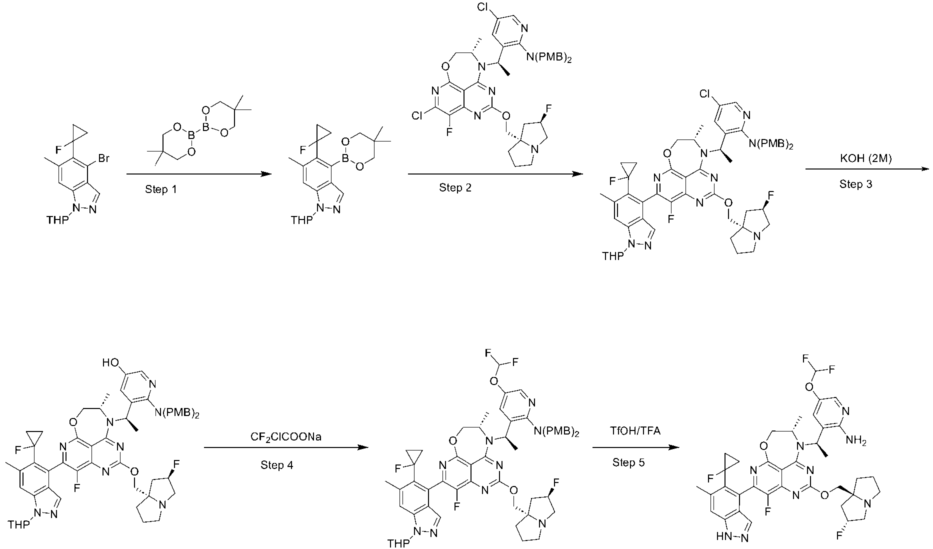

### Step 1: Synthesis of 4-(5,5-dimethyl-1,3,2-dioxaborinan-2-yl)-5-(1-fluorocyclopropyl)-6-methyl-1-(tetrahydro-2H-pyran-2-yl)-1H-indazole

The title compound was obtained as a yellow solid, 3.0 g, 94%, from Intermediate 12, step 4 and 2-(5,5-dimethyl-1,3,2-dioxaborinan-2-yl)-5,5-dimethyl-1,3,2-dioxaborinane, following a similar procedure to that described in Intermediate 10. ¹HNMR (400 MHz, CDCl₃) δ = 8.11 (s, 1H), 7.45 (s, 1H), 5.79 - 5.54 (m, 1H), 4.08 - 3.95 (m, 1H), 3.91 (s, 4H), 3.83 - 3.73 (m, 1H), 2.71 (s, 3H), 2.62 - 2.49 (m, 1H), 2.21 - 1.99 (m, 2H), 1.84 - 1.66 (m, 3H), 1.53 - 1.41 (m, 2H), 1.17 (s, 6H), 0.99 - 0.96 (m, 2H)

### Step 2: Synthesis of 5-chloro-3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

A mixture of the title compound from step 1 (1.05 g, 2.73 mmol), Intermediate 5 (2.2 g, 2.73 mmol), cataCXium^{®} A Pd G3 (198 mg, 272 µmol) and Cs₂CO₃ (2.67 g, 8.18 mmol) in dioxane (44 mL) and H₂O (4.4 mL) was degassed and purged with N₂ (3x), and the reaction mixture was stirred at 80 °C for 2 h under N₂. The mixture was filtered and concentrated *in vacuo.* The crude product was purified by prep- HPLC (column: Phenomenex luna C18 150*40mm* 15um; mobile phase: [H₂O (0.225% FA)-MeCN]; gradient:38%-68% B over 15.0 min) to give the title compound (900 mg, 31%) as a yellow solid.

### Step 3: Synthesis of 6-(bis(4-methoxybenzyl)amino)-5-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

The title compound was obtained as a yellow solid, 800 mg, 56%, from the title compound from step 2, following a similar procedure to that described in Example 15, step 3. LCMS: m/z = 1027 [M+H]+

### Step 4: Synthesis of 5-(difluoromethoxy)-3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid, 400 mg, 55%, from the title compound from step 3 and sodium chlorodifluoroacetate, following a similar procedure to that described in Example 15, step 4.

### Step 5: Synthesis of 5-(difluoromethoxy)-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine

A solution of the title compound from step 4 (400 mg, 371 µmol) in TfOH (0.6 mL) and TFA (6 mL) was stirred at 25°C for 1 h. The mixture was concentrated *in vacuo,* NH₃.H₂O was added at 0 °C and the mixture concentrated *in vacuo.* The residue was purified by prep-HPLC (Method A, gradient:19%-49% B over 10.0 min) to give the title compound (104 mg, 37%) as an off-white solid LCMS: m/z = 752 [M+H]+ ¹HNMR (400 MHz, DMSO-d₆) δ = 13.29 - 12.96 (m, 1H), 8.15 (s, 1H), 7.94 (d, 1H), 7.77 - 7.61 (m, 2H), 7.56 (d, 1H), 7.37 - 6.83 (m, 1H), 6.66 - 6.40 (m, 1H), 5.83 (s, 2H), 5.44 - 5.16 (m, 1H), 4.63 - 4.48 (m, 1H), 4.36 (d, 1H), 4.25 - 4.06 (m, 3H), 3.17 - 3.02 (m, 3H), 2.92 - 2.80 (m, 1H), 2.69 (d, 3H), 2.25 - 1.98 (m, 3H), 1.94 - 1.73 (m, 3H), 1.64 (d, 3H), 1.30 - 0.83 (m, 2H), 0.79 - 0.39 (m, 5H)

### Example 16b: Alternative synthesis of 5-(difluoromethoxy)-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 115)

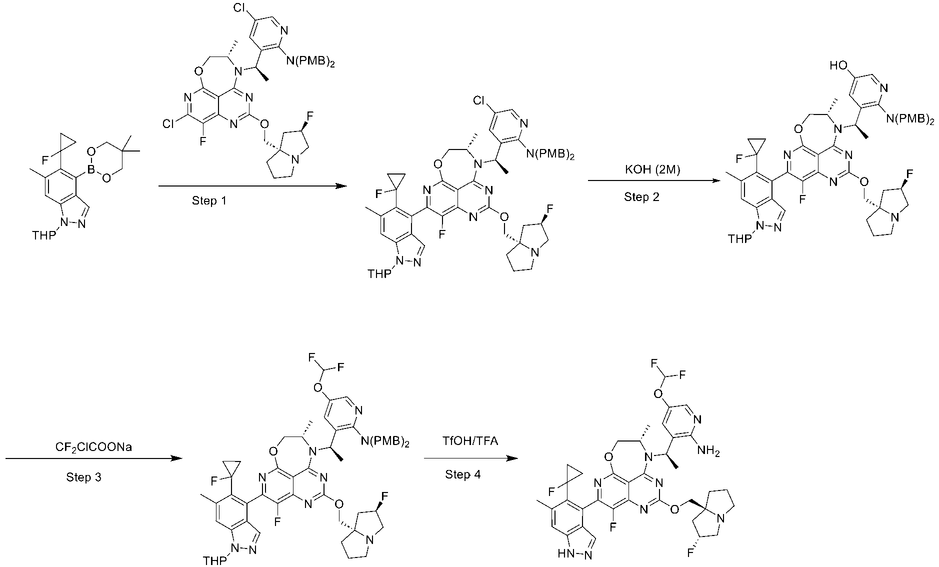

### Step 1: Synthesis of 5-chloro-3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

A mixture of Intermediate 18 (1.05 g, 2.73 mmol), Intermediate 5 (2.2 g, 2.73 mmol), cataCXium^{®} A Pd G3 (198 mg, 272 µmol) and Cs₂CO₃ (2.67 g, 8.18 mmol) in dioxane (44 mL) and H₂O (4.4 mL) was degassed and purged with N₂ (3x). The reaction mixture was stirred at 80 °C for 2 h under N₂. The mixture was filtered and concentrated *in vacuo.* The crude product was purified by prep-HPLC (column: Phenomenex luna C18 150*40mm* 15µm; mobile phase: [H₂O (0.225% FA)-MeCN]; gradient:38%-68% B over 15.0 min) to give the title compound (900 mg, 31%) as a yellow solid.

### Step 2: Synthesis of 6-(bis(4-methoxybenzyl)amino)-5-((1R)-1-((9S)-4-fluoro-5-(5-(1-fluorocyclopropyl)-6-methyl-1-(tetrahydro-2Hpyran-2yl)-1Hindazol-4yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

The title compound was obtained as a yellow solid, 800 mg, 56%, from the title compound from step 2, following a similar procedure to that described in Example 15, step 2. LCMS: m/z = 1027 [M+H]+

### Step 3: Synthesis of 5-(difluoromethoxy)-3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid, 400 mg, 55%, from the title compound from step 3 and sodium chlorodifluoroacetate, following a similar procedure to that described in Example 15, step 3.

### Step 4: Synthesis of 5-(difluoromethoxy)-3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine

A solution of the title compound from step 3 (400 mg, 371 µmol) in TfOH (0.6 mL) and TFA (6 mL) was stirred at 25°C for 1 h. The mixture was concentrated *in vacuo.* NH₃•H₂O was added at 0 °C and the mixture was concentrated *in vacuo.* The residue was purified by prep-HPLC (Method A, gradient: 19%-49% B over 10.0 min) to give the title compound (104 mg, 37%) as an off-white solid LCMS: m/z = 752 [M+H]+ 1H NMR (400 MHz, DMSO-d₆) δ = 13.29 - 12.96 (m, 1H), 8.15 (s, 1H), 7.94 (d, 1H), 7.77 - 7.61 (m, 2H), 7.56 (d, 1H), 7.37 - 6.83 (m, 1H), 6.66 - 6.40 (m, 1H), 5.83 (s, 2H), 5.44 - 5.16 (m, 1H), 4.63 - 4.48 (m, 1H), 4.36 (d, 1H), 4.25 - 4.06 (m, 3H), 3.17 - 3.02 (m, 3H), 2.92 - 2.80 (m, 1H), 2.69 (d, 3H), 2.25 - 1.98 (m, 3H), 1.94 - 1.73 (m, 3H), 1.64 (d, 3H), 1.30 - 0.83 (m, 2H), 0.79 - 0.39 (m, 5H)

### Example 17: Synthesis of 5-(difluoromethoxy)-3-((1R)-1-((9S)-4-fluoro-5-(6-fluoro-5-(1-fluorocyclopropyl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine (Compound 134)

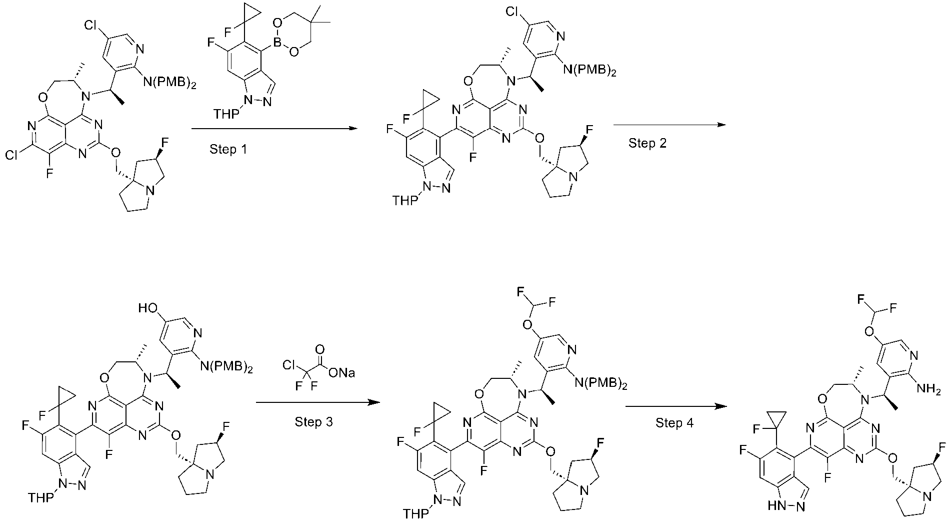

### Step 1: Synthesis of 5-chloro-3-((1R)-1-((9S)-4-fluoro-5-(6-fluoro-5-(1-fluorocyclopropyl)-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid (380 mg, 38%) from Intermediate 5 and Intermediate 13, following a similar procedure to that described in Example 3, step 1. LCMS m/z = 1049 [M+H]+

### Step 2: Synthesis of 6-(bis(4-methoxybenzyl)amino)-5-((1R)-1-((9S)-4-fluoro-5-(6-fluoro-5-(1-fluorocyclopropyl)-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-3-ol

The title compound was obtained as a yellow solid, 200 mg, 36%, from the title compound from step 1, following a similar procedure to that described in Example 15, step 2. LCMS m/z = 1031 [M+H]+

### Step 3: Synthesis of 5-(difluoromethoxy)-3-((1R)-1-((9S)-4-fluoro-5-(6-fluoro-5-(1-fluorocyclopropyl)-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid, 60 mg, 27%, from the title compound from step 2 and sodium chlorodifluoroacetate, following a similar procedure to that described in Example 15, step 3. LCMS m/z = 1081 [M+H]+

### Step 4: Synthesis of 5-(difluoromethoxy)-3-((1R)-1-((9S)-4-fluoro-5-(6-fluoro-5-(1-fluorocyclopropyl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)pyridin-2-amine formate

The title compound was obtained as an off-white solid, 22 mg, 62%, from the title compound from step 3, following a similar procedure to that described in Example 3, step 2. LCMS m/z = 756 [M+H]+

1H NMR (400 MHz, DMSO-d₆) δ 13.54 - 13.33 (m, 1H), 8.13 (s, 1H), 7.94 (d, 1H), 7.83 (d, 1H), 7.64 (d, 1H), 7.59 (d, 1H), 7.32 - 6.90 (m, 1H), 6.64 - 6.47 (m, 1H), 5.82 (s, 2H), 5.53 - 5.21 (m, 1H), 4.65 - 4.51 (m, 1H), 4.43 - 4.08 (m, 4H), 3.29 - 3.08 (m, 3H), 3.04 - 2.83 (m, 1H), 2.35 - 2.08 (m, 3H), 2.01 - 1.77 (m, 3H), 1.63 (d, 3H), 1.27 - 1.00 (m, 2H), 0.72 - 0.51 (m, 5H).

### Example 18: Synthesis of 3-((1R)-1-((9S)-5-(6-chloro-5-cyclopropyl-1H-pyrazolo[3,4-b]pyridin-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoropyridin-2-amine (Compound 116)

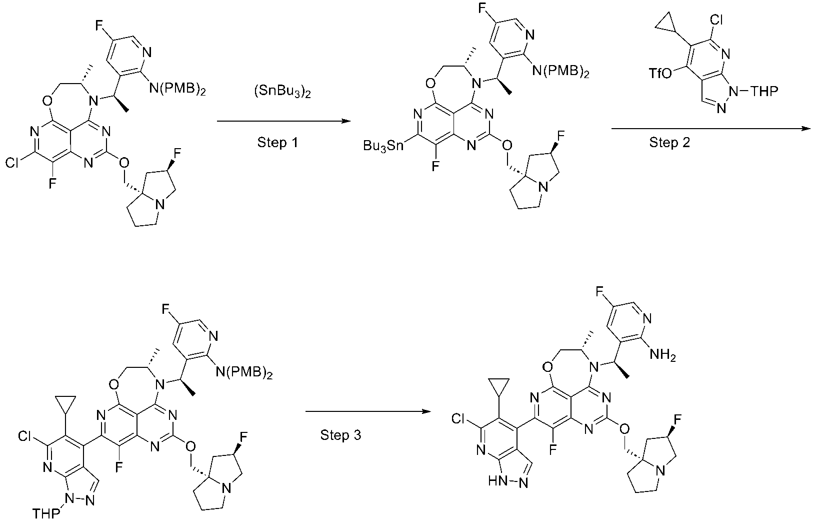

### Step 1: Synthesis of 5-fluoro-3-((R)-1-((S)-4-fluoro-2-(((2R,7aS)-2-fluorotetrahydro-1H-pyrrolizin-7a(5H)-yl)methoxy)-9-methyl-5-(tributylstannyl)-8,9-dihydro-10H-7-oxa-1,3,6,10-tetraazacyclohepta[de]naphthalen-10-yl)ethyl)-N,N-bis(4-methoxybenzyl)pyridin-2-amine

A mixture of Intermediate 3 (200 mg, 0.25 mmol), hexabutyldistannane (440.4 mg, 0.76 mmol), Pd₂(dba)₃•CHCl₃ (785.9 mg, 0.76 mmol), tricyclohexylphosphane (14.2 mg, 0.05 mmol) and LiCl (53.64 mg, 1.27 mmol) in dioxane (10 mL) was purged with N₂ (5x) and the reaction mixture was stirred at 110°C for 60 h. The mixture was concentrated and the residue purified by silica gel column (PE:EtOAc=60%) to afford the title compound (150 mg, 56.7%) as colorless oil. LCMS m/z = 1046 [M+H]+

### Step 2: Synthesis of 3-((1R)-1-((9S)-5-(6-chloro-5-cyclopropyl-1-(tetrahydro-2H-pyran-2-yl)-1H-pyrazolo[3,4-b]pyridin-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoro-N,N-bis(4-methoxybenzyl)pyridin-2-amine

To a mixture of Intermediate 20 (50 mg, 0.117 mmol) and the title compound from step 1 (245.4 mg, 0.234 mmol) in dioxane (1 mL), was added Pd(PPh₃)₄ (13.57 mg, 0.012 mmol), LiCl (24.89 mg, 0.585 mmol) and CuI (111.8 mg, 0.585 mmol) and the mixture was purged with N₂ (3 x). The reaction mixture was stirred at 80°C. The mixture was concentrated and the residue was purified by reverse-phase chromatography using a C18 silica gel column (mobile phase, 0.5% TFA in water/MeCN, 10% to 100% gradient in 40 min), to afford the title compound (50 mg, 41.3%) as yellow solid. LCMS m/z = 1031 [M+H]+

### Step 3: Synthesis of 3-((1R)-1-((9S)-5-(6-chloro-5-cyclopropyl-1H-pyrazolo[3,4-b]pyridin-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-fluoropyridin-2-amine

To a solution of the title compound from step 2 (35 mg, 0.034 mmol) in 1,2-DCE (3 mL) was added TFA (3 mL) and the solution was stirred at 80°C for 2h. The reaction was concentrated and purified by prep-HPLC (Method B, Gradient: 40% B to 50% B in 7 min) to afford the title compound as a white solid (4.1 mg, 17.1%). LCMS m/z = 707 [M+H]+ 1H NMR (400 MHz, DMSO-d₆) δ 13.84 (brs, 1H), 8.02 - 7.85 (m, 2H), 7.73 - 7.65 (m, 1H), 6.57 - 6.46 (m, 1H), 5.96 - 5.54 (m, 2H), 5.38 - 5.20 (m, 1H), 4.61 - 4.32 (m, 2H), 4.20 - 4.05 (m, 3H), 3.15 - 2.98 (m, 3H), 2.88 - 2.76 (m, 1H), 2.21 - 1.94 (m, 4H), 1.88 - 1.73 (m, 3H), 1.67 - 1.56 (m, 3H), 0.98 - 0.55 (m, 5H), 0.34 - 0.04 (m, 2H).

### Example 19: Synthesis of 3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-(trifluoromethoxy)pyridin-2-amine (Compound 135)

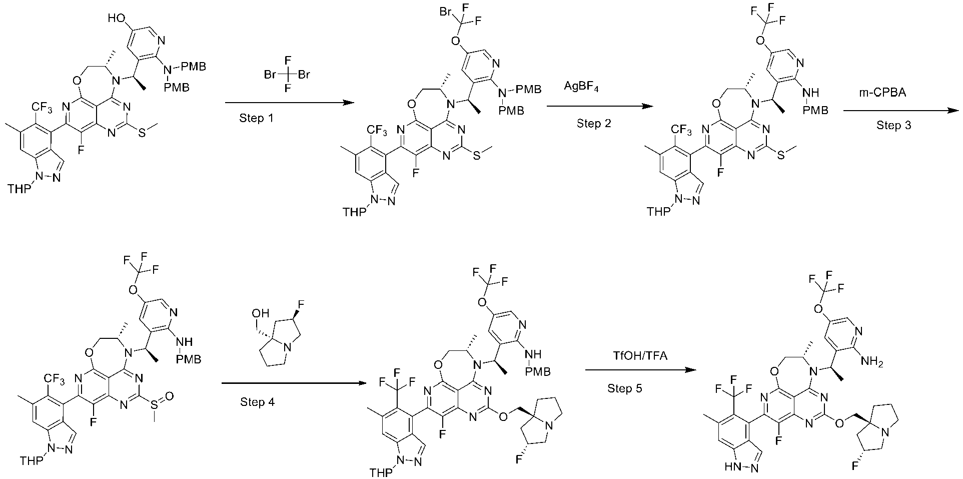

### Step 1: Synthesis of 5-(bromodifluoromethoxy)-3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

To a mixture of the product from Example 14, step 2 (2.24 g, 2.42 mmol) in DMF (20 mL) was added NaH (290 mg, 7.26 mmol, 60% purity) at 0 °C under N₂ and the reaction mixture stirred at 0 °C for 0.5 h. Dibromo(difluoro)methane (5.08 g, 24.2 mmol) was added at 0 °C and the mixture was stirred at 40 °C for 2 h under N₂. The mixture was quenched with 0.1% formic acid (FA) aqueous solution (0.5 mL), filtered and concentrated under reduced pressure. The crude product was purified by reverse-phase HPLC (column: SANTAI, SW-5231-120-SP, Spherical C 18, 20-45µm, 100A; mobile phase: water (0.1% FA) - MeCN; MeCN%: 30% - 80%, 20 min) to give the title compound (550 mg, 21%) as a white solid. LCMS m/z = 1055 [M+H]+

### Step 2: Synthesis of 3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylthio)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

To a solution of the title compound from step 1 (490 mg, 464 µmol) in DCM (5 mL) was added AgBF₄ (905 mg, 4.65 mmol) and the reaction mixture was stirred at 25 °C for 1 h. MeOH (10 mL) was added. The mixture was filtered and the filtrate was concentrated under reduced pressure. The crude product was purified by reverse-phase HPLC (column: SANTAI, SW-5231-120-SP, Spherical C 18, 20-45µm, 100A; mobile phase: water (0.1% FA) - MeCN; MeCN%: 30% - 80%, 20 min) to give the title compound (340 mg, 83%) as a white solid.

1H NMR (400 MHz, CD₃OD) δ 8.10 (s, 1H), 7.86 (s, 1H), 7.74 (d, 1H), 7.67 (s, 1H), 6.96 (d, 1H), 6.88 (d, 1H), 6.80 (d, 1H), 6.64 - 6.48 (m, 2H), 5.98 - 5.79 (m, 1H), 4.82 (d, 1H), 4.70 - 4.54 (m, 2H), 4.41 - 4.26 (m, 1H), 4.19 - 4.08 (m, 2H), 4.06 - 3.93 (m, 2H), 3.90 - 3.76 (m, 1H), 3.72 (d, 2H), 3.55 (d, 1H), 2.74 (s, 3H), 2.60 - 2.41 (m, 3H), 2.21 - 2.03 (m, 2H), 1.96 - 1.68 (m, 3H), 1.66 - 1.55 (m, 3H), 0.72 - 0.47 (m, 3H)

### Step 3: Synthesis of 3-((1R)-1-((9S)-4-fluoro-9-methyl-5-(6-methyl-1-(tetrahydro-2H-pyran-2-yl)-5-(trifluoromethyl)-1H-indazol-4-yl)-2-(methylsulfinyl)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

To a solution of the title compound from step 2 (300 mg, 343 µmol) in DCM (5 mL) was added m-CPBA (76.7 mg, 378 µmol, 85% purity) and the reaction mixture was stirred at 25 °C for 0.5 h. The mixture was poured into a saturated Na₂CO₃ solution (50 mL) and extracted with DCM (50 mL x 3). The combined organic layers were washed with brine (50 mL), dried over Na₂SO₄, filtered and evaporated under reduced pressure to give the title compound (0.3 g, 98%) as a yellow solid. LCMS m/z = 889 [M+H]+

### Step 4: Synthesis of 3-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

To a solution of ((2R,7aS)-2-fluorotetrahydro-1H-pyrrolizin-7a(5H)-yl)methanol (161 mg, 1.01 mmol) in THF (3 mL) was added t-BuOLi (2.2 M, THF, 306 µL) at 0 °C. The mixture was stirred at 0 °C for 15 min, then the title compound from step 3 (300 mg, 337 µmol) in THF (3 mL) was added dropwise and the reaction mixture was stirred at 25 °C for 0.5 h. The mixture was filtered and the filtrate was poured into aqueous NH₄Cl solution (30 mL) and extracted with EtOAc (30 mL x 3). The combined organic layers were dried over Na₂SO₄, filtered and concentrated under reduced pressure. The crude product was purified by reverse-phase HPLC (column: SANTAI, SW-8222-040-SP, Spherical C 18, 20-45 µm, 100Å; mobile phase: water (0.1% FA) - MeCN; MeCN%: 20% - 80%, 10 min) to give the title compound (230 mg, 69%) as a white solid. LCMS m/z = 984 [M+H]+

### Step 5: Synthesis of 3-((1R)-1-((9S)-4-fluoro-2-(((2R,7aS)-2-fluorotetrahydro-1H-pyrrolizin-7a(5SH)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-(trifluoromethoxy)pyridin-2-amine formate.

The title compound was obtained as a solid, 53.8 mg, 33.6%, from the title compound from step 4, following a similar procedure to that described in Example 16b, step 4. LCMS: m/z = 780 [M+H]+; 1H NMR (400 MHz, CD₃OD) δ 8.40 (s, 1H), 8.03 (d, 1H), 7.79 (s, 1H), 7.76 - 7.59 (m, 2H), 6.85 - 6.72 (m, 1H), 5.63 - 5.38 (m, 1H), 4.75 - 4.60 (m, 3H), 4.39 (d, 1H), 4.21 - 4.08 (m, 1H), 3.92 - 3.64 (m, 3H), 3.45 - 3.35 (m, 1H), 2.69 (d, 3H), 2.66 - 2.46 (m, 2H), 2.43 - 2.34 (m, 1H), 2.33 - 2.19 (m, 2H), 2.13 (s, 1H), 1.72 (d, 3H), 0.89 - 0.68 (m, 3H)

### Example 20: Synthesis of 5-(difluoromethoxy)-3-((1R)-1-((9S)-2-(((R)-1,2-dimethylpyrrolidin-2-yl)methoxy)-4-fluoro-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine formate (Compound 136)

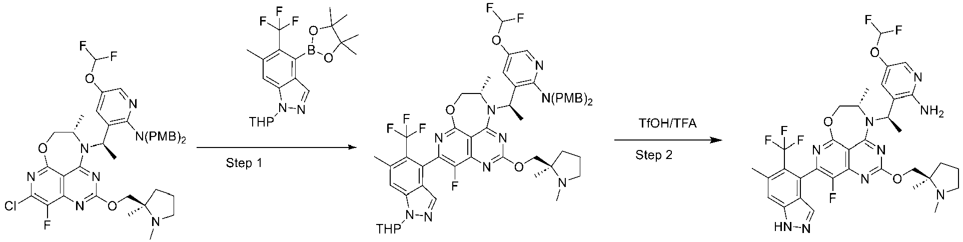

### Step 1: Synthesis of 5-(difluoromethoxy)-3-((1R)-1-((9S)-2-(((R)-1,2-dimethylpyrrolidin-2-yl)methoxy)-4-fluoro-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 30 mg, 22.9%, from Intermediate 23 and Intermediate 10, following a similar procedure to that described in Example 16b, step 1. LCMS m/z = 1057 [M+H]+

### Step 2: Synthesis of 5-(difluoromethoxy)-3-((1R)-1-((9S)-2-(((R)-1,2-dimethylpyrrolidin-2-yl)methoxy)-4-fluoro-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine formate.

The title compound was obtained as a yellow solid (4.57 mg, 21%) from the title compound from step 1, following a similar procedure to that described in Example 3, step 2. LCMS m/z = 732 [M+H]+; 1H NMR (400 MHz, DMSO-d₆) δ 13.52 (d, 1H), 8.15 (s, 1H), 7.94 (d, 1H), 7.73 - 7.71 (m, 2H), 7.64 (s, 1H), 7.11 (t, 1H), 6.57 - 6.54 (m, 1H), 5.88 (s, 1H), 5.79 (s, 1H), 4.56 - 4.53 (m, 1H), 4.41 - 4.32 (m, 3H), 4.16 - 4.14 (m, 1H), 3.10 - 3.05 (m, 1H), 2.82 - 2.80 (m, 1H), 2.64 (s, 3H), 2.45 (s, 3H), 2.03 - 2.00 (m, 1H), 1.82 - 1.69 (m, 3H), 1.64 (d, 3H), 1.14 (s, 3H), 0.67- 0.60 (m, 3H).

### Example 21: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-6-methyl-1H-indazol-4-yl)-9-methyl-8,9-dihydro-10H-7-oxa-1,3,6,10-tetraazacyclohepta[de]naphthalen-10-yl)ethyl)pyridin-2-amine (Compound 137)

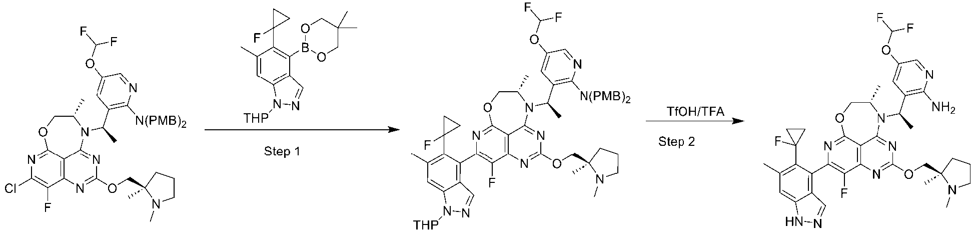

### Step 1: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-6-methyl-1-(tetrahydro-2H-pyran-2-yl)-1H-indazol-4-yl)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 90 mg, 46.3%, from Intermediate 23 and Intermediate 18, following a similar procedure to that described in Example 15, step 1. LCMS m/z = 1047 [M+H]+

### Step 2: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-6-methyl-1H-indazol-4-yl)-9-methyl-8,9-dihydro-10H-7-oxa-1,3,6,10-tetraazacyclohepta[de]naphthalen-10-yl)ethyl)pyridin-2-amine formate

The title compound was obtained as a white solid (14 mg, 31.8%) from the title compound from step 1, following a similar procedure to that described in Example 3, step 2. LCMS m/z = 722 [M+H]+ 1H NMR (400 MHz, DMSO-d₆) δ 13.19 (d, 1H), 8.17 (s, 1H), 7.94 (d, 1H), 7.66 - 7.63 (m, 2H), 7.55 (d, 1H), 7.12 (t, 1H), 6.59 - 6.53 (m, 1H), 5.84 (s, 2H), 4.59 - 4.54 (m, 1H), 4.39 - 4.36 (m, 1H), 4.32 - 4.22 (m, 2H), 4.15 - 4.13 (m, 1H), 2.92 - 2.88 (m, 1H), 2.69 (d, 3H), 2.63 - 2.60 (m, 1H), 2.31 (s, 3H), 1.98 - 1.95 (m, 1H), 1.75 - 1.70 (m, 2H), 1.65 - 1.60 (m, 4H), 1.25 - 0.95 (m, 5H), 0.68- 0.52 (m, 5H).

### Example 22: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-6-methyl-1H-pyrazolo[3,4-b]pyridin-4-yl)-9-methyl-8,9-dihydro-10H-7-oxa-1,3,6,10-tetraazacyclohepta[de]naphthalen-10-yl)ethyl)pyridin-2-amine (Compound 141)

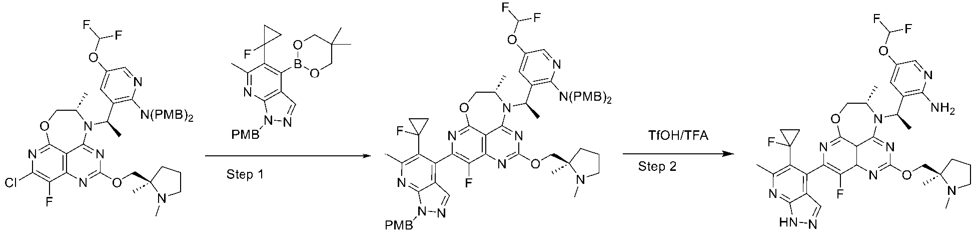

### Step 1: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-1-(4-methoxybenzyl)-6-methyl-1H-pyrazolo[3,4-b]pyridin-4-yl)-9-methyl-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid (80 mg, 39.8%) from Intermediate 23 and Intermediate 16, following a similar procedure to that described in Example 15, step 1. LCMS m/z = 1084 [M+1]+

### Step 2: Synthesis of 5-(difluoromethoxy)-3-((1R)-1-((9S)-2-(((R)-1,2-dimethylpyrrolidin-2-yl)methoxy)-4-fluoro-5-(5-(1-fluorocyclopropyl)-6-methyl-1H-pyrazolo[3,4-b]pyridin-4-yl)-9-methyl-8,9-dihydro-10H-7-oxa-1,3,6,10-tetraazacyclohepta[de]naphthalen-10-yl)ethyl)pyridin-2-amine

A solution of the title compound from step 1 (70 mg, 64.6 µmol) in TFA (1.4 mL) and TfOH (237 mg, 1.58 mmol) was stirred at 25 °C for 0.5 h. The solution was diluted with DCM (20 mL) and the pH was adjusted to 7~8 with saturated NaHCO₃ solution. The layers were separated and the organic layer was concentrated *in vacuo.* The residue was purified by prep-HPLC (column: Waters Xbridge 150 * 25 mm 10 um; mobile phase: [H₂O (10 mM NH₄HCO₃) - MeCN]; gradient: 35% - 65% B over 10.0 min), prep-HPLC (column: Welch Ultimate XB-SiOH 150 * 40 mm * 10 µm; mobile phase: [Hexane - EtOH]; gradient: 10% - 40% B over 12.0 min), prep-HPLC (column: Phenomenex Luna C18 150 * 25 mm * 10 µm; mobile phase: [H2O (0.1% TFA) - MeCN]; gradient: 1%-30% B over 13.0 min) and prep-HPLC (column: Waters Xbridge 150 * 25 mm 10 µm; mobile phase: [H₂O (10 mM NH₄HCO₃) - MeCN]; gradient: 30% - 60% B over 10.0 min) to give the title compound (16.9 mg, 36.1%) as a white solid. LCMS m/z = 723 [M+H]+ 1H NMR (400 MHz, DMSO-d₆) δ 13.83 - 13.73 (m, 1H), 7.94 (d, 1H), 7.85 (s, 1H), 7.64 (d, 1H), 7.12 (t, 1H), 6.58 - 6.55 (m, 1H), 5.82 (s, 2H), 4.59 - 4.55 (m, 1H), 4.39 - 4.30 (m, 3H), 4.17 - 4.13 (m, 1H), 3.15 - 2.90 (m, 1H), 2.88 (s, 3H), 2.78 - 2.68 (m, 1H), 2.39 - 2.33 (m, 3H), 2.03 - 2.00 (m, 1H), 1.80 - 1.78 (m, 2H), 1.70 - 1.64 (m, 4H), 1.24 - 1.07 (m, 5H), 0.77 - 0.64 (m, 5H).

### Example 23: Synthesis of 5-(difluoromethoxy)-3-((1R)-1-((9S)-4-fluoro-5-(5-(1-fluorocyclopropyl)-6-methyl-1H-indazol-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)pyridin-2-amine (Compound 139)

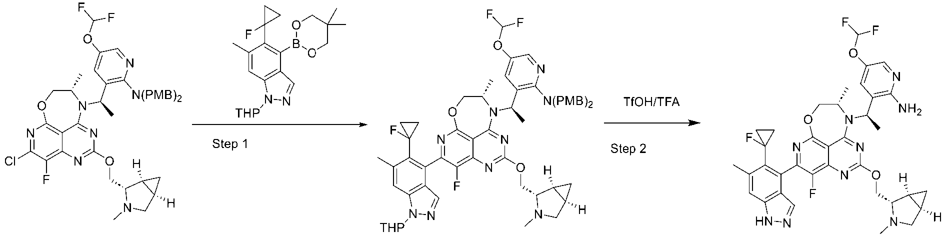

### Step 1: Synthesis of -fluoro-5-(5-(1-fluorocyclopropyl)-6-methyl-1-(tetrahydro-2H-pyran-2-yl)-1H-indazol-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid, 550 mg, 41.7%, from Intermediate 24 and Intermediate 18 following a similar procedure to that described in Example 15, step 1. LCMS m/z = 1045 [M+H]+

### Step 2: Synthesis of -fluoro-5-(5-(1-fluorocyclopropyl)-6-methyl-1H-indazol-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)pyridin-2-amine formate

The title compound was obtained as a white solid (42.4 mg, 58.3%) from the title compound from step 1, following a similar procedure to that described in Example 3, step 2. LCMS m/z = 720 [M+H]+; 1H NMR (400 MHz, DMSO-d₆) δ = 13.24 - 13.11 (m, 1H), 8.14 (s, 1H), 7.93 (d, 1H), 7.76 - 7.51 (m, 3H), 7.32 - 6.89 (m, 1H), 6.63 - 6.46 (m, 1H), 5.81 (s, 2H), 4.64 - 4.51 (m, 2H), 4.41 - 4.27 (m, 2H), 4.18 - 4.09 (m, 1H), 3.22 (s, 1H), 2.98 (d, 1H), 2.69 (d, 4H), 2.42 (s, 3H), 1.64 (d, 3H), 1.48 (d, 2H), 1.27 - 0.98 (m, 2H), 0.69 - 0.46 (m, 7H)

### Example 24: Synthesis of 5-(difluoromethoxy)-3-((1R)-1-((9S)-4-fluoro-5-(5-(1-fluorocyclopropyl)-6-methyl-1H-pyrazolo[3,4-b]pyridin-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)pyridin-2-amine (Compound 140)

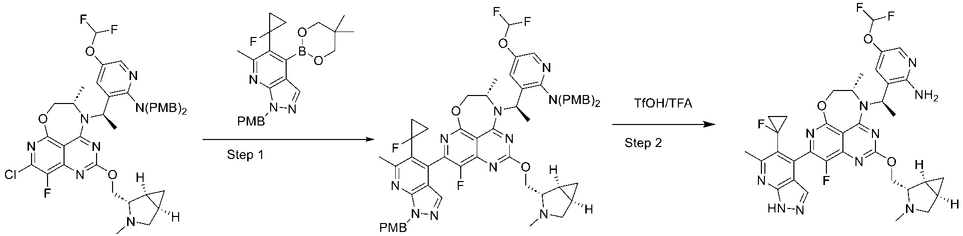

### Step 1: Synthesis of -fluoro-5-(5-(1-fluorocyclopropyl)-1-(4-methoxybenzyl)-6-methyl-1H-pyrazolo[3,4-b]pyridin-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a white solid (110 mg, 53.5%) from Intermediate 24 and Intermediate 16, following a similar procedure to that described in Example 3, step 1. LCMS m/z = 1082 [M+H]+

### Step 2: Synthesis of -fluoro-5-(5-(1-fluorocyclopropyl)-6-methyl-1H-pyrazolo[3,4-b]pyridin-4-yl)-9-methyl-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)pyridin-2-amine formate

The title compound was obtained as a white solid (40 mg, 60%) from the title compound from step 1, following a similar procedure to that described in Example 3, step 2. LCMS m/z = 721 [M+H]+ 1H NMR (400 MHz, DMSO-d₆) δ = 13.89 - 13.62 (m, 1H), 8.14 (s, 1H), 7.93 (d, 1H), 7.85 (s, 1H), 7.63 (d, 1H), 7.31 - 6.90 (m, 1H), 6.55 (s, 1H), 5.80 (s, 2H), 4.56 (d, 2H), 4.40 - 4.26 (m, 2H), 4.18 - 4.10 (m, 1H), 3.18 (t, 1H), 2.94 (d, 1H), 2.87 (s, 3H), 2.65 (d, 1H), 2.39 (s, 3H), 1.64 (d, 3H), 1.48 - 1.44 (m, 2H), 1.33 - 1.11 (m, 2H), 0.64 (s, 5H), 0.56 (td, 1H), 0.45 (q, 1H)

### Example 25: Synthesis of 5-(difluoromethoxy)-3-((1R)-1-((9S)-4-fluoro-9-methyl-2-(((1S,2S,5R)-3-methyl-3-azabicyclo[3.1.0]hexan-2-yl)methoxy)-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine (Compound 138)

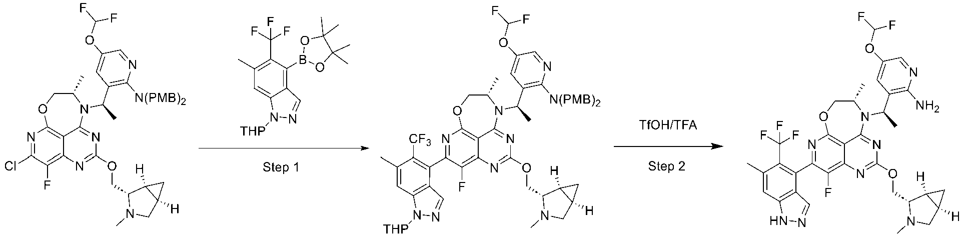

### Step 1: Synthesis of 5-(difluoromethoxy)-3-((1R)-1-((9S)-4-fluoro-9-methyl-5-(6-methyl-1-(tetrahydro-2H-pyran-2-yl)-5-(trifluoromethyl)-1H-indazol-4-yl)-2-(((1S,2S,5R)-3-methyl-3-azabicyclo[3.1.0]hexan-2-yl)methoxy)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 24 (80 mg, 99.2 µmol) and Intermediate 10 (101 mg, 248 µmol) in n-BuOH (5 mL) and H₂O (0.5 mL) was added SPhos Pd G3 (7.74 mg, 9.92 µmol) and K₃PO₄ (63.1 mg, 297 µmol). The reaction mixture was stirred at 40 °C for 16 h under N₂. The reaction mixture was quenched with water (10 mL) and extracted with EtOAc (10 mL x 3). The combined organic layers were washed with saturated brine (10 mL), dried over anhydrous Na₂SO₄, filtered and concentrated under reduced pressure. The residue was purified by prep-HPLC (Method A, gradient: 37% - 67% B over 10 min) and lyophilized to give the title compound (50 mg, 47.6%) as a yellow solid. LCMS m/z = 1055 [M+H]+

### Step 2: Synthesis of 5-(difluoromethoxy)-3-((1R)-1-((9S)-4-fluoro-9-methyl-2-(((1S,2S,5R)-3-methyl-3-azabicyclo[3.1.0]hexan-2-yl)methoxy)-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine formate

The title compound was obtained as a light-yellow solid (25.6 mg, 77.4%) from the title compound from step 1, following a similar procedure to that described in Example 3, step 2. LCMS m/z = 730 [M+H]+; 1H NMR (400 MHz, DMSO-d₆) δ = 13.59 - 13.41 (m, 1H), 8.13 (s, 1H), 7.93 (d, 1H), 7.76 - 7.68 (m, 2H), 7.63 (t, 1H), 7.32 - 6.89 (m, 1H), 6.56 (dd, 1H), 5.88 - 5.72 (m, 2H), 4.62 - 4.49 (m, 2H), 4.41 - 4.26 (m, 2H), 4.12 (q, 1H), 3.26 - 3.18 (m, 1H), 2.97 (dd, 1H), 2.64 (d, 4H), 2.41 (s, 3H), 1.63 (d, 3H), 1.48 (s, 2H), 0.69 - 0.40 (m, 5H)

### Example 26. Synthesis of 3-((1R)-1-((9S)-5-(6-chloro-5-(1-fluorocyclopropyl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)pyridin-2-amine. (Compound 142)

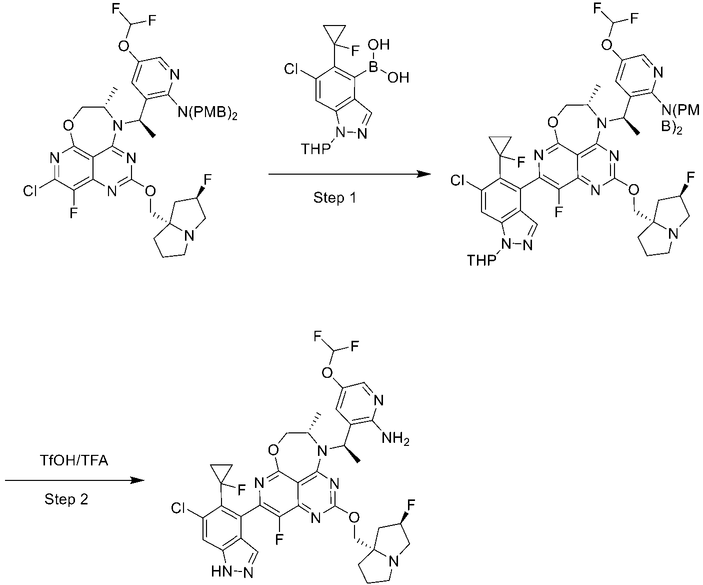

### Step 1: Synthesis of -fluorocyclopropyl)-1-(tetrahydro-2H-pyran-2-yl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)-N,N-bis(4-methoxybenzyl)pyridin-2-amine

A mixture of Intermediate 6 (100 mg, 119 µmol), Intermediate 25 (48.4 mg, 143 µmol), CataCXium^{®} A PD G3 (8.69 mg, 11.9 µmol) and K₂CO₃ (32.9 mg, 238 µmol) in dioxane (1 mL) and H₂O (0.1 mL) was degassed and purged with N₂ (3x). The reaction mixture was stirred at 130 °C for 0.5 h under N₂ and microwave irradiation. The mixture was filtered and the filtrate was concentrated under reduced pressure. The residue was purified by prep HPLC (Method C, 20% - 100%B, in 20 min) to give the title compound (70 mg, 53%) as a yellow solid. LCMS m/z = 1096 [M+H]⁺

### Step 2: Synthesis of 3-((1R)-1-((9S)-5-(6-chloro-5-(1-fluorocyclopropyl)-1H-indazol-4-yl)-4-fluoro-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(difluoromethoxy)pyridin-2-amine

To a solution from the title compound from Step 1 (70 mg, 63.8 µmol) in TFA (1 mL) was added TfOH (0.1 mL) and the reaction mixture was stirred at 25 °C for 10 mins. The mixture was concentrated under reduced pressure, the residue was diluted with DCM (2 mL), cooled to 0°C, then neutralized using NH₃H₂O. The mixture was concentrated under reduced pressure and the residue was purified by prep-HPLC (Method F, 36%-66% B over 15.0 min) to give the title compound (13.3 mg, 25%) as an off-white solid. LCMS m/z = 772 [M+H]⁺ 1H NMR (400 MHz, DMSO-d₆) δ = 13.65 - 13.26 (m, 1H), 7.94 (d, 1H), 7.90 (d, 1H), 7.84 (d, 1H), 7.63 (d, 1H), 7.11 (t, 1H), 6.62 - 6.47 (m, 1H), 5.82 (d, 2H), 5.43 - 5.17 (m, 1H), 4.64 - 4.47 (m, 1H), 4.41 - 4.29 (m, 1H), 4.24 - 4.06 (m, 3H), 3.17 - 3.02 (m, 3H), 2.92 - 2.74 (m, 1H), 2.18 - 2.01 (m, 3H), 1.92 - 1.76 (m, 3H), 1.64 (d, 3H), 1.29 - 1.05 (m, 2H), 0.77 - 0.50 (m, 5H).

### Example 27 and 28: Synthesis of 5-fluoro-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine or 5-fluoro-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine (Compound 143) , and

### Synthesis of 5-fluoro-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine or 5-fluoro-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine (Compound 144).

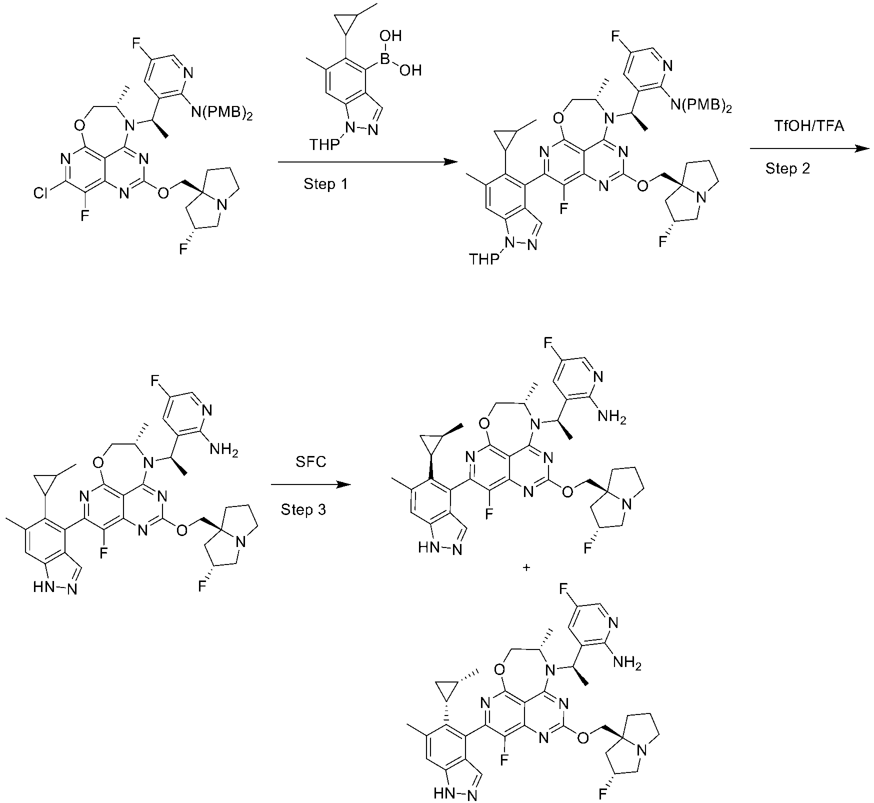

### Step 1: Synthesis of 5-fluoro-3-((1R)-1-((9S)-4-fluoro-2-(((2R,7aS)-2-fluorotetrahydro-1H-pyrrolizin-7a(5H)-yl)methoxy)-9-methyl-5-((4R)-6-methyl-5-((1SR,2RS)-2-methylcyclopropyl)-1-(tetrahydro-2H-pyran-2-yl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was obtained as a yellow solid, 260 mg, 83%, from Intermediate 3 and Intermediate 26 following a similar method to that described in Example 26, step 1. The residue was purified by reverse-phase HPLC (column: SANTAI, SW-5222-080-SP, Spherical C 18, 20 - 45 µm, 100A; mobile phase: water (0.1% NH₄HCO₃) - MeCN%: 1% - 100%, 20 min). LCMS: *m*/*z* = 1025 [M+H]⁺

### Step 2: Synthesis of 5-fluoro-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1SR,2RS)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine

The title compound was obtained as a yellow solid, 120 mg, 78%, from the title compound from step 1, following a similar method to that described in Example 10, step 2. The crude was purified by reverse-phase HPLC (column: SANTAI, SW-8222-040-SP, Spherical C₁₈, 20 - 45 µm, 100 A; mobile phase: water (0.1% NH₄HCO₃) - MeCN%: 30% - 70%, 20 min) to give the title compound (120 mg, 78%) as a yellow solid. LCMS: *m*/*z* = 700 [M+H]⁺

### Step 3: Synthesis of 5-fluoro-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine and 5-fluoro-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine

The title compound from step 2 (120 mg, 171 µmol) was separated by SFC (column: DAICEL CHIRALPAK IC (250 mm * 30 mm, 10 um); mobile phase: [CO₂-EtOH:MeCN = 4:1 (0.1% NH₃H₂O)]; B%: 42%, isocratic elution mode) to give Peak 1, Example 27 corresponding to structure of 143A or 143B (22.8 mg, 18%) as an off-white solid LCMS: *m*/*z* = 700 [M+H]^{+ 1}H NMR (400 MHz, DMSO-*d*₆) δ 12.96 (br s, 1H), 8.00 (d, 1H), 7.81 - 7.53 (m, 2H), 7.47 (s, 1H), 6.54 (d, 1H), 5.69 (s, 2H), 5.44 - 5.17 (m, 1H), 4.67 - 4.46 (m, 1H), 4.44 - 4.28 (m, 1H), 4.22 - 4.05 (m, 3H), 3.20 - 2.97 (m, 4H), 2.90 - 2.71 (m, 1H), 2.53 (s, 3H), 2.21 - 1.94 (m, 4H), 1.93 - 1.72 (m, 3H), 1.62 (d, 3H), 1.22 - 0.97 (m, 1H), 0.64 - 0.39 (m, 6H), -0.22 - -0.63 (m, 1H).
and Peak 2, Example 28 corresponding to structure of 144A or 144B (52.8 mg, 43%) as an off-white solid. LCMS: *m*/*z* = 700 [M+H]^{+ 1}H NMR (400 MHz, DMSO-d₆) δ 13.09 - 12.82 (m, 1H), 8.00 (d, 1H), 7.75 - 7.66 (m, 1H), 7.65 - 7.55 (m, 1H), 7.47 (d, 1H), 6.67 - 6.41 (m, 1H), 5.63 (d, 2H), 5.42 - 5.14 (m, 1H), 4.63 - 4.47 (m, 1H), 4.45 - 4.27 (m, 1H), 4.23 - 4.01 (m, 3H), 3.28 - 2.96 (m, 4H), 2.89 - 2.78 (m, 1H), 2.63 - 2.52 (m, 3H), 2.26 - 1.96 (m, 4H), 1.88 - 1.71 (m, 3H), 1.63 (d, 3H), 1.25 - 1.11 (m, 1H), 0.70 - 0.44 (m, 6H), -0.14 - -0.62 (m, 1H)

### Example 29 and 30: Synthesis of 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine or 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine (Compound 146) and Synthesis of 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine or 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine (Compound 145).

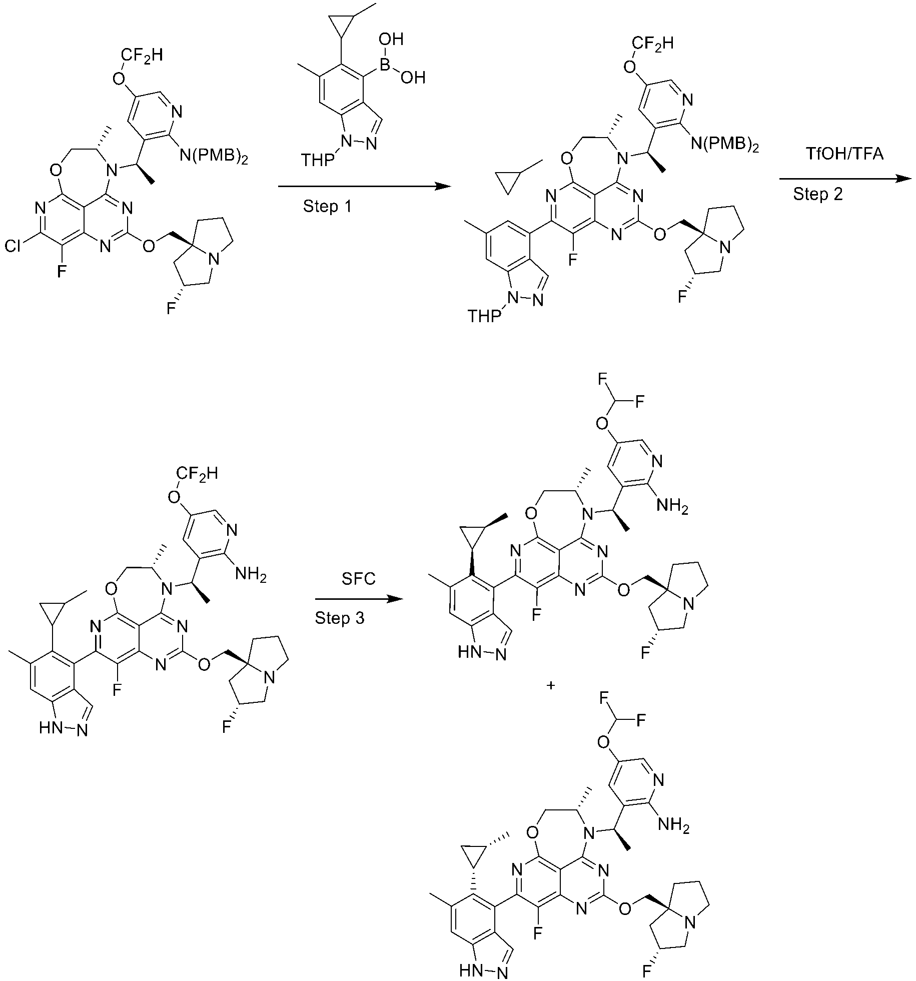

### Step 1: Synthesis of 5-(difluoromethoxy)-3-((1R)-1-((9S)-4-fluoro-2-(((2R, 7aS)-2-fluorotetrahydro-1H-pyrrolizin-7a(5H)-yl)methoxy)-9-methyl-5-((4S)-6-methyl-5-((1S,2R)-2-methylcyclopropyl)-1-(tetrahydro-2H-pyran-2-yl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

Intermediate 6 (290 mg, 346 µmol), Intermediate 26 (108.7 mg, 346 µmol), CataCXium^{®} A PD G3 (25.2 mg, 34.6 µmol) and K₂CO₃ (95.62 mg, 692 µmol) were placed in a microwave tube and dioxane (6 mL) and H₂O (2 mL) were added. The sealed tube was heated at 130 °C for 30 min under microwave irradiation under N₂. The reaction mixture was quenched with water (30 mL) and extracted with EtOAc (10 mL x 3). The combined organic layers were washed with saturated brine (10 mL), dried over anhydrous Na₂SO₄, filtered and concentrated under reduced pressure. The residue was purified by prep-HPLC (column: Waters Xbridge Prep OBD C18 150 * 40 mm * 10 um; mobile phase: [H₂O (10 mM NH₄HCO₃) - MeCN]; gradient: 70% - 100% B over 15.0 min) and lyophilized to give the title compound (300 mg, 80%) as a white solid. LCMS m/z = 1073 [M+H]⁺

### Step 2: Synthesis of 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine

The title compound was obtained from the title compound from step 1, following a similar method to that described in Example 3, step 2. The crude product was purified by prep HPLC (Method C, 1% - 36%B, over 15 min) and lyophilized to give the title compound (120 mg, 57.4%) as a white solid. LCMS: *m*/*z* = 748 [M+H]⁺

### Step 3: Synthesis of 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((R)-6-methyl-5-((1S,2R)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine and 5-(difluoromethoxy)-3-((R)-1-((S)-4-fluoro-2-(((2R,7aS)-2-fluorotetrahydro-1H-pyrrolizin-7a(5H)-yl)methoxy)-9-methyl-5-((S)-6-methyl-5-((1R,2S)-2-methylcyclopropyl)-1H-indazol-4-yl)-8,9-dihydro-10H-7-oxa-1,3,6,10-tetraazacyclohepta[de]naphthalen-10-yl)ethyl)pyridin-2-amine

The title compound from step 2 (120 mg, 160.5 µmol) was separated by SFC (column: DAICEL CHIRALPAK IK (250 mm * 25 mm, 10 um); mobile phase: [CO₂-IPA:MeCN = 4:1 (containing 0.2% NH₃H₂O)]; B%: 60%, isocratic elution mode) and lyophilized to give Peak 1, Example 29 corresponding to structure of 146A or 146B (39.9 mg, 33.3%) as a white solid. LCMS: *m*/*z* = 748 [M+H]^{+ 1}H NMR (400 MHz, DMSO-*d*₆) δ 12.96 (s, 1H), 7.93 (d, 1H), 7.72 - 7.56 (m, 2H), 7.47 (s, 1H), 7.30 - 6.90 (m, 1H), 6.62 - 6.48 (m, 1H), 5.85 (s, 2H), 5.42 - 5.18 (m, 1H), 4.63 - 4.47 (m, 1H), 4.43 - 4.29 (m, 1H), 4.25 - 4.01 (m, 3H), 3.25 - 3.02 (m, 3H), 2.92 - 2.79 (m, 1H), 2.53 (s, 3H), 2.23 - 1.95 (m, 4H), 1.92 - 1.76 (m, 3H), 1.63 (d, 3H), 1.18 - 1.06 (m, 1H), 0.84 - 0.51 (m, 5H), 0.48 (d, 2H), -0.50 - -0.49 (m, 1H).
and Peak 2, Example 30 corresponding to structure of 145A or 145B (39.9 mg, 33.3%) as a white solid. LCMS: *m*/*z* = 748 [M+H]^{+ 1}H NMR (400 MHz, DMSO-*d*₆) δ 13.03 - 12.87 (m, 1H), 7.93 (d, 1H), 7.60 (d, 2H), 7.49 - 7.42 (m, 1H), 7.31 - 6.90 (m, 1H), 6.62 - 6.48 (m, 1H), 5.79 (d, 2H), 5.40 - 5.18 (m, 1H), 4.58 - 4.47 (m, 1H), 4.41 - 4.31 (m, 1H), 4.20 - 4.05 (m, 3H), 3.14 - 3.06 (m, 2H), 3.02 (d, 1H), 2.88 - 2.79 (m, 1H), 2.54 (s, 3H), 2.20 - 2.00 (m, 4H), 1.88 - 1.76 (m, 3H), 1.63 (d, 3H), 1.22 - 1.06 (m, 1H), 0.73 - 0.60 (m, 4H), 0.53 (d, 2H), 0.45 (s, 1H), -0.24 - -0.52 (m, 1H).

### Example 31: Synthesis of 3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(trifluoromethoxy)pyridin-2-amine. (Compound 147)

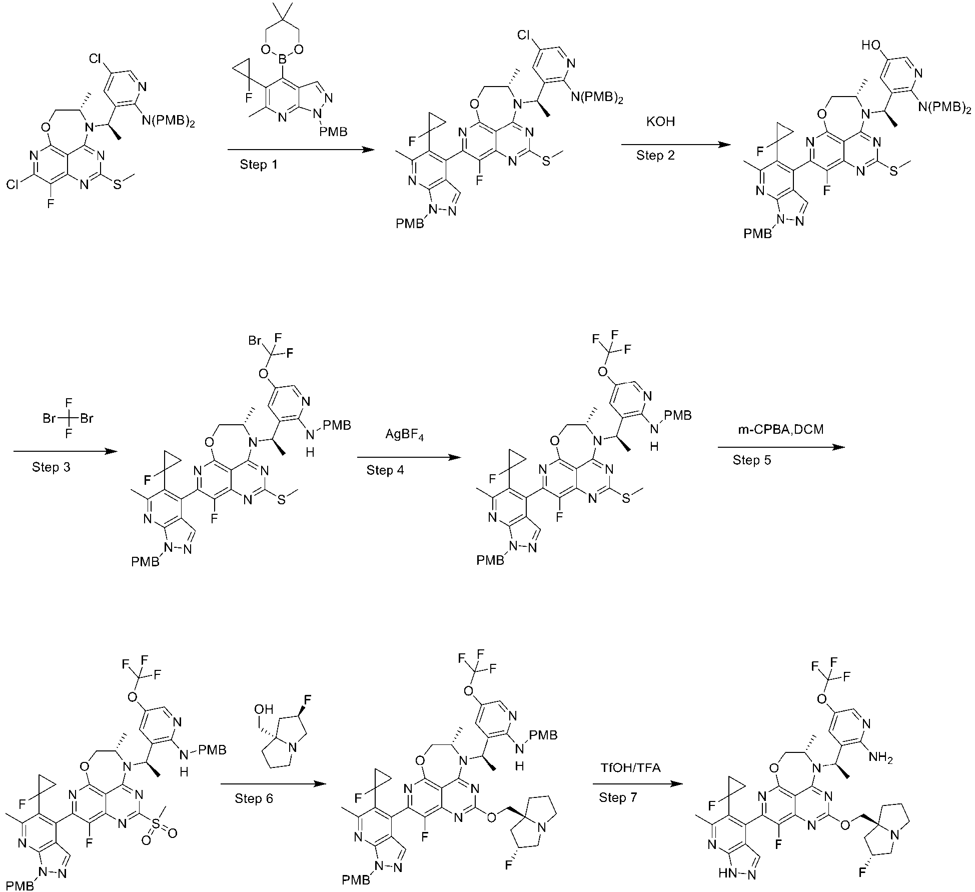

### Step 1: 5-chloro-3-((1R)-1-((9S)-4-fluoro-5-(5-(1-fluorocyclopropyl)-1-(4-methoxybenzyl)-6-methyl-1H-pyrazolo[3,4-b]pyridin-4-yl)-9-methyl-2-(methylthio)-8,9-dihydro-10H-7-oxa-1,3,6,10-tetraazacycloheptalde]naphthalen-10-yl)ethyl)-N,N-bis(4-methoxybenzyl)pyridin-2-amine

The title compound was prepared from Intermediate 4 and Intermediate 16, following a similar method to that described in Example 3, step 1. The crude product was purified by HPLC (Method G, gradient: 1% to 100% B over 15 min) to afford the title compound (1.6 g, 57%) as a yellow solid. LCMS m/z = 971 [M+H]⁺

### Step 2: 6-(bis(4-methoxybenzyl)amino)-5-((1R)-1-((9S)-4-fluoro-5-(5-(1-fluorocyclopropyl)-1-(4-methoxybenzyl)-6-methyl-lH-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)pyridin-3-ol

The title compound was prepared from the title compound from step 1 and KOH, following a similar procedure to that described in Example 15, step 2. The crude product was purified by reverse-phase HPLC (column: SANTAI SW-5231-120-SP, spherical C₁₈, 40-60 µm, 120 Å; mobile phase: water (0.1% FA)-MeCN gradient: 1% to 100% over 15 min) to afford the title compound (640 mg, 65%) as a yellow solid. ¹H NMR (400 MHz, DMSO-d₆) δ 9.99 - 9.64 (m, 1H), 8.16 - 7.87 (m, 2H), 7.37 (s, 1H), 7.22 (d, 2H), 6.87 (d, 2H), 6.81 - 6.35 (m, 8H), 5.55 - 5.54 (m, 1H), 5.62 (s, 2H), 4.37 - 4.24 (m, 1H), 4.13 - 3.91 (m, 2H), 3.77 (s, 3H), 3.72 - 3.50 (m, 9H), 3.02 - 2.79 (m, 3H), 2.67 (s, 3H), 1.55 - 1.20 (m, 4H), 1.04 - 0.43 (m, 3H), 0.38 - 0.12 (m, 4H).

### Step 3: 5-(bromodifluoromethoxy)-3-((1R)-1-((9S)-4-fluoro-5-(5-(1-fluorocyclopropyl)-1-(4-methoxybenzyl)-6-methyl-1H-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was prepared from the title compound from step 2 and dibromo(difluoro)methane, following a similar procedure to that described in Example 19, step 1. The crude product was purified by prep HPLC (Method D, 1% to 100% B over 20 min) to afford the title compound (350 mg, 48%) as a yellow solid. LCMS: *m*/*z* = 1083 [M+H]⁺

### Step 4: 3-((1R)-1-((9S)-4-fluoro-5-(5-(1-fluorocyclopropyl)-1-(4-methoxybenzyl)-6-methyl-1H-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was prepared from the title compound from step 3, following a similar procedure to that described in Example 19, step 2. The crude product was purified by prep HPLC (Method D, 50% to 100% over 20 min) to give the title compound (130 mg, 62%) as a yellow solid. LCMS: *m*/*z* = 901 [M+H]⁺

### Step 5: 3-((1R)-1-((9S)-4-fluoro-5-(5-(1-fluorocyclopropyl)-1-(4-methoxybenzyl)-6-methyl-1H-pyrazolo[3,4-b]pyridin-4-yl)-9-methyl-2-(methylsulfonyl)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

To a solution of the title compound from step 4 (180 mg, 200 µmol) in DCM (2 mL) was added m-CPBA (44.6 mg, 220 µmol, 85% purity) at 0 °C and the mixture was stirred at 0 °C for 1 h. The reaction mixture was quenched with aqueous Na₂SO₃ solution (20 mL) and extracted with DCM (20 mL x 3). The combined organic layers were dried over anhydrous Na₂SO₄, filtered, and concentrated under reduced pressure to afford the title compound (180 mg, crude) as a yellow solid. LCMS: *m*/*z* =932 [M+H]⁺

### Step 6: 3-((1R)-1-((9S)-4-fluoro-5-(5-(1-fluorocyclopropyl)-1-(4-methoxybenzyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was prepared from the title compound from step 5, following a similar procedure to that described in Example 19, step 4. The crude product was purified by prep HPLC (Method C, 1% to 60%B over 20 min) to give the title compound (60 mg, 30%) as a white solid. LCMS m/z = 1012 [M+H]⁺

### Step 7: 3-((1R)-1-((9S)-4-fluoro-5-(5-(1-fluorocyclopropyl)-6-methyl-1H-pyrazolo[3,4-b]pyridin-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(trifluoromethoxy)pyridin-2-amine

To a solution of the title compound from step 6 (50 mg, 49.45 µmol) in TFA (1 mL) was added TfOH (0.1 mL) and the reaction mixture was stirred at 25 °C for 10 min. The mixture was concentrated under reduced pressure, DCM (5 mL) and aqueous NH₃·H₂O (15% purity) were added to adjust the pH to 7 and the mixture concentrated under reduced pressure. The residue was purified by prep-HPLC (Method A, 13% to 43% MeCN over 14.0 min) to afford the title compound (22.5 mg, 58%) as an off-white solid. LCMS: m/z = 771 [M+H]^{+ 1}H NMR (400 MHz, DMSO-d₆) δ 13.93 - 13.55 (m, 1H), 8.08 (d, 1H), 7.85 (d, 1H), 7.77 (d, 1H), 6.54 (s, 1H), 6.07 (s, 2H), 5.47 - 5.10 (m, 1H), 4.57 (d, 1H), 4.37 (d, 1H), 4.24 - 3.99 (m, 3H), 3.13 - 3.02 (m, 3H), 2.93 - 2.80 (m, 4H), 2.23 - 1.98 (m, 3H), 1.91 - 1.74 (m, 3H), 1.64 (d, 3H), 1.34 - 1.05 (m, 2H), 0.77 - 0.53 (m, 5H).

### Example 32. Synthesis of 3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(trifluoromethoxy)pyridin-2-amine. (Compound 148)

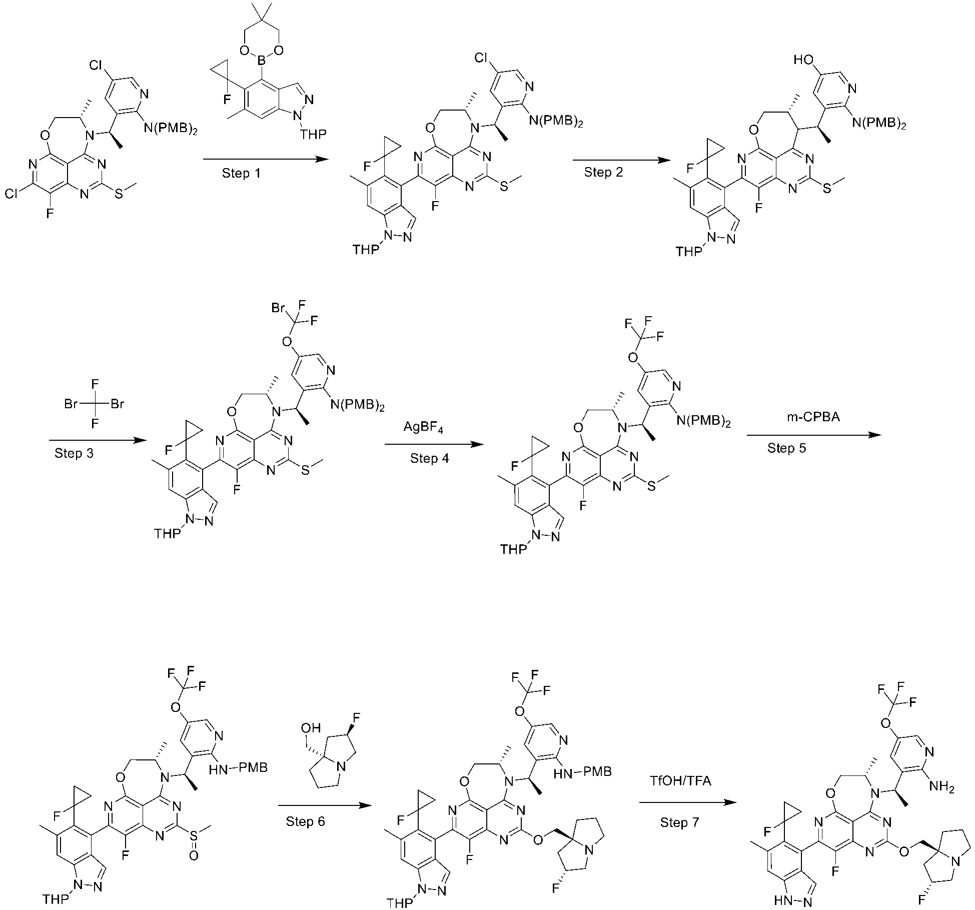

### Step 1: 5-chloro-3-((1R)-1-((9S)-4-fluoro-5-(5-(1-fluorocyclopropyl)-6-methyl-1-(tetrahydro-2H-pyran-2-yl)-1H-indazol-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution Intermediate 4 (1 g, 1.44 mmol) and Intermediate 18 (555 mg, 1.44 mmol) in n-BuOH (15 mL) and H₂O (3 mL) was added SPhos Pd G3 (112 mg, 144 µmol) and K₃PO₄ (915 mg, 4.31 mmol) and the reaction mixture was stirred at 80 °C for 4 h under N₂. The mixture was poured into water (50 mL) and extracted with EtOAc (50 mL x 2). The combined organic layers were concentrated in vacuo and the residue purified by column chromatography (SiO₂, PE/EtOAc = 10:1 to 3:1) to afford the title compound (0.81 g, 54%) as a yellow solid. LCMS *m*/*z* = 933 [M+H]⁺

### Step 2: Synthesis of 6-(bis(4-methoxybenzyl)amino)-5-((1R)-1-((9S)-4-fluoro-5-(5-(1-fluorocyclopropyl)-6-methyl-1-(tetrahydro-2H-pyran-2-yl)-1H-indazol-4-yl)-9-methyl-2-(methylthio)-8,9-dihydro-10H-7-oxa-1,3,6,10-tetraazacyclohepta[de]naphthalen-10-yl)ethyl)pyridin-3-ol

The title compound was prepared from the title compound from step 1 and KOH, following a similar procedure to that described in Example 15, step 2. The crude compound was purified by prep HPLC (Method D, 10% - 90%B, in 15 min) to give the title compound (0.44 g, 62%) as a yellow solid. LCMS *m*/*z* = 916 [M+H]⁺

### Step 3: Synthesis of 5-(bromodifluoromethoxy)-3-((1R)-1-((9S)-4-fluoro-5-(5-(1-fluorocyclopropyl)-6-methyl-1-(tetrahydro-2H-pyran-2-yl)-1H-indazol-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

To a mixture of the title compound from step 2 (0.44 g, 481 µmol) and dibromo(difluoro)methane (1.01 g, 4.81 mmol) in DMF (5 mL) was added NaH (57.7 mg, 1.44 mmol, 60% purity) at 0 °C and the reaction mixture was stirred at 40 °C for 1 h. The cooled reaction was quenched with ice water (30 mL) then extracted with EtOAc (30 mL x 2). The organic phase was concentrated *in vacuo* and the residue was purified by prep HPLC (Method C, 10% - 95%B, in 15 min) to give the title compound (0.2 g, 38%) as a yellow solid. LCMS *m*/*z* = 1045 [M+H]⁺

### Step 4: 3-((1R)-1-((9S)-4-fluoro-5-(5-(1-fluorocyclopropyl)-6-methyl-1-(tetrahydro-2H-pyran-2-yl)-1H-indazol-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)-5-(trifluoromethoxy)pyridin-2-amine

To a solution of the title compound from Step 3 (0.18 g, 172 µmol) in DCM (5 mL) was added AgBF₄ (336 mg, 1.72 mmol) and the reaction mixture was stirred at 25 °C for 2 h. The mixture was filtered and the filtrate was concentrated in vacuo. The residue was purified by prep HPLC (Method C, 10% - 95%B, in 20 min) to give the title compound (70 mg, 46%) as a yellow solid. LCMS m/z = 863 [M+H]⁺

### Step 5: 3-((1R)-1-((9S)-4-fluoro-5-(5-(1-fluorocyclopropyl)-6-methyl-1-(tetrahydro-2H-pyran-2-yl)-1H-indazol-4-yl)-9-methyl-2-(methylsulfinyl)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was obtained as a yellow solid, 75 mg, crude, from the title compound from step 4, following a similar procedure to that described in Example 31, step 5. LCMS m/z = 880 [M+H]⁺

### Step 6: 3-((1R)-1-((9S)-4-fluoro-5-(5-(1-fluorocyclopropyl)-6-methyl-1-(tetrahydro-2H-pyran-2-yl)-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was prepared from the title compound of step 5 and ((2R,7aS)-2-fluorotetrahydro-1H-pyrrolizin-7a(5H)-yl)methanol, following a similar procedure to that described in Example 19, step 4. The residue was purified by prep HPLC (Method C, 10% - 100% B, in 15 min) to give the title compound (30 mg, 39%) as a yellow solid. LCMS m/z = 975 [M+H]⁺

### Step 7: 3-((1R)-1-((9S)-4-fluoro-5-(5-(1-fluorocyclopropyl)-6-methyl-1H-indazol-4-yl)-2-(((2R,7aS)-2-fluorotetrahydro-1H-pyrrolizin-7a(5H)-yl)methoxy)-9-methyl-8,9-dihydro-10H-7-oxa-1,3,6,10-tetraazacyclohepta[de]naphthalen-10-yl)ethyl)-5-(trifluoromethoxy)pyridin-2-amine

The title compound was prepared from the title compound from step 6, following a similar procedure to that described in Example 31, step 7. The residue was purified by prep-HPLC (Method A, 24% to 54% B over 10.0 min) to afford the title compound (13 mg, 55%) as a white solid. LCMS m/z = 770 [M+H]⁺ 1H NMR (400 MHz, DMSO-d₆) δ 13.24 - 13.12 (m, 1H), 8.08 (d, 1H), 7.77 (s, 1H), 7.69 (d, 1H), 7.55 (d, 1H), 6.61 - 6.49 (m, 1H), 6.10 (s, 2H), 5.39 - 5.19 (m, 1H), 4.63 - 4.48 (m, 1H), 4.36 (d, 1H), 4.20 - 4.07 (m, 3H), 3.14 - 3.05 (m, 2H), 3.02 (s, 1H), 2.87 - 2.78 (m, 1H), 2.68 (d, 3H), 2.18 - 2.11 (m, 1H), 2.10 - 1.99 (m, 2H), 1.90 - 1.74 (m, 3H), 1.64 (d, 3H), 1.26 - 0.95 (m, 2H), 0.63 (dd, 5H).

### Example 33. Synthesis of 3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)-5-(trifluoromethoxy)pyridin-2-amine. Compound 151

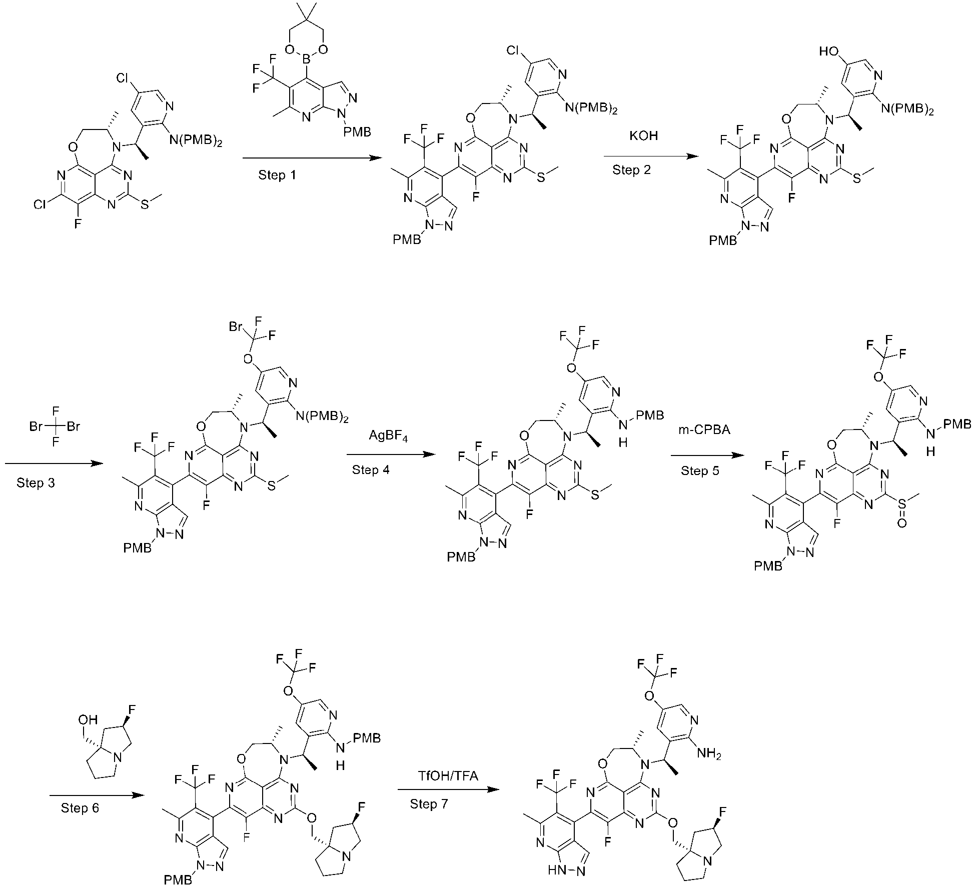

### Step 1: Synthesis of 5-chloro-3-((1R)-1-((9S)-4-fluoro-5-(1-(4-methoxybenzyl)-6-methyl-5-(trifluoromethyl)-1H-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

A solution of Intermediate 4 (1.5 g, 2.16 mmol) and Intermediate 11 (1.03 g, 2.37 mmol) in H₂O (2 mL) and toluene (20 mL) was added SPhos Pd G3 (336 mg, 431µmol) and Na₂CO₃ (457 mg, 4.31 mmol) and the reaction mixture was stirred at 80 °C for 4 h under N₂. The cooled mixture was poured into water (10 mL) and extracted with EtOAc (30 mL x 3). The combined organic layers were dried over anhydrous Na₂SO₄, filtered and concentrated under reduced pressure. The crude product was purified by HPLC (Method G, gradient: 50% to 100%B over 15 min) to give the title compound (580 mg, 27%) as a yellow solid. LCMS m/z = 981 [M+H]⁺

### Step 2: Synthesis of 6-(bis(4-methoxybenzyl)amino)-5-((1R)-1-((9S)-4-fluoro-5-(1-(4-methoxybenzyl)-6-methyl-5-(trifluoromethyl)-1H-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)pyridin-3-ol

The title compound was prepared from the title compound from Step 1 and KOH, following a similar procedure to that described in Example 15, step 2. The crude product was purified by prep HPLC (Method D, 50% to 100%B over 20 min) to give the title compound (250 mg, 53%) as a yellow solid. LCMS m/z = 962 [M+H]⁺

### Step 3: Synthesis of 5-(bromodifluoromethoxy)-3-((1R)-1-((9S)-4-fluoro-5-(1-(4-methoxybenzyl)-6-methyl-5-(trifluoromethyl)-1H-pyrazolo[3,4-b]pyridin-4-yl)-9-methyl-2-(methylthio)-8,9-dihydro-10H-7-oxa-1,3,6,10-tetraazacyclohepta[de]naphthalen-10-yl)ethyl)-N,N-bis(4-methoxybenzyl)pyridin-2-amine

The title compound was prepared from the title compound from Step 2 and dibromo(difluoro)methane, following a similar procedure to that described in Example 19, step 1. The crude product was purified by prep HPLC (Method D, 1% to 100%B over 20 min) to give the title compound (90 mg, 26%) as a yellow solid. LCMS m/z = 1092 [M+H]⁺ *Step 4: Synthesis of 3-((1R)-1-((9S)-4-fluoro-5-(1-(4-methoxybenzyl)-6-methyl-5-(trifluoromethyl)-1H-pyrazolo[3, 4-b]pyridin-4-yl)-9-methyl-2-(methylthio)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine*

The title compound was prepared from the title compound from step 3, following a similar procedure to that described in Example 19, step 2. The crude product was purified by prep HPLC (Method D, 50% to 100%B over 20 min) to give the title compound as a white solid. LCMS m/z = 910 [M+H]⁺

### Step 5: Synthesis of 3-((1R)-1-((9S)-4-fluoro-5-(1-(4-methoxybenzyl)-6-methyl-5-(trifluoromethyl)-1H-pyrazolo[3,4-b]pyridin-4-yl)-9-methyl-2-(methylsulfinyl)-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was obtained as a yellow solid, from the title compound from step 4, following the procedure described in Example 31, step 5. LCMS: m/z = 926 [M+H]⁺

### Step 6: Synthesis of 3-((1R)-1-((9S)-4-fluoro-2-(((2R,7aS)-2-fluorotetrahydro-1H-pyrrolizin-7a(5H)-yl)methoxy)-5-(1-(4-methoxybenzyl)-6-methyl-5-(trifluoromethyl)-1H-pyrazolo[3,4-b]pyridin-4-yl)-9-methyl-8,9-dihydro-10H-7-oxa-1,3,6,10-tetraazacyclohepta[de]naphthalen-10-yl)ethyl)-N-(4-methoxybenzyl)-5-(trifluoromethoxy)pyridin-2-amine

The title compound was prepared from the title compound from step 5, following a similar procedure to that described in Example 31, step 6. The crude product was purified by prep HPLC (Method C, 1% to 60% B over 20 min) to give the title compound (60 mg, 30%) as a white solid. LCMS m/z = 1012 [M+H]⁺

### Step 7: Synthesis of 3-((1R)-1-((9S)-4-fluoro-2-(((2R, 7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)-5-(trifluoromethoxy)pyridin-2-amine

The title compound was prepared from the title compound from step 6, following a similar procedure to that described in Example 31, step 7. The residue was purified by prep-HPLC (Method A, 13% to 43% B over 14.0 min) to afford the title compound (22.5 mg, 58%) as an off-white solid. LCMS m/z = 781 [M+H]⁺ 1H NMR (400 MHz, DMSO-d₆) δ 13.93 - 13.55 (m, 1H), 8.08 (d, 1H), 7.85 (d, 1H), 7.77 (d, 1H), 6.54 (s, 1H), 6.07 (s, 2H), 5.47 - 5.10 (m, 1H), 4.57 (d, 1H), 4.37 (d, 1H), 4.24 - 3.99 (m, 3H), 3.13 - 3.02 (m, 3H), 2.93 - 2.80 (m, 4H), 2.23 - 1.98 (m, 3H), 1.91 - 1.74 (m, 3H), 1.64 (d, 3H), 1.34 - 1.05 (m, 2H), 0.77 - 0.53 (m, 5H).

### Example 34. Synthesis of 5-(difluoromethoxy)-3-((1R)-1-((9S)-4-fluoro-2-(((2R,7aS)-2-fluorotetrahydro-1H-pyrrolizin-7a(5H)-yl-5,5-d2)methoxy-d2)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine. (Compound 149)

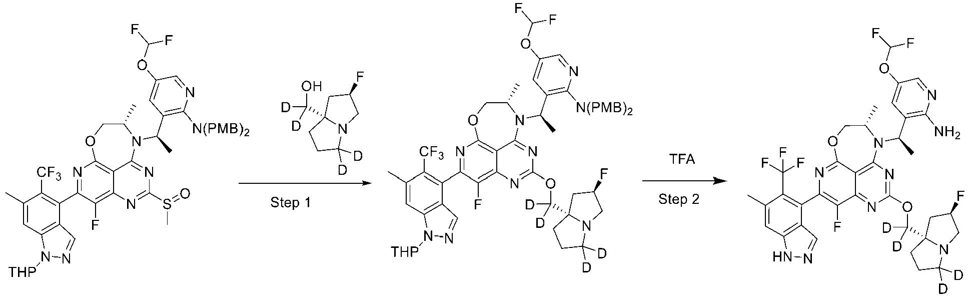

### Step 1: Synthesis of 5-(difluoromethoxy)-3-((1R)-1-((9S)-4-fluoro-2-(((2R,7aS)-2-fluorotetrahydro-1H-pyrrolizin-7a(5H)-yl-5,5-d2)methoxy-d2)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

t-BuONa (2.83 mL, 5.66 mmol) was added dropwise at 0°C to a solution of Example 14, step 4 (850 mg, 0.86 mmol) and Intermediate 27 (214 mg, 1.3 mmol) in DCM (150 mL) and the solution was stirred at 0°C for 30 mins. The reaction was quenched with ice water (100 mL) at 0°C and the resulting mixture was extracted with DCM (2 x 100 mL). The combined organic layers were washed with brine (100 mL), dried over anhydrous Na₂SO₄, filtered and the filtrate was concentrated under reduced pressure. The residue was purified by Prep-TLC (DCM / MeOH, 20:1) to afford the title compound (310 mg, 33%) as a yellow solid. LCMS m/z = 1090 [M+H]⁺

### Step 2: Synthesis of 5-(difluoromethoxy)-3-((1R)-1-((9S)-4-fluoro-2-(((2R,7aS)-2-fluorotetrahydro-1H-pyrrolizin-7a(5H)-yl-5,5-d2)methoxy-d2)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine

TFA (12 mL) was added to a solution of the title compound from step 1 (310 mg, 0.28 mmol) in DCE (12 mL) and the reaction mixture was stirred at 80°C for 2.5 h. The resulting mixture was concentrated under reduced pressure. The residue was purified by Prep-HPLC (Column: Xselect CSH C18 OBD Column 30*150mm 5µm; Mobile Phase A: Water (0.1% FA), Mobile Phase B: MeCN; Flow rate: 60 mL/min; Gradient (B%): 15% to 45% B in 7 min) to afford the title compound (110.3 mg, 51%) as a white solid. LCMS m/z = 766 [M+H]⁺ 1H NMR (300 MHz, DMSO-d₆) δ 13.51 (d, 1H), 7.94 (d, 1H), 7.83 - 7.62 (m, 3H), 7.10 (t, 1H), 6.54 (d, 1H), 5.82 (d, 2H), 5.41 (d, 1H), 4.56 (dt, 1H), 4.34 (t, 1H), 4.22 - 4.06 (m, 1H), 2.64 (dd, 4H), 2.23 (m, 4H), 1.93 (m, 3H), 1.62 (d, 3H), 0.63 (dd, 3H).

### Example 35: Synthesis of 5-(difluoromethoxy)-3-((1R)-1-((9S)-4-fluoro-2-(((2R,7aS)-2-fluorotetrahydro-1H-pyrrolizin-7a(5H)-yl-2,5,5-d3)methoxy-d2)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine (Compound 152)

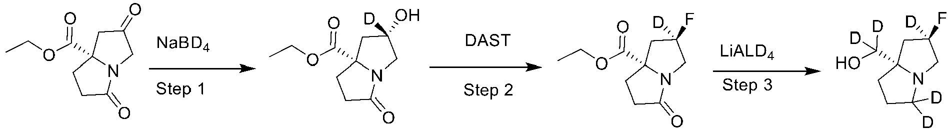

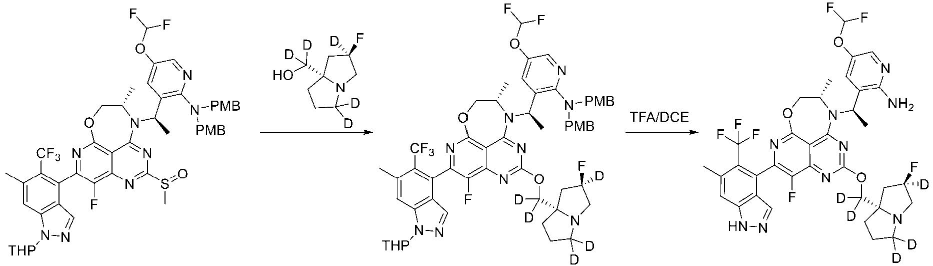

### Step 1: Synthesis of ethyl (2S,7aS)-2-hydroxy-5-oxotetrahydro-1H-pyrrolizine-7a(5H)-carboxylate-2-d

To a stirred solution of ethyl (S)-2,5-dioxotetrahydro-1H-pyrrolizine-7a(5H)-carboxylate (6 g, 28.4 mmol) in EtOH (50 mL) was added NaBD₄ (0.437 g, 10.51 mmol) portion wise at 0 °C under N₂ and the reaction mixture was stirred at 0°C for 30 min. The reaction mixture was quenched with ice cold water (5 mL) and evaporated under reduced pressure. The residue was purified by prep-HPLC (100G c18 GOLD column, Mobile Phase A: 10mM NH₄HCO₃ in water, Mobile Phase B: MeCN, @25 mL/min, Gradient: 10% to 45%B) to afford the title compound (2 g, 32.9%) as an off white semi-solid and Peak-2, ethyl (2R,7aS)-2-hydroxy-5-oxotetrahydro-1H-pyrrolizine-7a(5H)-carboxylate-2-d (0.9 g, 14.8%) as an off white solid. LCMS m/z = 215 [M+H]⁺

### Step 2: Synthesis of ethyl (2R,7aS)-2-fluoro-5-oxotetrahydro-1H-pyrrolizine-7a(5H)-carboxylate-2-d

To a stirred solution of the title compound from step 1, Peak 1 (1.9 g, 8.6 mmol) in DCM (50 mL) was added DAST (3.5 mL, 26 mmol) drop wise at -15°C under N₂ and the reaction mixture was stirred at 10°C for 3 h. The reaction mixture was quenched with MeOH (4 mL) and diluted with water (50 mL). The mixture was extracted with DCM (2 x 50 mL), the combined organic layers were washed with water (50 mL), brine (30 mL), dried over Na₂SO₄, filtered and the filtrate was evaporated under reduced pressure. The residue was purified by column chromatography on silica gel, eluting with 2% of MeOH/ DCM to afford the title compound (1.45 g, 68%) as a pale yellow liquid. LCMS m/z = 217 [M+H]⁺

### Step 3: Synthesis of ((2R, 7aS)-2-fluorotetrahydro-1H-pyrrolizin-7a(5H)-yl-2,5,5-d3)methan-d2-ol

To a stirred solution of the title compound from step 2 (1 g, 4.02 mmol) in dry THF (20 mL) was added LiAlD₄ (0.52 g, 12.07 mmol) portion wise at rt under N₂ and the reaction mixture was stirred at 80°C for 2 h. The reaction mixture was cooled to 10°C, slowly quenched with MeOD (2 mL), the mixture was stirred for 10 min, then was diluted with THF (50 mL) and filtered. The filtrate was evaporated under reduced pressure and the crude product was co-distilled with toluene (2 x 5 mL) to give the title compound (550 mg, 71%).

### Step 4: Synthesis of 5-(difluoromethoxy)-3-((1R)-1-((9S)-4-fluoro-2-(((2R,7aS)-2-fluorotetrahydro-1H-pyrrolizin-7a(5H)-yl-2,5,5-d3)methoxy-d2)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

To a stirred solution of Example 14, step 4 (800 mg, 0.80 mmol) in DCM (10 mL) was added the title compound from step 3 (0.46 g, 2.42 mmol), followed by NaOtBu (2 M in THF, 1.21 mL, 2.42 mmol) drop wise at 0°C under N₂ and the reaction mixture was stirred at this temperature for 30 min. The reaction mixture was quenched with ice cold water (10 mL) and extracted with DCM (3 x 20 mL). The combined organic layers were dried over Na₂SO₄, filtered and the filtrate was evaporated under reduced pressure. The residue was purified by column chromatography on silica gel eluting with 2% MeOH in DCM to afford the title compound (500 mg, 52.8%) as a pale yellow sold. 1H NMR (400 MHz, DMSO-d₆) δ = 8.43 - 8.34 (m, 1H), 8.05 (d, 1H), 7.04 (q, 1H), 6.67 (d, 4H), 6.55 (d, 4H), 5.33 - 5.11 (m, 1H), 4.51 - 4.41 (m, 1H), 4.28 - 4.16 (m, 4H), 4.10 - 4.05 (m, 1H), 4.02 - 3.94 (m, 1H), 3.83 (d, 2H), 3.67 - 3.63 (m, 6H), 3.17 (d, 1H), 3.09 - 3.03 (m, 2H), 2.83 - 2.75 (m, 1H), 2.06 - 1.98 (m, 2H), 1.92 - 1.65 (m, 4H), 1.44 (d, 3H), -0.04 (d, 3H).

### Step 5: Synthesis of 5-(difluoromethoxy)-3-((1R)-1-((9S)-4-fluoro-2-(((2R, 7aS)-2-fluorotetrahydro-1H-pyrrolizin-7a(5H)-yl-2,5,5-d3)methoxy-d2)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine

To a stirred solution of the title compound from step 4 (1.85 g, 1.67 mmol) in DCE (20 mL) in a sealed tube, was added TFA (20 mL) and the reaction mixture was stirred at 85°C for 3 h. The reaction mixture was evaporated under reduced pressure and the residue purified by HPLC (275 g c18 gold column, Mobile Phase A:10mM NH₄HCO₃ in Water, Mobile Phase B: MeCN, @ 50 mL/min, Gradient 10% to 98% B) to afford the title compound as an off-white solid (500 mg, 38.5%). 1H NMR (400 MHz, DMSO-d₆) δ 13.48 (d, 1H), 7.93 (s, 1H), 7.71 (t, 2H), 7.62 (s, 1H), 7.10 (t, 1H), 6.54 (t, 1H), 5.85 (s, 1H), 5.76 (s, 1H), 4.57 - 4.50 (m, 1H), 4.38 - 4.30 (m, 1H), 4.12 - 4.08 (m, 1H), 3.13 - 3.01 (m, 2H), 2.65 - 2.63 (m, 3H), 2.19 - 2.02 (m, 3H), 1.85 - 1.78 (m, 3H), 1.62 (d, 3H), 0.62 (dd, 3H).

### Example 36. Synthesis of 3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-isopropoxypyridin-2-amine formate. (Compound 150)

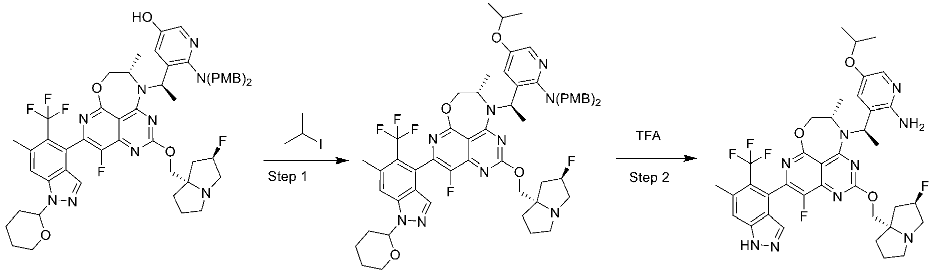

### Step 1: Synthesis of 3-((1R)-1-((9S)-4-fluoro-2-(((2R, 7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-5-isopropoxy-N,N-bis(4-methoxybenzyl)pyridin-2-amine

To a solution of Intermediate 28 (50 mg, 48.3 µmol) and Cs₂CO₃ (47.2 mg, 144.8 µmol) in DMF (0.7 mL) was added 2-iodopropane (20.5 mg, 120.7 µmol) and the reaction mixture was stirred at 50 °C for 3 h. The mixture was filtered and the filtrate purified by prep HPLC (Method E, 35% - 50%B, in 20 min) to give the title compound as pale yellow solid. LCMS m/z = 1079 [M+H]⁺

### Step 2: Synthesis of -fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-isopropoxypyridin-2-amine formate

A solution of the title compound from step 1 (40 mg, 37.1 µmol) in DCE (0.8 mL) and TFA (1.23 g, 10.8 mmol) was stirred at 80°C for 2 h. The solution was concentrated and the residue was purified by prep-HPLC (Method A, 13%-43% B over 14.0 min) then lyophilized, to give the title compound (18.8 mg, 63 %) as a white solid. LCMS m/z = 754 [M+H]⁺ 1H NMR (400 MHz, DMSO-d₆) δ = 13.50 (d, 1H), 8.14 (s, 1HCO₂H), 7.75 - 7.71 (m, 1H), 7.43 - 7.41 (m, 1H), 6.56 - 6.53 (m, 1H), 5.38 - 5.25 (m, 3H), 4.55 - 4.49 (m, 2H), 4.37 - 4.32 (m, 1H), 4.17 - 4.11 (m, 3H), 3.18 - 3.06 (m, 3H), 2.87 - 2.85 (m, 1H), 2.66 - 2.63 (m, 3H), 2.19 - 2.05 (m, 3H), 1.89 - 1.81 (m, 3H), 1.62 (d, 3H), 1.27 - 1.25 (m, 6H), 0.61 (dd, 3H).

### Example 37. Synthesis of 2-((6-amino-5-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-lH-indazol-4-yl)-8,9-dihydro-10H-7-oxa-1,3,6,10-tetraazacyclohepta[de]naphthalen-10-yl)ethyl)pyridin-3-yl)oxy)acetonitrile. Compound 153

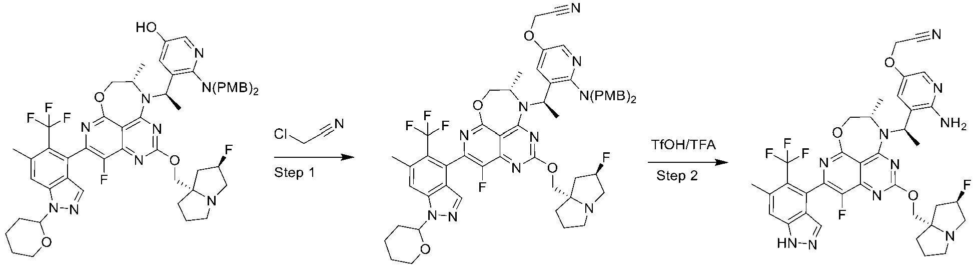

### Step 1: Synthesis of 2-((6-(bis(4-methoxybenzyl)amino)-5-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)pyridin-3-yl)oxy)acetonitrile

To a solution of Intermediate 28 (50 mg, 48.3 µmol) and Cs₂CO₃ (47.2 mg, 144.8 µmol) in DMF (0.7 mL) was added 2-chloroacetonitrile (9.11 mg, 120.7 µmol) and NaI (1.45 mg, 9.65 µmol) and the reaction mixture was stirred at 70 °C for 2 h. The mixture was filtered and the filtrate was purified by prep HPLC (35% - 50%B, in 20 min) to give the title compound (35 mg, 67%) as white solid. LCMS: *m*/*z* = 1076 [M+H]⁺

### Step 2: Synthesis of 2-((6-amino-5-((1R)-1-((9S)-4-fluoro-2-(((2R, 7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-3-yl)oxy)acetonitrile

A solution of the title compound from step 1 (25 mg, 23.25 µmol) in TFA (0.5 mL) and TfOH (84.8 mg, 565 µmol) was stirred at 15°C for 30 min. The solution was concentrated *in vacuo,* the residue was diluted with DCM (3 mL) and then adjusted to pH 7~8 with NH₃.H₂O solution. The solution was concentrated *in vacuo* and the residue was purified by prep-HPLC (Method A, 14% - 44% B over 14.0 min). The product was further purified by prep-HPLC (Method F, gradient: 35%-65% MeCN over 9.0 min) to give the title compound (6.4 mg, 37%) as a white solid. LCMS m/z = 751 [M+H]⁺ 1H NMR (400 MHz, DMSO-d₆) δ = 13.57 - 13.44 (m, 1H), 7.90 (d, 1H), 7.73 - 7.71 (m, 2H), 7.59 - 7.57 (m, 1H), 6.57 - 6.54 (m, 1H), 5.59 - 5.51 (m, 2H), 5.38 - 5.22 (m, 1H), 5.17 (s, 2H), 4.54 - 4.50 (m, 1H), 4.40 - 4.35 (m, 1H), 4.15 - 4.10 (m, 3H), 3.12 - 3.02 (m, 3H), 2.85 - 2.80 (m, 1H), 2.68 - 2.64 (m, 3H), 2.15 - 2.03 (m, 3H), 1.84 - 1.79 (m, 3H), 1.63 (d, 3H), 0.61 (dd, 3H).

### Example 38. Synthesis of 3-((1R)-1-((9S)-4-fluoro-2-(((2R,7aS)-2-fluorotetrahydro-1H-pyrrolizin-7a(SH)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-methoxypyridin-2-amine. (Compound 154)

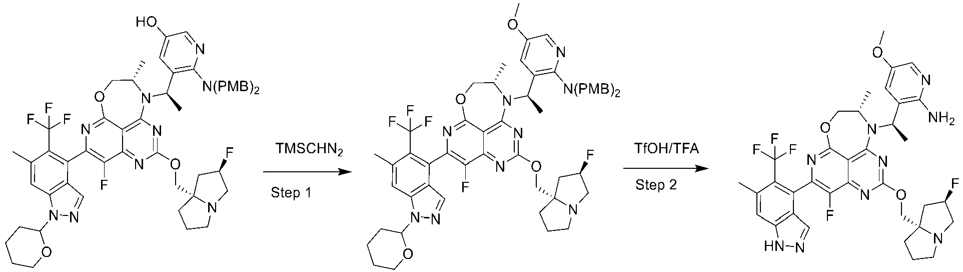

### Step 1: Synthesis of 3-((1R)-1-((9S)-4-fluoro-2-(((2R, 7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-5-methoxy-N,N-bis(4-methoxybenzyl)pyridin-2-amine

To a solution of Intermediate 28 (50 mg, 48.3 µmol) in DCM (1 mL) and MeOH (0.5 mL) was added TMSCHN₂ (2 M, 72.4 µL) and the mixture was stirred at 15°C for 16 h. TMSCHN₂ (2 M, 72.4 µL) was added and the reaction mixture stirred at 30°C for a further 2 h. Nitrogen was blown through the solution to give a residue. The residue was purified by prep HPLC (Method E, 35% - 50%B, in 20 min) to give the title compound (35 mg, 69%) as pale yellow solid. LCMS: *m*/*z* = 1051 [M+H]⁺

### Step 2: Synthesis of 3-((1R)-]-((9S)-4-fluoro-2-(((2R, 7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-methoxypyridin-2-amine

The title compound was obtained as a white solid, 9.1 mg, 53%, from the title compound from step 1, following a similar method to that described in Example 37, step 2. LCMS m/z = 726 [M+H]⁺ 1H NMR (400 MHz, DMSO-d₆) δ = 13.50 (d, 1H), 7.78 (d, 1H), 7.74 - 7.70 (m, 2H), 7.43 - 7.41 (m, 1H), 6.56 - 6.52 (m, 1H), 5.36 - 5.21 (m, 3H), 4.56 - 4.52 (m, 1H), 4.38 - 4.30 (m, 1H), 4.15 - 4.10 (m, 3H), 3.80 (s, 3H), 3.11 - 3.02 (m, 3H), 2.86 - 2.82 (m, 1H), 2.65 - 2.64 (m, 3H), 2.17 - 2.03 (m, 3H), 1.86 - 1.78 (m, 3H), 1.62 (d, 3H), 0.61 (dd, 3H).

### Example 39. Synthesis of 5-cyclopropoxy-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine (Compound 155)

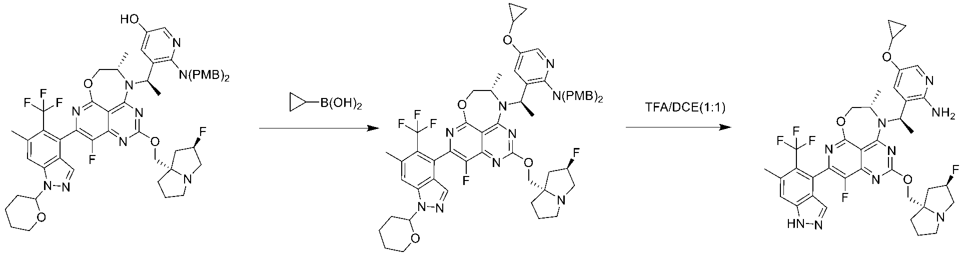

### Step 1: Synthesis of 5-cyclopropoxy-3-((1R)-1-((9S)-4-fluoro-2-(((2R,7aS)-2-fluorotetrahydro-1H-pyrrolizin-7a(5H)-yl)methoxy)-9-methyl-5-(6-methyl-1-(tetrahydro-2H-pyran-2-yl)-5-(trifluoromethyl)-1H-indazol-4-yl)-8,9-dihydro-10H-7-oxa-1,3,6,10-tetraazacyclohepta[de]naphthalen-10-yl)ethyl)-N,N-bis(4-methoxybenzyl)pyridin-2-amine

To a solution of Intermediate 28 (100 mg, 96.5 µmol), cyclopropylboronic acid (41.45 mg, 482.6 µmol) and di-µ-hydroxo-bis-[(N,N,N',N'-tetramethylethylenediamine)copper(II)]chloride (13.45 mg, 29 µmol) in DCM (4 mL) was added K₂CO₃ (40.0 mg, 290 µmol) and the reaction mixture was stirred at 40 °C for 16 h under an O₂. The mixture was filtrated and the filtrate was concentrated. The residue was purified by prep HPLC (Method E, 35% - 50B%, 20 min) and prep-HPLC (Method A, 35% - 65% B over 12.0 min) to give the title compound (30 mg, 7.51%) as a pale yellow solid. LCMS: m/z = 1077 [M+H]⁺.

### Step 2: Synthesis of 5-cyclopropoxy-3-((1R)-1-((9S)-4-fluoro-2-(((2R, 7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine

The title compound was prepared from the title compound from step 1, following a similar procedure to that described in Example 36, step 2. The crude product was further purified by prep HPLC (Method F, 38% - 68% B over 9.0 min) to give the title compound (1.57 mg, 35%) as off-white solid. LCMS: *m*/*z* = 752 [M+H]^{+ 1}H NMR (400 MHz, DMSO-*d*₆) δ = 13.58 - 13.46 (m, 1H), 7.91 (d, 1H), 7.75 - 7.71 (m, 2H), 7.46 - 7.45 (m, 1H), 6.58 - 6.53 (m, 1H), 5.36 - 5.22 (m, 3H), 4.59 - 4.52 (m, 2H), 4.37 - 4.32 (m, 1H), 4.12 - 4.06 (m, 2H), 3.91 - 3.90 (m, 1H), 3.11 - 3.02 (m, 3H), 2.84 - 2.82 (m, 1H), 2.65 - 2.63 (m, 3H), 2.16 - 2.02 (m, 3H), 1.86 - 1.79 (m, 3H), 1.61 (d, 3H), 0.77 - 0.75 (m, 2H), 0.68 - 0.57 (m, 5H).

### Example 40. Synthesis of (2R,4S,7aS)-7a-((((9S)-10-((R)-1-(2-amino-5-(difluoromethoxy)pyridin-3-yl)ethyl)-4-fluoro-9-methyl-5-(6-methyl-5-(trifluoromethyl)-1H-indazol-4-yl)-9,10-dihydro-8H-7-oxa-1,3,6,10-tetraazacyclohepta[de]naphthalen-2-yl)oxy)methyl)-2-fluorohexahydropyrrolizine 4(1H)-oxide (Compound No: 170) and

### Example 41. Synthesis of (2R,4R,7aS)-7a-((((9S)-10-((R)-1-(2-amino-5-(difluoromethoxy)pyridin-3-yl)ethyl)-4-fluoro-9-methyl-5-(6-methyl-5-(trifluoromethyl)-1H-indazol-4-yl)-9,10-dihydro-8H-7-oxa-1,3,6,10-tetraazacyclohepta[de]naphthalen-2-yl)oxy)methyl)-2-fluorohexahydropyrrolizine 4(1H)-oxide (Compound No: 171)

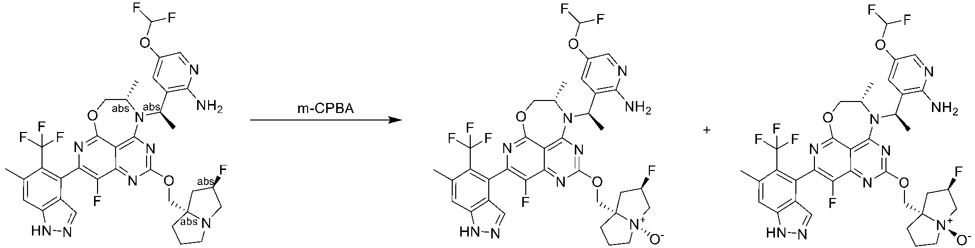

To a solution of 5-(difluoromethoxy)-3-[(1R)-1-[(12S)-6-fluoro-3-[[(2R,8S)-2-fluoro-1,2,3,5,6,7-hexahydropyrrolizin-8-yl]methoxy]-12-methyl-7-[6-methyl-5-(trifluoromethyl)-1H-indazol-4-yl]-10-oxa-2,4,8,13-tetrazatricyclo[7.4.1.0^{5,14}]tetradeca-1 (14),2,4,6,8-pentaen-13-yl]ethyl]pyridin-2-amine (30 mg, 39.39 µ mol) in DCM (1 mL) was added m-CPBA (9.60 mg, 47.26 1 mol, 85% purity). The mixture was stirred at 25 ° C for 1 hour. The reaction mixture was concentrated under reduced pressure (≤ 30 ° C) to give a residue. The residue was purified by prep-HPLC (column: Waters Xbridge C18 150 * 25 mm * 5 um; mobile phase: [H₂O (10 mM NH₄HCO₃)-ACN]; gradient: 13% - 43% B over 15.0 min) to give 5-(difluoromethoxy)-3-[(1R)-1-[(12S)-6-fluoro-3-[[(2R,8S)-2-fluoro-4-oxido-1,2,3,5,6,7-hexahydropyrrolizin-4-ium-8-yl]methoxy]-12-methyl-7-[6-methyl-5-(trifluoromethyl)-1H-indazol-4-yl]-10-oxa-2,4,8,13-tetrazatricyclo[7.4.1.0^{5,14}]tetradeca-1(14),2,4,6,8-pentaen-13-yl]ethyl]pyridin-2-amine (8.36 mg, 10.46 µmol, 27% yield) as a gray solid and 5-(difluoromethoxy)-3-[(1R)-1-[(12S)-6-fluoro-3-[[(2R,8S)-2-fluoro-4-oxido-1,2,3,5,6,7-hexahydropyrrolizin-4-ium-8-yl]methoxy]-12-methyl-7-[6-methyl-5-(trifluoromethyl)-1H-indazol-4-yl]-10-oxa-2,4,8,13-tetrazatricyclo[7.4.1.0^{5,14}]tetradeca-1(14),2,4,6,8-pentaen-13-yl]ethyl]-1-oxido-pyridin-1-ium-2-amine (7.69 mg, 9.69 µmol, 25% yield) as a white solid. LCMS: m/z ESI [M+1]⁺ = 778.3 ¹H NMR (400 MHz, DMSO-d₆) δ 13.65 - 13.55 (m, 1H), 7.93 (d, *J* = 2.4 Hz, 1H), 7.73 - 7.70 (m, 2H), 7.64 - 7.63 (m, 1H), 7.11 (t, *J =* 74.8 Hz, 1H), 6.61 - 6.53 (m, 1H), 5.97 - 5.85 (m, 2H), 5.57 - 5.43 (m, 1H), 5.00 - 4.93 (m, 1H), 4.57 - 4.50 (m, 2H), 4.40 - 4.33 (m, 1H), 4.18 - 4.00 (m, 2H), 3.85 - 3.65 (m, 2H), 3.46 - 3.42 (m, 1H), 2.89 - 2.86 (m, 1H), 2.68 - 2.63 (m, 3H), 2.35 - 2.06 (m, 5H), 1.64 - 1.61 (m, 3H), 0.67 - 0.59 (m, 3H).

### Example 42: KRAS G12D SOS1-mediated TR-FRET Assay

The ability of compounds to inhibit SOS1-mediated nucleotide exchange was measured using a TR-FRET assay. In a 10-µl final assay volume, 5 µL of GST-tagged KRAS G12D and 1 nM anti-GST-Tb were added to pre-dispensed compounds. The assay buffer consisted of 10 mM HEPES, pH 7.4, 150 mM NaCl, 0.05% BSA, 0.0025% Igepal and 5 mM MgCl2. Then, 5 µL of SOS1 and 10 nM EDA-GTP-DY-647P1 were added to initiate the nucleotide exchange reaction. After a 60-minute incubation at room temperature, the reaction was measured in a plate reader using excitation and emission wavelengths of 340 nm and 620/665 nm, respectively. IC₅₀ values were established using a four-parameter fit model. The results are shown in Table 2, where A is ≤ 10 nM; B is > 10 nM and ≤ 100 nM; C is > 100 nM and ≤ 1000 nM; and D is > 1000 nM.

**Table 2.**

| Comp. No. | KRAS-G12D- SOS-NEA | KRAS-G12V- SOS-NEA | KRAS-G12C- SOS-NEA | KRAS-WT-SOS-NEA | KRAS-G12R- SOS-NEA |
|---|---|---|---|---|---|
| 101 | A | A | A | A | |
| 102 | A | A | A | | |
| 103 | A | A | A | | |
| 104 | A | A | A | | |
| 105 | A | A | A | | |
| 106 | A | A | A | A | |
| 107 | A | A | | | |
| 108 | A | A | A | A | |
| 116 | A | | A | A | A |
| 134 | A | | A | A | A |
| 124 | A | | | A | |
| 121 | A | | | A | |
| 113 | A | A | | | |
| 115 | A | | | A | |
| 114 | A | A | | | |
| 123 | A | A | A | A | |
| 122 | A | A | A | A | |
| 125 | A | | | A | |
| 110 | A | A | A | A | |
| 127 | A | A | A | A | |
| 128 | A | A | A | A | |
| 129 | A | A | | | |
| 120 | A | A | A | | |
| 132 | A | A | A | | |
| 135 | A | | | | |
| 136 | A | | | | |
| 137 | A | | | | |
| 138 | A | | | | |
| 139 | A | | | | |
| 140 | B | | | | |
| 141 | A | | | | |

### Example 43: Active KRAS G12D / CRAF RBD TR-FRET Binding Assay

The ability of compounds to inhibit activation of KRAS G12D (GMPPNP loaded) and its interaction with effector protein, CRAF, was measured using a TR-FRET assay. In a 10-µl final assay volume, 2.5 µL of biotinylated KRAS G12D and 2.5 µL FLAG-tagged CRAF-RBD were added to pre-dispensed compounds. The assay buffer consisted of 25 mM HEPES, pH 7.4, 0.002% Tween20, 0.1% BSA, 100 mM NaCl and 5 mM MgCl₂. Then, 1 nM Tb-anti-FLAG and 5 nM SA-XL665 were added to detect the interaction. After a 30-minute incubation at room temperature, the reaction was measured in a plate reader using excitation and emission wavelengths of 340 nm and 620/665 nm, respectively. IC50 values were established using a four-parameter fit model. The results are shown in Table 3, where A is ≤ 10 nM; B is > 10 nM and ≤ 100 nM; C is > 100 nM and ≤ 1000 nM; and D is > 1000 nM.

**Table 3.**

| Comp. No. | KRAS-G12D-GMPPNP-RAF | KRAS-G12V-GMPPNP-RAF | KRAS-G12C-GMPPNP-RAF | KRAS-WT-GMPPNP-RAF |
|---|---|---|---|---|
| 101 | B | C | D | |
| 102 | A | B | B | |
| 103 | | | | |
| 104 | A | B | | |
| 105 | B | B | B | |
| 106 | B | C | C | |
| 107 | B | C | C | |
| 108 | B | C | C | |
| 116 | B | | | C |
| 134 | B | | | C |
| 124 | B | | | |
| 121 | B | | | |
| 113 | B | C | | |
| 115 | B | | | |
| 114 | B | C | | |
| 123 | A | B | B | |
| 122 | B | C | C | |
| 125 | C | | | |
| 110 | B | C | C | |
| 127 | C | C | D | |
| 128 | C | C | D | |
| 129 | C | D | | |
| 135 | C | | | |
| 136 | C | | | |
| 137 | C | | | |
| 138 | C | | | |
| 139 | D | | | |
| 140 | D | | | |
| 141 | D | | | |

### Example 44: in vitro pERK HTRF Assay

The potency of test compounds in inhibiting phosphorylation of ERK was determined by pERK HTRF assay in human cancer cells (GP2D KRAS G12D-driven CRC model) expressing either mutant or wild-type KRAS. Cells (8000 cells per well) were plated in 384-well cell culture plates. After overnight incubation at 37 °C and 5% CO2, cells were treated with a 3-fold, 10-point dilution of indicated compounds or DMSO (0.1% DMSO final) for 3 hours. Cells were then lysed, and the lysates transferred to assay plates and incubated with phospho-ERK HTRF detection reagents according to the manufacturer's protocol (HTRF Human & Mouse Phospho-ERK (Thr202/Ty204) Detection Kit; Revvity Health Sciences). The HTRF signal was measured using a BMG PHERAstar FSX multimode plate reader. Curves were fitted and IC50 and IC90 values calculated using a four-parameter logistic model (XLFit; equation 201). The results are shown in Table 4 and Table 4a, where A is ≤ 10 nM; B is > 10 nM and ≤ 100 nM; C is > 100 nM and ≤ 1000 nM; and D is > 1000 nM. Table 4 represents IC50 values and Table 4a represents IC90 values.

**Table 4.**

| Comp. No. | GP2D | SW620 | NCIH358 | KE39 | A375 |
|---|---|---|---|---|---|
| 101 | A | A | A | A | D |
| 102 | A | A | A | A | D |
| 103 | A | A | A | A | D |
| 104 | A | A | A | A | D |
| 105 | A | A | A | A | D |
| 106 | A | A | A | A | D |
| 107 | A | A | A | A | D |
| 108 | A | A | A | A | D |
| 134 | A | A | | | |
| 124 | A | | A | A | D |
| 121 | A | A | A | A | D |
| 113 | A | A | A | A | D |
| 115 | A | A | A | A | D |
| 114 | A | A | A | A | D |
| 123 | A | A | A | A | D |
| 122 | A | A | A | A | D |
| 125 | A | A | A | B | D |
| 126 | B | B | A | B | D |
| 110 | A | A | A | A | D |
| 127 | A | A | A | A | D |
| 128 | A | A | | | |
| 129 | A | A | A | B | D |
| 130 | A | A | A | A | D |
| 131 | B | A | | | |
| 120 | A | A | A | A | D |
| 132 | A | A | A | A | D |
| 133 | A | A | A | A | D |
| 119 | B | C | | | |
| 116 | A | A | | | |
| 135 | A | | | | |
| 136 | A | | | | |
| 137 | A | | | | |
| 138 | A | | | | |
| 139 | A | | | | |
| 140 | B | | | | |
| 141 | A | | | | |
| 142 | A | A | A | | |
| 143 | A | A | | | |
| 144 | A | A | | | |
| 145 | A | A | A | | |
| 146 | A | A | | | |
| 147 | A | A | A | | |
| 148 | A | A | A | | |
| 150 | A | A | A | | |
| 151 | A | A | A | | |
| 152 | A | A | A | | |
| 153 | A | A | A | | |
| 154 | A | A | A | | |
| 155 | A | A | A | | |
| 109 | A | A | A | | |
| 160 | A | A | A | | |
| 161 | A | A | A | | |
| 170 | A | A | B | | |
| 171 | A | A | A | | |

**Table 4a.**

| Parent ID | GP2D |
|---|---|
| 141 | 37.9 |
| 134 | 1.37 |
| 116 | 2.65 |
| 135 | 0.85 |
| 136 | 8.8 |
| 137 | 3.75 |
| 138 | 27.1 |
| 139 | 12.7 |
| 140 | 90.8 |
| 101 | 0.90 |
| 106 | 0.51 |
| 160 | 0.44 |
| 161 | 1.00 |
| 109 | 0.85 |
| Reference Compound | 0.89 |

The reference compound is a compound having the following structure: 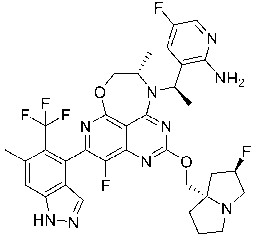 (See e.g., WO2024/235286).

### Example 45: Cell Proliferation Assay

The anti-proliferative activity of compounds was assessed by CellTiter-Glo^{®} Luminescent Cell Viability Assay (CTG; Promega Corp.) in human cancer cell lines expressing either mutant or wild-type KRAS. Cells were seeded into 384-well plates at densities of 500-2000 cells per well in complete growth medium. After overnight incubation at 37 °C and 5% CO2, cells were treated with a 3-fold, 10-point dilution of indicated compounds or DMSO (0.1% DMSO final) and cell viability was assessed 3 days post-treatment according to the manufacturer's protocol. The luminescent signal was measured using an EnVision multimode plate reader. Curves were fitted and IC50 values calculated using a four-parameter logistic model (XLFit; equation 201). The results are shown in Table 5, where A is ≤ 10 nM; B is > 10 nM and ≤ 100 nM; C is > 100 nM and ≤ 1000 nM; and D is > 1000 nM.

**Table 5.**

| Comp. No. | GP2D | SW620 | NCIH358 | KE39 | A375 |
|---|---|---|---|---|---|
| 101 | A | A | A | A | D |
| 102 | A | A | A | A | D |
| 103 | A | A | A | A | D |
| 104 | A | A | A | A | D |
| 105 | A | A | A | A | D |
| 106 | A | A | A | A | D |
| 107 | A | A | A | A | D |
| 108 | A | A | A | A | D |
| 111 | A | A | A | | |
| 113 | A | A | A | | |
| 114 | A | A | A | | |
| 115 | A | A | A | | |
| 116 | A | A | A | | |
| 117 | A | A | A | | |
| 118 | A | A | A | | |
| 124 | A | A | A | | |
| 130 | A | A | A | | |
| 132 | A | A | A | | |
| 133 | A | A | A | | |
| 134 | A | A | A | | |
| 135 | A | | A | | |
| 145 | A | A | A | | |
| 146 | A | A | A | | |

### Example 46: SPR Assay

SPR measurements were performed using a Biacore 8K equipped with CAP sensor chips (Cytiva). Avi-tagged KRAS4b^{G12D}-(1-169) was biotinylated, loaded with either GDP or GMPPNP, and immobilized to the sensor chip using standard biotin-capture procedures. The resulting immobilization levels ranged from 500 to 700 resonance units with estimated surface activity of ~100%. Interactions with KRAS were analyzed in 9-point or 11-point dose response using twofold serial dilutions. The top dose of the dilution series was adjusted from 10 µM to 200 nM depending on the anticipated potency range of the compound. The running buffer for all experiments was 20 mM HEPES pH8, 50 mM NaCl, 2mM MgCl₂, 1 mM DTT, 0.05 % Tween, 2%DMSO. For binding experiments run in multicycle kinetic mode, the association phase was 60 s and the dissociation phase was 300 s. For experiments using single cycle kinetic mode, the contact time was 90 s and the dissociation phase was 1200 s. After each cycle, needles were washed with 50% DMSO. For data analyses, the data were fit using a 1:1 kinetic model after reference signal subtraction using Biacore 8K evaluation software. The results are shown in Table 6, where A is ≤ 10 nM; B is > 10 nM and ≤ 100 nM; C is > 100 nM and ≤ 1000 nM; and D is > 1000 nM.

**Table 6.**

| Comp. No. | KRAS-G12D-GDP | KRAS-G12D-GMPPNP | KRAS-G12V-GMPPNP |
|---|---|---|---|
| 101 | A | | |
| 102 | A | B | C |
| 103 | A | B | D |
| 104 | A | B | C |
| 105 | A | B | B |
| 106 | A | C | D |
| 107 | A | C | D |

### Example 47: 2D Cell Viability Assay

The anti-proliferative activity of compounds was assessed by a CellTiter-^{®} Luminescent Cell Viability Assay (CTG; Promega Corp.) in human cancer cell lines expressing mutant KRAS. Cells were seeded into 384-well plates at densities of 1000-2000 cells per well in complete growth medium. After overnight incubation at 37 °C and 5% CO2, cells were treated with a 3-fold, 10-point dilution of indicated compounds or DMSO (0.1% DMSO final) and cell viability was assessed 3 days post-treatment according to the manufacturer's protocol. The luminescent signal was measured using an EnVision multimode plate reader. Curves were fitted and IC90 values calculated using a four-parameter logistic model (XLFit; equation 201) (see Table 7 and Table 7a).

The reference compound is a compound having the following structure: 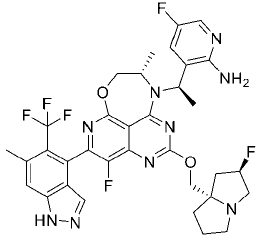 (See e.g., WO2024/235286).

**Table 7.**

| Comp. No. | 2D CTG SW620 | 2D CTG GP2D | 2D CTG KE39 | 2D CTG NCIH358 |
|---|---|---|---|---|
| | IC90 (nM) | IC90 (nM) | IC90 (nM) | IC90 (nM) |
| 101 | 2.8 | 14.9 | 2.7 | 29.2 |
| 102 | 0.8 | 2.6 | 0.5 | 7.1 |
| 103 | 7.0 | 16.5 | 4.8 | 15.0 |
| 106 | 1.6 | 4.2 | 1.7 | 10.8 |
| 110 | 14.8 | 60.7 | 10.5 | 55.3 |
| 113 | 5.0 | 15.3 | | 28.9 |
| 114 | 17.6 | 63.5 | 35.5 | 78.0 |
| 115 | 0.7 | 1.4 | 0.8 | 7.7 |
| 117 | 3.7 | 31.4 | | |
| 118 | 9.2 | 42.5 | | |
| 119 | >1000 | 271.0 | | |
| 120 | 4.3 | 23.2 | 3.1 | 110.4 |
| 121 | 9.7 | 42.2 | 5.9 | 37.9 |
| 122 | 4.5 | 15.9 | 2.2 | 12.0 |
| 123 | 0.8 | 1.5 | 0.4 | 2.2 |
| 125 | 83.7 | 175.0 | 54.2 | 321.0 |
| 124 | 2.0 | 10.0 | 1.9 | 55.8 |
| 126 | 113.0 | 485.0 | 113.0 | 621.0 |
| 127 | 23.2 | 89.2 | 7.1 | 65.8 |
| 128 | 39.1 | 62.7 | | |
| 129 | 85.3 | 280.0 | 189.0 | 541.0 |
| 130 | 5.4 | 15.1 | 10.2 | 43.2 |
| 131 | 214.0 | 340.0 | | |
| 132 | 12.4 | 72.1 | 25.2 | 215.0 |
| 133 | 23.4 | 117.0 | 39.4 | 56.9 |
| 134 | | 24.0 | | |
| 116 | | 13.9 | | |
| 141 | | 206 | | |
| 135 | | 9.2 | | |
| 136 | | 73.3 | | |
| 137 | | 42.2 | | |
| 138 | | 645 | | |
| 139 | | 268 | | |
| Reference | 2.8 | 18.8 | 2.0 | 9.1 |

**Table 7a.**

| Comp. No. | 3D CTG GP2D | 3D CTG KE39 | 3D CTG NCIH358 | 3D CTG SW620 |
|---|---|---|---|---|
| | IC90 (nM) | IC90 (nM) | IC90 (nM) | IC90 (nM) |
| 117 | 5.96 | | | |
| 106 | 6.52 | 5.22 | 1.18 | 17.88 |
| 120 | 4.17 | | | |
| 102 | 1.24 | | | |
| 101 | 20.46 | 14.72 | 1.798 | 62.17 |
| 127 | 49.68 | | | |
| 110 | 20.25 | | | |
| 115 | | | 8.47 | |
| 113 | 45.96 | | | |
| 124 | 8.79 | | | |
| 135 | 15.2 | | | |
| 148 | 19.7 | | | |
| 151 | 23.1 | | | |
| 142 | 56.4 | | | |
| Reference | 16.99 | 5.65 | 1.74 | 56.50 |

### Example 48: Pharmacokinetic (PK) Studies

Pharmacokinetics following IV and oral administration of compounds of the disclosure were investigated in dogs and monkeys. Beagle dogs were administered compounds of the disclosure intravenously at a dose of 0.5 mg/kg and orally at a dose of 1.5 mg/kg. Monkeys were administered compounds of the disclosure intravenously at a dose of 0.5 mg/kg. Plasma samples for pharmacokinetics were collected at preplanned time points.

The results of the study in dogs are summarized in Table 8, Figure 1 and Figure 4. Figure 1 shows the mean plasma concentration of compound 106 as a function of time. The mean plasma concentration was measured in beagle dogs orally administered a 1.5 mg/kg dose of compound 106. The IC₅₀ and IC₉₀ concentrations of compound 106 are also displayed. The mean plasma concentration of compound 106 exceeds its IC₅₀ and IC₉₀ for extended periods of time (~20 h and ~4 h respectively). **Figure 4** shows the mean plasma concentration of compound 101 as a function of time. The mean plasma concentration was measured in beagle dogs orally administered a 1.5 mg/kg dose of compound 101. The IC₅₀ and IC₉₀ concentrations of compound 101 are also displayed. The mean plasma concentration (PO) of compound 101 exceeds its IC₃₀ and IC₉₀ for extended periods of time.

The reference compound is a compound having the following structure: 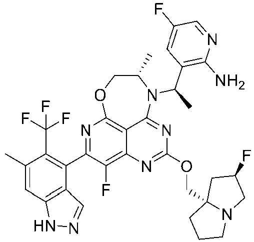 (See e.g., WO2024/235286).

**Table 8.**

| PK Parameter (Dog) | Reference | Comp. No. | | |
|---|---|---|---|---|
| | | 106 | 101 | 107 |
| IV Dose (mg/kg) | 0.5 | 0.5 | 0.5 | 0.5 |
| t½ (h) | 3.3 | 6.1 | 5.6 | 3.0 |
| V_{d} (L/kg) | 6.0 | 5.7 | 6.1 | 6.4 |
| Cl (mL/min/kg) | 28.7 | 15.5 | 15.8 | 38.6 |
| AUC_{inf} (ng·h/mL) | 303 | 550 | 537 | 432 |
| | | | | |
| Oral Dose (mg/kg) | 1.5 | 1.5 | 1.5 | 1.5 |
| Cₘₐₓ (ng/mL) | 63 | 100 | 76 | 56 |
| AUC_{inf} (ng·h/mL) | 173 | 503 | 691 | 352 |
| Bioavailability (F %) | 19 | 31 | 33 | 16 |
| PPB (fᵤ %) | 3.8 | 3.1 | 6.5 | 7.3 |

The results of the study in monkeys are summarized in Table 9.

**Table 9.**

| PK Parameter (Monkey) | Reference | Comp. No. | | |
|---|---|---|---|---|
| | | 106 | 101 | 107 |
| IV Dose (mg/kg) | 0.5 | 0.5 | 0.5 | 0.5 |
| t½ (h) | 3.5 | 6.3 | 4.1 | 2.3 |
| V_{d} (L/kg) | 9.3 | 8.4 | 5.1 | 4.7 |
| Cl (mL/min/kg) | 39.4 | 23 | 21.8 | 28.7 |

### Example 49: In vivo Efficacy Studies

The *in vivo* efficacy of compounds of the disclosure was evaluated in a GP2D KRAS G12D-driven CRC model. Six-to 8-week old, female, BALB/c nude mice were injected subcutaneously on the right flank with tumor cells (1x10⁷) in 100 uL DMEM Matrigel mixture (1:1 ratio). When tumors reached an average tumor volume of ~ 150 mm³ mice were randomized into treatment groups. Mice were dosed once daily by oral gavage over a 2-week treatment period. The results of the study are shown in Table 10 and tumor volume results shown in Figures 2A and Figure 2B. Figure 2C shows the *in vivo* efficacy of the reference compound below.

The reference compound is a compound having the following structure: 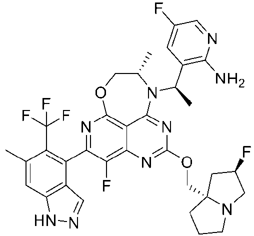 (See e.g., WO2024/235286).

**Table 10.**

| Comp. No | Reference | 101 | 106 |
|---|---|---|---|
| % TGI (% TR) at 5 mg/kg BID | - | 101 (3) | 99 (0) |
| % TGI (% TR) at 10 mg/kg QD | 80 (0) | - | 101 (5) |
| % TGI (% TR) at 10 mg/kg BID | 101 (4) | 109 (33) | 111 (42) |
| % TGI (% TR) at 30 mg/kg BID | 111 (49) | 114 (51) | 115 (58) |

The data demonstrates efficacy of the tested compounds at low doses (i.e., 10 mg/kg or less). Figure 2A shows the effect of compounds of the disclosure on tumor volume over time in a GP2D KRAS G12D CRC model. Female (6-8 week old) Balb/c nude mice were administered a control (vehicle only), 5 mg/kg, 10 mg/kg, or 30 mg/kg dose of compound 101 twice a day (BID), or a 5 mg/kg dose of compound 106 twice a day (BID). Figure 2B shows the effect of compounds of the disclosure on tumor volume over time in a GP2D KRAS G12D CRC xenograft tumor model. Female (6-8 week old) Balb/c nude mice were administered a control (vehicle only), a 10 mg/kg or 30 mg/kg dose of compound 106 twice a day, or a 10 mg/kg dose of compound 106 once a day. Figure 2C shows the effect of a reference compound (as defined in Example 47) on tumor volume over time in a GP2D KRAS G12D CRC xenograft tumor model. Female (6-8 week old) Balb/c nude mice were administered a control (vehicle only), a 10 mg/kg, or 30 mg/kg dose of the reference compound twice a day, or a 10 mg/kg dose of the reference compound once a day.

Compound plasma exposure over time is shown in Figure 5A and Figure 5B. Figure 5A shows antiproliferative activities and plasma exposure of compound 106 measured in mouse plasma over time in a GP2D KRAS G12D CRC xenograft tumor model. The IC₅₀ and IC₉₀ concentrations of compound 106 are also displayed. Female (6-8 week old) Balb/c nude mice were administered varied doses of compound 106 once a day (QD). Figure 5B shows antiproliferative activities of compound 101 measured in mouse plasma over time in a GP2D KRAS G12D CRC xenograft tumor model. The IC₅₀ and IC₉₀ concentrations of compound 101 are also displayed. Female (6-8 week old) Balb/c nude mice were administered varied doses of compound 101 once a day (QD).

### Example 50: In vivo pERK Inhibition Assay

The potency of test compounds in inhibiting phosphorylation of ERK (p-ERK) *in vivo* was evaluated in a GP2D KRAS G12D xenograft tumor model at predetermined time points after administration. Six-to 8-week old, female, BALB/c nude mice were injected subcutaneously on the right flank with tumor cells (1x10⁷) in 100 uL DMEM Matrigel mixture (1:1 ratio). When tumors reached an average tumor volume of ~ 300 mm³ mice were randomized into treatment groups. Mice received a single dose of indicated treatments, administered by oral gavage, and tumor samples were subsequently collected for analysis at indicated post-treatment time points. ERK phosphorylation was detected by standard immunohistochemical staining (p-ERK antibody #4370S; Cell Signaling Technology). Tumor sections were scanned by Pannoramic MIDI and analyzed by software Image Pro 10.0. The positive expression area was selected and expressed as positive area / density per µm². Data are presented as percentage pERK positivity relative to vehicle controls. The results are shown in Figure 3A and demonstrate sustained inhibition of p-ERK 24 h after a single dose of compound 106. Figure 3B shows the results for a reference compound. The reference compound is a compound having the following structure: 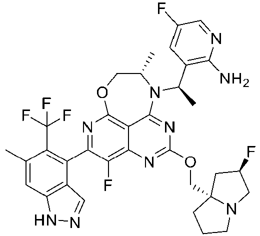 (See e.g., WO2024/235286).

Sustained inhibition of p-ERK after 24 h was not observed with the reference compound. pERK inhibition after single dosing results are also shown in Figure 6A and Figure 6B for Compound 101 and Compound 106.

### Example 51: in vivo tumor gene expression (DUSP6 inhibition) assay

The potency of test compounds in inhibiting tumor gene expression was determined by a DUSP6 mRNA inhibition assay in mice. GP2d colorectal adenocarcinoma (KRAS G12D mutant) tumor cell lines were maintained *in vitro* as monolayer cultures in Roswell Park Memorial Institute 1640 medium or Dulbecco's Modified Eagle Medium supplemented with 10% fetal bovine serum at 37 °C in an atmosphere of 5% CO₂ in air. Each mouse was inoculated subcutaneously into the right flank with 5 or 10 million tumor cells to establish tumors. When the mean tumor volume reached 300 to 400 mm³, mice were randomized into groups. Vehicle or compound was administered as a single oral dose (3 mg/kg, 10 mg/kg, 30 mg/kg, or 60 mg/kg) as indicated (n = 9 or 12/group). Tumor tissue samples were harvested and snap frozen at indicated timepoints post-treatment for qPCR analysis of DUSP6. Total RNA was extracted from snap frozen tumor tissues using a RNeasy Plus mini kit (Qiagen #74134) following the manufacturer's protocol. The concentration and quality of total RNA was measured using the NanoDrop One spectrophotometer. Reverse transcription was carried out using a High-Capacity RNA-to-complementary DNA (cDNA) kit (Invitrogen #4387406) according to the manufacturer's protocol. The cDNA product was used for qPCR analysis using TaqMan FAST Gene Expression Master Mix (Invitrogen #4444557), TaqMan Gene Expression Assays for DUSP6, and TATA-box binding protein (TBP) to detect the levels from each sample in duplicate. For qPCR, cycle threshold (Ct) values of DUSP6 and TBP were obtained for analysis. The DUSP6 Ct value was normalized to TBP, and then the mean relative messenger RNA (mRNA) expression levels of each group were normalized to the vehicle control group. Values were plotted as relative change in mRNA expression compared with vehicle control as means ± SEM. Results are shown in FIG. 7A and FIG. 7B.

### ENUMERATED EMBODIMENTS

**Enumerated Embodiment 1.** A compound represented by Formula I: 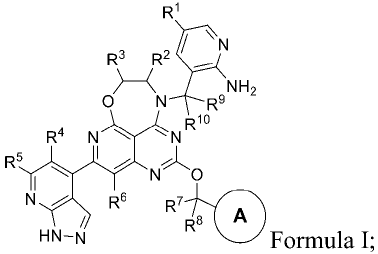 or a pharmaceutically acceptable salt, stereoisomer, or tautomer thereof, wherein:
R¹ is selected from the group consisting of halogen, C₁-C₆alkoxy, C₃-C₄cycloalkyl, C₁-C₆alkyl, C₂-C₆alkenyl, and C₂-C₆alkynyl, wherein the alkoxy, cycloalkyl, alkyl, alkenyl, and alkynyl are optionally substituted with one or more halogens;
R² and R³ are each independently selected from the group consisting of C₁-C₆alkyl and hydrogen, wherein the alkyl is optionally substituted with one or more halogens or hydroxyl; or
R² and R³, together with the atoms to which they are attached, may be joined together to form a 4-7 membered heterocyclyl or a 5-7 membered cycloalkyl; wherein the heterocyclyl and cycloalkyl are optionally substituted with one or more substituents each independently selected from the group consisting of halogen, deuterium, hydroxyl, -NR^{a}R^{b}, C₁-C₆alkyl, and C₁-C₆alkoxy;
R⁴ and R⁵ are each independently selected from the group consisting of hydrogen, halogen, deuterium, hydroxyl, -CN, -NO₂, -NR^{a}R^{b}, C₁-C₆alkyl, C₂-C₆alkenyl, C₂-C₆alkynyl, C₁-C₆alkoxy, C₃-C₆cycloalkyl, phenyl, -C(=O)NR^{a}R^{b}, -NR^{a}(C=O)R^{b}, -O(C=O)NR^{a}R^{b}, - NR^{a}(C=O)OR^{b}, -NR^{a}(C=O)NR^{a}R^{b}, -(C=O)C₁-C₆alkyl, -(C=O)OC₁-C₆alkyl, -O(C=O)C₁-C₆alkyl, -O(C=O)OC₁-C₆alkyl, -SH, -SC₁-C₆alkyl, -S(O)C₁-C₆alkyl, -S(O)₂C₁-C₆alkyl, - S(O)₂NR^{a}R^{b}, and -NR^{a}S(O)₂C₁-C₆alkyl, wherein the alkyl, alkenyl, alkynyl, alkoxy, cycloalkyl, and phenyl are optionally substituted with one or more substituents each independently selected from the group consisting of halogen, deuterium, hydroxyl, oxo, - NR^{a}R^{b}, C₁-C₆alkyl, and C₁-C₆alkoxy;
R⁶ is selected from the group consisting of halogen, hydrogen, deuterium, and C₁-C₃alkyl;
R⁷ and R⁸ are each independently selected from the group consisting of hydrogen, deuterium, and C₁-C₃alkyl;
R⁹ and R¹⁰ are each independently selected from the group consisting of hydrogen, deuterium, and C₁-C₂alkyl optionally substituted with one or more halogens;
Ring A is selected from the group consisting of 4-12 membered heterocyclyl containing at least one ring nitrogen; wherein ring A is optionally substituted with one, two, or three substituents each independently selected from R^{A};
R^{A} is independently selected for each occurrence from the group consisting of halogen, deuterium, hydroxyl, oxo, -CN, -NR^{a}R^{b}, -COOH, C₁-C₆alkyl, C₁-C₆alkoxy, C₃-C₆cycloalkyl, =CHF, and =CH-C₁-C₆alkyl, wherein the alkyl is optionally substituted with one, two, or three substituents each independently selected from the group consisting of hydroxyl, halogen, and C₁-C₃alkoxy; and
R^{a} and R^{b} are independently selected for each occurrence from the group consisting of hydrogen and C₁-C₆alkyl, wherein the alkyl is optionally substituted with one or more substituents each independently selected from the group consisting of halogen, deuterium, hydroxyl, and C₁-C₆alkoxy; or
R^{a} and R^{b}, at each occurrence, together with the nitrogen to which they are attached, are joined together to form a 4-7 membered heterocyclyl optionally substituted by one or more substituents each independently selected from the group consisting of halogen, deuterium, hydroxyl, -NR^{a1}R^{b1}, C₁-C₆alkyl, and C₁-C₆alkoxy; wherein R^{a1} and R^{b1} are independently, at each occurrence, selected from the group consisting of hydrogen and C₁-C₆alkyl, wherein the alkyl is optionally substituted with one or more substituents which are each independently selected from the group consisting of halogen, deuterium, hydroxyl, and C₁-C₆alkoxy, or R^{a1} and R^{b1}, at each occurrence, together with the nitrogen to which they are attached, are joined together to form a 4-7 membered heterocyclyl.

**Enumerated Embodiment 2.** The compound or a pharmaceutically acceptable salt, stereoisomer, or tautomer thereof of Enumerated Embodiment 1, wherein the compound is represented by Formula IA:

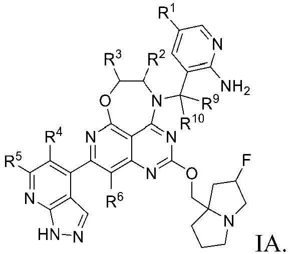

**Enumerated Embodiment 3.** The compound or a pharmaceutically acceptable salt, stereoisomer, or tautomer thereof of Enumerated Embodiment 1 or Enumerated Embodiment 2, wherein R¹ is selected from the group consisting of fluoro, chloro, -OCH₃, - OCHF₂, and -OCF₃.

**Enumerated Embodiment 4.** The compound or a pharmaceutically acceptable salt, stereoisomer, or tautomer thereof of any one of Enumerated Embodiments 1-3, wherein R² is selected from the group consisting of -CH₃ and hydrogen.

**Enumerated Embodiment 5.** The compound or a pharmaceutically acceptable salt, stereoisomer, or tautomer thereof of any one of Enumerated Embodiments 1-4, wherein R⁴ and R⁵ are each independently selected from the group consisting of halogen, hydroxyl, cyano, -NH₂, -C(O)NH₂, C₁-C₄alkyl, C₂-C₄alkynyl, C₁-C₄alkoxy, and C₃-C₆cycloalkyl, wherein the alkyl, alkynyl, alkoxy, and cycloalkyl are optionally substituted with one, two, or three halogens or -CN.

**Enumerated Embodiment 6.** The compound or a pharmaceutically acceptable salt, stereoisomer, or tautomer thereof of any one of Enumerated Embodiments 1-5, wherein R⁴ is selected from the group consisting of C₁-C₄alkyl and C₃-C₆cycloalkyl, wherein the alkyl and cycloalkyl are optionally substituted with one, two, or three halogens.

**Enumerated Embodiment 7.** The compound or a pharmaceutically acceptable salt, stereoisomer, or tautomer thereof of any one of Enumerated Embodiments 1-6, wherein R⁴ is selected from the group consisting of -CF₃,

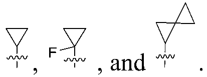

**Enumerated Embodiment 8.** The compound or a pharmaceutically acceptable salt, stereoisomer, or tautomer thereof of any one of Enumerated Embodiments 1-7, wherein R⁵ is selected from the group consisting of halogen and C₁-C₄alkyl, wherein the alkyl is optionally substituted with one, two, or three halogens.

**Enumerated Embodiment 9.** The compound or a pharmaceutically acceptable salt, stereoisomer, or tautomer thereof of any one of Enumerated Embodiments 1-8, wherein R⁵ is selected from the group consisting of chloro, fluoro, and -CH₃.

**Enumerated Embodiment 10.** The compound or a pharmaceutically acceptable salt, stereoisomer, or tautomer thereof of any one of Enumerated Embodiments 1-9, wherein the moiety 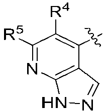 is selected from the group consisting of:

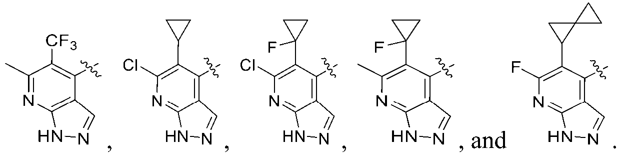

**Enumerated Embodiment 11.** The compound or a pharmaceutically acceptable salt, stereoisomer, or tautomer thereof of any one of Enumerated Embodiments 1-10, wherein R⁶ is fluoro.

**Enumerated Embodiment 12.** The compound or a pharmaceutically acceptable salt, stereoisomer, or tautomer thereof of any one of Enumerated Embodiments 1-11, wherein R⁹ is hydrogen and R¹⁰ is -CH₃.

**Enumerated Embodiment 13.** A compound represented by Formula II: 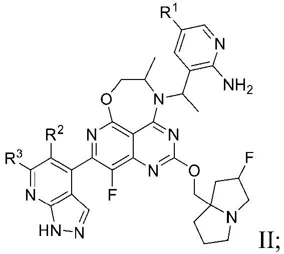 or a pharmaceutically acceptable salt, stereoisomer, or tautomer thereof, wherein:
R¹ is selected from the group consisting of halogen, cyano, and C₁-C₆alkoxy, wherein the alkoxy is optionally substituted with one or more halogens;
R² is selected from the group consisting of C₁-C₄alkyl and C₃-C₆cycloalkyl, wherein the alkyl and cycloalkyl are optionally substituted with one, two, or three halogens; and
R³ is selected from the group consisting of halogen and C₁-C₄alkyl, wherein the alkyl is optionally substituted with one, two, or three halogens.

**Enumerated Embodiment 14.** The compound or a pharmaceutically acceptable salt, stereoisomer, or tautomer thereof of Enumerated Embodiment 13, wherein the compound is represented by Formula II-1:

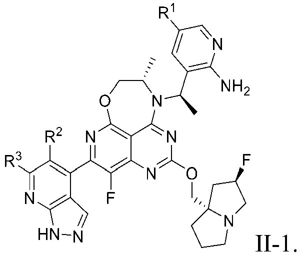

**Enumerated Embodiment 15.** The compound or a pharmaceutically acceptable salt, stereoisomer, or tautomer thereof of Enumerated Embodiment 13 or Enumerated Embodiment 14, wherein R¹ is selected from the group consisting of fluoro, chloro, -OCHF₂, and -OCF₃.

**Enumerated Embodiment 16.** The compound or a pharmaceutically acceptable salt, stereoisomer, or tautomer thereof of any one of Enumerated Embodiments 13-15, wherein R² is selected from the group consisting of -CF₃,

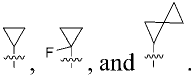

**Enumerated Embodiment 17.** The compound or a pharmaceutically acceptable salt, stereoisomer, or tautomer thereof of any one of Enumerated Embodiments 13-16, wherein R³ is selected from the group consisting of chloro, fluoro, and -CH₃.

**Enumerated Embodiment 18.** The compound or a pharmaceutically acceptable salt, stereoisomer, or tautomer thereof of any one of Enumerated Embodiments 13-17, wherein the moiety 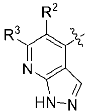 is selected from the group consisting of:

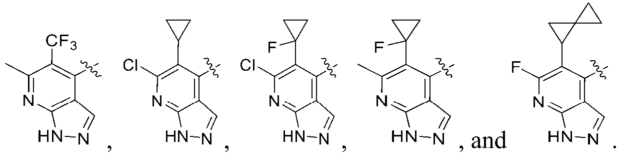

**Enumerated Embodiment 19.** A compound represented by Formula III: 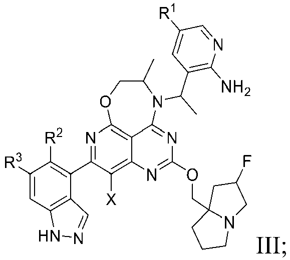 or a pharmaceutically acceptable salt, stereoisomer, or tautomer thereof, wherein:
X is F or Cl;
R¹ is cyano, -C(O)NH₂, isopropyl, C₁-C₄alkoxy, -OCH₃, -OCF₃, or -OCHF₂, wherein the alkyl of the alkoxy is optionally -CH₂CN or a C₃-C₆cycloalkyl group;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens, or -CN;
R³ is selected from the group consisting of halogen and C₁-C₄alkyl, wherein the alkyl is optionally substituted with one, two, or three halogens,
   or:
R¹ is fluoro;
R² is selected from the group consisting of C₁-C₄alkoxy and C₃-C₆cycloalkyl, wherein alkoxy and cycloalkyl are optionally substituted with one, two, or three halogens, or -CN;
R³ is selected from the group consisting of halogen and C₁-C₄alkyl.

**Enumerated Embodiment 20.** The compound or a pharmaceutically acceptable salt or tautomer thereof of Enumerated Embodiment 19, wherein the compound is represented by Formula III-1:

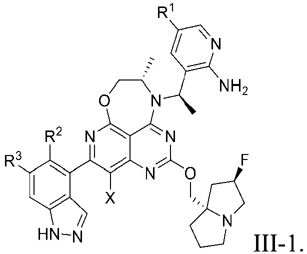

**Enumerated Embodiment 21.** A compound represented by Formula IIIa: 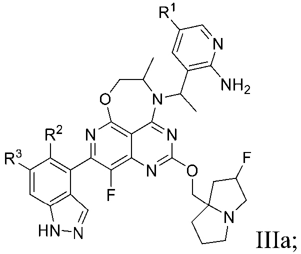 or a pharmaceutically acceptable salt, stereoisomer, or tautomer thereof, wherein:
R¹ is cyano, -C(O)NH₂, isopropyl, C₁-C₄alkoxy, -OCH₃, -OCF₃, or -OCHF₂, wherein the alkyl of the C₁-C₄alkoxy is optionally -CH₂CN, or a C₃-C₆cycloalkyl group, and wherein the alkyl may be substituted with one or more deuterium;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens, or -CN;
R³ is selected from the group consisting of halogen and C₁-C₄alkyl, wherein the alkyl is optionally substituted with one, two, or three halogens;
   or:
R¹ is fluoro;
R² is selected from the group consisting of C₁-C₄alkoxy and C₃-C₆cycloalkyl, wherein alkoxy and cycloalkyl are optionally substituted with one, two, or three halogens; and
R³ is selected from the group consisting of halogen and C₁-C₄alkyl.

**Enumerated Embodiment 22.** The compound or a pharmaceutically acceptable salt or tautomer thereof of Enumerated Embodiment 19, wherein the compound is represented by Formula IIIa-1:

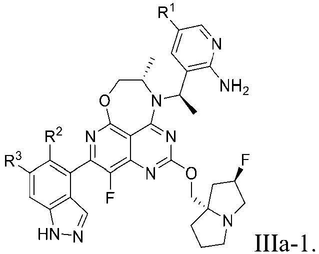

**Enumerated Embodiment 23.** The compound or a pharmaceutically acceptable salt, stereoisomer, or tautomer thereof of any one of Enumerated Embodiments 19-22, wherein R¹ is -OCHF₂.

**Enumerated Embodiment 24.** The compound or a pharmaceutically acceptable salt, stereoisomer, or tautomer thereof of any one of Enumerated Embodiments 19-22, wherein R¹ is -OCF₃.

**Enumerated Embodiment 25.** The compound or a pharmaceutically acceptable salt, stereoisomer, or tautomer thereof of any one of Enumerated Embodiments 19-24, wherein R² is selected from the group consisting of -CF₃,

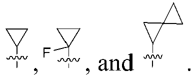

**Enumerated Embodiment 26.** The compound or a pharmaceutically acceptable salt, stereoisomer, or tautomer thereof of any one of Enumerated Embodiments 19-25, wherein R³ is selected from the group consisting of chloro, fluoro, and -CH₃.

**Enumerated Embodiment 27.** The compound or a pharmaceutically acceptable salt, stereoisomer, or tautomer thereof of any one of Enumerated Embodiments 19-22, wherein R¹ is fluoro.

**Enumerated Embodiment 28.** The compound or a pharmaceutically acceptable salt, stereoisomer, or tautomer thereof of any one of Enumerated Embodiments 19-22 or 27, wherein R² is selected from the group consisting of -OCHF₂,

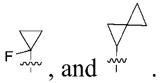

**Enumerated Embodiment 29.** The compound or a pharmaceutically acceptable salt, stereoisomer, or tautomer thereof of any one of any one of Enumerated Embodiments 19-22 and 27-28, wherein R³ is selected from the group consisting of fluoro, and -CH₃.

**Enumerated Embodiment 30.** The compound or a pharmaceutically acceptable salt, stereoisomer, or tautomer thereof of any one of Enumerated Embodiments 19-29, wherein the moiety 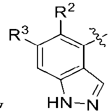 is selected from the group consisting of:

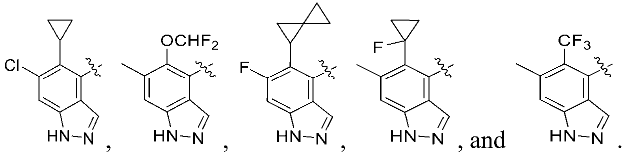

**Enumerated Embodiment 31.** The compound or a pharmaceutically acceptable salt, stereoisomer, or tautomer thereof of any one of Enumerated Embodiments 19-22, wherein R¹ is cyano or -C(O)NH₂.

**Enumerated Embodiment 32.** The compound or a pharmaceutically acceptable salt, stereoisomer, or tautomer thereof of any one of Enumerated Embodiments 19-22, wherein R¹ is isopropyl.

**Enumerated Embodiment 33.** The compound or a pharmaceutically acceptable salt, stereoisomer, or tautomer thereof of Enumerated Embodiment 31 or Enumerated Embodiment 32, wherein R² is CF₃.

**Enumerated Embodiment 34.** A compound selected from the group consisting of: 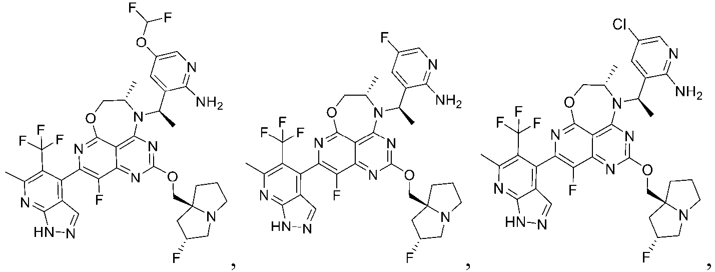 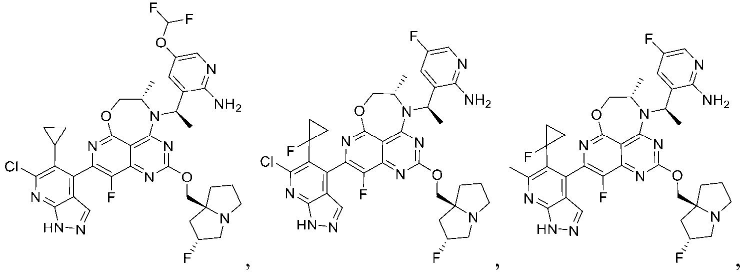 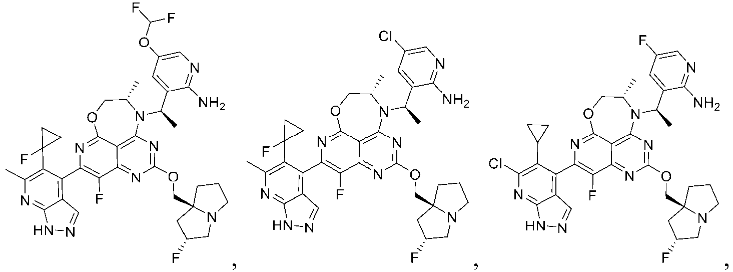 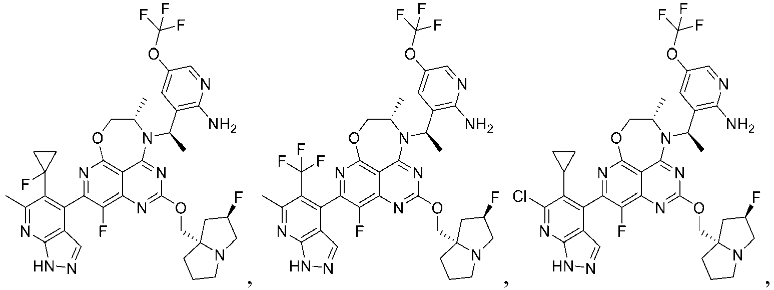 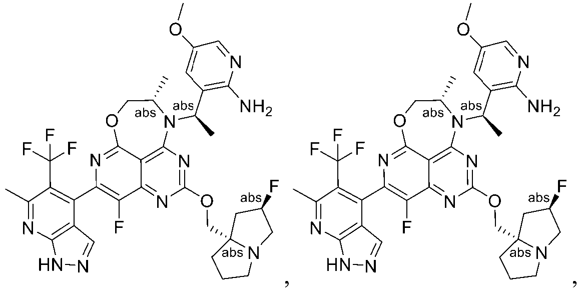 and 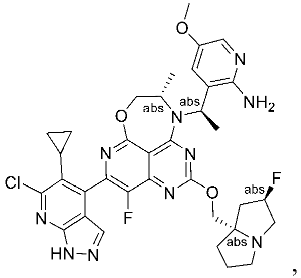 or a pharmaceutically acceptable salt, stereoisomer, or tautomer thereof.

**Enumerated Embodiment 35.** A compound selected from the group consisting of: 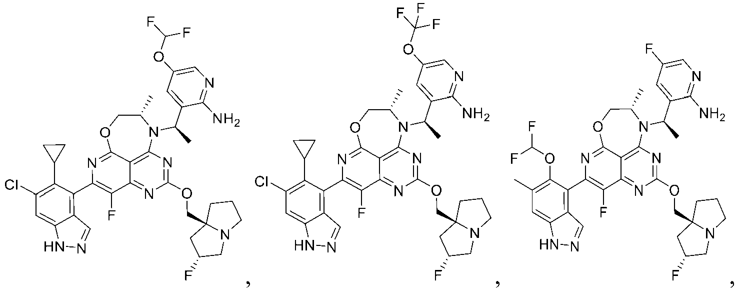 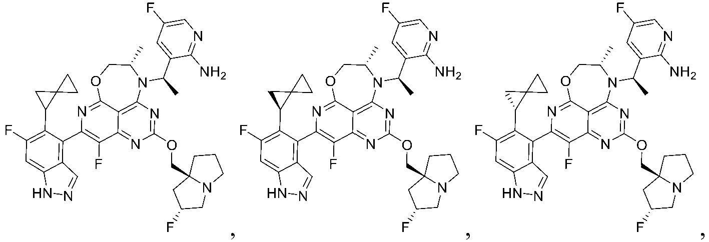 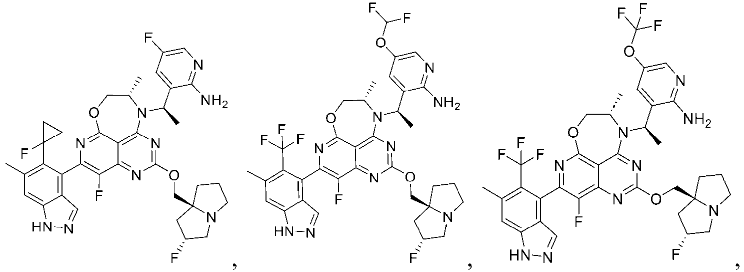 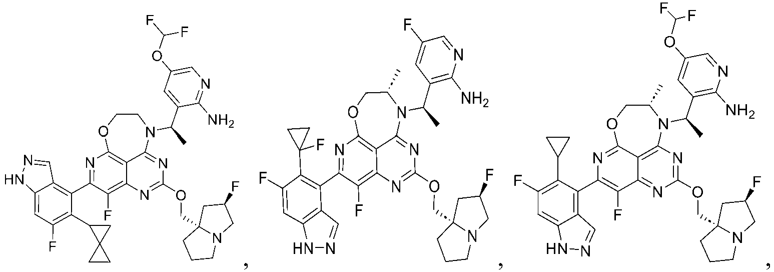 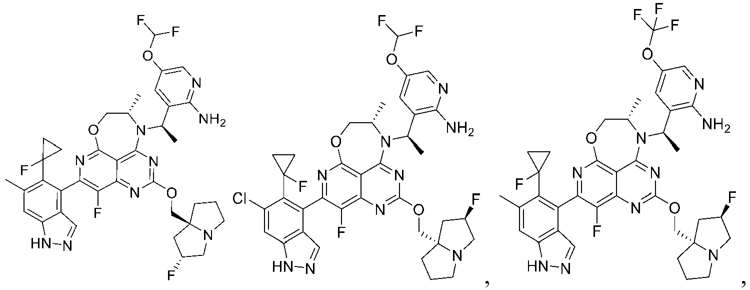 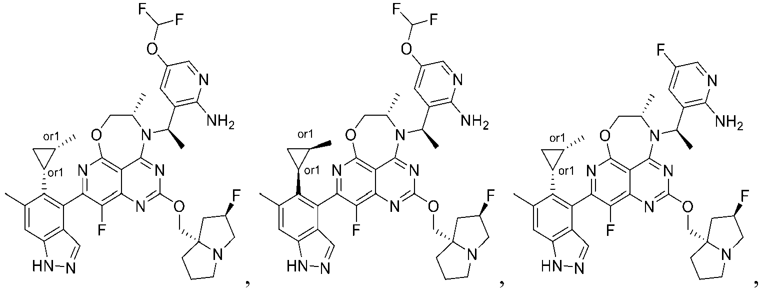 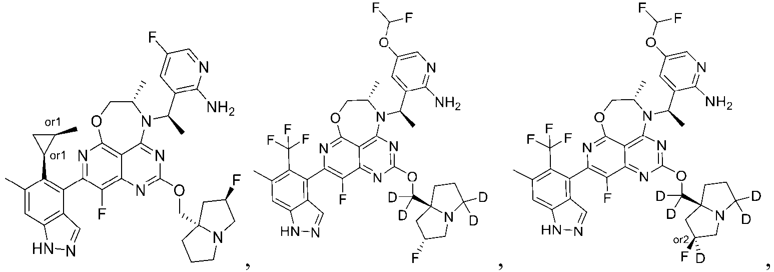 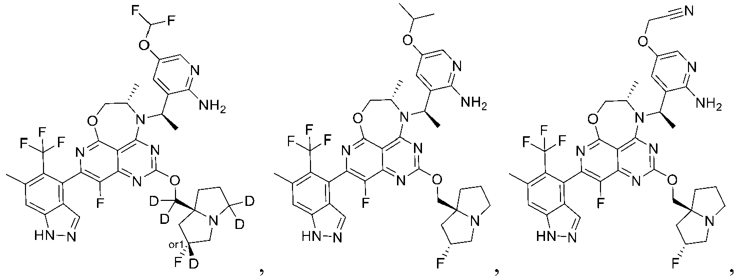 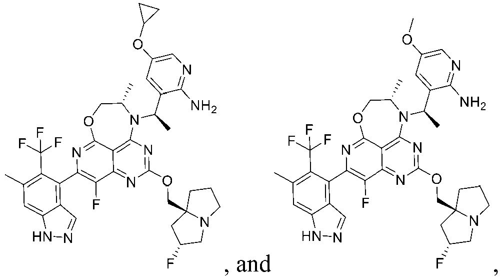 or a pharmaceutically acceptable salt, stereoisomer, or tautomer thereof.

**Enumerated Embodiment 36.** A compound selected from the group consisting of: 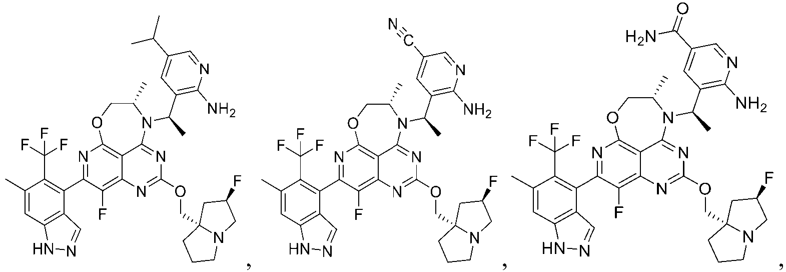 and 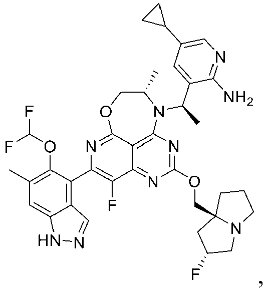 or a pharmaceutically acceptable salt or tautomer thereof.

**Enumerated Embodiment 37.** A compound selected from the group consisting of: 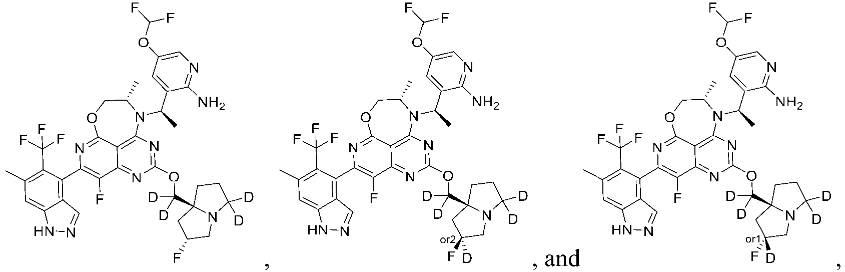 or a pharmaceutically acceptable salt or tautomer thereof.

**Enumerated Embodiment 38.** A compound selected from the group consisting of: 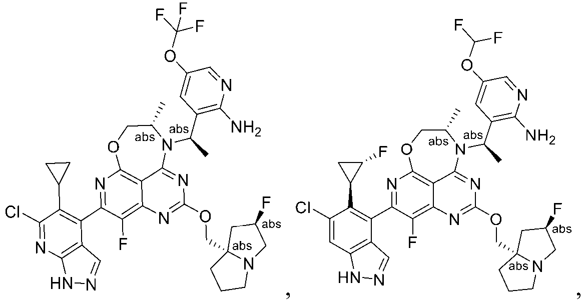 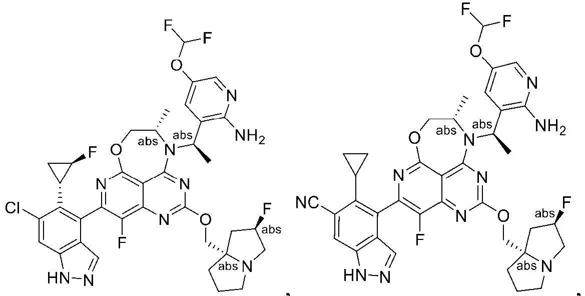 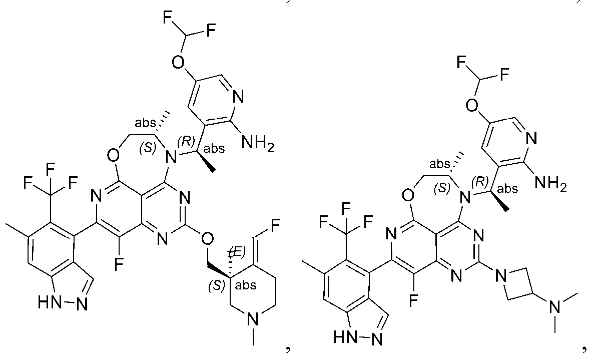 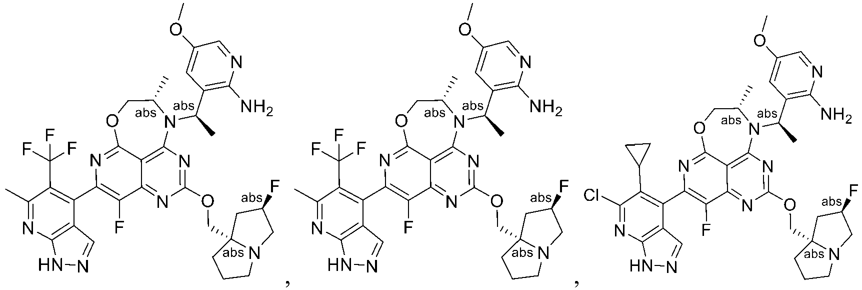 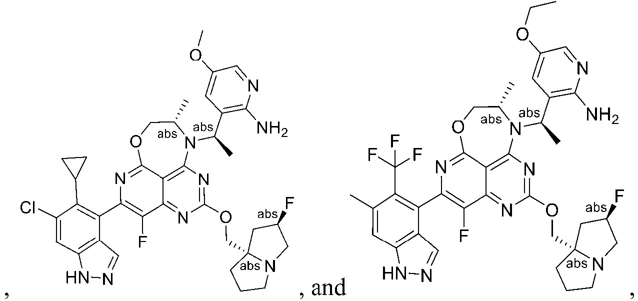 or a pharmaceutically acceptable salt **or** tautomer thereof.

**Enumerated Embodiment 39.** A compound selected from the group consisting of: 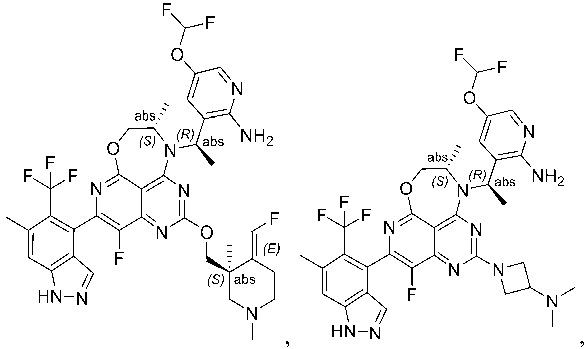 and 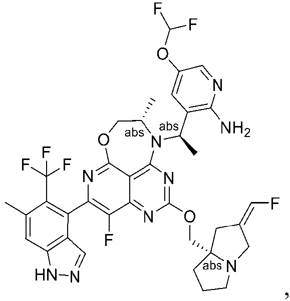 or a pharmaceutically acceptable salt or tautomer thereof.

**Enumerated Embodiment 40.** A compound represented by Formula IV: 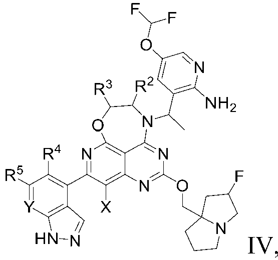 or a pharmaceutically acceptable salt, stereoisomer, or tautomer thereof, wherein
X is F or Cl;
Y is N or CH;
R² and R³ are each independently selected from C₁-C₆ alkyl and hydrogen; and
R⁴ and R⁵ are independently selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogens, or one to three methyls.

**Enumerated Embodiment 41.** The compound or a pharmaceutically acceptable salt, stereoisomer, or tautomer thereof of Enumerated Embodiment 40, wherein the compound is represented by Formula IV-1:

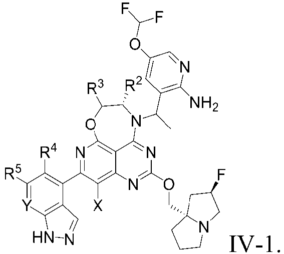

**Enumerated Embodiment 42.** The compound or a pharmaceutically acceptable salt or tautomer thereof of Enumerated Embodiment 36, wherein the compound is represented by Formula IV-2:

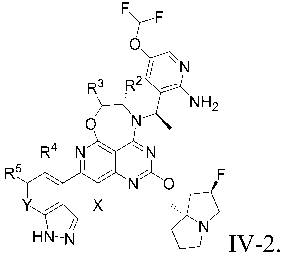

**Enumerated Embodiment 43.** The compound or a pharmaceutically acceptable salt, stereoisomer, or tautomer thereof of any one of Enumerated Embodiments 40-42, wherein R⁴ is halogen, C₁-C₆ alkyl, or C₃-C₆ cycloalkyl, wherein the alkyl and cycloalkyl are optionally substituted with one to three halogens or one to three methyls.

**Enumerated Embodiment 44.** The compound or a pharmaceutically acceptable salt, stereoisomer, or tautomer thereof of any one of Enumerated Embodiments 40-43, wherein R⁵ is -C₁-C₃ alkyl.

**Enumerated Embodiment 45.** The compound or a pharmaceutically acceptable salt, stereoisomer, or tautomer thereof of any one of Enumerated Embodiments 40-44, wherein R⁵ is -CH₃.

**Enumerated Embodiment 46.** The compound or a pharmaceutically acceptable salt, stereoisomer, or tautomer thereof of any one of Enumerated Embodiments 40-43, wherein R⁵ is -Cl or -F.

**Enumerated Embodiment 47.** The compound or a pharmaceutically acceptable salt, stereoisomer, or tautomer thereof of Enumerated Embodiment 40, wherein the compound is represented by Formula IV-a1 or Formula IV-b1: 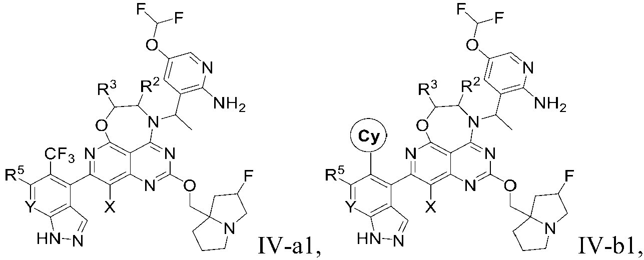 wherein 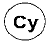 is a C₃-C₆ cycloalkyl, optionally substituted with one to three halogens or one to three methyls.

**Enumerated Embodiment 48.** The compound or a pharmaceutically acceptable salt, stereoisomer, or tautomer thereof of Enumerated Embodiment 40, wherein the compound is represented by Formula IV-a2 or Formula IV-b2: 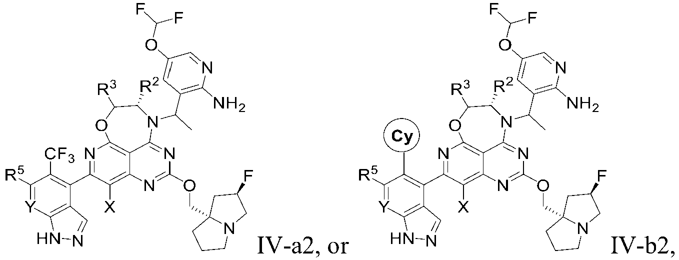 wherein 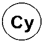 is a C₃-C₆ cycloalkyl, optionally substituted with one to three halogens or one to three methyls.

**Enumerated Embodiment 49.** The compound or a pharmaceutically acceptable salt, stereoisomer, or tautomer thereof of Enumerated Embodiment 40, wherein the compound is represented by Formula IV-a3 or Formula IV-b3: 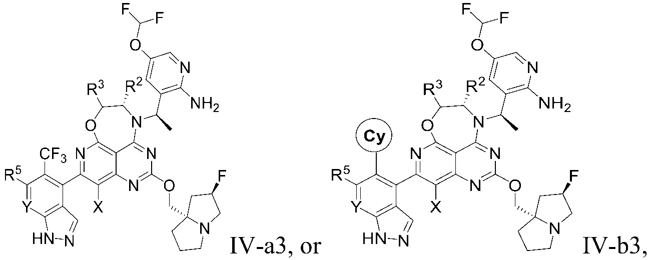 wherein 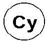 is a C₃-C₆ cycloalkyl, optionally substituted with one to three halogen.

**Enumerated Embodiment 50.** The compound or a pharmaceutically acceptable salt, stereoisomer, or tautomer thereof of any one of Enumerated Embodiments 47-49, wherein 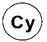 is substituted with one halogen.

**Enumerated Embodiment 51.** compound or a pharmaceutically acceptable salt, stereoisomer, or tautomer thereof of any one of Enumerated Embodiments 47-48, wherein 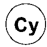 is cyclopropyl.

**Enumerated Embodiment 52.** The compound or a pharmaceutically acceptable salt, stereoisomer, or tautomer thereof of any one of Enumerated Embodiments 47-49, wherein 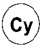 is 1-fluorocyclopropyl.

**Enumerated Embodiment 53.** The compound or a pharmaceutically acceptable salt, stereoisomer, or tautomer thereof of any one of Enumerated Embodiments 47-50, wherein 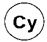 is spiro[2.2]pentan-1-yl.

**Enumerated Embodiment 54.** The compound of any one of Enumerated Embodiments 47-53, wherein R³ is H.

**Enumerated Embodiment 55.** The compound or a pharmaceutically acceptable salt, stereoisomer, or tautomer thereof of any one of Enumerated Embodiments 47-54, wherein R² is -C₁-C₃ alkyl.

**Enumerated Embodiment 56.** The compound or a pharmaceutically acceptable salt, stereoisomer, or tautomer thereof of any one of claims 47-54, wherein R² is -CH₃.

**Enumerated Embodiment 57.** The compound of Enumerated Embodiment 40, wherein the compound is: 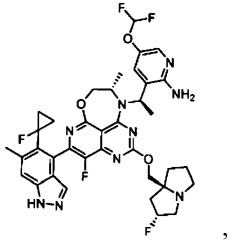 or a pharmaceutically acceptable salt or tautomer thereof.

**Enumerated Embodiment 58.** The compound of Enumerated Embodiment 40, wherein the compound is: 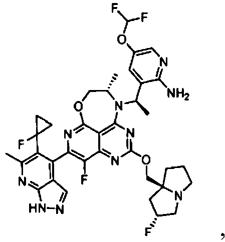 or a pharmaceutically acceptable salt or tautomer thereof.

**Enumerated Embodiment 59.** The compound of Enumerated Embodiment 40, wherein the compound is: 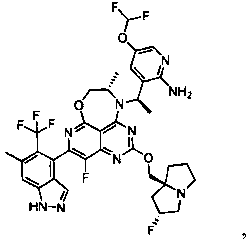 or a pharmaceutically acceptable salt or tautomer thereof.

**Enumerated Embodiment 60.** The compound of Enumerated Embodiment 40, wherein the compound is: 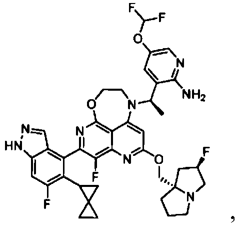 or a pharmaceutically acceptable salt or tautomer thereof.

**Enumerated Embodiment 61.** The compound of Enumerated Embodiment 40, wherein the compound is: 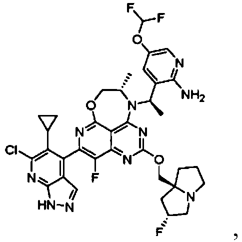 or a pharmaceutically acceptable salt or tautomer thereof.

**Enumerated Embodiment 62.** The compound of Enumerated Embodiment 40, wherein the compound is: 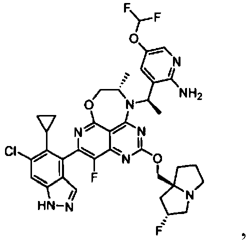 or a pharmaceutically acceptable salt or tautomer thereof.

**Enumerated Embodiment 63.** The compound of Enumerated Embodiment 40, wherein the compound is: 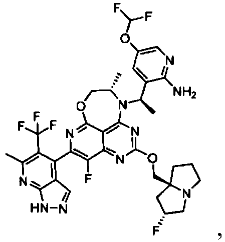 or a pharmaceutically acceptable salt or tautomer thereof.

**Enumerated Embodiment 64.** The compound of Enumerated Embodiment 40, wherein the compound is: 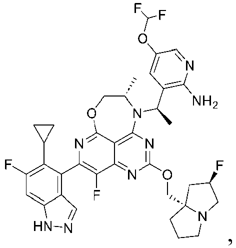 or a pharmaceutically acceptable salt or tautomer thereof.

**Enumerated Embodiment 65.** The compound of Enumerated Embodiment 40, wherein the compound is: 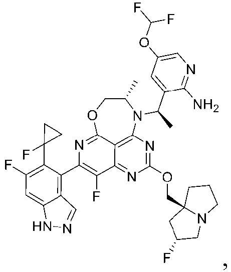 or a pharmaceutically acceptable salt or tautomer thereof.

**Enumerated Embodiment 66.** The compound of Enumerated Embodiment 40, wherein the compound is: 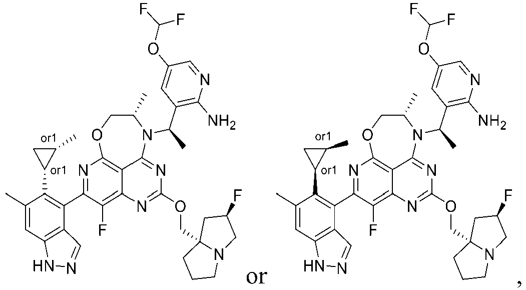 or a pharmaceutically acceptable salt or tautomer thereof.

**Enumerated Embodiment 67.** A compound represented by Formula V: 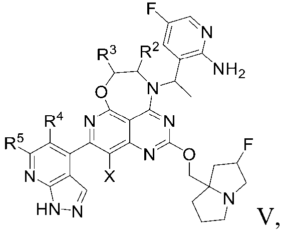 or a pharmaceutically acceptable salt, stereoisomer, or tautomer thereof, wherein
X is F or Cl;
R² and R³ are each independently selected from C₁-C₆ alkyl and hydrogen; and
R⁴ and R⁵ are each independently selected from hydrogen, halogen, deuterium, C₁-C₆ alkyl, C₁-C₆ alkoxy, and C₃-C₆ cycloalkyl, wherein the alkyl, alkoxy, and cycloalkyl are optionally substituted with one to three halogen.

**Enumerated Embodiment 68.** The compound or a pharmaceutically acceptable salt, stereoisomer, or tautomer thereof of Enumerated Embodiment 67, wherein the compound is represented by Formula V-1:

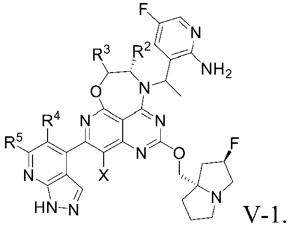

**Enumerated Embodiment 69.** The compound or a pharmaceutically acceptable salt, stereoisomer, or tautomer thereof of Enumerated Embodiment 67, wherein the compound is represented by Formula V-2:

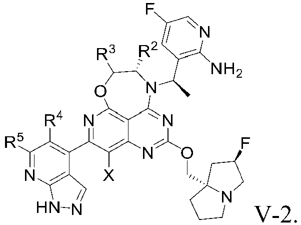

**Enumerated Embodiment 70.** The compound or a pharmaceutically acceptable salt, stereoisomer, or tautomer thereof of any one of Enumerated Embodiments 67-69, wherein R⁴ is halogen, C₁-C₆ alkyl, C₁-C₆ alkoxy, or C₃-C₆ cycloalkyl wherein the alkyl, alkoxy, and cycloalkyl are optionally substituted with one to three halogen.

**Enumerated Embodiment 71.** The compound or a pharmaceutically acceptable salt, stereoisomer, or tautomer thereof of any one of Enumerated Embodiments 67-70, wherein R⁵ is -C₁-C₃ alkyl.

**Enumerated Embodiment 72.** The compound or a pharmaceutically acceptable salt, stereoisomer, or tautomer thereof of any one of Enumerated Embodiments 67-71, wherein R⁵ is -CH₃.

**Enumerated Embodiment 73.** The compound or a pharmaceutically acceptable salt, stereoisomer, or tautomer thereof of any one of Enumerated Embodiments 67-70, wherein R⁵ is -Cl or -F.

**Enumerated Embodiment 74.** The compound or a pharmaceutically acceptable salt, stereoisomer, or tautomer thereof of Enumerated Embodiment 67, wherein the compound is represented by Formula V-a, V-b, or V-c: 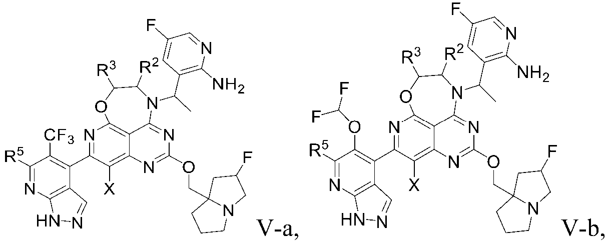 or 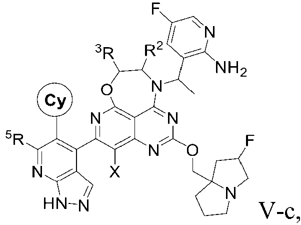 wherein 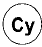 is a C₃-C₆ cycloalkyl, wherein the C₃-C₆ cycloalkyl is optionally substituted with one to three halogen.

**Enumerated Embodiment 75.** The compound or a pharmaceutically acceptable salt, stereoisomer, or tautomer thereof of Enumerated Embodiment 67, wherein the compound is represented by Formula V-a1, V-b1, or V-c1: 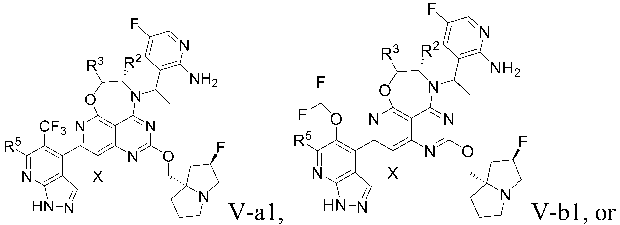 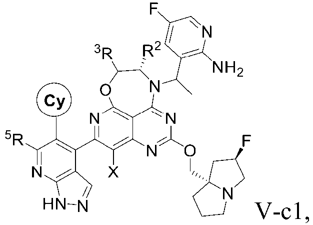 wherein 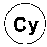 is a C₃-C₆ cycloalkyl, wherein the C₃-C₆ cycloalkyl is optionally substituted with one to three halogen.

**Enumerated Embodiment 76.** The compound or a pharmaceutically acceptable salt or tautomer thereof of Enumerated Embodiment 67, wherein the compound is represented by Formula V-a2, V-b2, or V-c2: 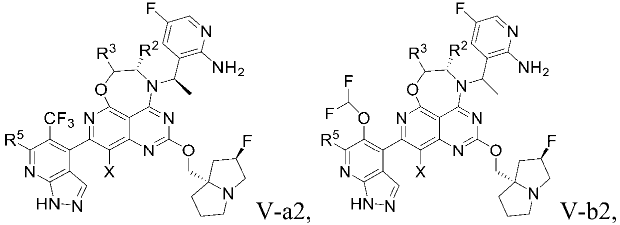 or 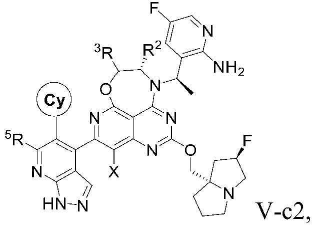 wherein 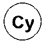 is a C₃-C₆ cycloalkyl, wherein the C₃-C₆ cycloalkyl is optionally substituted with one to three halogen.

**Enumerated Embodiment 77.** The compound or a pharmaceutically acceptable salt, stereoisomer, or tautomer thereof of any one of Enumerated Embodiments 74-76, wherein 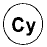 is substituted with one halogen.

**Enumerated Embodiment 78.** The compound or a pharmaceutically acceptable salt, stereoisomer, or tautomer thereof of any one of Enumerated Embodiments 74-77, wherein 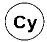 is 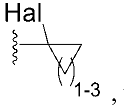 wherein 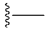 is the point of attachment to aza-indazole moiety, and Hal is a halogen.

**Enumerated Embodiment 79.** The compound or a pharmaceutically acceptable salt, stereoisomer, or tautomer thereof of any one of Enumerated Embodiments 74-78, wherein the halogen is fluoro.

**Enumerated Embodiment 80.** The compound or a pharmaceutically acceptable salt, stereoisomer, or tautomer thereof of any one of Enumerated Embodiments 74-77, wherein 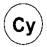 is 1-fluorocyclopropyl.

**Enumerated Embodiment 81.** The compound or a pharmaceutically acceptable salt, stereoisomer, or tautomer thereof of any one of Enumerated Embodiments 74-77, wherein 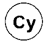 is spiro[2.2]pentan-1-yl.

**Enumerated Embodiment 82.** The compound or a pharmaceutically acceptable salt, stereoisomer, or tautomer thereof of any one of Enumerated Embodiments 74-77, wherein 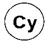 is cyclopropyl.

**Enumerated Embodiment 83.** The compound or a pharmaceutically acceptable salt, stereoisomer, or tautomer thereof of any one of Enumerated Embodiments 67-82, wherein R³ is H.

**Enumerated Embodiment 84.** The compound or a pharmaceutically acceptable salt, stereoisomer, or tautomer thereof of any one of Enumerated Embodiments 67-83, wherein R² is -C₁-C₃ alkyl.

**Enumerated Embodiment 85.** The compound or a pharmaceutically acceptable salt, stereoisomer, or tautomer thereof of any one of Enumerated Embodiments 67-84, wherein R² is -CH₃.

**Enumerated Embodiment 86.** The compound of Enumerated Embodiment 67, wherein the compound is: 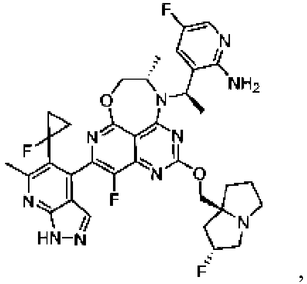 or a pharmaceutically acceptable salt or tautomer thereof.

**Enumerated Embodiment 87.** The compound of Enumerated Embodiment 67, wherein the compound is: 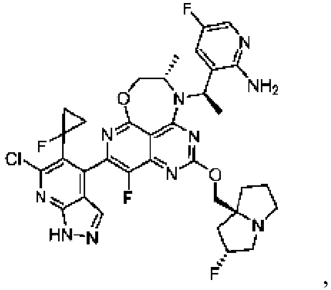 or a pharmaceutically acceptable salt or tautomer thereof.

**Enumerated Embodiment 88.** The compound of Enumerated Embodiment 67, wherein the compound is: 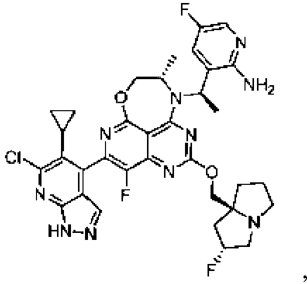 or a pharmaceutically acceptable salt or tautomer thereof.

**Enumerated Embodiment 89.** The compound of Enumerated Embodiment 67, wherein the compound is: 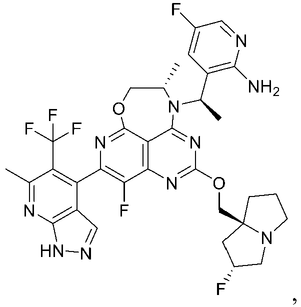 or a pharmaceutically acceptable salt or tautomer thereof.

**Enumerated Embodiment 90.** The compound of Enumerated Embodiment 67, wherein the compound is: 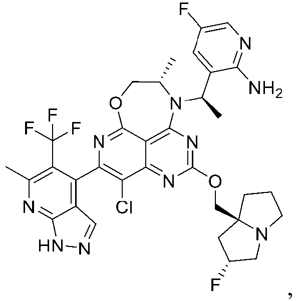 or a pharmaceutically acceptable salt or tautomer thereof.

**Enumerated Embodiment 91.** The compound of Enumerated Embodiment 67, wherein the compound is: 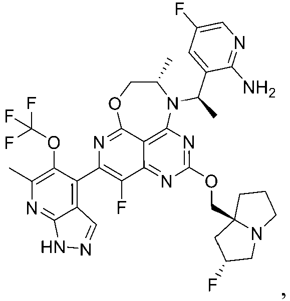 or a pharmaceutically acceptable salt or tautomer thereof.

**Enumerated** Embodiment 92. The compound of Enumerated Embodiment 67, wherein the compound is: 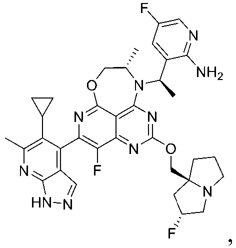 or a pharmaceutically acceptable salt or tautomer thereof.

**Enumerated** Embodiment 93. A compound represented by Formula VI: 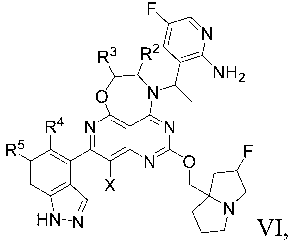 or a pharmaceutically acceptable salt, stereoisomer, or tautomer thereof, wherein
X is F or Cl;
R² and R³ are each independently selected from C₁-C₆ alkyl and hydrogen;
R⁴ is C₁-C₆ alkoxy or C₃-C₆ cycloalkyl, optionally substituted with one to three halogens or one to three methyls;
R⁵ is selected from hydrogen, deuterium, fluoro, and C₁-C₆ alkyl; and
wherein the compound is not:

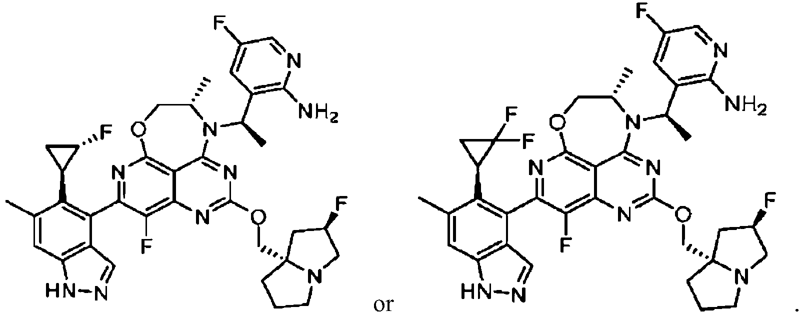

**Enumerated** Embodiment 94. The compound or a pharmaceutically acceptable salt, stereoisomer, or tautomer thereof of Enumerated Embodiment 93, wherein the compound is represented by Formula VI-1:

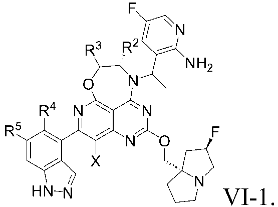

**Enumerated** Embodiment 95. The compound or a pharmaceutically acceptable salt, stereoisomer, or tautomer thereof of Enumerated Embodiment 93, wherein the compound is represented by Formula VI-2:

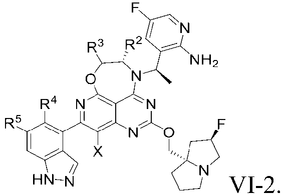

**Enumerated Embodiment 96.** The compound or a pharmaceutically acceptable salt, stereoisomer, or tautomer thereof of any one of Enumerated Embodiments 93-95, wherein R² is -C₁-C₃ alkyl.

**Enumerated Embodiment 97.** The compound or a pharmaceutically acceptable salt, stereoisomer, or tautomer thereof of any one of Enumerated Embodiments 93-95, wherein R² is -CH₃.

**Enumerated Embodiment 98.** The compound or a pharmaceutically acceptable salt, stereoisomer, or tautomer thereof of any one of claims 93-97, wherein R⁴ is C₁-C₆ alkoxy or C₄-C₆ cycloalkyl optionally substituted with one to three halogen.

**Enumerated Embodiment 99.** The compound or a pharmaceutically acceptable salt, stereoisomer, or tautomer thereof of any one of Enumerated Embodiments 93-97, wherein R⁴ is C₃-C₆ cycloalkyl optionally substituted with one to three halogen.

**Enumerated Embodiment 100.** The compound or a pharmaceutically acceptable salt, stereoisomer, or tautomer thereof of any one of Enumerated Embodiments 93-97, wherein R⁴ is 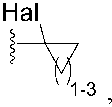 wherein 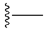 is the point of attachment to indazole, and Hal is a halogen.

**Enumerated Embodiment** 101. The compound or a pharmaceutically acceptable salt, stereoisomer, or tautomer thereof of any one of Enumerated Embodiments 98-100, wherein the halogen is fluoro.

**Enumerated Embodiment** 102. The compound of Enumerated Embodiment 93, wherein the compound is: 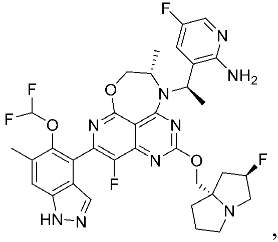 or pharmaceutically acceptable salt or tautomer thereof.

**Enumerated Embodiment 103.** The compound of Enumerated Embodiment 93, wherein the compound is: 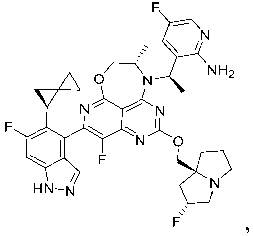 or pharmaceutically acceptable salt or tautomer thereof.

**Enumerated Embodiment 104.** The compound of Enumerated Embodiment 93, wherein the compound is: 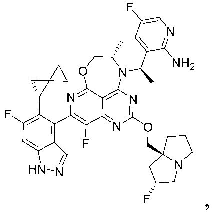 or pharmaceutically acceptable salt or tautomer thereof.

**Enumerated Embodiment 105.** The compound of Enumerated Embodiment 93, wherein the compound is: 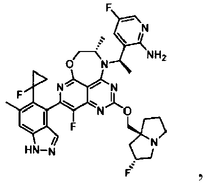 or pharmaceutically acceptable salt or tautomer thereof.

**Enumerated Embodiment 106.** The compound of Enumerated Embodiment 93, wherein the compound **is:** 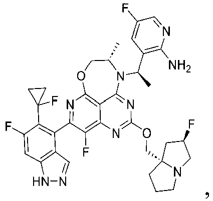 or pharmaceutically acceptable salt or tautomer thereof.

**Enumerated Embodiment 107.** The compound of Enumerated Embodiment 93, wherein the compound is: 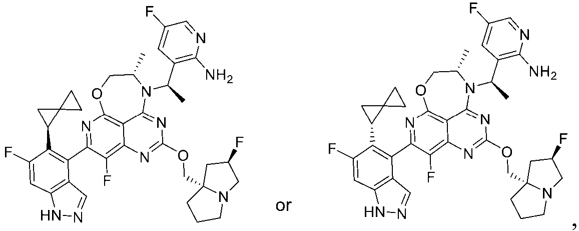 or pharmaceutically acceptable salt or tautomer thereof.

**Enumerated Embodiment 108.** The compound of Enumerated Embodiment 107, wherein the compound is the compound eluting after about 26 min from a chiral highpressure liquid chromatography (HPLC) using a first mobile phase comprising hexane with 0.2% isopropylamine and a second mobile phase comprising ethanol and dichloromethane in a 1:1 ratio.

**Enumerated Embodiment 109.** The compound of Enumerated Embodiment 107, wherein the compound is the compound eluting after about 30 min from a chiral highpressure liquid chromatography (HPLC) using a first mobile phase comprising hexane with 0.2% isopropylamine and a second mobile phase comprising ethanol and dichloromethane in a 1:1 ratio.

**Enumerated Embodiment 110.** The compound of Enumerated Embodiment 107 or 108, wherein the compound has a nuclear magnetic resonance (NMR) spectrum as shown in Example 12.

**Enumerated Embodiment 111.** The compound of Enumerated Embodiment 107 or 109, wherein the compound has an nuclear magnetic resonance (NMR) spectrum as shown in Example 13.

**Enumerated Embodiment 112.** The compound of Enumerated Embodiment 93, wherein the compound is: 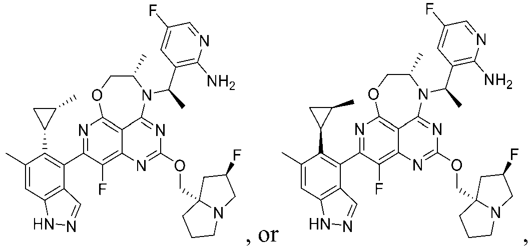 or pharmaceutically acceptable salt or tautomer thereof.

**Enumerated Embodiment 113.** A compound represented by Formula VII: 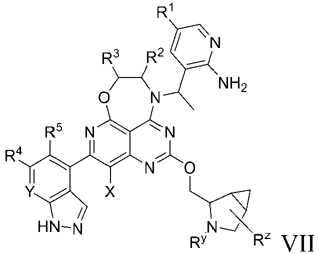 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen;
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen;
R⁵ is C₃-C₆ cycloalkyl optionally substituted with one to three halogen, or C₁-C₆ alkoxy substituted with one to three halogen; and
wherein when R¹ is halogen, R⁵ is C₁-C₆ alkoxy substituted with one to three halogen, or C₄-C₆ cycloalkyl.

**Enumerated Embodiment 114.** The compound or a pharmaceutically acceptable salt, stereoisomer, or tautomer thereof of Enumerated Embodiment 113, wherein the compound is represented by Formula VII-1 or VII-2:

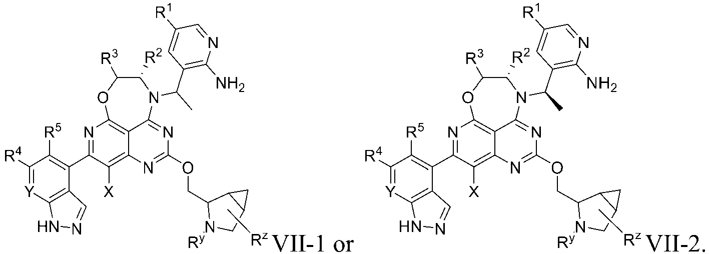

**Enumerated Embodiment 115.** The compound or a pharmaceutically acceptable salt, stereoisomer, or tautomer thereof of Enumerated Embodiment 113, wherein the compound is represented by Formula VII-3, VII-4, or VII-5:

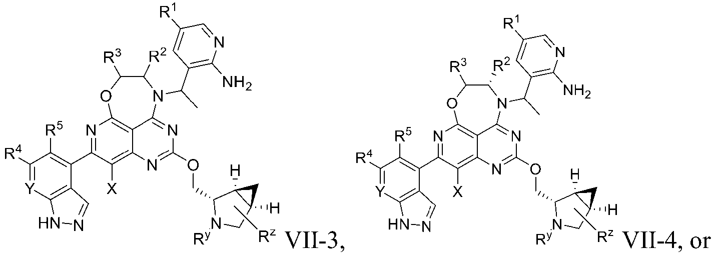

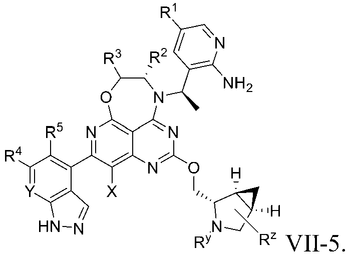

**Enumerated** Embodiment 116. A compound represented by Formula VIII: 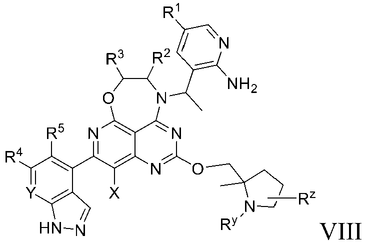 or a pharmaceutically acceptable salt, stereoisomer, or tautomer thereof, wherein:
X is F or Cl;
Y is N or CH;
R^{y} is C₁-C₆ alkyl;
R^{z} is hydrogen, halogen or OH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen;
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen; and
R⁵ is C₃-C₆ cycloalkyl optionally substituted with one to three halogen, or C₁-C₆ alkoxy substituted with one to three halogen;
wherein when R¹ is halogen, R⁵ is C₁-C₆ alkoxy substituted with one to three halogen, or C₄-C₆ cycloalkyl.

**Enumerated Embodiment 117.** The compound or a pharmaceutically acceptable salt, stereoisomer, or tautomer thereof of Enumerated Embodiment 116, wherein the compound is represented by Formula VIII-1 or VIII-2:

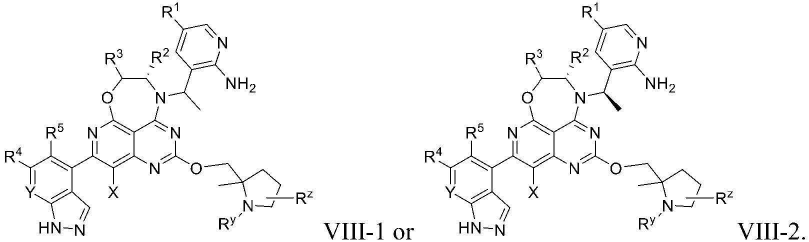

**Enumerated Embodiment 118.** The compound or a pharmaceutically acceptable salt, stereoisomer, or tautomer thereof of Enumerated Embodiment 116, wherein the compound is represented by Formula VIII-3, VIII-4, or VIII-5: 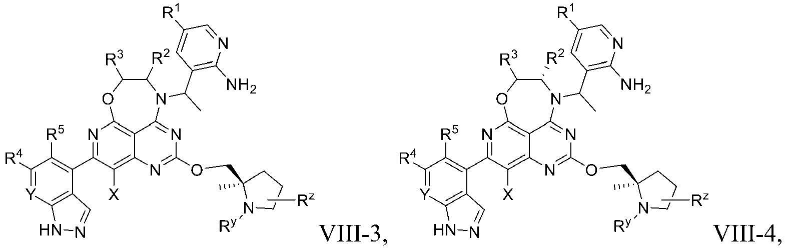 or

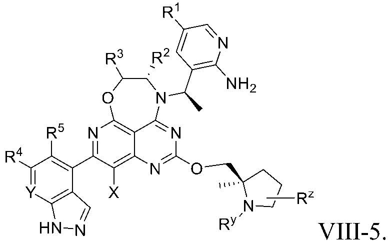

**Enumerated Embodiment 119.** The compound or a pharmaceutically acceptable salt, stereoisomer, or tautomer thereof of any one of Enumerated Embodiments 113-118, wherein Y is CH.

**Enumerated Embodiment 120.** The compound or a pharmaceutically acceptable salt, stereoisomer, or tautomer thereof of any one of Enumerated Embodiments 113-118, wherein Y is N.

**Enumerated Embodiment 121.** The compound or a pharmaceutically acceptable salt, stereoisomer, or tautomer thereof of any one of Enumerated Embodiments 113-120, wherein X is F.

**Enumerated Embodiment 122.** The compound or a pharmaceutically acceptable salt, stereoisomer, or tautomer thereof of any one of Enumerated Embodiments 113-121, wherein R^{z} is H.

**Enumerated Embodiment 123.** The compound or a pharmaceutically acceptable salt, stereoisomer, or tautomer thereof of any one of Enumerated Embodiments 113-121, wherein R^{z} is halogen or OH.

**Enumerated Embodiment 124.** The compound or a pharmaceutically acceptable salt, stereoisomer, or tautomer thereof of any one of Enumerated Embodiments 113-121, wherein R^{z} is Cl or F.

**Enumerated Embodiment 125.** The compound or a pharmaceutically acceptable salt, stereoisomer, or tautomer thereof of any one of Enumerated Embodiments 113-124, wherein R ^{y} is C₁-C₃ alkyl.

**Enumerated Embodiment 126.** The compound or a pharmaceutically acceptable salt, stereoisomer, or tautomer thereof of any one of Enumerated Embodiments 113-124, wherein R^{y} is -CH₃.

**Enumerated Embodiment 127.** The compound or a pharmaceutically acceptable salt, stereoisomer, or tautomer thereof of any one of Enumerated Embodiments 113-126, wherein R¹ is unsubstituted C₃-C₆ cycloalkyl.

**Enumerated Embodiment 128.** The compound or a pharmaceutically acceptable salt, stereoisomer, or tautomer thereof of any one of Enumerated Embodiments 113-127, wherein the C₃-C₆ cycloalkyl of R¹ is cyclopropyl.

**Enumerated Embodiment 129.** The compound or a pharmaceutically acceptable salt, stereoisomer, or tautomer thereof of any one of Enumerated Embodiments 113-126, wherein R¹ is C₁-C₆ alkoxy substituted with two or three halogen.

**Enumerated Embodiment 130.** The compound or a pharmaceutically acceptable salt, stereoisomer, or tautomer thereof of any one of Enumerated Embodiments 113-126, wherein R¹ is C₁-C₃ alkoxy substituted with two or three halogen.

**Enumerated Embodiment 131.** The compound or a pharmaceutically acceptable salt, stereoisomer, or tautomer thereof of any one of claims 113-124, wherein R¹ is methoxy substituted with two or three halogen.

**Enumerated Embodiment 132.** The compound or a pharmaceutically acceptable salt, stereoisomer, or tautomer thereof of any one of Enumerated Embodiments 113-124, wherein R¹ is -OCHF₂.

**Enumerated Embodiment 133.** The compound or a pharmaceutically acceptable salt, stereoisomer, or tautomer thereof of any one of Enumerated Embodiments 113-124, wherein R¹ is -OCF₃.

**Enumerated Embodiment 134.** The compound or a pharmaceutically acceptable salt, stereoisomer, or tautomer thereof of any one of Enumerated Embodiments 113-133, wherein R² and R³ are each independently selected from C₁-C₃ alkyl and hydrogen.

**Enumerated Embodiment 135.** The compound or a pharmaceutically acceptable salt, stereoisomer, or tautomer thereof of any one of Enumerated Embodiments 113-133, wherein R² and R³ are each independently selected from -CH₃ and hydrogen.

**Enumerated Embodiment 136.** The compound or a pharmaceutically acceptable salt, stereoisomer, or tautomer thereof of any one of Enumerated Embodiments 113-135, wherein R³ is hydrogen.

**Enumerated Embodiment 137.** The compound or a pharmaceutically acceptable salt, stereoisomer, or tautomer thereof of any one of Enumerated Embodiments 113-136, wherein R² is -CH₃.

**Enumerated Embodiment 138.** The compound or a pharmaceutically acceptable salt, stereoisomer, or tautomer thereof of any one of Enumerated Embodiments 113-137, wherein R⁴ is halogen.

**Enumerated Embodiment 139.** The compound or a pharmaceutically acceptable salt, stereoisomer, or tautomer thereof of any one of Enumerated Embodiments 113-137, wherein R⁴ is chloro.

**Enumerated Embodiment 140.** The compound or a pharmaceutically acceptable salt, stereoisomer, or tautomer thereof of any one of Enumerated Embodiments 113-137, wherein R⁴ is unsubstituted C₁-C₆ alkyl.

**Enumerated Embodiment 141.** The compound or a pharmaceutically acceptable salt, stereoisomer, or tautomer thereof of any one of Enumerated Embodiments 113-137, wherein R⁴ is unsubstituted C₁-C₃ alkyl.

**Enumerated Embodiment 142.** The compound or a pharmaceutically acceptable salt, stereoisomer, or tautomer thereof of any one of Enumerated Embodiments 113-137, wherein R⁴ is -CH₃.

**Enumerated Embodiment 143.** The compound or a pharmaceutically acceptable salt, stereoisomer, or tautomer thereof of any one of Enumerated Embodiments 113-142, wherein R⁵ is C₃-C₆ cycloalkyl optionally substituted with one to three halogen.

**Enumerated Embodiment 144.** The compound or a pharmaceutically acceptable salt, stereoisomer, or tautomer thereof of any one of Enumerated Embodiments 113-142, wherein R⁵ is cyclopropyl optionally substituted with one to three halogen.

**Enumerated Embodiment 145.** The compound or a pharmaceutically acceptable salt, stereoisomer, or tautomer thereof of any one of Enumerated Embodiments 113-142, wherein R⁵ is cyclopropyl substituted with one halogen.

**Enumerated Embodiment 146.** The compound or a pharmaceutically acceptable salt, stereoisomer, or tautomer thereof of any one of Enumerated Embodiments 113-145, wherein the halogen is fluoro.

**Enumerated Embodiment 147.** The compound or a pharmaceutically acceptable salt, stereoisomer, or tautomer thereof of any one of Enumerated Embodiments 113-145, wherein R⁵ is unsubstituted cyclopropyl.

**Enumerated Embodiment 148.** The compound or a pharmaceutically acceptable salt, stereoisomer, or tautomer thereof of any one of Enumerated Embodiments 113-145, wherein R⁵ is methoxy optionally substituted with one to three halogen.

**Enumerated Embodiment 149.** The compound or a pharmaceutically acceptable salt, stereoisomer, or tautomer thereof of any one of Enumerated Embodiments 113-145, wherein R⁵ is -OCHF₂.

**Enumerated Embodiment 150.** The compound or a pharmaceutically acceptable salt, stereoisomer, or tautomer thereof of any one of Enumerated Embodiments 113-145, wherein R⁵ is -OCF₃.

**Enumerated Embodiment 151.** The compound of Enumerated Embodiment 113, wherein the compound is: 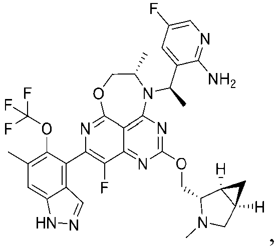 or pharmaceutically acceptable salt or tautomer thereof.

**Enumerated Embodiment 152.** The compound of Enumerated Embodiment 113, wherein the compound is: 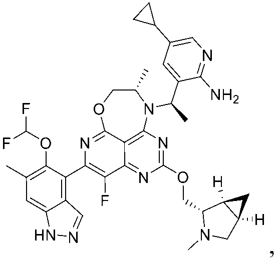 or pharmaceutically acceptable salt or tautomer thereof.

**Enumerated Embodiment 153.** The compound of Enumerated Embodiment 113, wherein the compound is: 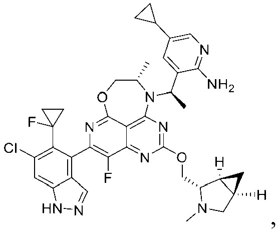 or pharmaceutically acceptable salt or tautomer thereof.

**Enumerated Embodiment 154.** The compound of Enumerated Embodiment 113, wherein the compound is: 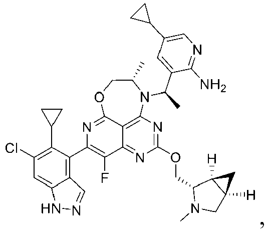 or pharmaceutically acceptable salt or tautomer thereof.

**Enumerated Embodiment 155.** The compound of Enumerated Embodiment 113, wherein the compound is: 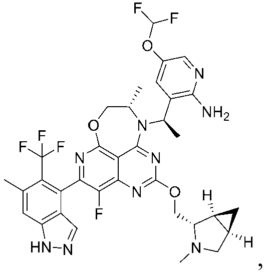 or pharmaceutically acceptable salt or tautomer thereof.

**Enumerated Embodiment 156.** The compound of Enumerated Embodiment 113, wherein the compound is: 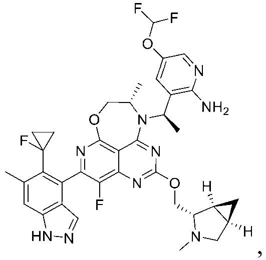 or pharmaceutically acceptable salt or tautomer thereof.

**Enumerated Embodiment 157.** The compound of Enumerated Embodiment 113, wherein the compound is: 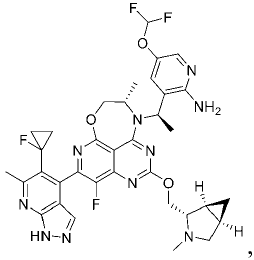 or pharmaceutically acceptable salt or tautomer thereof.

Enumerated Embodiment 158. The compound of Enumerated Embodiment 116, wherein the compound is: 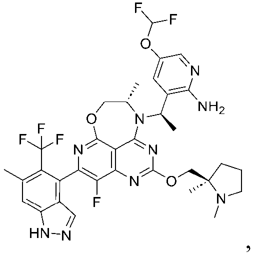 or pharmaceutically acceptable salt or tautomer thereof.

**Enumerated Embodiment 159.** The compound of Enumerated Embodiment 116, wherein the compound is: 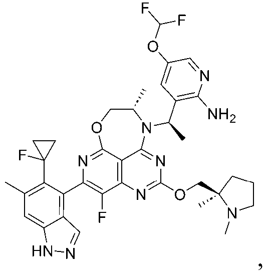 or pharmaceutically acceptable salt or tautomer thereof.

Enumerated Embodiment 160. The compound of Enumerated Embodiment 116, wherein the compound is: 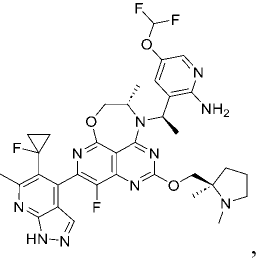 or pharmaceutically acceptable salt or tautomer thereof.

**Enumerated Embodiment 161.** A compound represented by Formula IX: 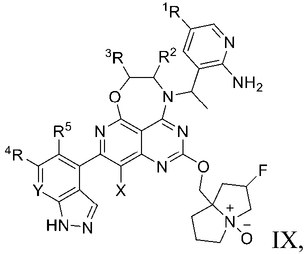 or a pharmaceutically acceptable salt, stereoisomer, or tautomer thereof, wherein:
X is F or Cl;
Y is N or CH;
R¹ is halogen, C₃-C₆ cycloalkyl optionally substituted with one to three halogen; or C₁-C₆ alkoxy substituted with one to three halogen;
R² and R³ are each independently selected from C₁-C₆ alkyl and hydrogen;
R⁴ is selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogen;
R⁵ is C₃-C₆ cycloalkyl optionally substituted with one to three halogen, or C₁-C₆ alkoxy substituted with one to three halogen; and
wherein when R¹ is halogen, R⁵ is C₁-C₆ alkoxy substituted with one to three halogen, or C₄-C₆ cycloalkyl.

**Enumerated Embodiment 162.** The compound or a pharmaceutically acceptable salt, stereoisomer, or tautomer thereof of Enumerated Embodiment 161, wherein the compound is represented by Formula IX-1, IX-2, or IX-3: 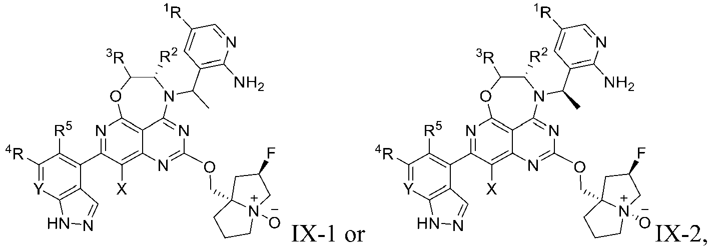 or

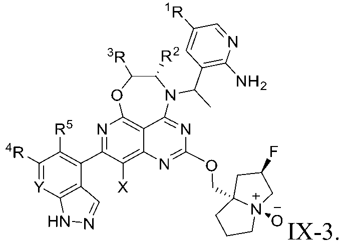

**Enumerated Embodiment 163.** The compound or a pharmaceutically acceptable salt, stereoisomer, or tautomer thereof of any one of Enumerated Embodiments 160-162, wherein Y is CH.

**Enumerated Embodiment 164.** The compound or a pharmaceutically acceptable salt, stereoisomer, or tautomer thereof of any one of Enumerated Embodiments 160-162, wherein Y is N.

**Enumerated Embodiment 165.** The compound or a pharmaceutically acceptable salt, stereoisomer, or tautomer thereof of any one of Enumerated Embodiments 160-164, wherein X is F.

**Enumerated Embodiment 166.** The compound or a pharmaceutically acceptable salt, stereoisomer, or tautomer thereof of any one of Enumerated Embodiments 160-165, wherein R¹ is unsubstituted C₃-C₆ cycloalkyl.

**Enumerated Embodiment 167.** The compound or a pharmaceutically acceptable salt, stereoisomer, or tautomer thereof of any one of Enumerated Embodiments 160-166, wherein the C₃-C₆ cycloalkyl of R¹ is cyclopropyl.

**Enumerated Embodiment 168.** The compound or a pharmaceutically acceptable salt, stereoisomer, or tautomer thereof of any one of Enumerated Embodiments 160-165, wherein R¹ is C₁-C₆ alkoxy substituted with two or three halogen.

**Enumerated Embodiment 169.** The compound or a pharmaceutically acceptable salt, stereoisomer, or tautomer thereof of any one of Enumerated Embodiments 160-165, wherein R¹ is C₁-C₃ alkoxy substituted with two or three halogen.

**Enumerated Embodiment 170.** The compound or a pharmaceutically acceptable salt, stereoisomer, or tautomer thereof of any one of Enumerated Embodiments 160-165, wherein R¹ is methoxy substituted with two or three halogen.

**Enumerated Embodiment 171.** The compound or a pharmaceutically acceptable salt, stereoisomer, or tautomer thereof of any one of Enumerated Embodiments 160-165, wherein R¹ is -OCHF₂.

**Enumerated Embodiment 172.** The compound or a pharmaceutically acceptable salt, stereoisomer, or tautomer thereof of any one of Enumerated Embodiments 160-165, wherein R¹ is -OCF₃.

**Enumerated Embodiment 173.** The compound or a pharmaceutically acceptable salt, stereoisomer, or tautomer thereof of any one of Enumerated Embodiments 160-172, wherein R² and R³ are each independently selected from C₁-C₃ alkyl and hydrogen.

**Enumerated Embodiment 174.** The compound or a pharmaceutically acceptable salt, stereoisomer, or tautomer thereof of any one of Enumerated Embodiments 160-172, wherein R² and R³ are each independently selected from -CH₃ and hydrogen.

**Enumerated Embodiment 175.** The compound or a pharmaceutically acceptable salt, stereoisomer, or tautomer thereof of any one of Enumerated Embodiments 160-174, wherein R³ is hydrogen.

**Enumerated Embodiment 176.** The compound or a pharmaceutically acceptable salt, stereoisomer, or tautomer thereof of any one of Enumerated Embodiments 160-175, wherein R² is -CH₃.

**Enumerated Embodiment 177.** The compound or a pharmaceutically acceptable salt, stereoisomer, or tautomer thereof of any one of Enumerated Embodiments 160-176, wherein R⁴ is unsubstituted C₁-C₆ alkyl.

**Enumerated Embodiment 178.** The compound or a pharmaceutically acceptable salt, stereoisomer, or tautomer thereof of any one of Enumerated Embodiments 160-176, wherein R⁴ is halogen.

**Enumerated Embodiment 179.** The compound or a pharmaceutically acceptable salt, stereoisomer, or tautomer thereof of any one of Enumerated Embodiments 160-176, wherein R⁴ is unsubstituted C₁-C₃ alkyl.

**Enumerated Embodiment 180.** The compound or a pharmaceutically acceptable salt, stereoisomer, or tautomer thereof of any one of Enumerated Embodiments 160-176, wherein R⁴ is -CH₃.

**Enumerated Embodiment 181.** The compound or a pharmaceutically acceptable salt, stereoisomer, or tautomer thereof of any one of Enumerated Embodiments 160-180, wherein R⁵ is C₃-C₆ cycloalkyl optionally substituted with one to three halogen.

**Enumerated Embodiment 182.** The compound or a pharmaceutically acceptable salt, stereoisomer, or tautomer thereof of any one of Enumerated Embodiments 160-180, wherein R⁵ is cyclopropyl optionally substituted with one to three halogen.

**Enumerated Embodiment 183.** The compound or a pharmaceutically acceptable salt, stereoisomer, or tautomer thereof of any one of Enumerated Embodiments 160-180, wherein R⁵ is cyclopropyl substituted with one halogen.

**Enumerated Embodiment 184.** The compound or a pharmaceutically acceptable salt, stereoisomer, or tautomer thereof of any one of Enumerated Embodiments 160-183, wherein the halogen is fluoro.

**Enumerated Embodiment 185.** The compound or a pharmaceutically acceptable salt, stereoisomer, or tautomer thereof of any one of Enumerated Embodiments 160-180, wherein R⁵ is unsubstituted cyclopropyl.

**Enumerated Embodiment 186.** The compound or a pharmaceutically acceptable salt, stereoisomer, or tautomer thereof of any one of Enumerated Embodiments 160-180, wherein R⁵ is methoxy optionally substituted with one to three halogen.

**Enumerated Embodiment 187.** The compound or a pharmaceutically acceptable salt, stereoisomer, or tautomer thereof of any one of Enumerated Embodiments 160-180, wherein R⁵ is -OCHF₂ or -OCF₃.

**Enumerated Embodiment 188.** The compound or a pharmaceutically acceptable salt, stereoisomer, or tautomer thereof of any one of Enumerated Embodiments 160-180, wherein R⁵ is -CF₃.

**Enumerated Embodiment 189.** The compound of Enumerated Embodiment 160, wherein the compound is: 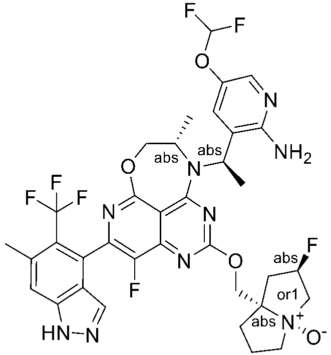 , or pharmaceutically acceptable salt or tautomer thereof.

**Enumerated Embodiment 190.** The compound of Enumerated Embodiment 160, wherein the compound is: 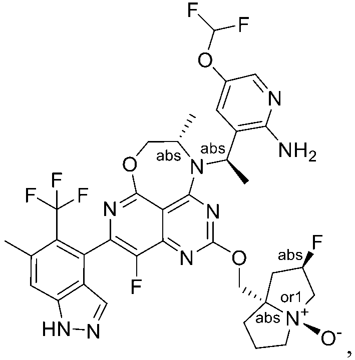 or pharmaceutically acceptable salt or tautomer thereof.

**Enumerated Embodiment 191.** The compound or a pharmaceutically acceptable salt, stereoisomer, or tautomer thereof of any one of the preceding Enumerated Embodiments, wherein the compound or pharmaceutically acceptable salt, stereoisomer, or tautomer thereof is a pan-KRAS inhibitor.

**Enumerated Embodiment 192.** A pharmaceutical composition comprising the compound or a pharmaceutically acceptable salt, stereoisomer, or tautomer thereof of any one of Enumerated Embodiments 1-191, and a pharmaceutically acceptable excipient.

**Enumerated Embodiment 193.** A method of treating a Ras-related disease or disorder in a patient in need thereof, comprising administering to the patient a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 194.** Use of the compound or a pharmaceutically acceptable salt, stereoisomer, or tautomer thereof of any one of Enumerated Embodiments 1-191 in the manufacture of a medicament for the treatment of a Ras-related disease or disorder in a patient in need thereof.

**Enumerated Embodiment 195.** The method of Enumerated Embodiment 193 or the use of Enumerated Embodiment 194, wherein the disease or disorder is characterized by aberrant Ras activity in the patient due to a Ras mutation.

**Enumerated Embodiment 196.** The method of Enumerated Embodiment 193 or 195 or the use of claim 164, wherein the Ras mutation is a K-Ras mutation, a H-Ras mutation, or a N-Ras mutation.

**Enumerated Embodiment 197.** The method or use of Enumerated Embodiment 196, wherein the Ras mutation is a K-Ras G12C mutation.

**Enumerated Embodiment 198.** The method or use of Enumerated Embodiment 196, wherein the Ras mutation is a K-Ras G12D mutation.

**Enumerated Embodiment 199.** The method or use of Enumerated Embodiment 196, wherein the Ras mutation is a K-Ras G12V mutation.

**Enumerated Embodiment 200.** The method or use of any one of Enumerated Embodiments 193-199, wherein the disease or disorder is a cancer.

**Enumerated Embodiment 201.** A method of treating a cancer in a patient in need thereof, comprising administering to the patient a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 202.** Use of the compound or a pharmaceutically acceptable salt, stereoisomer, or tautomer thereof of any one of Enumerated Embodiments 1-191 in the manufacture of a medicament for the treatment of cancer in a patient in need thereof.

**Enumerated Embodiment 203.** The method of Enumerated Embodiment 201 or the use of Enumerated Embodiment 202, wherein the cancer is a Ras-mutated cancer.

**Enumerated Embodiment 204.** The method or use of any one of Enumerated Embodiments 201-203, wherein the cancer is a K-Ras-mutated cancer.

**Enumerated Embodiment 205.** The method or use of Enumerated Embodiment 204, wherein the cancer is a K-Ras G12C-mutated cancer.

**Enumerated Embodiment 206.** The method or use of Enumerated Embodiment 204, wherein the cancer is a K-Ras G12D-mutated cancer.

**Enumerated Embodiment 207.** The method or use of Enumerated Embodiment 204, wherein the cancer is a K-Ras G12V-mutated cancer.

**Enumerated Embodiment 208.** A method for inhibiting K-Ras G12C in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 202.

**Enumerated Embodiment 209.** Use of the compound or a pharmaceutically acceptable salt, stereoisomer, or tautomer thereof of any one of Enumerated Embodiments 1-191 in the manufacture of a medicament for inhibiting K-Ras G12C in a subject in need thereof.

**Enumerated Embodiment 210.** A method for inhibiting K-Ras G12D in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 211.** Use of the compound or a pharmaceutically acceptable salt, stereoisomer, or tautomer thereof of any one of Enumerated Embodiments 1-191 in the manufacture of a medicament for inhibiting K-Ras G12D in a subject in need thereof.

**Enumerated Embodiment 212.** A method for inhibiting K-Ras G12V in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 213.** Use of the compound or a pharmaceutically acceptable salt, stereoisomer, or tautomer thereof of any one of Enumerated Embodiments 1-191 in the manufacture of a medicament for inhibiting K-Ras G12V in a subject in need thereof.

**Enumerated Embodiment 214.** A method for inhibiting K-Ras G12C and G12D in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 215.** Use of the compound or a pharmaceutically acceptable salt, stereoisomer, or tautomer thereof of any one of Enumerated Embodiments 1-191 in the manufacture of a medicament for inhibiting K-Ras G12C and G12D in a subject in need thereof.

**Enumerated Embodiment 216.** A method for inhibiting K-Ras G12D and G12V in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 217.** Use of the compound or a pharmaceutically acceptable salt, stereoisomer, or tautomer thereof of any one of Enumerated Embodiments 1-191 in the manufacture of a medicament for inhibiting K-Ras G12D and G12V in a subject in need thereof.

**Enumerated Embodiment 218.** A method for inhibiting K-Ras G12C and G12V in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 219.** Use of the compound or a pharmaceutically acceptable salt, stereoisomer, or tautomer thereof of any one of Enumerated Embodiments 1-191 in the manufacture of a medicament for inhibiting K-Ras G12C and G12V in a subject in need thereof.

**Enumerated Embodiment 220.** A method for inhibiting K-Ras G12C, G12D, and G12V in a subject in need thereof, comprising administering to the subject a therapeutically effective amount of the compound or a pharmaceutically acceptable salt, stereoisomer, or tautomer thereof of any one of Enumerated Embodiments 1-191, or the pharmaceutical composition of Enumerated Embodiment 192.

**Enumerated Embodiment 221.** Use of the compound or a pharmaceutically acceptable salt, stereoisomer, or tautomer thereof of any one of Enumerated Embodiments 1-191 in the manufacture of a medicament for inhibiting K-Ras G12C, G12D, and G12V in a subject in need thereof.

**Enumerated Embodiment 222.** The method or use of any one of Enumerated Embodiments 208-211 wherein the subject has cancer.

**Enumerated Embodiment 223.** The method or use of Enumerated Embodiment 222, wherein the cancer is a Ras-mutated cancer.

**Enumerated Embodiment 224.** The method or use of Enumerated Embodiment 222 or 223, wherein the cancer is a K-Ras-mutated cancer.

**Enumerated Embodiment 225.** The method or use of Enumerated Embodiment 224, wherein the cancer is a K-Ras G12C-mutated cancer.

**Enumerated Embodiment 226.** The method or use of Enumerated Embodiment 224, wherein the cancer is a K-Ras G12D-mutated cancer.

**Enumerated Embodiment 227.** The method or use of Enumerated Embodiment 224, wherein the cancer is a K-Ras G12V-mutated cancer.

**Enumerated Embodiment 228.** A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of any one of claims 1-13, or the pharmaceutical composition of claim 14.

**Enumerated Embodiment 229.** A compound according to Formulae I-IX, or a pharmaceutically acceptable salt, stereoisomer, or tautomer thereof.

**Enumerated Embodiment 230.** A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of the compound according to Formulae I-IX.

**Enumerated Embodiment 231.** A process for preparing 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine, or a pharmaceutically acceptable salt thereof, the process comprising:
(i) reacting 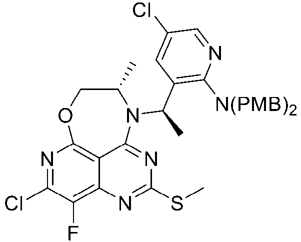 with 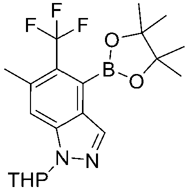 to form 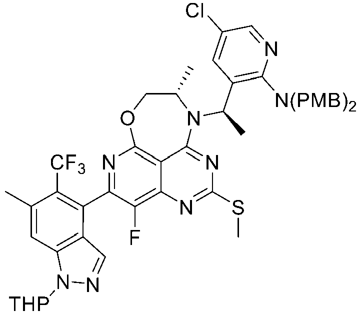 or a salt thereof;
(ii) reacting 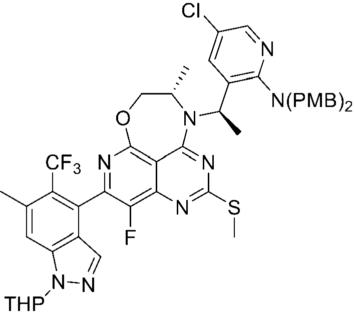 with KOH in the presence of a catalyst to form 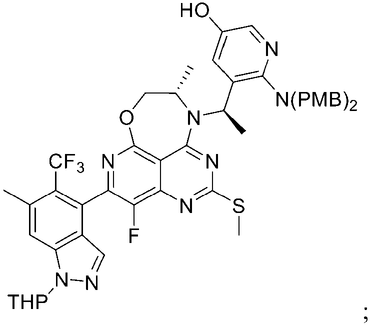
(iii) reacting 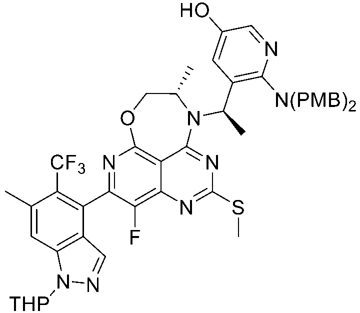 with ClCF₂COONa, or an alternative salt thereof to form 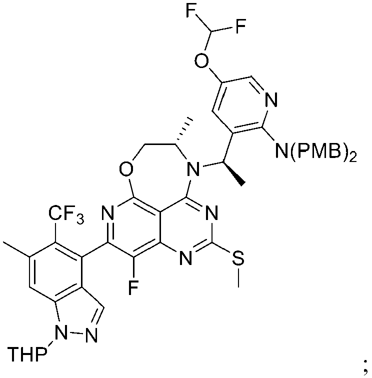
(iv) reacting 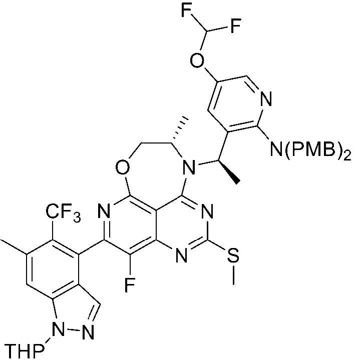 with m-CPBA to form 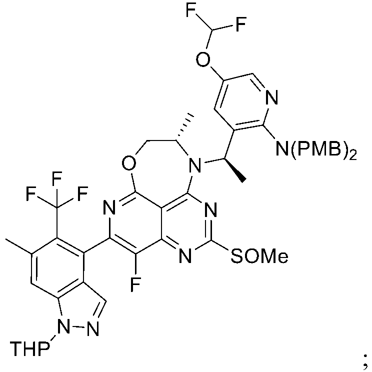
(v) reacting 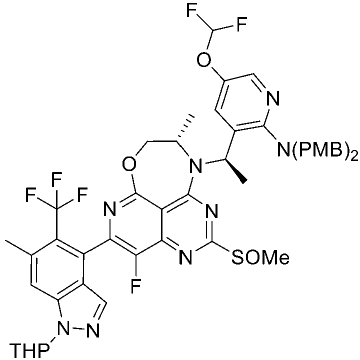 with 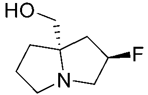 to form 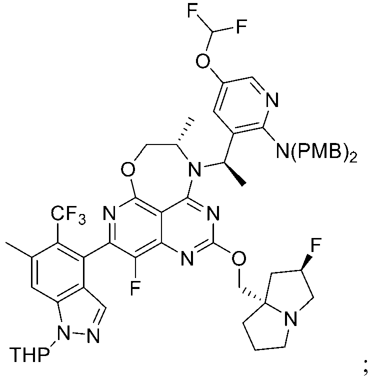 and
(vi) deprotecting 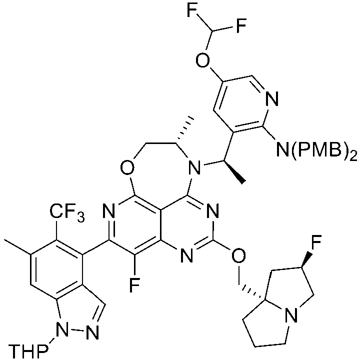 with triflic acid to form

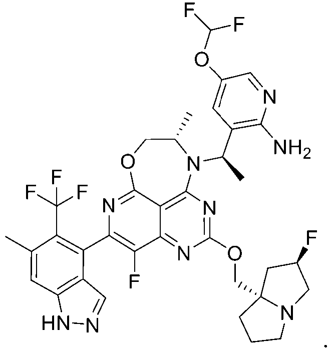

**Enumerated Embodiment 232.** A process for preparing 3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)-5-isopropoxypyridin-2-amine, or a pharmaceutically acceptable salt thereof, the process comprising:
(i) reacting 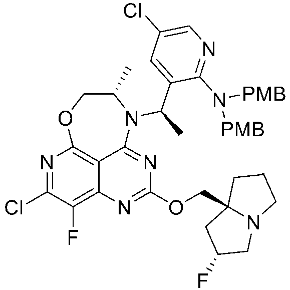 with 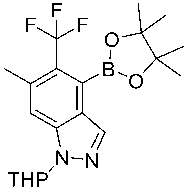 to form 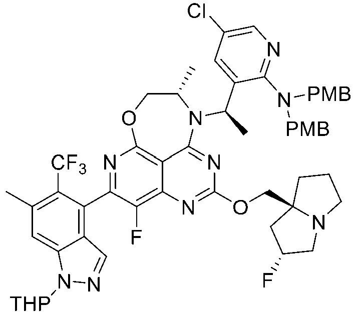 or a salt thereof;
(ii) reacting 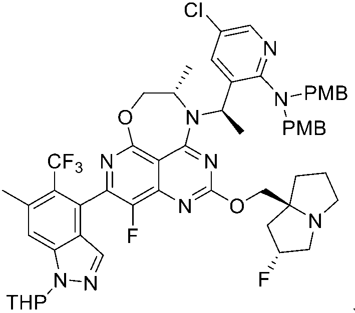 with KOH in the presence of a catalyst to form 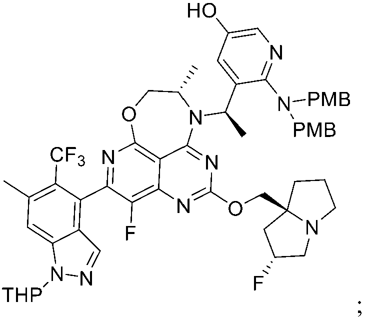
(iii) reacting 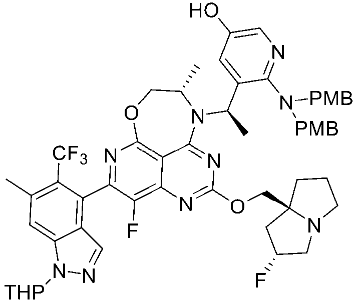 with 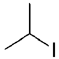 to form 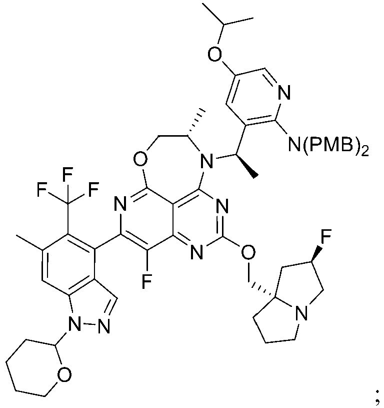
(iv) deprotecting 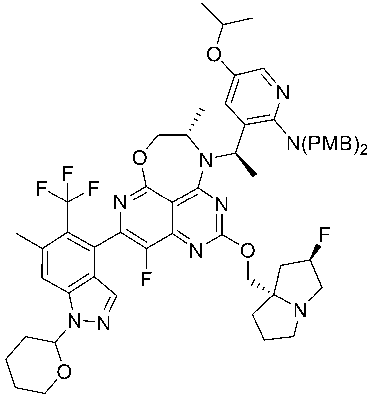 with trifluoroacetic acid to form

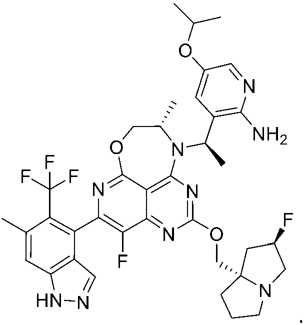

**Enumerated Embodiment 233.** A compound represented by Formula IV: 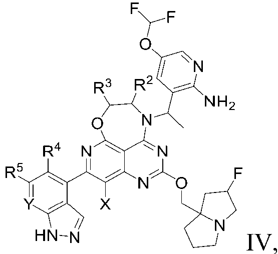 or a pharmaceutically acceptable salt, stereoisomer, or tautomer thereof, wherein
X is F or Cl;
Y is N or CH;
R² and R³ are each independently selected from C₁-C₆ alkyl and hydrogen; and
R⁴ and R⁵ are independently selected from hydrogen, halogen, deuterium, C₁-C₆ alkyl, C₁-C₆ alkoxy, and C₃-C₆ cycloalkyl wherein the alkyl, alkoxy, and cycloalkyl are optionally substituted with one to three halogens, or one to three methyls.

**Enumerated Embodiment 234.** The compound or a pharmaceutically acceptable salt, stereoisomer, or tautomer thereof of Enumerated Embodiment 233, wherein R⁴ is halogen, C₁-C₆ alkyl, or C₃-C₆ cycloalkyl, wherein the alkyl and cycloalkyl are optionally substituted with one to three halogens or one to three methyls.

**Enumerated Embodiment 235.** The compound or a pharmaceutically acceptable salt, stereoisomer, or tautomer thereof of Enumerated Embodiment 233, wherein R5 is - C1-C3 alkyl.

**Enumerated Embodiment 236.** The compound or a pharmaceutically acceptable salt, stereoisomer, or tautomer thereof of Enumerated Embodiment 233 or Enumerated Embodiment 235, wherein the compound is represented by Formula IV-a3 or Formula IV-b3: 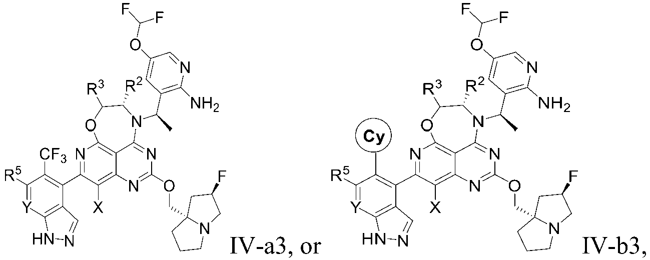 wherein 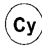 is a C3-C6 cycloalkyl, optionally substituted with one to three halogens.

**Enumerated Embodiment 237.** The compound or a pharmaceutically acceptable salt, stereoisomer, or tautomer thereof of any one of Enumerated Embodiments 233-236, wherein R3 is H.

**Enumerated Embodiment 238.** The compound or a pharmaceutically acceptable salt, stereoisomer, or tautomer thereof of any one of Enumerated Embodiment 233-237, wherein R² is -C₁-C₃ alkyl.

**Enumerated Embodiment 239.** A compound selected from the group consisting of: 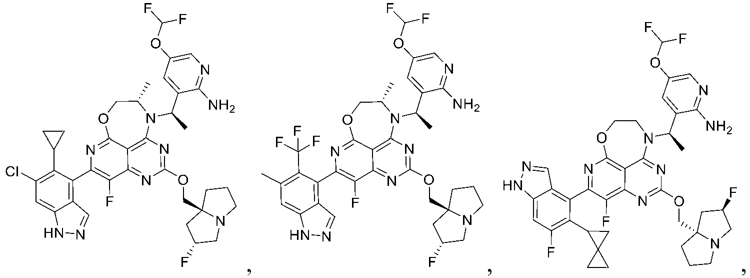 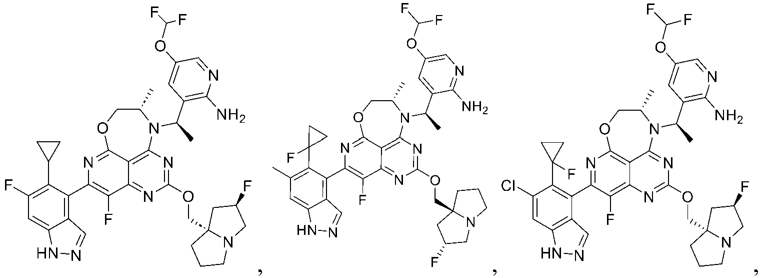 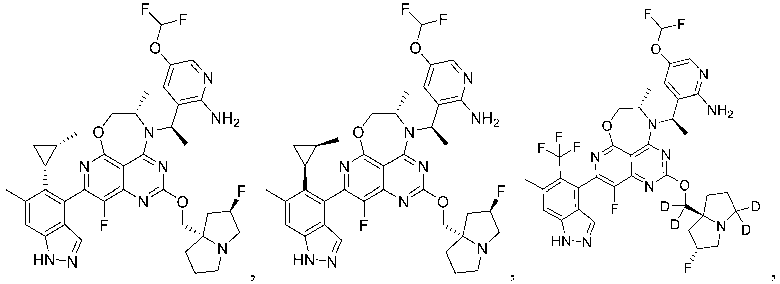 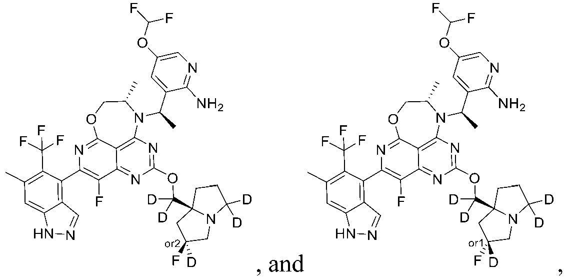 or a pharmaceutically acceptable salt, stereoisomer, or tautomer thereof.

**Enumerated Embodiment 240.** The compound of Enumerated Embodiment 239, wherein the compound is

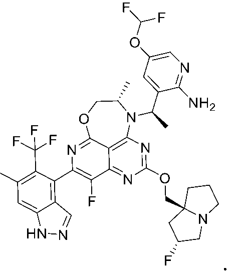

**Enumerated Embodiment 241.** The compound of Enumerated Embodiment 239, wherein the compound is a pharmaceutically acceptable salt of

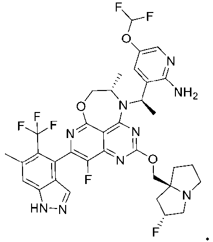

**Enumerated Embodiment 242.** A compound selected from the group consisting of: 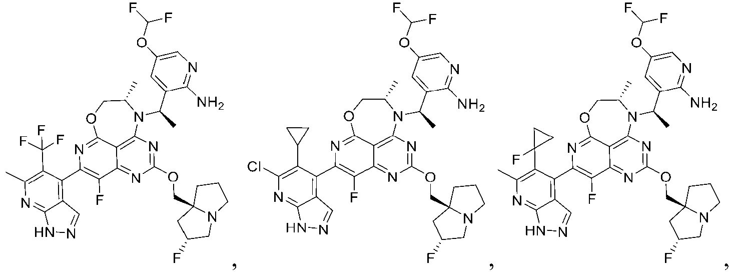 or a pharmaceutically acceptable salt, stereoisomer, or tautomer thereof.

**Enumerated Embodiment 243.** The compound of Enumerated Embodiment 242, wherein the compound is

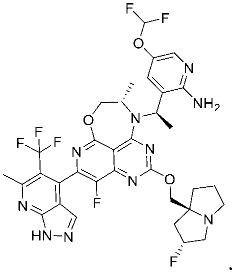

**Enumerated Embodiment 244.** The compound of Enumerated Embodiment 242, wherein the compound is a pharmaceutically acceptable salt of

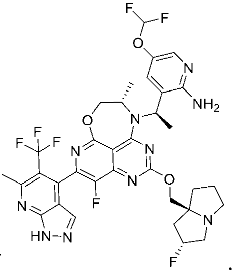

**Enumerated Embodiment 245.** The compound of Enumerated Embodiment 242, wherein the compound is

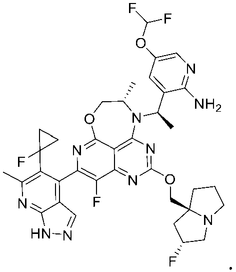

**Enumerated Embodiment 246.** The compound of Enumerated Embodiment 242,
wherein the compound is a pharmaceutically acceptable salt of

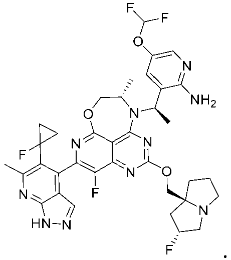

**Enumerated Embodiment 247.** A pharmaceutical composition comprising the compound or a pharmaceutically acceptable salt, stereoisomer, or tautomer thereof of any one of Enumerated Embodiments 233-246, and a pharmaceutically acceptable excipient.

**Enumerated Embodiment 248.** A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of any one of Enumerated Embodiments 233-246, or the pharmaceutical composition of Enumerated Embodiments 247.

**Enumerated Embodiment 249.** A compound according to Formulae I-IX, or a pharmaceutically acceptable salt, stereoisomer, or tautomer thereof.

**Enumerated Embodiment 250.** A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of the compound of Enumerated Embodiments 249.

**Enumerated Embodiment 251.** A process for preparing 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-pyrazolo[3,4-b]pyridin-4-yl)-8,9-dihydro-10H-7-oxa-1,3,6,10-tetraazacyclohepta[de]naphthalen-10-yl)ethyl)pyridin-2-amine, or a pharmaceutically acceptable salt thereof, the process comprising:
(i) brominating 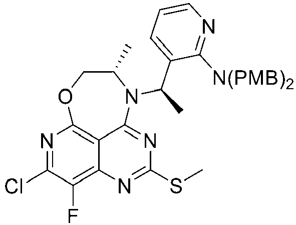 with NBS to form 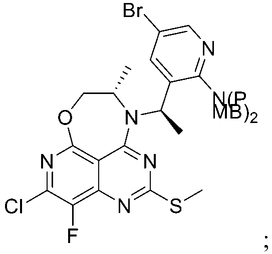
(ii) oxidizing 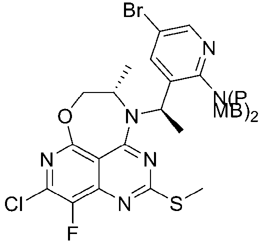 with m-CPBA to form 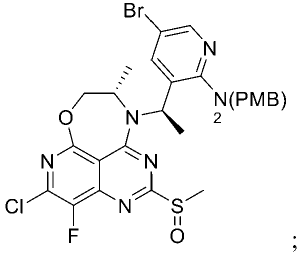
(iii) reacting 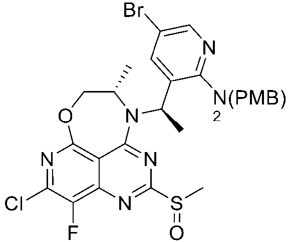 with 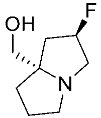 to form 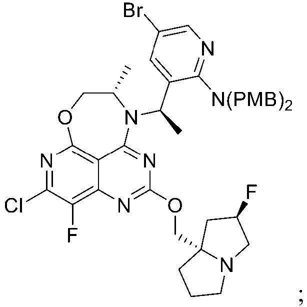
(iv) reacting 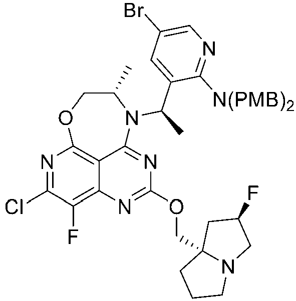 with 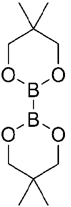 to form 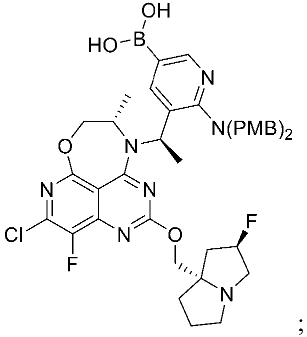
(v) reacting 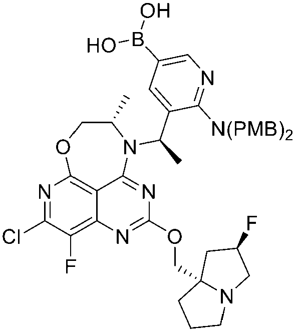 with hydrogen peroxide under acidic conditions to form 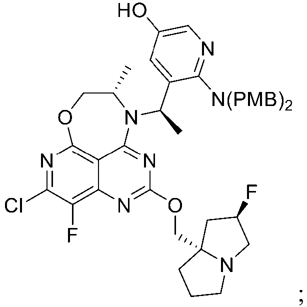
(vi) reacting 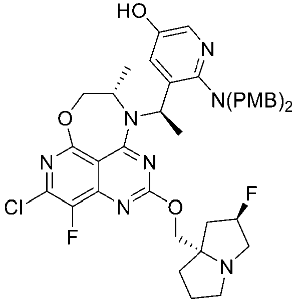 with 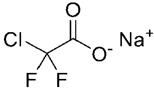 to form 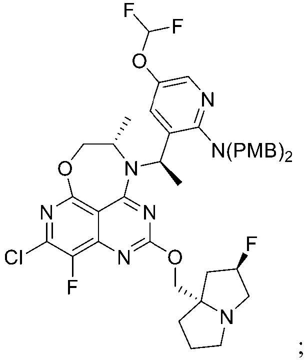
(vii) reacting 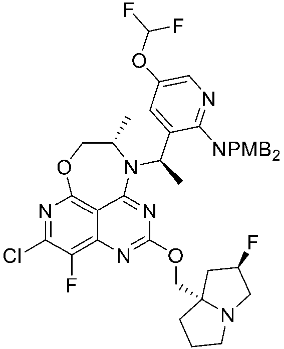 with 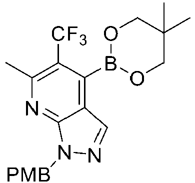 to form 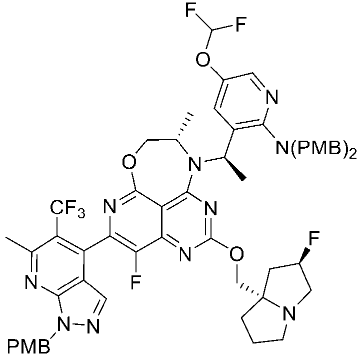 or a salt thereof;
(viii) reacting 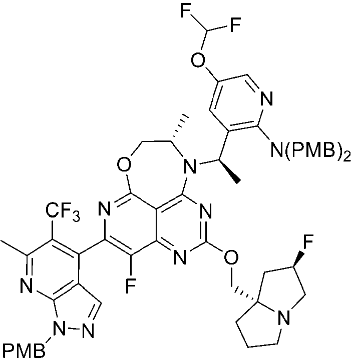 with trifluoromethanesulfonic acid in the presence of an aprotic solvent to form

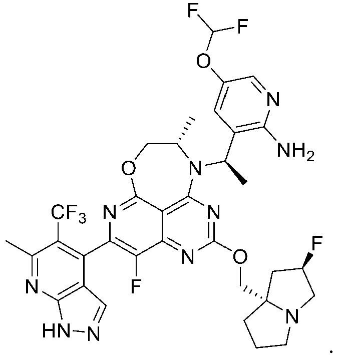

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.*, in Markush group format, each subgroup of the elements is also disclosed, and any element(s) can be removed from the group. It should be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

### INCORPORATION BY REFERENCE

All publications and patents mentioned herein, including those items listed below, are hereby incorporated by reference in their entirety for all purposes as if each individual publication or patent was specifically and individually incorporated by reference. In case of conflict, the present application, including any definitions herein, will control.

## Claims

1. A compound represented by Formula III: 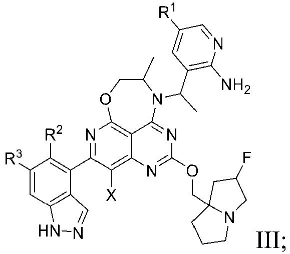 or a pharmaceutically acceptable salt, stereoisomer, or tautomer thereof, wherein:
X is F or Cl;
R¹ is cyano, -C(O)NH₂, isopropyl, C₁-C₄alkoxy, -OCH₃, -OCF₃, or -OCHF₂, wherein the alkyl of the alkoxy is optionally -CH₂CN or a C₃-C₆cycloalkyl group;
R² is selected from the group consisting of C₁-C₄alkyl, C₁-C₄alkoxy, and C₃-C₆cycloalkyl, wherein the alkyl, alkoxy, and cycloalkyl are optionally substituted with one, two, or three halogens, or -CN;
R³ is selected from the group consisting of halogen and C₁-C₄alkyl, wherein the alkyl is optionally substituted with one, two, or three halogens,
or:
R¹ is fluoro;
R² is selected from the group consisting of C₁-C₄alkoxy and C₃-C₆cycloalkyl, wherein alkoxy and cycloalkyl are optionally substituted with one, two, or three halogens, or -CN;
R³ is selected from the group consisting of halogen and C₁-C₄alkyl.

2. The compound or a pharmaceutically acceptable salt, stereoisomer, or tautomer thereof of claim 1 wherein R¹ is -OCHF₂.

3. The compound or a pharmaceutically acceptable salt, stereoisomer, or tautomer thereof of claim 1, wherein R¹ is -OCF₃.

4. The compound or a pharmaceutically acceptable salt, stereoisomer, or tautomer thereof of any one of claims 1-3, wherein R² is selected from the group consisting of -CF₃,

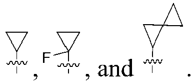

5. The compound or a pharmaceutically acceptable salt, stereoisomer, or tautomer thereof of any one of claims 1-4, wherein R³ is selected from the group consisting of chloro, fluoro, and -CH₃.

6. The compound or a pharmaceutically acceptable salt, stereoisomer, or tautomer thereof of any one of claims 1-5, wherein the moiety 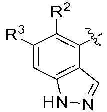 is selected from the group consisting of:

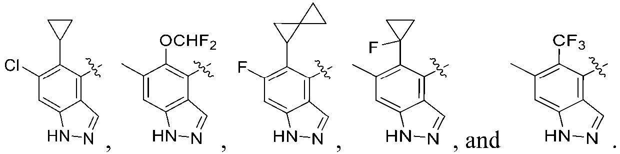

7. A compound selected from the group consisting of: 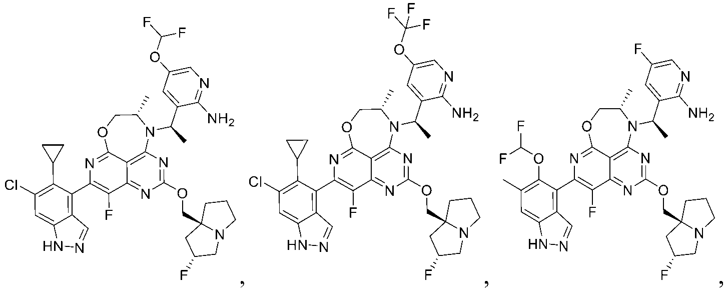 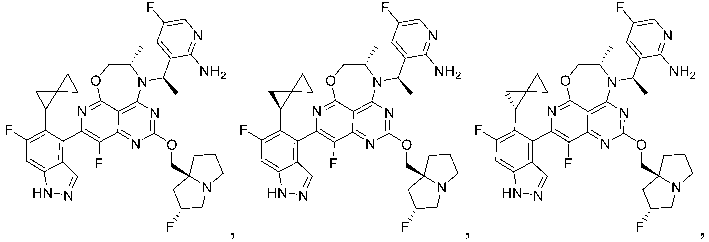 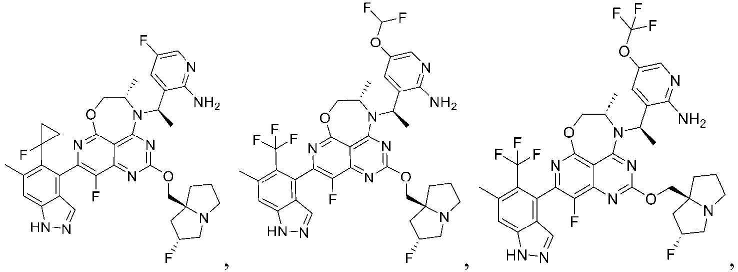 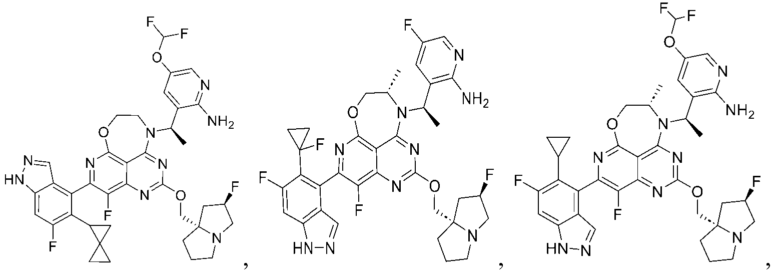 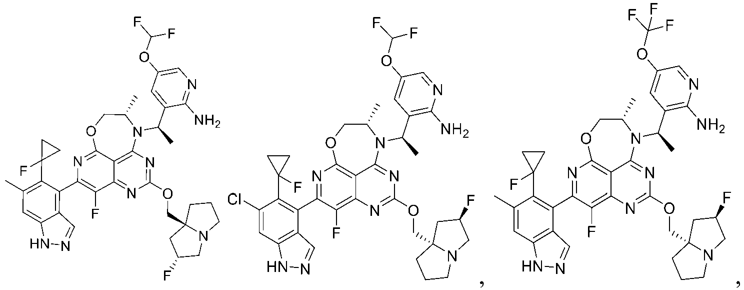 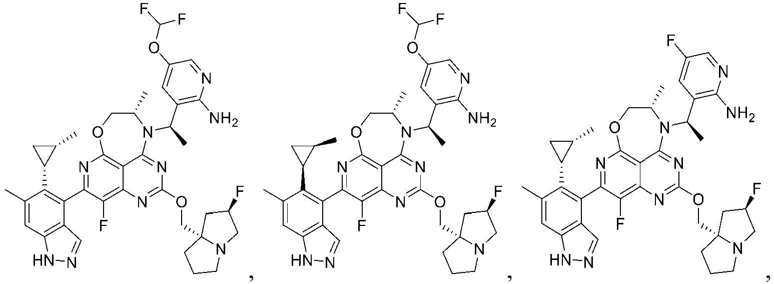 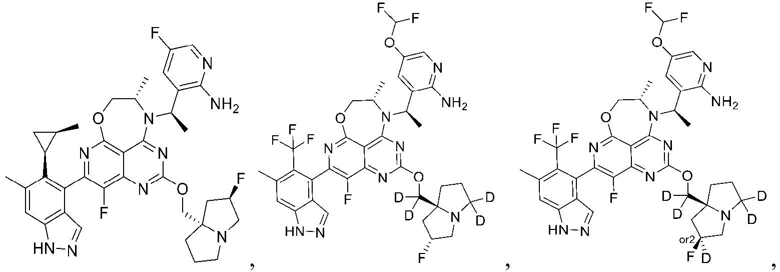 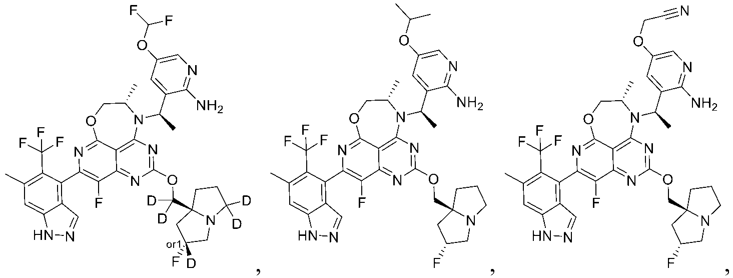 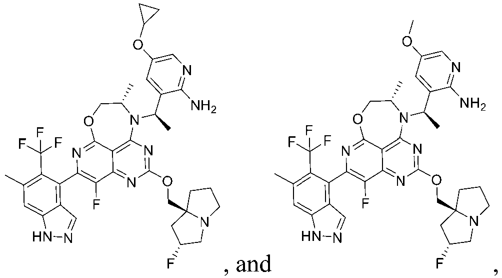 or a pharmaceutically acceptable salt, stereoisomer, or tautomer thereof.

8. The compound of claim 7, wherein the compound is

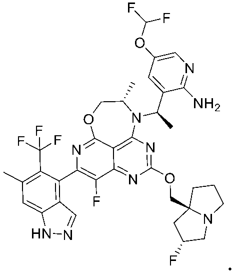

9. The compound of claim 7, wherein the compound is a pharmaceutically acceptable salt of

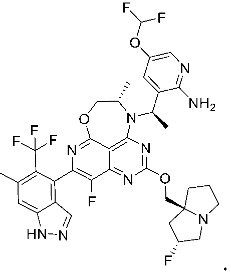

10. The compound of claim 7, wherein the compound is

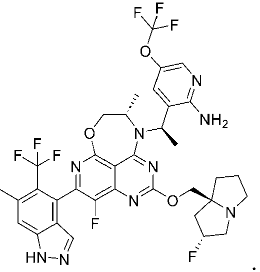

11. The compound of claim 7, wherein the compound is a pharmaceutically acceptable salt of

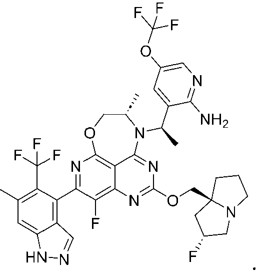

12. The compound of claim 7, wherein the compound is

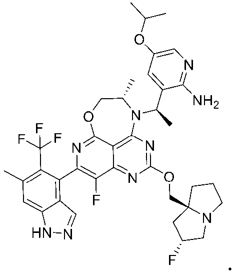

13. The compound of claim 7, wherein the compound is a pharmaceutically acceptable salt of

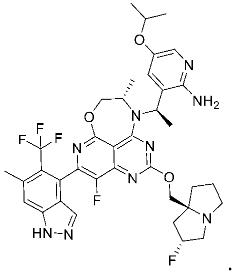

14. A pharmaceutical composition comprising the compound or a pharmaceutically acceptable salt, stereoisomer, or tautomer thereof of any one of claims 1-13, and a pharmaceutically acceptable excipient.

15. A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of any one of claims 1-13, or the pharmaceutical composition of claim 14.

16. A compound according to Formulae I-IX, or a pharmaceutically acceptable salt, stereoisomer, or tautomer thereof.

17. A method of treating pancreatic cancer, colorectal cancer, or non-small cell lung cancer in a patient in need thereof, comprising administering to the patient a therapeutically effective amount of the compound or a pharmaceutically acceptable salt, stereoisomer, or tautomer thereof of the compound of claim 16.

18. A process for preparing 5-(difluoromethoxy)-3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-oxa-1,3,6,10-tetraazacyclohepta[de]naphthalen-10-yl)ethyl)pyridin-2-amine, or a pharmaceutically acceptable salt thereof, the process comprising:
(i) reacting 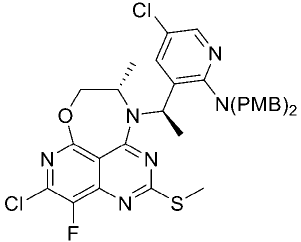 with 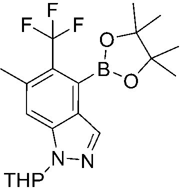 to form 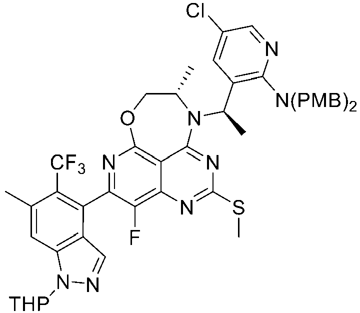 or a salt thereof;
(ii) reacting 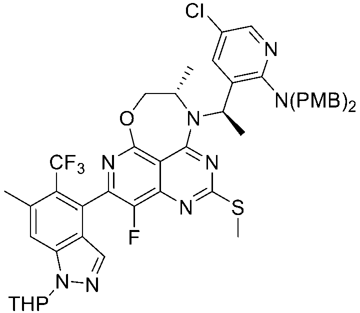 with KOH in the presence of a catalyst to form 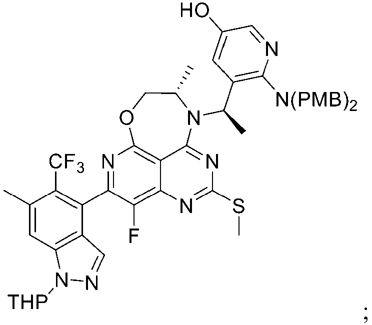
(iii) reacting 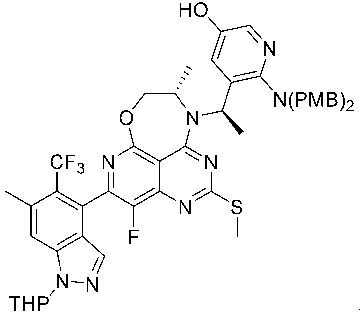 with ClCF₂COONa, or an alternative salt thereof to form 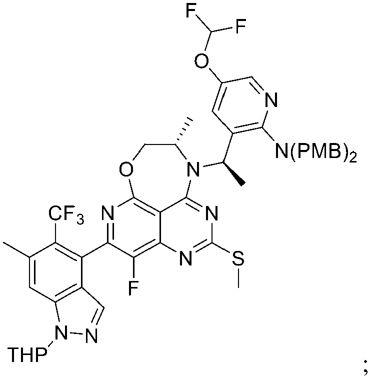
(iv) reacting 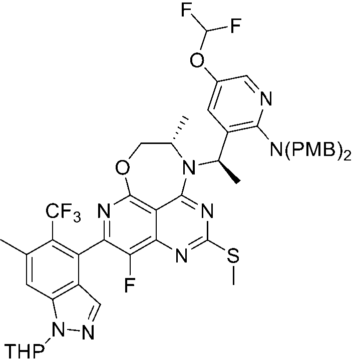 with m-CPBA to form 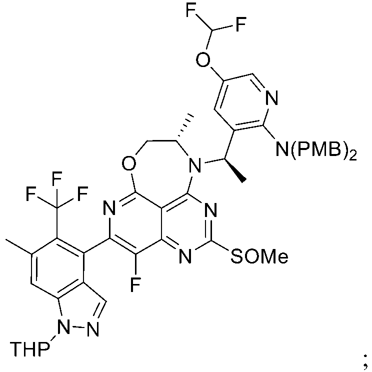
(v) reacting 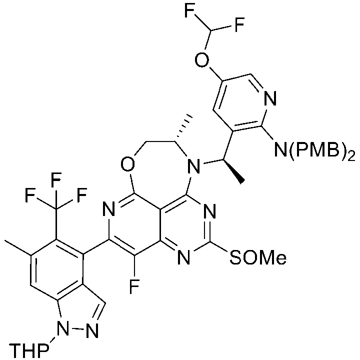 with 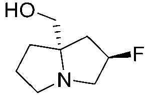 to form 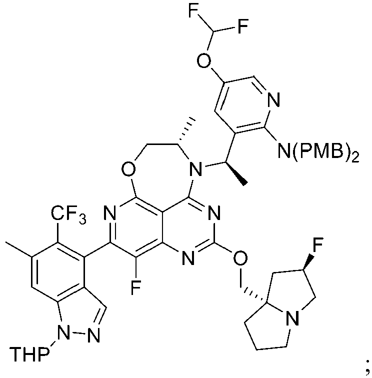 and
(vi) deprotecting 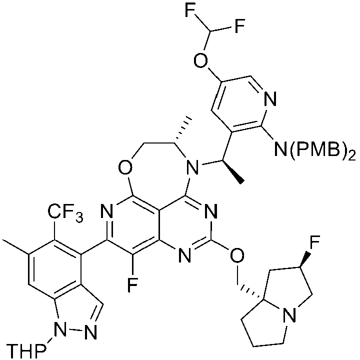 with triflic acid to form

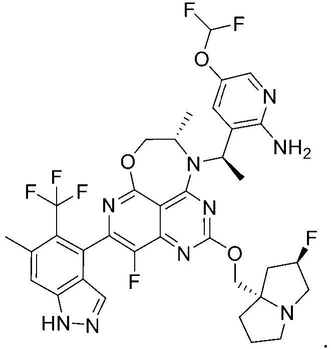

19. A process for preparing 3-((1R)-1-((9S)-4-fluoro-2-(((2R,7aS)-2-fluorotetrahydro-1H-pyrrolizin-7a(5H)-yl)methoxy)-9-methyl-5-(6-methyl-5-(trifluoromethyl)-1H-indazol-4-yl)-8,9-dihydro-10H-7-o0xa-1,3,6,10-tetraazacyclohepta[de]naphthalen-10-yl)ethyl)-5-isopropoxypyridin-2-amine, or a pharmaceutically acceptable salt thereof, the process comprising:
(i) reacting 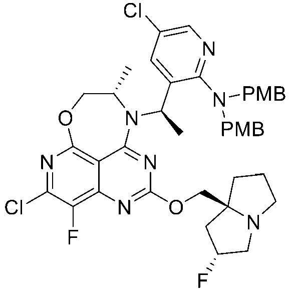 with 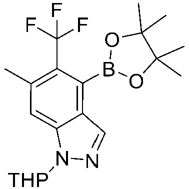 to form 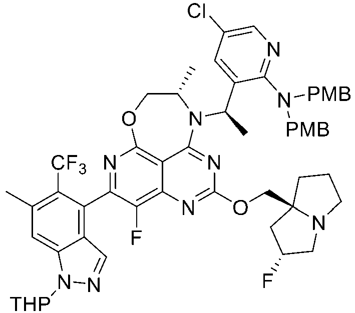 or a salt thereof;
(ii) reacting 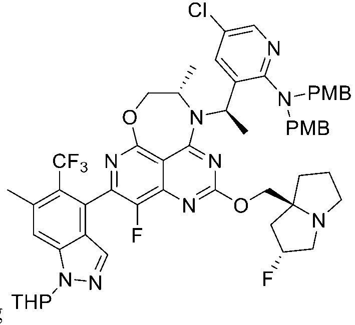 with KOH in the presence of a catalyst to form 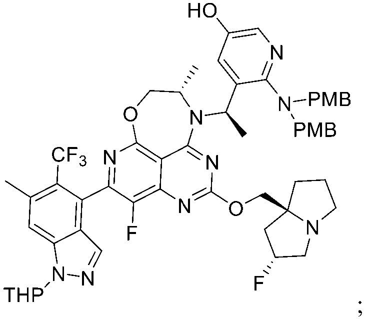
(iii) reacting 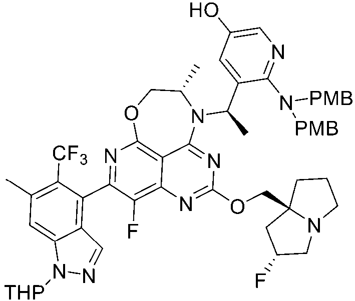 with 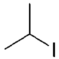 to form 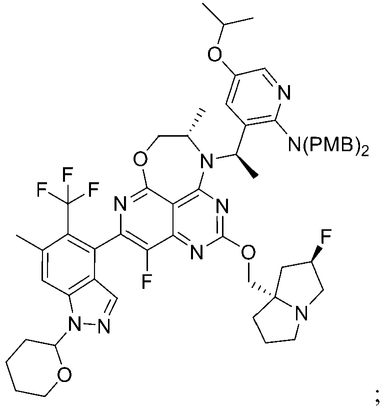
(iv) deprotecting 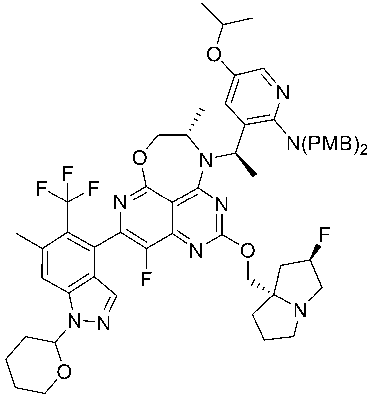 with trifluoroacetic acid to form
